(19) Europäisches Patentamt
European Patent Office
Office européen des brevets

(11) **EP 2 771 021 B1**

(12) # EUROPEAN PATENT SPECIFICATION

(45) Date of publication and mention of the grant of the patent:
**29.08.2018 Bulletin 2018/35**

(21) Application number: **12844456.9**

(22) Date of filing: **24.10.2012**

(51) Int Cl.:
***C07K 5/103*** *(2006.01)* ***C07K 5/078*** *(2006.01)*
***C07K 5/097*** *(2006.01)* ***A61K 38/07*** *(2006.01)*
***A61K 38/00*** *(2006.01)* ***A61P 25/24*** *(2006.01)*

(86) International application number:
**PCT/US2012/061696**

(87) International publication number:
**WO 2013/063120 (02.05.2013 Gazette 2013/18)**

(54) **NMDA RECEPTOR MODULATORS AND USES THEREOF**

NMDA-REZEPTORMODULATOREN UND IHRE VERWENDUNG

MODULATEURS DES RÉCEPTEURS NMDA ET UTILISATIONS DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.10.2011 US 201161550782 P**

(43) Date of publication of application:
**03.09.2014 Bulletin 2014/36**

(73) Proprietor: **North Western University**
**Evanston, IL 60208 (US)**

(72) Inventors:
- **KHAN, Amin, M.**
  **Evanston, IL 60611 (US)**
- **MOSKAL, Joseph**
  **Evanston, IL 60201 (US)**

(74) Representative: **Hoffmann Eitle**
**Patent- und Rechtsanwälte PartmbB**
**Arabellastraße 30**
**81925 München (DE)**

(56) References cited:
**EP-A1- 2 371 375**
**WO-A1-2010/033757**
**WO-A1-2010/033757**
**WO-A2-2007/027559**
**WO-A2-2011/044089**
**WO-A2-2011/044089**
**JP-B2- 3 318 622**
**US-A- 4 683 221**
**US-A1- 2005 176 649**
**US-B1- 6 897 028**
**US-B1- 6 902 886**

- **OHMORI T ET AL: "Isolation of Prolylendopeptidase-Inhibiting Peptides from Bovine Brain", BIOCHEMICAL AND BIOPHYSICAL RESEARCH COMMUNICATIONS, ACADEMIC PRESS INC. ORLANDO, FL, US, vol. 202, no. 2, 29 July 1994 (1994-07-29) , pages 809-815, XP024766199, ISSN: 0006-291X, DOI: 10.1006/BBRC.1994.2002 [retrieved on 1994-07-29]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 771 021 B1

## Description

### BACKGROUND

An N-methyl-d-aspartate (NMDA) receptor is a postsynaptic, ionotropic receptor that is responsive to, *inter alia,* the excitatory amino acids glutamate and glycine and the synthetic compound NMDA. The NMDA receptor controls the flow of both divalent and monovalent ions into the postsynaptic neural cell through a receptor associated channel (Foster et al., Nature 1987, 329:395-396; Mayer et al., Trends in Pharmacol. Sci. 1990, 11:254-260). The NMDA receptor has been implicated during development in specifying neuronal architecture and synaptic connectivity, and may be involved in experience-dependent synaptic modifications. In addition, NMDA receptors are also thought to be involved in long term potentiation and central nervous system disorders.

The NMDA receptor plays a major role in the synaptic plasticity that underlies many higher cognitive functions, such as memory acquisition, retention and learning, as well as in certain cognitive pathways and in the perception of pain (Collingridge et al., The NMDA Receptor, Oxford University Press, 1994). In addition, certain properties of NMDA receptors suggest that they may be involved in the information-processing in the brain that underlies consciousness itself.

The NMDA receptor has drawn particular interest since it appears to be involved in a broad spectrum of CNS disorders. For instance, during brain ischemia caused by stroke or traumatic injury, excessive amounts of the excitatory amino acid glutamate are released from damaged or oxygen deprived neurons. This excess glutamate binds to the NMDA receptors which opens their ligand-gated ion channels; in turn the calcium influx produces a high level of intracellular calcium which activates a biochemical cascade resulting in protein degradation and cell death. This phenomenon, known as excitotoxicity, is also thought to be responsible for the neurological damage associated with other disorders ranging from hypoglycemia and cardiac arrest to epilepsy. In addition, there are preliminary reports indicating similar involvement in the chronic neurodegeneration of Huntington's, Parkinson's, and Alzheimer's diseases. Activation of the NMDA receptor has been shown to be responsible for post-stroke convulsions, and, in certain models of epilepsy, activation of the NMDA receptor has been shown to be necessary for the generation of seizures. Neuropsychiatric involvement of the NMDA receptor has also been recognized since blockage of the NMDA receptor $Ca^{++}$ channel by the animal anesthetic PCP (phencyclidine) produces a psychotic state in humans similar to schizophrenia (reviewed in Johnson, K. and Jones, S., 1990). Further, NMDA receptors have also been implicated in certain types of spatial learning.

The NMDA receptor is believed to consist of several protein chains embedded in the postsynaptic membrane. The first two types of subunits discovered so far form a large extracellular region, which probably contains most of the allosteric binding sites, several transmembrane regions looped and folded so as to form a pore or channel, which is permeable to $Ca^{++}$, and a carboxyl terminal region. The opening and closing of the channel is regulated by the binding of various ligands to domains (allosteric sites) of the protein residing on the extracellular surface. The binding of the ligands is thought to affect a conformational change in the overall structure of the protein which is ultimately reflected in the channel opening, partially opening, partially closing, or closing.

NMDA receptor compounds may exert dual (agonist/antagonist) effect on the NMDA receptor through the allosteric sites. These compounds are typically termed "partial agonists". In the presence of the principal site ligand, a partial agonist will displace some of the ligand and thus decrease $Ca^{++}$ flow through the receptor. In the absence of or lowered level of the principal site ligand, the partial agonist acts to increase $Ca^{++}$ flow through the receptor channel.

A need continues to exist in the art for novel and more specific/potent compounds that are capable of binding the glycine binding site of NMDA receptors, and provide pharmaceutical benefits. In addition, a need continues to exist in the medical arts for an orally deliverable forms of such compounds.

### SUMMARY

Provided herein, at least in part, are compounds that are NMDA modulators, for example, partial agonists of NMDA. For example, disclosed herein are compounds represented by the formula:

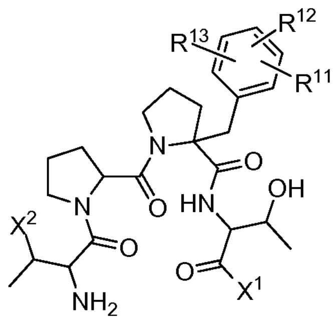

wherein

$R^{11}$, $R^{12}$ and $R^{13}$ are each independently selected from the group consisting of H, halogen, $C_{1-3}$alkoxy, and $C_{1-3}$alkyl (optionally substituted by one, two or three halogens);
$X^1$ is OH or $NH_2$,
$X^2$ is H or OH;
and pharmaceutically acceptable salts, stereoisomers and hydrates thereof.

In some embodiments, a halogen of a contemplated compound may be independently selected from the group consisting of Cl, Br, and F. In some instances, $X^2$ in the above structure may be OH and $X^1$ in the above structure may be $NH_2$.

In some cases, each of $R^{11}$, $R^{12}$, and $R^{13}$ in the above structure may be H. Alternatively, $R^{11}$ and $R^{13}$ in the above structure may be H. $R^{12}$ in the above structure may be selected from the group consisting of H, halogen, $C_{1-3}$alkoxy, and $C_{1-3}$alkyl (optionally substituted by one, two or three halogens). For example, $R^{11}$ and $R^{13}$ in the above structure may be H, and $R^{12}$ in the above structure may be selected from the group consisting of F, Br, Cl, $CH_3$, $CF_3$, and $-OCH_3$.

In certain embodiments, a contemplated compound may be substantially more efficacious when administered orally as compared to

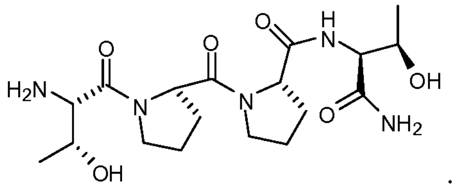

In another aspect, a compound is provided represented by:

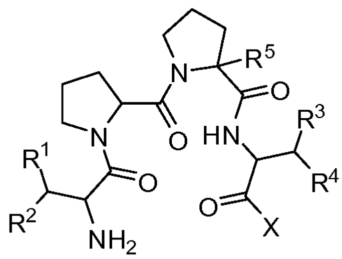

wherein:

$R^1$ is selected from the group consisting of hydrogen, -OH, $C_1$-$C_6$alkyl, and halogen;
$R^2$ and $R^4$ are each independently selected from the group consisting of $C_{1-6}$alkyl, hydrogen, halogen, and -OH;
$R^3$ is selected from the group consisting of -OH, hydrogen, $C_1$-$C_6$alkyl, and halogen;
$R^5$ is $-CH_2$-phenyl (wherein the phenyl is optionally substituted by one, two, or three substituents selected from the group consisting of halogen, $C_{1-3}$alkoxy, and $C_{1-3}$alkyl (optionally substituted by one, two or three halogens); and
X is selected from the group consisting of $OR^x$ and $NR^xR^x$, wherein $R^X$ is independently selected, for each occurrence,

from the group consisting of hydrogen, and $C_{1-}C_6$alkyl; pharmaceutically acceptable salts, stereoisomers, and hydrates thereof.

For example, a contemplated compound may be represented by:

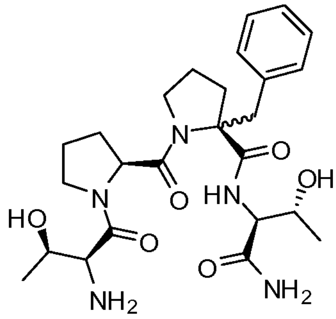 or 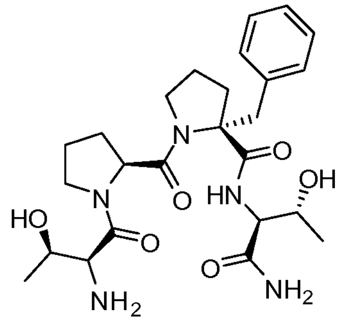 .

In other examples, a contemplated compound may be represented by:

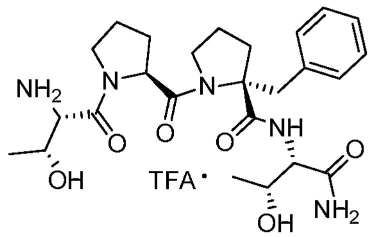 , 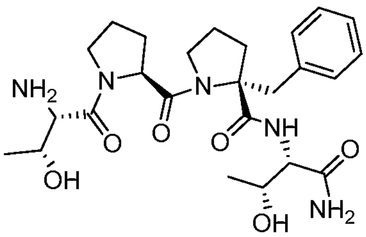 ,

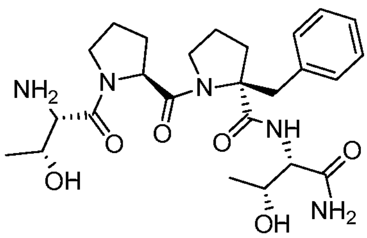 , 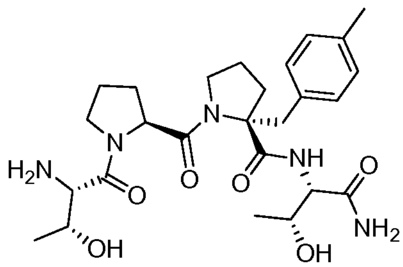 ,

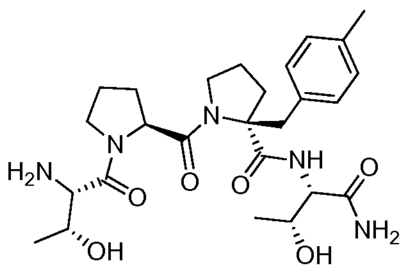 , 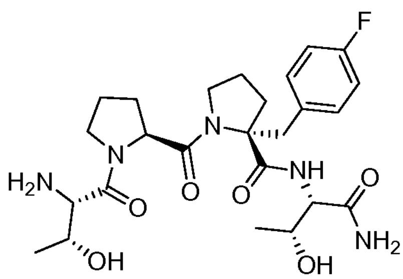 ,

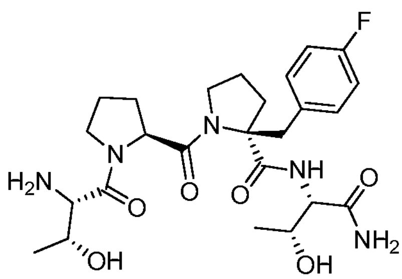 , 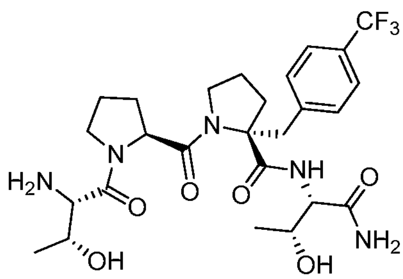 ,

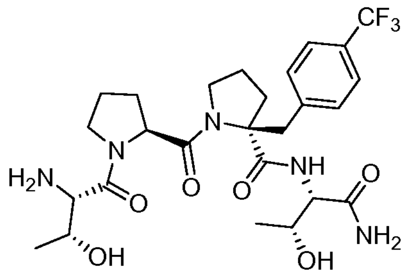,
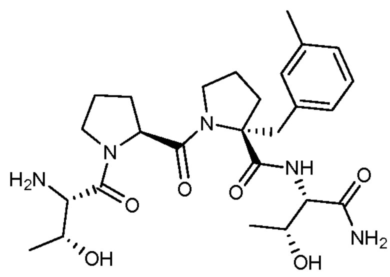,

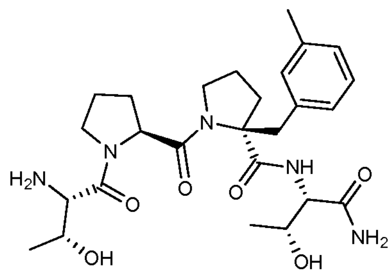,
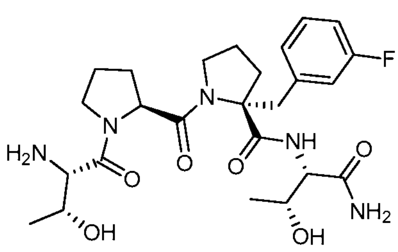,

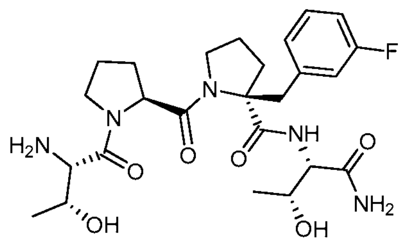,
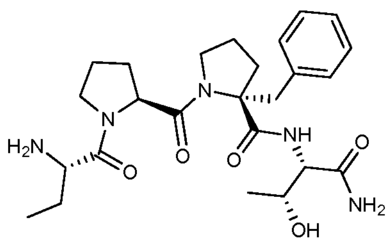,

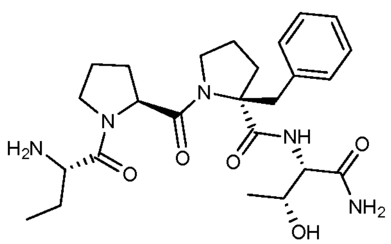,
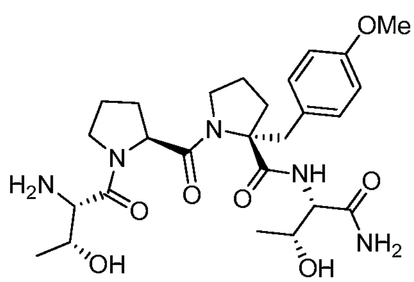,

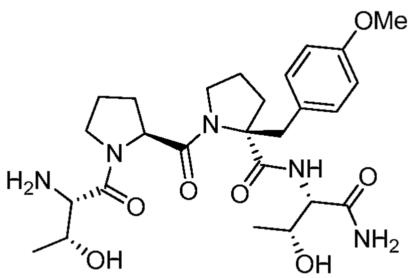,
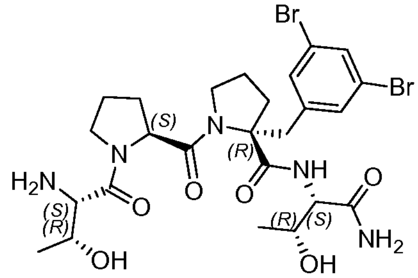,

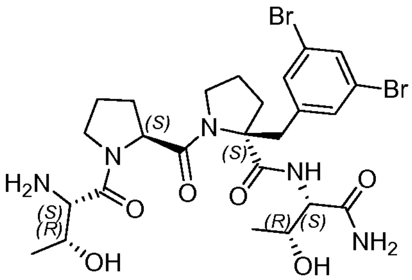
,
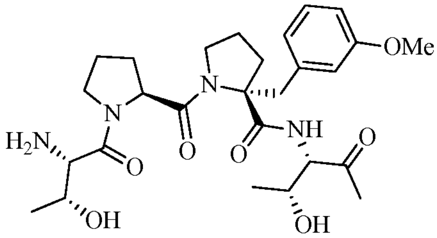
,

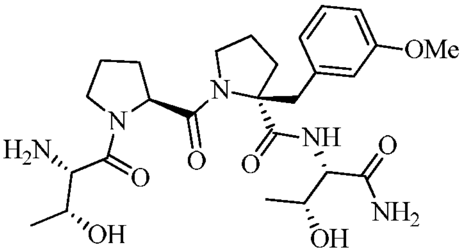
,
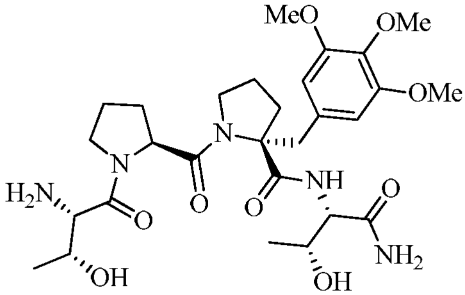
,

or

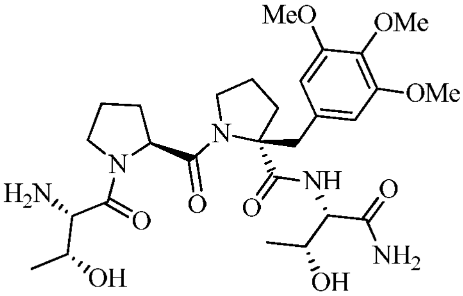
.

Also provided herein are pharmaceutically acceptable compositions comprising a disclosed compound, and a pharmaceutically acceptable excipient. For example, such compositions may be suitable for oral administration to a patient. In other embodiments, such compositions may be suitable for injection.

In another aspect, a contemplated compound for use in a method of treating depression, Alzheimer's disease, attention deficit disorder, ADHD, schizophrenia, or anxiety, in a patient in need thereof, is provided comprising, e.g., oral, intravenous, or subcutaneous administration.

**BRIEF DESCRIPTION OF THE FIGURES**

FIG. 1 shows a plot of plasma concentration of CM-4A as a function of post-dosing time.

FIG. 2 shows dose-dependent plots of a [$^3$H] MK-801 binding assay for assessing agonistic properties of contemplated compounds.

FIG. 3 shows dose-dependent plots of a [$^3$H] MK-801 binding assay for assessing agonistic properties of contemplated compounds.

FIG. 4 shows a dose-dependent plot of a [$^3$H] MK-801 binding assay for assessing agonistic properties of a contemplated compound.

FIG. 5 shows a plot depicting a time course of the effect of CM-4A on single shock Schaffer collateral-evoked

pharmacologically-isolated NMDA excitatory postsynaptic currents (EPSCs) recorded in CA1 pyramidal neurons.

FIG. 6 shows a plot depicting the effect of CM-4A concentration on long-term potentiation after high frequency stimulation of Schaffer collateral-evoked NMDA EPSCs recorded in CA1 pyramidal neurons.

FIG. 7 shows a plot depicting a dose-response relationship of CM-4A on long-term depression (LTD).

FIG. 8 shows a bar graph of percent long-term potentiation of field excitatory postsynaptic potential (fEPSP) slope for contemplated compounds.

FIG. 9 shows a plot depicting Porsolt test mean floating time as a function of CM-4A concentration.

FIG. 10 shows a bar graph of Porsolt test mean floating times for CM-4A and CM-4A in combination with AMPA receptor antagonist 2,3-dihydroxy-6-nitro-7-sulfamoyl-benzo[f]quinoxaline-2,3-dione (NBQX).

FIGs. 11A-11C show bar graphs depicting the results of the Porsolt test for contemplated compounds.

FIG. 12 shows a bar graph depicting the results of the Porsolt test for contemplated compounds after 1 hour and 24 hours.

FIG. 13 shows a bar graph depicting the results of the rat novelty-induced hypophagia (NIH) test for CM-4A.

FIGs. 14A and 14B show bar graphs depicting the results of the rat ultrasonic vocalizations (USVs) test for CM-4A. FIG. 14A quantifies positive emotional learning, and FIG. 14B quantifies hedonic and aversive USVs and reward.

FIG. 15 shows a bar graph depicting the results of the rat open field test for CM-4A.

FIG. 16 shows a bar graph depicting the results of the rat accelerating rota-rod test for CM-4A.

## DETAILED DESCRIPTION

This disclosure is generally directed to compounds that are capable of modulating NMDA, e.g., NMDA antagonists or partial agonists, and compositions and/or methods of using the disclosed compounds.

### Definitions

In some embodiments, the compounds, as described herein, may be substituted with any number of substituents or functional moieties. In general, the term "substituted" whether preceded by the term "optionally" or not, and substituents contained in formulas, refer to the replacement of hydrogen radicals in a given structure with the radical of a specified substituent.

In some instances, when more than one position in any given structure may be substituted with more than one substituent selected from a specified group, the substituent may be either the same or different at every position.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. In some embodiments, heteroatoms such as nitrogen may have hydrogen substituents and/or any permissible substituents of organic compounds described herein which satisfy the valencies of the heteroatoms. Examples of substituents include acyl; aliphatic; heteroaliphatic; aryl; heteroaryl; arylalkyl; heteroarylalkyl; alkoxy; cycloalkoxy; heterocyclylalkoxy; heterocyclyloxy; heterocyclyloxyalkyl; alkenyloxy; alkynyloxy; aryloxy; heteroalkoxy; heteroaryloxy; alkylthio; arylthio; heteroarylthio; oxo; -F;-Cl; -Br; -I; -OH; $-NO_2$; $-N_3$; -CN; -SCN; $-SR^x$; $-CF_3$; $-CH_2CF_3$; $-CHCl_2$; $-CH_2OH$;$-CH_2CH_2OH$; $-CH_2NH_2$; $-CH_2SO_2CH_3$; $-OR^x$, $-C(O)R^x$; $-CO_2(R^x)$; $-C(O)N(R^x)_2$;$-C(NR^x)N(R^x)_2$; $-OC(O)R^x$; $-OCO_2R^x$; $-OC(O)N(R^x)_2$; $-N(R^x)_2$; $-SOR^x$; $-S(O)_2R^x$; $-NR^xC(O)R^x$; $-NR^xC(O)N(R^x)_2$; $-NR^xC(O)OR^x$; $-NR^xC(NR^x)N(R^x)_2$; and $-C(R^x)_3$; wherein each occurrence of $R^x$ independently includes hydrogen, halogen, acyl, aliphatic, heteroaliphatic, aryl, heteroaryl, arylalkyl, or heteroarylalkyl, wherein any of the aliphatic, heteroaliphatic, arylalkyl, or heteroarylalkyl substituents described above and herein may be substituted or unsubstituted, branched or unbranched, cyclic or acyclic, and wherein any of the aryl or heteroaryl substituents described above and herein may be substituted or unsubstituted. In some embodiments, combinations of substituents and variables described herein may be preferably those that result

in the formation of stable compounds. The term "stable," as used herein, refers to compounds which possess stability

sufficient to allow manufacture and which maintain the integrity of the compound for a sufficient period of time to be detected and preferably for a sufficient period of time to be useful for the purposes detailed herein.

The term "aliphatic," as used herein, includes both saturated and unsaturated, straight chain (i.e., unbranched), branched, acyclic, cyclic, or polycyclic aliphatic hydrocarbons, which are optionally substituted with one or more functional groups. As will be appreciated by one of ordinary skill in the art, "aliphatic" is intended herein to include alkyl, alkenyl, alkynyl, cycloalkyl, cycloalkenyl, and cycloalkynyl moieties.

The terms "aryl" and "heteroaryl," as used herein, refer to mono- or polycyclic unsaturated moieties having preferably 3-14 carbon atoms, each of which may be substituted or unsubstituted. In certain embodiments, "aryl" refers to a mono- or bicyclic carbocyclic ring system having one or two aromatic rings including phenyl, naphthyl, tetrahydronaphthyl, indanyl and indenyl. In certain embodiments, "heteroaryl" refers to a mono- or bicyclic heterocyclic ring system having one or two aromatic rings in which one, two, or three ring atoms are heteroatoms independently selected from the group consisting of S, O, and N and the remaining ring atoms are carbon. Examples of heteroaryl groups include pyridyl, pyrazinyl, pyrimidinyl, pyrrolyl, pyrazolyl, imidazolyl, thiazolyl, oxazolyl, isooxazolyl, thiadiazolyl,oxadiazolyl, thiophenyl, furanyl, quinolinyl and isoquinolinyl.

The term "alkenyl" as used herein refers to an unsaturated straight or branched hydrocarbon having at least one carbon-carbon double bond, such as a straight or branched group of 2-12, 2-10, or 2-6 carbon atoms, referred to herein as $C_2$-$C_{12}$alkenyl, $C_2$-$C_{10}$alkenyl, and $C_2$-$C_6$alkenyl, respectively. Exemplary alkenyl groups include vinyl, allyl, butenyl, pentenyl, hexenyl, butadienyl, pentadienyl, hexadienyl, 2-ethylhexenyl, 2-propyl-2-butenyl, 4-(2-methyl-3-butene)-pentenyl, etc.

The term "alkoxy" as used herein refers to an alkyl group attached to an oxygen (-O-alkyl). Exemplary alkoxy groups include groups with an alkyl group of 1-12, 1-8, or 1-6 carbon atoms, referred to herein as $C_1$-$C_{12}$alkoxy, $C_1$-$C_8$alkoxy, and $C_1$-$C_6$alkoxy, respectively. Exemplary alkoxy groups include methoxy, ethoxy, etc. Similarly, exemplary "alkenoxy" groups include vinyloxy, allyloxy, butenoxy, etc.

The term "alkoxycarbonyl" as used herein refers to a straight or branched alkyl group attached to oxygen, attached to a carbonyl group (alkyl-O-C(O)-). Exemplary alkoxycarbonyl groups include alkoxycarbonyl groups of 1-6 carbon atoms, referred to herein as $C_{1-6}$alkoxycarbonyl. Exemplary alkoxycarbonyl groups include methoxycarbonyl, ethoxycarbonyl, t-butoxycarbonyl, etc.

The term "alkynyloxy" used herein refers to a straight or branched alkynyl group attached to an oxygen (alkynyl-O)). Exemplary alkynyloxy groups include propynyloxy.

The term "alkyl" as used herein refers to a saturated straight or branched hydrocarbon, for example, such as a straight or branched group of 1-6, 1-4, or 1-3 carbon atom, referred to herein as $C_1$-$C_6$alkyl, $C_1$-$C_4$alkyl, and $C_1$-$C_3$alkyl, respectively. Exemplary alkyl groups include methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl, octyl, etc.

The term "alkylcarbonyl" as used herein refers to a straight or branched alkyl group attached to a carbonyl group (alkyl-C(O)-). Exemplary alkylcarbonyl groups include alkylcarbonyl groups of 1-6 atoms, referred to herein as $C_1$-$C_6$alkylcarbonyl groups. Exemplary alkylcarbonyl groups include acetyl, propanoyl, isopropanoyl, butanoyl, etc.

The term "alkynyl" as used herein refers to an unsaturated straight or branched hydrocarbon having at least one carbon-carbon triple bond, such as a straight or branched group of 2-6, or 3-6 carbon atoms, referred to herein as $C_{2-6}$alkynyl, and $C_{3-6}$alkynyl, respectively. Exemplary alkynyl groups include ethynyl, propynyl, butynyl, pentynyl, hexynyl, methylpropynyl, etc.

Alkyl, alkenyl and alkynyl groups can optionally be substituted, if not indicated otherwise, with one or more groups selected from alkoxy, alkyl, cycloalkyl, amino, halogen, and -C(O)alkyl. In certain embodiments, the alkyl, alkenyl, and alkynyl groups are not substituted, i.e., they are unsubstituted.

The term "amide" or "amido" as used herein refers to a radical of the form $-R^aC(O)N(R^b)-$, $-R^aC(O)N(R^b)R^c-$, or $-C(O)NR^bR^c$, wherein $R^a$, $R^b$, and $R^c$ are each independently selected from alkoxy, alkyl, alkenyl, alkynyl, amide, amino, aryl, arylalkyl, carbamate, cycloalkyl, ester, ether, formyl, halogen, haloalkyl, heteroaryl, heterocyclyl, hydrogen, hydroxyl, ketone, and nitro. The amide can be attached to another group through the carbon, the nitrogen, $R^b$, $R^c$, or $R^a$. The amide also may be cyclic, for example $R^b$ and $R^c$, $R^a$ and $R^b$, or $R^a$ and $R^c$ may be joined to form a 3- to 12-membered ring, such as a 3- to 10-membered ring or a 5- to 6-membered ring. The term "carboxamido" refers to the structure $-C(O)NR^bR^c$.

The term "amine" or "amino" as used herein refers to a radical of the form $-NR^dR^e$, where $R^d$ and $R^e$ are independently selected from hydrogen, alkyl, alkenyl, alkynyl, aryl, arylalkyl, cycloalkyl, haloalkyl, heteroaryl, and heterocyclyl. The amino also may be cyclic, for example, $R^d$ and $R^e$ are joined together with the N to form a 3- to 12-membered ring, e.g., morpholino or piperidinyl. The term amino also includes the corresponding quaternary ammonium salt of any amino group, e.g., $-[N(R^d)(R^e)(R^f)]+$. Exemplary amino groups include aminoalkyl groups, wherein at least one of $R^d$, $R^e$, or $R^f$ is an alkyl group. In certain embodiment, $R^d$ and $R^e$ are hydrogen or alkyl.

The term "cycloalkyl" as used herein refers to a monocyclic saturated or partially unsaturated hydrocarbon group of for example 3-6, or 4-6 carbons, referred to herein, e.g., as $C_{3-6}$cycloalkyl or $C_{4-6}$cycloalkyl and derived from a cycloalkane. Exemplary cycloalkyl groups include cyclohexyl, cyclohexenyl, cyclopentyl, cyclobutyl or, cyclopropyl.

The terms "halo" or "halogen" or "Hal" as used herein refer to F, Cl, Br, or I. The term "haloalkyl" as used herein refers to an alkyl group substituted with one or more halogen atoms.

The terms "heterocyclyl" or "heterocyclic group" are art-recognized and refer to saturated or partially unsaturated 3- to 10-membered ring structures, alternatively 3- to 7-membered rings, whose ring structures include one to four heteroatoms, such as nitrogen, oxygen, and sulfur. Heterocycles may also be mono-, bi-, or other multi-cyclic ring systems. A heterocycle may be fused to one or more aryl, partially unsaturated, or saturated rings. Heterocyclyl groups include, for example, biotinyl, chromenyl, dihydrofuryl, dihydroindolyl, dihydropyranyl, dihydrothienyl, dithiazolyl, homopiperidinyl, imidazolidinyl, isoquinolyl, isothiazolidinyl, isoxazolidinyl, morpholinyl, oxolanyl, oxazolidinyl, phenoxanthenyl, piperazinyl, piperidinyl, pyranyl, pyrazolidinyl, pyrazolinyl, pyridyl, pyrimidinyl, pyrrolidinyl, pyrrolidin-2-onyl, pyrrolinyl, tetrahydrofuryl, tetrahydroisoquinolyl, tetrahydropyranyl, tetrahydroquinolyl, thiazolidinyl, thiolanyl, thiomorpholinyl, thiopyranyl, xanthenyl, lactones, lactams such as azetidinones and pyrrolidinones, sultams and sultones. The heterocyclic ring may be substituted at one or more positions with substituents such as alkanoyl, alkoxy, alkyl, alkenyl, alkynyl, amido, amidino, amino, aryl, arylalkyl, azido, carbamate, carbonate, carboxy, cyano, cycloalkyl, ester, ether, formyl, halogen, haloalkyl, heteroaryl, heterocyclyl, hydroxyl, imino, ketone, nitro, phosphate, phosphonato, phosphinato, sulfate, sulfide, sulfonamido, sulfonyl and thiocarbonyl. In certain embodiments, the heterocyclic group is not substituted, i.e., the heterocyclic group is unsubstituted.

The term "heterocyclylalkoxy" as used herein refers to a heterocyclyl-alkyl-O-group.

The term "heterocyclyloxy" refers to a heterocyclyl-O- group.

The term "heterocyclyloxyalkyl" refers to a heterocyclyl-O-alkyl- group.

The terms "hydroxy" and "hydroxyl" as used herein refers to the radical -OH.

The term "oxo" as used herein refers to the radical =O.

"Pharmaceutically or pharmacologically acceptable" include molecular entities and compositions that do not produce an adverse, allergic or other untoward reaction when administered to an animal, or a human, as appropriate. "For human administration, preparations should meet sterility, pyrogenicity, general safety and purity standards as required by FDA Office of Biologics standards.

As used in the present disclosure, the term "partial NMDA receptor agonist" is defined as a compound that is capable of binding to a glycine binding site of an NMDA receptor; at low concentrations a NMDA receptor agonist acts substantially as agonist and at high concentrations it acts substantially as an antagonist. These concentrations are experimentally determined for each and every "partial agonist.

As used herein "pharmaceutically acceptable carrier" or "excipient" includes any and all solvents, dispersion media, coatings, antibacterial and antifungal agents, isotonic and absorption delaying agents that are physiologically compatible. In one embodiment, the carrier is suitable for parenteral administration. Alternatively, the carrier can be suitable for intravenous, intraperitoneal, intramuscular, sublingual or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

The term "pharmaceutically acceptable salt(s)" as used herein refers to salts of acidic or basic groups that may be present in compounds used in the present compositions. Compounds included in the present compositions that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, including malate, oxalate, chloride, bromide, iodide, nitrate, sulfate, bisulfate, phosphate, acid phosphate, isonicotinate, acetate, lactate, salicylate, citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, *p*-toluenesulfonate and pamoate (i.e., 1,1'-methylene-bis-(2-hydroxy-3-naphthoate)) salts. Compounds included in the present compositions that include an amino moiety may form pharmaceutically acceptable salts with various amino acids, in addition to the acids mentioned above. Compounds included in the present compositions that are acidic in nature are capable of forming base salts with various pharmacologically acceptable cations. Examples of such salts include alkali metal or alkaline earth metal salts and, particularly, calcium, magnesium, sodium, lithium, zinc, potassium, and iron salts.

The compounds of the disclosure may contain one or more chiral centers and/or double bonds and, therefore, exist as stereoisomers, such as geometric isomers, enantiomers or diastereomers. The term "stereoisomers" when used herein consist of all geometric isomers, enantiomers or diastereomers. These compounds may be designated by the

symbols "R" or "S," depending on the configuration of substituents around the stereogenic carbon atom. The present invention encompasses various stereoisomers of these compounds and mixtures thereof. Stereoisomers include enantiomers and diastereomers. Mixtures of enantiomers or diastereomers may be designated "(±)" in nomenclature, but the skilled artisan will recognize that a structure may denote a chiral center implicitly.

Individual stereoisomers of compounds of the present invention can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, or (3) direct separation of the mixture of optical enantiomers on chiral chromatographic columns. Stereoisomeric mixtures can also be resolved into their component stereoisomers by well known methods, such as chiral-phase gas chromatography, chiral-phase high performance liquid chromatography, crystallizing the compound as a chiral salt complex, or crystallizing the compound in a chiral solvent. Stereoisomers can also be obtained from stereomerically-pure intermediates, reagents, and catalysts by well known asymmetric synthetic methods.

Geometric isomers can also exist in the compounds of the present invention. The symbol ⎓ denotes a bond that may be a single, double or triple bond as described herein. The present invention encompasses the various geometric isomers and mixtures thereof resulting from the arrangement of substituents around a carbon-carbon double bond or arrangement of substituents around a carbocyclic ring. Substituents around a carbon-carbon double bond are designated as being in the *"Z"* or *"E"* configuration wherein the terms *"Z"* and *"E"* are used in accordance with IUPAC standards. Unless otherwise specified, structures depicting double bonds encompass both the *"E"* and *"Z"* isomers.

Substituents around a carbon-carbon double bond alternatively can be referred to as "cis" or "trans," where "cis" represents substituents on the same side of the double bond and "trans" represents substituents on opposite sides of the double bond. The arrangement of substituents around a carbocyclic ring are designated as "cis" or "trans." The term "cis" represents substituents on the same side of the plane of the ring and the term "trans" represents substituents on opposite sides of the plane of the ring. Mixtures of compounds wherein the substituents are disposed on both the same and opposite sides of plane of the ring are designated "cis/trans."

The compounds disclosed herein can exist in solvated as well as unsolvated forms with pharmaceutically acceptable solvents such as water and ethanol, and it is intended that the invention embrace both solvated and unsolvated forms. In one embodiment,
the compound is amorphous. In one embodiment, the compound is a polymorph. In another embodiment, the compound is in a crystalline form.

The invention also embraces isotopically labeled compounds of the invention which are identical to those recited herein, except that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorus, fluorine and chlorine, such as $^{2}H$, $^{3}H$, $^{13}C$, $^{14}C$, $^{15}N$, $^{18}O$, $^{17}O$, $^{31}P$, $^{32}P$, $^{35}S$, $^{18}F$, and $^{36}Cl$, respectively.

Certain isotopically-labeled disclosed compounds (*e.g.,* those labeled with $^{3}H$ and $^{14}C$) are useful in compound and/or substrate tissue distribution assays. Tritiated (*i.e.,* $^{3}H$) and carbon-14 (*i.e.,* $^{14}C$) isotopes are particularly preferred for their ease of preparation and detectability. Further, substitution with heavier isotopes such as deuterium (*i.e.,* $^{2}H$) may afford certain therapeutic advantages resulting from greater metabolic stability (*e.g.,* increased *in vivo* half-life or reduced dosage requirements) and hence may be preferred in some circumstances. Isotopically labeled compounds of the invention can generally be prepared by following procedures analogous to those disclosed in the e.g., Examples herein by substituting an isotopically labeled reagent for a non-isotopically labeled reagent.

As used in the present disclosure, "NMDA" is defined as N-methyl-d-aspartate.

In the present specification, the term "therapeutically effective amount" means the amount of the subject compound that will elicit the biological or medical response of a tissue, system, animal or human that is being sought by the researcher, veterinarian, medical doctor or other clinician. The compounds of the invention are administered in therapeutically effective amounts to treat a disease. Alternatively, a therapeutically effective amount of a compound is the quantity required to achieve a desired therapeutic and/or prophylactic effect, such as an amount which results in defined as that amount needed to give maximal enhancement of a behavior (for example, learning), physiological response (for example, LTP induction), or inhibition of neuropathic pain.

**Compounds**

Disclosed reference compounds include those represented by the formula:

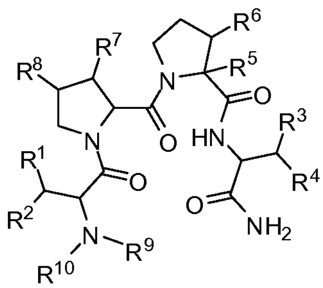

and pharmaceutically acceptable salts, stereoisomers, metabolites, and hydrates thereof, wherein:

$R^1$, $R^2$, $R^3$, and $R^4$ may be independently selected from the group consisting of hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted aryl; substituted or unsubstituted heteroaryl; $-OR^x$; $-NO_2$; $-N_3$; -CN; -SCN; $-SR^X$; $-C(O)R^x$; $-CO_2(R^x)$; $-C(O)N(R^x)_2$; $-C(NR^x)N(R^x)_2$; $-OC(O)R^x$;$-OCO_2R^x$; $-OC(O)N(R^x)_2$; $-N(R^x)_2$; $-SOR^x$; $-S(O)_2R^x$; $-NR^xC(O)R^x$; $-NR^xC(O)N(R^x)_2$;$-NR^xC(O)OR^x$; $-NR^xC(NR^x)N(R^x)_2$; and $-C(R^x)_3$; wherein each occurrence of $R^x$ is independently selected from the group consisting of hydrogen; halogen; acyl; optionally substituted aliphatic; optionally substituted heteroaliphatic; optionally substituted aryl; and optionally substituted heteroaryl;

$R^5$ and $R^6$ may be independently selected from the group consisting of -Q-Ar and hydrogen, provided that at least one of $R^5$ and $R^6$ is -Q-Ar; wherein Q is independently selected from the group consisting of cyclic or acyclic, substituted or unsubstituted, branched or unbranched aliphatic; cyclic or acyclic, substituted or unsubstituted, branched or unbranched heteroaliphatic; and a bond; and wherein Ar is selected from the group consisting substituted or unsubstituted aryl, and substituted or unsubstituted heteroaryl; or $R^5$ and $R^6$, together with the atoms to which they are attached, form a substituted or unsubstituted 4-6 membered heterocyclic or cycloalkyl ring;

$R^7$ and $R^8$ may be independently selected from the group consisting of hydrogen; halogen; hydroxyl; substituted or unsubstituted $C_1$-$C_6$ alkyl; substituted or unsubstituted $C_1$-$C_6$ alkoxy; and substituted or unsubstituted aryl; or $R^7$ and $R^8$, together with the atoms to which they are attached, form a substituted or unsubstituted 4-6 membered heterocyclic or cycloalkyl ring;

$R^9$ and $R^{10}$ may be independently selected from the group consisting of hydrogen; $C_1$-$C_6$ alkyl, optionally substituted by one or more substituents each independently selected from the group consisting of halogen, oxo, and hydroxyl; $C_{2-6}$alkenyl, optionally substituted by one or more substituents each independently selected from the group consisting of halogen, oxo, and hydroxyl; $C_{2-6}$alkynyl, optionally substituted by one or more substituents each independently selected from the group consisting of halogen, oxo, and hydroxyl; $C_{3-6}$cycloalkyl, optionally substituted by one or more substituents each independently selected from the group consisting of $C_{1-6}$alkyl, halogen, oxo, and hydroxyl; phenyl, optionally substituted by one or more substituents each independently selected from the group consisting of $C_{1-6}$alkyl; $C_{1-6}$alkoxy; halogen; hydroxyl; $-C(O)R^x$; $-CO_2(R^x)$; $-C(O)N(R^x)_2$; $-C(NR^x)N(R^x)_2$; and $-C(R^x)_3$; wherein each occurrence of $R^x$ is independently selected from the group consisting of hydrogen; halogen; $C_{1-6}$alkyl; $C_{2-6}$alkenyl; $C_{2-6}$alkynyl; $C_{3-6}$cycloalkyl; and phenyl; or $R^9$ and $R^{10}$, together with N, form a 4-6 membered heterocyclic ring, optionally substituted by one or more substituents each independently selected from the group consisting of $C_{1-6}$alkyl, halogen, oxo, and hydroxyl.

In some embodiments, $R^1$, $R^2$, $R^3$, and $R^4$ may be independently selected from the group consisting of hydrogen; halogen; $C_{1-6}$alkyl; $C_{2-6}$alkenyl; $C_{2-6}$alkynyl; $C_{3-6}$cycloalkyl; phenyl; naphthyl; heteroaryl; heterocyclyl; $C_{3-6}$cycloalkyl-$C_{1-6}$alkyl-; phenyl-$C_{1-6}$alkyl-; naphthyl-$C_{1-6}$alkyl-; heteroaryl-$C_{1-6}$alkyl-; and heterocyclyl-$C_{1-6}$alkyl-; $-OR^x$; $-NO_2$; $-N_3$; -CN;-SCN; $-SR^X$; $-C(O)R^x$; $-CO_2(R^x)$; $-C(O)N(R^x)_2$; $-C(NR^x)N(R^x)_2$; $-OC(O)R^x$; $-OCO_2R^x$;$-OC(O)N(R^x)_2$; $-N(R^x)_2$; $-SOR^x$; $-S(O)_2R^x$; $-NR^xC(O)R^x$; $-NR^xC(O)N(R^x)_2$; $-NR^xC(O)OR^x$;$-NR^xC(NR^x)N(R^x)_2$; and $-C(R^x)_3$; wherein heteroaryl is a 5-6 membered ring having one, two, or three heteroatoms each independently selected from N, O, or S; wherein heteroaryl is optionally substituted with one or more substituents each independently selected from $R^b$; wherein heterocyclyl is a 4-7 membered ring optionally substituted by one or more substituents each independently selected from $R^c$; wherein when heterocyclyl contains a -NH- moiety, that -NH- moiety is optionally substituted by $R^d$; wherein $C_{2-6}$alkenyl and $C_{2-6}$alkynyl, are each independently optionally substituted by one or more substituents each independently selected from $R^e$; wherein $C_{1-6}$alkyl is optionally substituted by one or more substituents each independently selected from $R^f$; wherein $C_{3-6}$cycloalkyl is independently optionally substituted by one or more substituents each independently selected from $R^g$;

$R^b$ may be selected, independently for each occurrence, from the group consisting of halogen; hydroxyl; $-NO_2$; $-N_3$; -CN;

-SCN; $C_{1-6}$alkyl; $C_{2-6}$alkenyl; $C_{2-6}$alkynyl; $C_{3-6}$cycloalkyl; $C_{1-6}$alkoxy; $C_{3-6}$alkenyloxy; $C_{3-6}$alkynyloxy; $C_{3-6}$cycloalkoxy; $C_{1-6}$alkyl-S(O)$_w$-, where w is 0, 1, or 2; $C_{1-6}$alkyl$C_{3-6}$cycloalkyl-; $C_{3-6}$cycloalkyl-$C_{1-6}$alkyl-; $C_{1-6}$alkoxycarbonyl-N($R^a$)-; $C_{1-6}$alkylN($R^a$)-; $C_{1-6}$alkyl-N($R^a$)carbonyl-; $R^aR^{a'}$N-; $R^aR^{a'}$N-carbonyl-; $R^aR^{a'}$N-carbonyl-N($R^a$)-; $R^aR^{a'}$N-$SO_2$-; and $C_{1-6}$alkyl-carbonyl-N(R)-;

$R^a$ and $R^{a'}$ may be selected, independently for each occurrence, from the group consisting of hydrogen and $C_{1-6}$alkyl, or $R^a$ and $R^{a'}$ when taken together with the nitrogen to which they are attached form a 4-6 membered heterocyclic ring, wherein $C_{1-6}$alkyl is optionally substituted by one or more substituents each independently selected from the group consisting of halogen, oxo, and hydroxyl, and wherein the heterocyclic ring is optionally substituted by one or more substituents each independently selected from the group consisting of halogen, alkyl, oxo, or hydroxyl;

$R^c$ may be selected, independently for each occurrence, from the group consisting of halogen; hydroxyl; -$NO_2$; -$N_3$; -CN; -SCN; oxo; $C_{1-6}$alkyl; $C_{2-6}$alkenyl; $C_{2-6}$alkynyl; $C_{3-6}$cycloalkyl; $C_{1-6}$alkoxy; $C_{3-6}$alkenyloxy; $C_{3-6}$alkynyloxy; $C_{3-6}$cycloalkoxy; $C_{1-6}$alkyl-S(O)$_w$-, where w is 0, 1, or 2; $C_{1-6}$alkyl$C_{3-6}$cycloalkyl-; $C_{3-6}$cycloalkyl-$C_{1-6}$alkyl-; $C_{1-6}$alkoxycarbonyl-N($R^a$)-; $C_{1-6}$alkylN($R^a$)-; $C_{1-6}$alkyl-N($R^a$)carbonyl-; $R^aR^{a'}$N-; $R^aR^{a'}$N-carbonyl-; $R^aR^{a'}$N-carbonyl-N($R^a$)-; $R^aR^{a'}$N-$SO_2$-; and $C_{1-6}$alkyl-carbonyl-N($R^a$)-;

$R^d$ may be selected, independently for each occurrence, from the group consisting of $C_{1-6}$alkyl, $C_{1-6}$alkylcarbonyl, and $C_{1-6}$alkylsulfonyl, wherein $C_{1-6}$alkyl is optionally substituted by one or more substituents each independently selected from halogen, hydroxyl, and $R^aR^{a'}$N-;

$R^e$ may be selected, independently for each occurrence, from the group consisting of halogen; hydroxyl; -$NO_2$; -$N_3$; -CN; -SCN; $C_{1-4}$alkoxy; $C_{1-4}$alkoxycarbonyl; $R^aR^{a'}$N-; $R^aR^{a'}$N-carbonyl; $R^aR^{a'}$N-$SO_2$-; and $C_{1-4}$alkylS(O)$_w$-, where w is 0, 1, or 2;

$R^f$ may be selected, independently for each occurrence, from the group consisting of halogen; hydroxyl; -$NO_2$; -$N_3$; -CN; -SCN; $C_{1-4}$alkoxy; $C_{1-4}$alkoxycarbonyl; $R^aR^{a'}$N-; $R^aR^{a'}$N-carbonyl; $R^aR^{a'}$N-$SO_2$-; and $C_{1-4}$alkylS(O)$_w$-, where w is 0, 1, or 2;

$R^g$ may be selected, independently for each occurrence, from the group consisting of halogen, hydroxyl, -$NO_2$; -$N_3$; -CN; -SCN; $C_{1-6}$alkyl; $C_{1-4}$alkoxy; $C_{1-4}$alkoxycarbonyl; $R^aR^{a'}$N-; $R^aR^{a'}$N-carbonyl; $R^aR^{a'}$N-$SO_2$-; and $C_{1-4}$alkylS(O)$_w$-, where w is 0, 1, or 2;

$R^x$ may be selected, independently, from the group consisting of hydrogen; halogen; $C_{1-6}$alkyl; $C_{2-6}$alkenyl; $C_{2-6}$alkynyl; $C_{3-6}$cycloalkyl; phenyl; naphthyl; heteroaryl; heterocyclyl; $C_{3-6}$cycloalkyl-$C_{1-6}$alkyl-; phenyl-$C_{1-6}$alkyl-; naphthyl-$C_{1-6}$alkyl-; heteroaryl-$C_{1-6}$alkyl-; and heterocyclyl-$C_{1-6}$alkyl-; wherein heteroaryl is a 5-6 membered ring having one, two, or three heteroatoms each independently selected from N, O, or S; wherein heteroaryl is optionally substituted with one or more substituents each independently selected from $R^b$; wherein heterocyclyl is a 4-7 membered ring optionally substituted by one or more substituents each independently selected from $R^c$; wherein when heterocyclyl contains a -NH- moiety, that - NH- moiety is optionally substituted by $R^d$; wherein $C_{2-6}$alkenyl and $C_{2-6}$alkynyl, are each independently optionally substituted by one or more substituents each independently selected from $R^e$; wherein $C_{1-6}$alkyl is optionally substituted by one or more substituents each independently selected from $R^f$; wherein $C_{3-6}$cycloalkyl is independently optionally substituted by one or more substituents each independently selected from $R^g$.

In certain embodiments, at least one of $R^1$, $R^2$, $R^3$, and $R^4$ may be hydroxyl.

In some instances, at least one of $R^1$, $R^2$, $R^3$, and $R^4$ may be $C_1$-$C_6$ alkyl, optionally substituted with one, two, or three substituents selected independently from the group consisting of halogen, hydroxyl, -$NH_2$, and cyano.

In some embodiments, at least one of $R^5$ and $R^6$ may be -($C_1$-$C_6$ alkylene)-Ar. At least one of $R^5$ and $R^6$ may also be -$CH_2$-Ar. In some cases, at least one of $R^5$ and $R^6$ is -Q-phenyl. In certain examples, one of $R^5$ and $R^6$ may be hydrogen.

In some cases, $R^7$ and $R^8$ may be independently selected from the group consisting of hydrogen; halogen; hydroxyl; $C_1$-$C_6$ alkyl; phenyl; and naphthyl; or $R^7$ and $R^8$, together with the atoms to which they are attached, form a 4-6 membered heterocyclic or cycloalkyl ring; wherein $C_1$-$C_6$ alkyl, phenyl, naphthyl, the cycloalkyl ring, and the heterocyclic ring each may be substituted independently by one or more substituents selected from the group consisting of halogen; hydroxyl; -$NO_2$; -$N_3$; -CN; -SCN; $C_{1-4}$alkoxy; $C_{1-4}$alkoxycarbonyl; $R^aR^{a'}$N-; $R^aR^{a'}$N-carbonyl; $R^aR^{a'}$N-$SO_2$-; and $C_{1-4}$alkylS(O)$_w$-, where w is 0, 1, or 2; wherein $R^a$ and $R^{a'}$ may be selected, independently for each occurrence, from the group consisting of hydrogen and $C_{1-6}$alkyl, or $R^a$ and $R^{a'}$ when taken together with the nitrogen to which they are attached form a 4-6 membered heterocyclic ring, wherein $C_{1-6}$alkyl is optionally substituted by one or more substituents each independently selected from the group consisting of halogen, oxo, and hydroxyl, and wherein the heterocyclic ring is optionally substituted by one or more substituents each independently selected from the group consisting of halogen, alkyl, oxo, or hydroxyl.

In some cases, $R^7$ and $R^8$ may be hydrogen.

In an exemplary embodiment, a compound may be represented by:

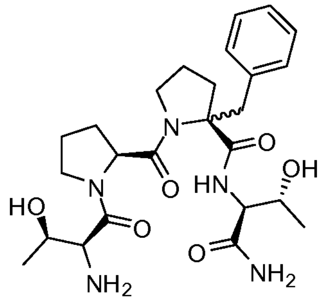

.

In another exemplary embodiment, a compound may be represented by:

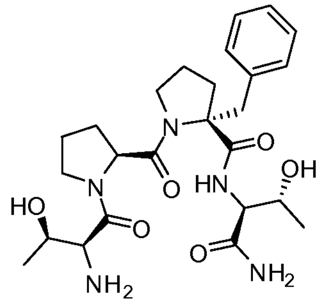

.

In yet another exemplary embodiment, a compound may be represented by:

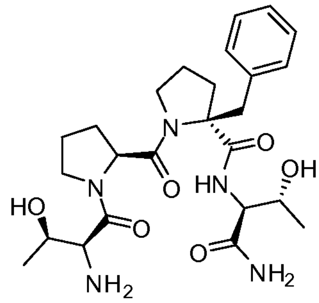

.

In still another exemplary embodiment, a compound may be represented by:

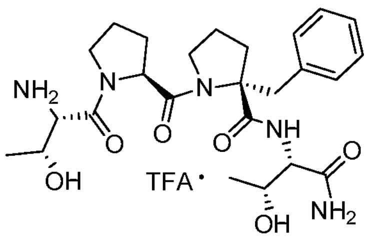
,
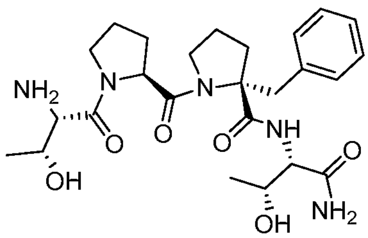
,
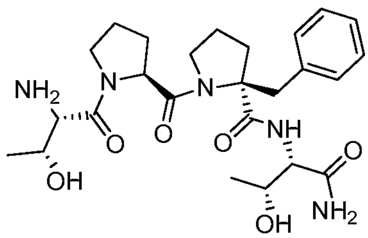
,

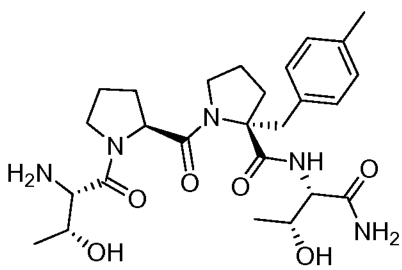
,
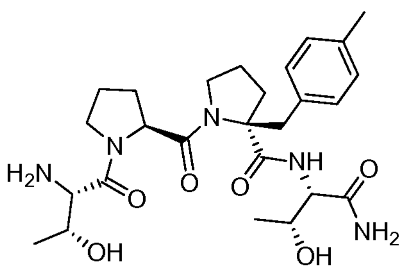
,

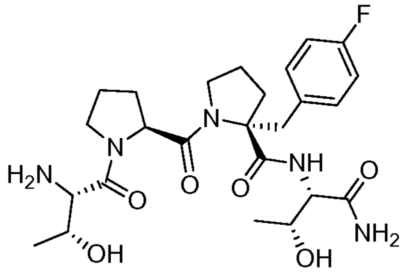 , 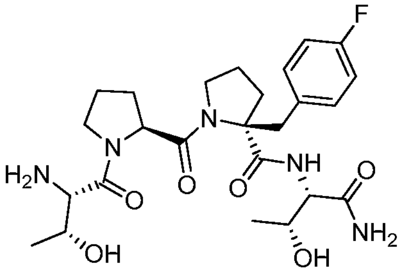 ,

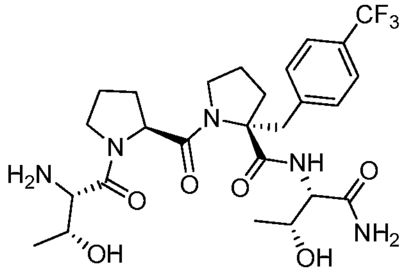 , 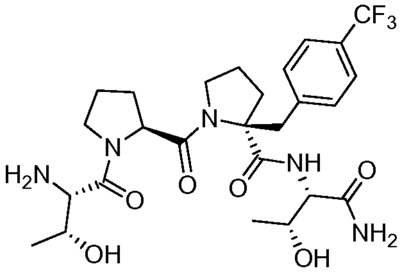 ,

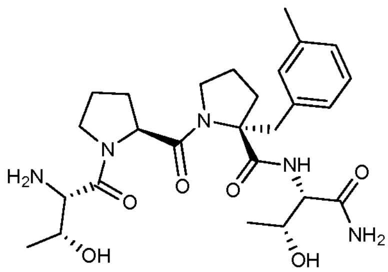 , 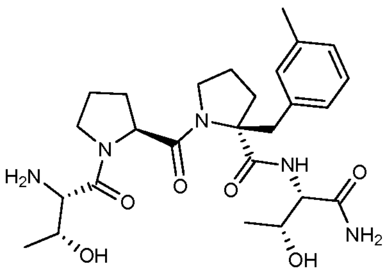 ,

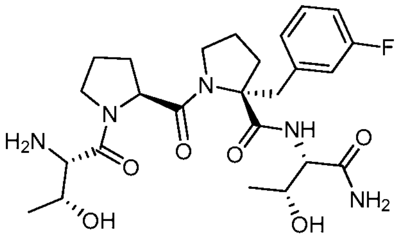 , 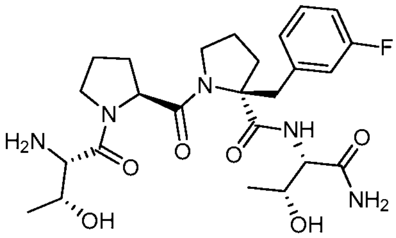 ,

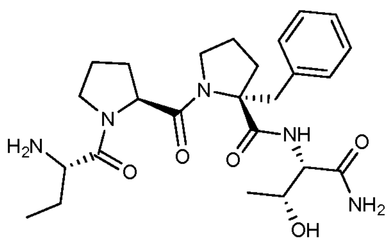 , 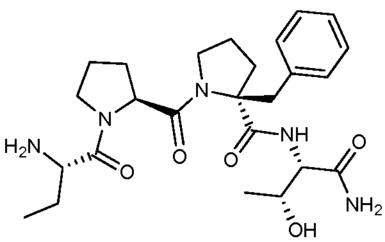 ,

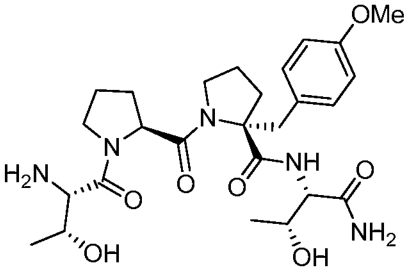 , 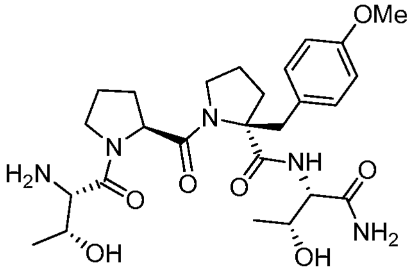 ,

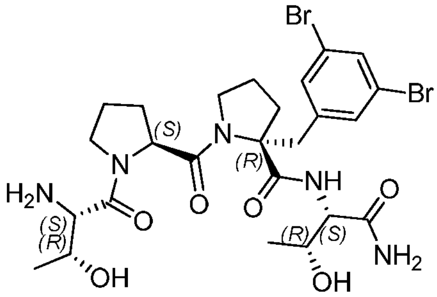 , 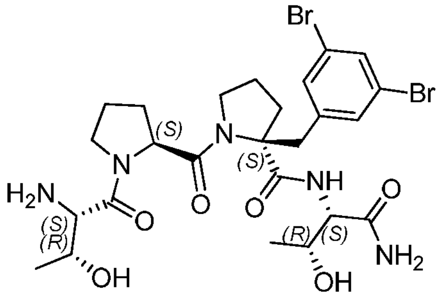 ,

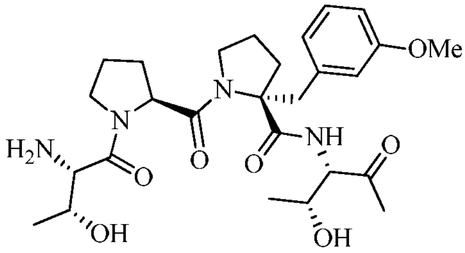 , 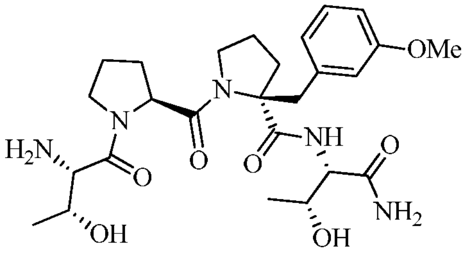 ,

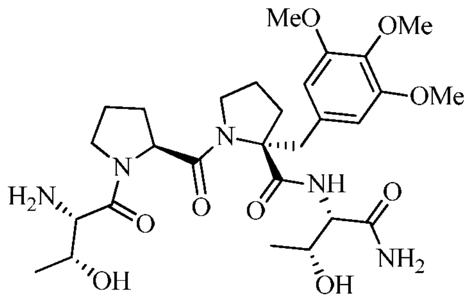 , or 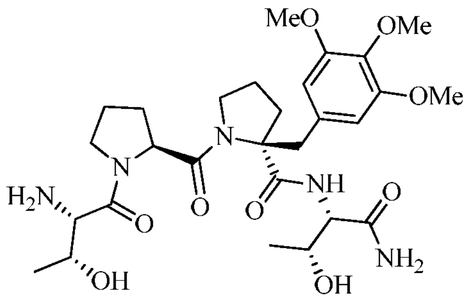 .

Disclosed reference compounds also include those represented by the formula:

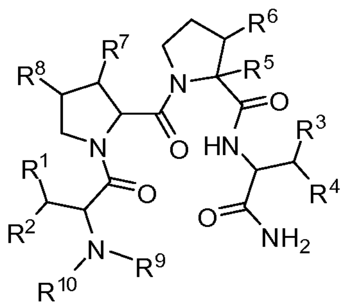

and pharmaceutically acceptable salts, stereoisomers, metabolites, and hydrates thereof, wherein:

$R^1$ and $R^3$ may be independently selected from the group consisting of hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted aryl; substituted or unsubstituted heteroaryl; $-OR^x$; $-NO_2$; $-N_3$; -CN; -SCN; $-SR^x$; $-C(O)R^x$; $-CO_2(R^x)$; $-C(O)N(R^x)_2$; $-C(NR^x)N(R^x)_2$; $-OC(O)R^x$; $-OCO_2R^x$;$-OC(O)N(R^x)_2$; $-N(R^x)_2$; $-SOR^x$; $-S(O)_2R^x$; $-NR^xC(O)R^x$; $-NR^xC(O)N(R^x)_2$; $-NR^xC(O)OR^x$;$-NR^xC(NR^x)N(R^x)_2$; and $-C(R^x)_3$; wherein each occurrence of $R^x$ is independently selected from the group consisting of hydrogen; halogen; acyl; optionally substituted aliphatic; optionally substituted heteroaliphatic; optionally substituted aryl; and optionally substituted heteroaryl;

$R^2$ and $R^4$ may be independently selected from the group consisting of hydrogen and - $OR^x$, provided that at least one of $R^2$ and $R^4$ is hydrogen, wherein $R^x$ is selected from the group consisting of hydrogen; halogen; acyl; optionally substituted aliphatic; optionally substituted heteroaliphatic; optionally substituted aryl; and optionally substituted heteroaryl;

$R^5$ and $R^6$ may be independently selected from the group consisting of -Q-Ar and hydrogen; wherein Q is independently selected from the group consisting of cyclic or acyclic, substituted or unsubstituted, branched or unbranched aliphatic; cyclic or acyclic, substituted or unsubstituted, branched or unbranched heteroaliphatic; and a bond; and wherein Ar is selected from the group consisting substituted or unsubstituted aryl, and substituted or unsubstituted heteroaryl; or $R^5$ and $R^6$, together with the atoms to which they are attached, form a substituted or unsubstituted 4-6 membered heterocyclic or cycloalkyl ring;

$R^7$ and $R^8$ are independently selected from the group consisting of hydrogen; halogen; hydroxyl; substituted or unsubstituted $C_1$-$C_6$ alkyl; substituted or unsubstituted $C_1$-$C_6$ alkoxy; and substituted or unsubstituted aryl; or $R^7$ and $R^8$, together with the atoms to which they are attached, form a substituted or unsubstituted 4-6 membered heterocyclic or cycloalkyl ring;

$R^9$ and $R^{10}$ may be independently selected from the group consisting of hydrogen; $C_1$-$C_6$ alkyl, optionally substituted by one or more substituents each independently selected from the group consisting of halogen, oxo, and hydroxyl; $C_{2-6}$alkenyl, optionally substituted by one or more substituents each independently selected from the group consisting of halogen, oxo, and hydroxyl; $C_{2-6}$alkynyl, optionally substituted by one or more substituents each independently selected from the group consisting of halogen, oxo, and hydroxyl; $C_{3-6}$cycloalkyl, optionally substituted by one or more substituents each independently selected from the group consisting of $C_{1-6}$alkyl, halogen, oxo, and hydroxyl; phenyl, optionally substituted by one or more substituents each independently selected from the group consisting of $C_{1-6}$alkyl; $C_{1-6}$alkoxy; halogen; hydroxyl; -C(O)$R^x$; -$CO_2$($R^x$); -C(O)N($R^x$)$_2$; -C(N$R^x$)N($R^x$)$_2$; and -C($R^x$)$_3$; wherein each occurrence of $R^x$ is independently selected from the group consisting of hydrogen; halogen; $C_{1-6}$alkyl; $C_{2-6}$alkenyl; $C_{2-6}$alkynyl; $C_{3-6}$cycloalkyl; and phenyl; or $R^9$ and $R^{10}$, together with N, form a 4-6 membered heterocyclic ring, optionally substituted by one or more substituents each independently selected from the group consisting of $C_{1-6}$alkyl, halogen, oxo, and hydroxyl.

In some embodiments, $R^1$ and $R^3$ may be independently selected from the group consisting of hydrogen; halogen; $C_{1-6}$alkyl; $C_{2-6}$alkenyl; $C_{2-6}$alkynyl; $C_{3-6}$cycloalkyl; phenyl; naphthyl; heteroaryl; heterocyclyl; $C_{3-6}$cycloalkyl-$C_{1-6}$alkyl-; phenyl-$C_{1-6}$alkyl-; naphthyl-$C_{1-6}$alkyl-; heteroaryl-$C_{1-6}$alkyl-; and heterocyclyl-$C_{1-6}$alkyl-; -O$R^x$; -$NO_2$; -$N_3$; -CN; -SCN; -S$R^X$;-C(O)$R^x$; -$CO_2$($R^x$); -C(O)N($R^x$)$_2$; -C(N$R^x$)N($R^x$)$_2$; -OC(O)$R^x$; -$OCO_2R^x$; -OC(O)N($R^x$)$_2$;-N($R^x$)$_2$; -SO$R^x$; -S(O)$_2R^x$; -N$R^x$C(O)$R^x$; -N$R^x$C(O)N($R^x$)$_2$; -N$R^x$C(O)O$R^x$; -N$R^x$C(N$R^x$)N($R^x$)$_2$; and -C($R^x$)$_3$; wherein heteroaryl is a 5-6 membered ring having one, two, or three heteroatoms each independently selected from N, O, or S; wherein heteroaryl is optionally substituted with one or more substituents each independently selected from $R^b$; wherein heterocyclyl is a 4-7 membered ring optionally substituted by one or more substituents each independently selected from $R^c$; wherein when heterocyclyl contains a -NH- moiety, that -NH- moiety is optionally substituted by $R^d$; wherein $C_{2-6}$alkenyl and $C_{2-6}$alkynyl, are each independently optionally substituted by one or more substituents each independently selected from $R^e$; wherein $C_{1-6}$alkyl is optionally substituted by one or more substituents each independently selected from $R^f$; wherein $C_{3-6}$cycloalkyl is independently optionally substituted by one or more substituents each independently selected from $R^g$;

$R^b$ may be selected, independently for each occurrence, from the group consisting of halogen; hydroxyl; -$NO_2$; -$N_3$; -CN; -SCN; $C_{1-6}$alkyl; $C_{2-6}$alkenyl; $C_{2-6}$alkynyl; $C_{3-6}$cycloalkyl; $C_{1-6}$alkoxy; $C_{3-6}$alkenyloxy; $C_{3-6}$alkynyloxy; $C_{3-6}$cycloalkoxy; $C_{1-6}$alkyl-S(O)$_w$-, where w is 0, 1, or 2; $C_{1-6}$alkyl$C_{3-6}$cycloalkyl-; $C_{3-6}$cycloalkyl-$C_{1-6}$alkyl-; $C_{1-6}$alkoxycarbonyl-N($R^a$)-; $C_{1-6}$alkylN($R^a$)-; $C_{1-6}$alkyl-N($R^a$)carbonyl-; $R^aR^{a'}$N-; $R^aR^{a'}$N-carbonyl-; $R^aR^{a'}$N-carbonyl-N($R^a$)-; $R^aR^{a'}$N-$SO_2$-; and $C_{1-6}$alkyl-carbonyl-N($R^a$)-;

$R^a$ and $R^{a'}$ may be selected, independently for each occurrence, from the group consisting of hydrogen and $C_{1-6}$alkyl, or $R^a$ and $R^{a'}$ when taken together with the nitrogen to which they are attached form a 4-6 membered heterocyclic ring, wherein $C_{1-6}$alkyl is optionally substituted by one or more substituents each independently selected from the group consisting of halogen, oxo, and hydroxyl, and wherein the heterocyclic ring is optionally substituted by one or more substituents each independently selected from the group consisting of halogen, alkyl, oxo, or hydroxyl;

$R^c$ may be selected, independently for each occurrence, from the group consisting of halogen; hydroxyl; -$NO_2$; -$N_3$; -CN; -SCN; oxo; $C_{1-6}$alkyl; $C_{2-6}$alkenyl; $C_{2-6}$alkynyl; $C_{3-6}$cycloalkyl; $C_{1-6}$alkoxy; $C_{3-6}$alkenyloxy; $C_{3-6}$alkynyloxy; $C_{3-6}$cycloalkoxy; $C_{1-6}$alkyl-S(O)$_w$-, where w is 0, 1, or 2; $C_{1-6}$alkyl$C_{3-6}$cycloalkyl-; $C_{3-6}$cycloalkyl-$C_{1-6}$alkyl-; $C_{1-6}$alkoxycarbonyl-N($R^a$)-; $C_{1-6}$alkylN($R^a$)-; $C_{1-6}$alkyl-N($R^a$)carbonyl-; $R^aR^{a'}$N-; $R^aR^{a'}$N-carbonyl-; $R^aR^{a'}$N-carbonyl-N($R^a$)-; $R^aR^{a'}$N-$SO_2$-; and $C_{1-6}$alkyl-carbonyl-N($R^a$)-;

$R^d$ may be selected, independently for each occurrence, from the group consisting of $C_{1-6}$alkyl, $C_{1-6}$alkylcarbonyl, and $C_{1-6}$alkylsulfonyl, wherein $C_{1-6}$alkyl is optionally substituted by one or more substituents each independently selected from halogen, hydroxyl, and $R^aR^{a'}$N-;

$R^e$ may be selected, independently for each occurrence, from the group consisting of halogen; hydroxyl; -$NO_2$; -$N_3$; -CN; -SCN; $C_{1-4}$alkoxy; $C_{1-4}$alkoxycarbonyl; $R^aR^{a'}$N-; $R^aR^{a'}$N-carbonyl; $R^aR^{a'}$N-$SO_2$-; and $C_{1-4}$alkylS(O)$_w$-, where w is 0, 1, or 2;

$R^f$ may be selected, independently for each occurrence, from the group consisting of halogen; hydroxyl; -$NO_2$; -$N_3$; -CN;

-SCN; $C_{1-4}$alkoxy; $C_{1-4}$alkoxycarbonyl; $R^aR^{a'}N$-; $R^aR^{a'}N$-carbonyl; $R^aR^{a'}N-SO_2$-; and $C_{1-4}$alkylS(O)$_w$-, where w is 0, 1, or 2;

$R^g$ may be selected, independently for each occurrence, from the group consisting of halogen, hydroxyl, -$NO_2$; -$N_3$; -CN; -SCN; $C_{1-6}$alkyl; $C_{1-4}$alkoxy; $C_{1-4}$alkoxycarbonyl; $R^aR^{a'}N$-; $R^aR^{a'}N$-carbonyl; $R^aR^{a'}N-SO_2$-; and $C_{1-4}$alkylS(O)$_w$-, where w is 0, 1, or 2;

$R^x$ may be selected, independently, from the group consisting of hydrogen; halogen; $C_{1-6}$alkyl; $C_{2-6}$alkenyl; $C_{2-6}$alkynyl; $C_{3-6}$cycloalkyl; phenyl; naphthyl; heteroaryl; heterocyclyl; $C_{3-6}$cycloalkyl-$C_{1-6}$alkyl-; phenyl-$C_{1-6}$alkyl-; naphthyl-$C_{1-6}$alkyl-; heteroaryl-$C_{1-6}$alkyl-; and heterocyclyl-$C_{1-6}$alkyl-; wherein heteroaryl is a 5-6 membered ring having one, two, or three heteroatoms each independently selected from N, O, or S; wherein heteroaryl is optionally substituted with one or more substituents each independently selected from $R^b$; wherein heterocyclyl is a 4-7 membered ring optionally substituted by one or more substituents each independently selected from $R^c$; wherein when heterocyclyl contains a -NH- moiety, that - NH- moiety is optionally substituted by $R^d$; wherein $C_{2-6}$alkenyl and $C_{2-6}$alkynyl, are each independently optionally substituted by one or more substituents each independently selected from $R^e$; wherein $C_{1-6}$alkyl is optionally substituted by one or more substituents each independently selected from $R^f$; wherein $C_{3-6}$cycloalkyl is independently optionally substituted by one or more substituents each independently selected from $R^g$.

In some cases, $R^2$ and $R^4$ may be independently selected from the group consisting of hydrogen and -$OR^x$, provided that at least one of $R^2$ and $R^4$ is hydrogen, wherein $R^x$ may be selected from the group consisting of hydrogen; halogen; $C_{1-6}$alkyl; $C_{2-6}$alkenyl; $C_{2-6}$alkynyl; $C_{3-6}$cycloalkyl; phenyl; naphthyl; heteroaryl; heterocyclyl; $C_{3-6}$cycloalkyl-$C_{1-6}$alkyl-; phenyl-$C_{1-6}$alkyl-; naphthyl-$C_{1-6}$alkyl-; heteroaryl-$C_{1-6}$alkyl-; and heterocyclyl-$C_{1-6}$alkyl-; wherein heteroaryl is a 5-6 membered ring having one, two, or three heteroatoms each independently selected from N, O, or S; wherein heteroaryl is optionally substituted with one or more substituents each independently selected from $R^b$; wherein heterocyclyl is a 4-7 membered ring optionally substituted by one or more substituents each independently selected from $R^c$; wherein when heterocyclyl contains a -NH- moiety, that -NH- moiety is optionally substituted by $R^d$; wherein $C_{2-6}$alkenyl and $C_{2-6}$alkynyl, are each independently optionally substituted by one or more substituents each independently selected from $R^e$; wherein $C_{1-6}$alkyl is optionally substituted by one or more substituents each independently selected from $R^f$; wherein $C_{3-6}$cycloalkyl is independently optionally substituted by one or more substituents each independently selected from $R^g$;

$R^b$ may be selected, independently for each occurrence, from the group consisting of halogen; hydroxyl; -$NO_2$; -$N_3$; -CN; -SCN; $C_{1-6}$alkyl; $C_{2-6}$alkenyl; $C_{2-6}$alkynyl; $C_{3-6}$cycloalkyl; $C_{1-6}$alkoxy; $C_{3-6}$alkenyloxy; $C_{3-6}$alkynyloxy; $C_{3-6}$cycloalkoxy; $C_{1-6}$alkyl-S(O)$_w$-, where w is 0, 1, or 2; $C_{1-6}$alkyl$C_{3-6}$cycloalkyl-; $C_{3-6}$cycloalkyl-$C_{1-6}$alkyl-; $C_{1-6}$alkoxycarbonyl-N($R^a$)-; $C_{1-6}$alkylN($R^a$)-; $C_{1-6}$alkyl-N($R^a$)carbonyl-; $R^aR^{a'}N$-; $R^aR^{a'}N$-carbonyl-; $R^aR^{a'}N$-carbonyl-N($R^a$)-; $R^aR^{a'}N-SO_2$-; and $C_{1-6}$alkyl-carbonyl-N($R^a$)-;

$R^a$ and $R^{a'}$ may be selected, independently for each occurrence, from the group consisting of hydrogen and $C_{1-6}$alkyl, or $R^a$ and $R^{a'}$ when taken together with the nitrogen to which they are attached form a 4-6 membered heterocyclic ring, wherein $C_{1-6}$alkyl is optionally substituted by one or more substituents each independently selected from the group consisting of halogen, oxo, and hydroxyl, and wherein the heterocyclic ring is optionally substituted by one or more substituents each independently selected from the group consisting of halogen, alkyl, oxo, or hydroxyl;

$R^c$ may be selected, independently for each occurrence, from the group consisting of halogen; hydroxyl; -$NO_2$; -$N_3$; -CN; -SCN; oxo; $C_{1-6}$alkyl; $C_{2-6}$alkenyl; $C_{2-6}$alkynyl; $C_{3-6}$cycloalkyl; $C_{1-6}$alkoxy; $C_{3-6}$alkenyloxy; $C_{3-6}$alkynyloxy; $C_{3-6}$cycloalkoxy; $C_{1-6}$alkyl-S(O)$_w$-, where w is 0, 1, or 2; $C_{1-6}$alkyl$C_{3-6}$cycloalkyl-; $C_{3-6}$cycloalkyl-$C_{1-6}$alkyl-; $C_{1-6}$alkoxycarbonyl-N($R^a$)-; $C_{1-6}$alkylN($R^a$)-; $C_{1-6}$alkyl-N($R^a$)carbonyl-; $R^aR^{a'}N$-; $R^aR^{a'}N$-carbonyl-; $R^aR^{a'}N$-carbonyl-N($R^a$)-; $R^aR^{a'}N-SO_2$-; and $C_{1-6}$alkyl-carbonyl-N($R^a$)-;

$R^d$ may be selected, independently for each occurrence, from the group consisting of $C_{1-6}$alkyl, $C_{1-6}$alkylcarbonyl, and $C_{1-6}$alkylsulfonyl, wherein $C_{1-6}$alkyl is optionally substituted by one or more substituents each independently selected from halogen, hydroxyl, and $R^aR^{a'}N$-;

$R^e$ may be selected, independently for each occurrence, from the group consisting of halogen; hydroxyl; -$NO_2$; -$N_3$; -CN; -SCN; $C_{1-4}$alkoxy; $C_{1-4}$alkoxycarbonyl; $R^aR^{a'}N$-; $R^aR^{a'}N$-carbonyl; $R^aR^{a'}N-SO_2$-; and $C_{1-4}$alkylS(O)$_w$-, where w is 0, 1, or 2;

$R^f$ may be selected, independently for each occurrence, from the group consisting of halogen; hydroxyl; -$NO_2$; -$N_3$; -CN; -SCN; $C_{1-4}$alkoxy; $C_{1-4}$alkoxycarbonyl; $R^aR^{a'}N$-; $R^aR^{a'}N$-carbonyl; $R^aR^{a'}N-SO_2$-; and $C_{1-4}$alkylS(O)$_w$-, where w is 0, 1, or 2;

$R^g$ may be selected, independently for each occurrence, from the group consisting of halogen, hydroxyl, -$NO_2$; -$N_3$; -CN; -SCN; $C_{1-6}$alkyl; $C_{1-4}$alkoxy; $C_{1-4}$alkoxycarbonyl; $R^aR^{a'}N$-; $R^aR^{a'}N$-carbonyl; $R^aR^{a'}N-SO_2$-; and $C_{1-4}$alkylS(O)$_w$-, where w is 0, 1, or 2.

In certain embodiments, $R^5$ and $R^6$ may be independently selected from the group consisting of -Q-Ar and hydrogen; wherein Q is independently selected from the group consisting of $C_{1-6}$alkyl; $C_{2-6}$alkenyl; $C_{2-6}$alkynyl; $C_{3-6}$cycloalkyl; heterocyclyl; $C_{3-6}$cycloalkyl-$C_{1-6}$alkyl-; heterocyclyl-$C_{1-6}$alkyl-; and a bond; and wherein Ar is selected from the group consisting substituted or unsubstituted phenyl, naphthyl, and heteroaryl; or $R^5$ and $R^6$, together with the

atoms to which they are attached, form a 4-6 membered heterocyclic or cycloalkyl ring, optionally substituted by one or more substituents each independently selected from halogen, hydroxyl, $-NO_2$; $-N_3$; -CN; -SCN; $C_{1-6}$alkyl; $C_{1-4}$alkoxy; $C_{1-4}$alkoxycarbonyl; $R^aR^{a'}N$-; $R^aR^{a'}N$-carbonyl; $R^aR^{a'}N-SO_2$-; and $C_{1-4}$alkylS(O)$_w$-, where w is 0, 1, or 2; and wherein $R^a$ and $R^{a'}$ may be selected, independently for each occurrence, from the group consisting of hydrogen and $C_{1-6}$alkyl, or $R^a$ and $R^{a'}$ when taken together with the nitrogen to which they are attached form a 4-6 membered heterocyclic ring, wherein $C_{1-6}$alkyl is optionally substituted by one or more substituents each independently selected from the group consisting of halogen, oxo, and hydroxyl, and wherein the heterocyclic ring is optionally substituted by one or more substituents each independently selected from the group consisting of halogen, alkyl, oxo, or hydroxyl.

In certain embodiments, at least one of $R^1$, $R^2$, $R^3$, and $R^4$ may be hydroxyl.

In some instances, at least one of $R^1$, $R^2$, $R^3$, and $R^4$ may be $C_1$-$C_6$ alkyl, optionally substituted with one, two, or three substituents selected independently from the group consisting of halogen, hydroxyl, $-NH_2$, and cyano.

In some embodiments, at least one of $R^5$ and $R^6$ may be -($C_1$-$C_6$ alkylene)-Ar, e.g., one of $R^5$ and $R^6$ may also be $-CH_2$-Ar. In some cases, at least one of $R^5$ and $R^6$ is -Q-phenyl. In certain examples, one of $R^5$ and $R^6$ may be hydrogen.

In some cases, $R^7$ and $R^8$ may be independently selected from the group consisting of hydrogen; halogen; hydroxyl; $C_1$-$C_6$ alkyl; phenyl; and naphthyl; or $R^7$ and $R^8$, together with the atoms to which they are attached, form a 4-6 membered heterocyclic or cycloalkyl ring; wherein $C_1$-$C_6$ alkyl, phenyl, naphthyl, the cycloalkyl ring, and the heterocyclic ring each may be substituted independently by one or more substituents selected from the group consisting of halogen; hydroxyl; $-NO_2$; $-N_3$; -CN; -SCN; $C_{1-4}$alkoxy; $C_{1-4}$alkoxycarbonyl; $R^aR^{a'}N$-; $R^aR^{a'}N$-carbonyl; $R^aR^{a'}N-SO_2$-; and $C_{1-4}$alkylS(O)$_w$-, where w is 0, 1, or 2; wherein $R^a$ and $R^{a'}$ may be selected, independently for each occurrence, from the group consisting of hydrogen and $C_{1-6}$alkyl, or $R^a$ and $R^{a'}$ when taken together with the nitrogen to which they are attached form a 4-6 membered heterocyclic ring, wherein $C_{1-6}$alkyl is optionally substituted by one or more substituents each independently selected from the group consisting of halogen, oxo, and hydroxyl, and wherein the heterocyclic ring is optionally substituted by one or more substituents each independently selected from the group consisting of halogen, alkyl, oxo, or hydroxyl.

In some cases, $R^7$ and $R^8$ may be hydrogen.

In a reference embodiment, a compound may be represented by:

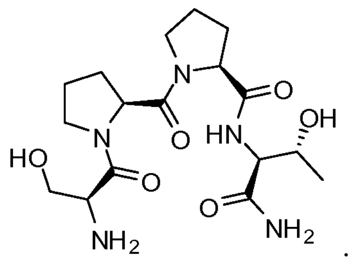

In another reference embodiment, a compound may be represented by:

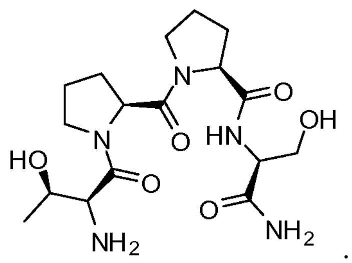

In yet another reference embodiment, a compound may be represented by:

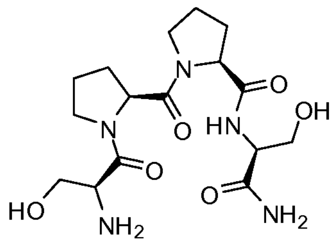

.

The compounds of the present disclosure and formulations thereof may have a plurality of chiral centers. Each chiral center may be independently *R, S,* or any mixture of *R* and S. For example, in some embodiments, a chiral center may have an *R*:*S* ratio of between about 100:0 and about 50:50, between about 100:0 and about 75:25, between about 100:0 and about 85:15, between about 100:0 and about 90:10, between about 100:0 and about 95:5, between about 100:0 and about 98:2, between about 100:0 and about 99:1, between about 0:100 and 50:50, between about 0:100 and about 25:75, between about 0:100 and about 15:85, between about 0:100 and about 10:90, between about 0:100 and about 5:95, between about 0:100 and about 2:98, between about 0:100 and about 1:99, between about 75:25 and 25:75, and about 50:50. Formulations of the disclosed compounds comprising a greater ratio of one or more isomers (i.e., R and/or S) may possess enhanced therapeutic characteristic relative to racemic formulations of a disclosed compounds or mixture of compounds.

Disclosed compounds may provide for efficient cation channel opening at the NMDA receptor, e.g. may bind or associate with the glutamate site of the NMDA receptor to assist in opening the cation channel. The disclosed compounds may be used to regulate (turn on or turn off) the NMDA receptor through action as an agonist.

The compounds as described herein may be glycine site NMDA receptor partial agonists. A partial agonist as used in this context will be understood to mean that at a low concentration, the analog acts as an agonist and at a high concentration, the analog acts as an antagonist. Glycine binding is not inhibited by glutamate or by competitive inhibitors of glutamate, and also does not bind at the same site as glutamate on the NMDA receptor. A second and separate binding site for glycine exists at the NMDA receptor. The ligand-gated ion channel of the NMDA receptor is, thus, under the control of at least these two distinct allosteric sites. Disclosed compounds may be capable of binding or associating with the glycine binding site of the NMDA receptor. In some embodiments, disclosed compounds may possess a potency that is 10-fold or greater than the activity of existing NMDA receptor glycine site partial agonists. For example, disclosed compounds may possess a 10-fold to 20-fold enhanced potency compared to GLYX-13. GLYX-13 is represented by:

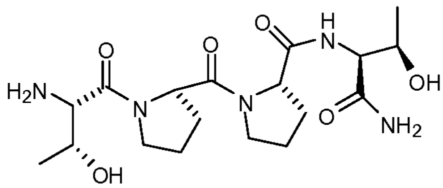

.

For example, provided herein are compounds that may be at least about 20-fold more potent as compared to GLYX-13, as measured by burst activated NMDA receptor-gated single neuron conductance ($I_{NMDA}$) in a culture of hippocampal CA1 pyramidal neurons at a concentration of 50 nM. In another embodiment, a provided compound may be capable of generating an enhanced single shock evoked NMDA receptor-gated single neuron conductance ($I_{NMDA}$) in hippocampal CA1 pyramidal neurons at concentrations of 100 nM to 1 μM. Disclosed compounds may have enhanced potency as compared to GLYX-13 as measured by magnitude of long term potentiation (LTP) at Schaffer collateral-CA-1 synapses in *in vitro* hippocampal slices.

The disclosed compounds may exhibit a high therapeutic index. The therapeutic index, as used herein, refers to the ratio of the dose that produces a toxicity in 50% of the population (i.e., $TD_{50}$) to the minimum effective dose for 50% of the population (i.e., $ED_{50}$). Thus, the therapeutic index = ($TD_{50}$):($ED_{50}$). In some embodiments, a disclosed compound may have a therapeutic index of at least about 10:1, at least about 50:1, at least about 100:1, at least about 200:1, at least about 500:1, or at least about 1000:1.

## Compositions

In other aspects, formulations and compositions comprising the disclosed compounds and optionally a pharmaceutically acceptable excipient are provided. In some embodiments, a contemplated formulation comprises a racemic mixture of one or more of the disclosed compounds.

Contemplated formulations may be prepared in any of a variety of forms for use. By way of example, the compounds may be prepared in a formulation suitable for oral administration, subcutaneous injection, or other methods for administering an active agent to an animal known in the pharmaceutical arts.

Amounts of a disclosed compound as described herein in a formulation may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier.

The specification for the dosage unit forms of the invention are dictated by and directly dependent on (a) the unique characteristics of the compound selected and the particular therapeutic effect to be achieved, and (b) the limitations inherent in the art of compounding such an active compound for the treatment of sensitivity in individuals.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin.

The compounds can be administered in a time release formulation, for example in a composition which includes a slow release polymer. The compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are generally known to those skilled in the art.

Sterile injectable solutions can be prepared by incorporating the compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

In some embodiments, certain disclosed compounds are capable of delivering an efficiacous amount of compound when administered to a patient orally. For example, in certain embodiments, certain disclosed compounds are more efficacious when administered orally to a patient as compared to oral administration to a patient of a peptidyl compound represented by:

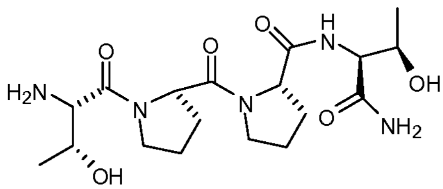

.

In accordance with an alternative aspect of the invention, a compound may be formulated with one or more additional compounds that enhance the solubility of the compound.

**Methods**

One or more of the disclosed compounds for use in methods for treating cognitive disorders and for enhancing learning are provided. Such methods include administering a pharmaceutically acceptable formulation of one or more of the disclosed compounds to a patient in need thereof. Also contemplated are methods of treating patients suffering from, memory deficits associated with aging, schizophrenia, special learning disorders, seizures, post-stroke convulsions,

brain ischemia, hypoglycemia, cardiac arrest, epilepsy, migraine, as well as Huntington's, Parkinson's, and Alzheimer's disease.

Other methods contemplated include the treatment of cerebral ischemia, stroke, brain trauma, brain tumors, acute neuropathic pain, chronic neuropathic pain, sleep disorders, drug addiction, depression, certain vision disorders, ethanol withdrawal, anxiety, memory and learning disabilities, autism, epilepsy, AIDS dementia, multiple system atrophy, progressive supra-nuclear palsy, Friedrich's ataxia, Down's syndrome, fragile X syndrome, tuberous sclerosis, olivio-ponto-cerebellar atrophy, cerebral palsy, drug-induced optic neuritis, peripheral neuropathy, myelopathy, ischemic retinopathy, diabetic retinopathy, glaucoma, cardiac arrest, behavior disorders, impulse control disorders, Alzheimer's disease, memory loss that accompanies early stage Alzheimer's disease, attention deficit disorder, ADHD, schizophrenia, amelioration of opiate, nicotine addiction, ethanol addition, traumatic brain injury, spinal cord injury, post-traumatic stress syndrome, and Huntington's chorea.

For example, provided herein is a disclosed compound for use in a method of treating depression in a patient in need thereof, comprising administering a disclosed compound, e.g., by acutely administering a disclosed compound. In certain embodiments, the treatment-resistant patient is identified as one who has been treated with at least two types of antidepressant treatments prior to administration of a disclosed compound. In other embodiments, the treatment-resistant patient is one who is identified as unwilling or unable to tolerate a side effect of at least one type of antidepressant treatment.

The most common depression conditions include Major Depressive Disorder and Dysthymic Disorder. Other depression conditions develop under unique circumstances. Such depression conditions include Psychotic depression, Postpartum depression, Seasonal affective disorder (SAD), mood disorder, depressions caused by chronic medical conditions such as cancer or chronic pain, chemotherapy, chronic stress, post traumatic stress disorders, and Bipolar disorder (or manic depressive disorder).

Refractory depression occurs in patients suffering from depression who are resistant to standard pharmacological treatments, including tricyclic antidepressants, MAOIs, SSRIs, and double and triple uptake inhibitors and/or anxiolytic drugs, as well non-pharmacological treatments such as psychotherapy, electroconvulsive therapy, vagus nerve stimulation and/or transcranial magnetic stimulation. A treatment-resistant patient may be identified as one who fails to experience alleviation of one or more symptoms of depression (e.g., persistent anxious or sad feelings, feelings of helplessness, hopelessness, pessimism) despite undergoing one or more standard pharmacological or non-pharmacological treatment. In certain embodiments, a treatment-resistant patient is one who fails to experience alleviation of one or more symptoms of depression despite undergoing treatment with two different antidepressant drugs. In other embodiments, a treatment-resistant patient is one who fails to experience alleviation of one or more symptoms of depression despite undergoing treatment with four different antidepressant drugs. A treatment-resistant patient may also be identified as one who is unwilling or unable to tolerate the side effects of one or more standard pharmacological or non-pharmacological treatment.

In yet another aspect, a disclosed compound for use in a method for enhancing pain relief and for providing analgesia to an animal is provided.

In certain embodiments, a disclosed compound for use in methods for treating schizophrenia is provided. For example, paranoid type schizophrenia, disorganized type schizophrenia (i.e., hebephrenic schizophrenia), catatonic type schizophrenia, undifferentiated type schizophrenia, residual type schizophrenia, post-schizophrenic depression, and simple schizophrenia may be treated using the methods and compositions contemplated herein. Psychotic disorders such as schizoaffective disorders, delusional disorders, brief psychotic disorders, shared psychotic disorders, and psychotic disorders with delusions or hallucinations may also be treated using the compositions contemplated herein.

Paranoid schizophrenia may be characterized where delusions or auditory hallucinations are present, but thought disorder, disorganized behavior, or affective flattening are not. Delusions may be persecutory and/or grandiose, but in addition to these, other themes such as jealousy, religiosity, or somatization may also be present.

Disorganized type schizophrenia may be characterized where thought disorder and flat affect are present together.

Catatonic type schizophrenia may be characterized where the subject may be almost immobile or exhibit agitated, purposeless movement. Symptoms can include catatonic stupor and waxy flexibility.

Undifferentiated type schizophrenia may be characterized where psychotic symptoms are present but the criteria for paranoid, disorganized, or catatonic types have not been met.

Residual type schizophrenia may be characterized where positive symptoms are present at a low intensity only.

Post-schizophrenic depression may be characterized where a depressive episode arises in the aftermath of a schizophrenic illness where some low-level schizophrenic symptoms may still be present.

Simple schizophrenia may be characterized by insidious and progressive development of prominent negative symptoms with no history of psychotic episodes.

In some embodiments, a disclosed compound for use in methods is provided for treating psychotic symptoms that may be present in other mental disorders, including bipolar disorder, borderline personality disorder, drug intoxication,

and drug-induced psychosis.

In another embodiment, a disclosed compound for use in methods for treating delusions (e.g., "non-bizarre") that may be present in, for example, delusional disorder is provided.

Also provided is a disclosed compound for use in methods for treating social withdrawal in conditions including social anxiety disorder, avoidant personality disorder, and schizotypal personality disorder.

Additionally, a disclosed compound for use in methods is provided for treating obsessive-compulsive disorder (OCD).

## EXAMPLES

The following examples are provided for illustrative purposes only, and are not intended to limit the scope of the disclosure.

Reference Example 1 - Synthesis of (*S*)-*N*-((2*S*,3*R*)-1-amino-3-hydroxy-1-oxobutan-2-yl)-1-((*S*)-1-((*S*)-2-amino-3-hydroxypropanoyl)pyrrolidine-2-carbonyl)pyrrolidine-2-carboxamide (CM-1).

The following reaction sequence was used (Scheme A) to synthesize (*S*)-*N*-((2*S*,3*R*)-1-amino-3-hydroxy-1-oxobutan-2-yl)-1-((*S*)-1-((*S*)-2-amino-3-hydroxypropanoyl)pyrrolidine-2-carbonyl)pyrrolidine-2-carboxamide (CM-1):

Scheme A. Synthesis of CM-1.

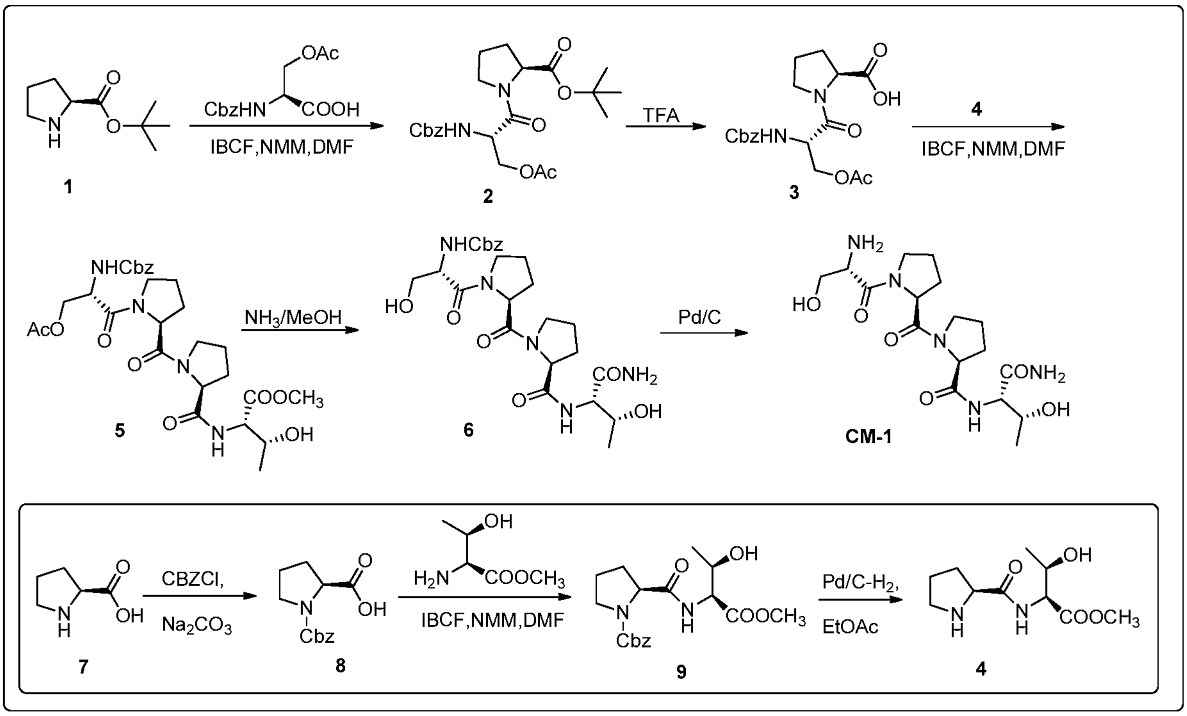


Synthesis of (*S*)-tert-butyl 1-((*S*)-3-acetoxy-2-(benzyloxycarbonylamino)-propanoyl)-pyrrolidine-2-carboxylate (**2**):

(*S*)-3-Acetoxy-2-(benzyloxycarbonylamino)-propanoic acid (1.5 g, 5.33 mmol) was dissolved in $CH_2Cl_2$ (15 mL). *N*-Methylmorpholine (NMM) (0.64 mL, 5.87 mmol) and isobutyl chloroformate (IBCF) (0.72 mL, 6.12 mmol) were added at -15 °C and stirred for 30 minutes under inert atmosphere. A mixture of (*S*)-tert-butyl pyrrolidine-2-carboxylate (**1**) (998 mg, 5.87 mmol) and NMM (0.64 mL, 5.87 mmol) in DMF (5 mL) were added drop wise to the reaction mixture and stirring was continued for another 3 h at RT. The reaction mixture was diluted with DCM (200 mL), washed with water (50 mL), citric acid solution (10 mL) and brine (10 mL). The separated organic layer was dried over anhydrous $Na_2SO_4$ and concentrated under reduced pressure. The obtained crude residue was purified by silica gel column chromatography eluting with 30% EtOAc/Hexane to afford compound 2 (1.6 g, 69.5%).

**$^1$H-NMR:** (200 MHz, DMSO-$d_6$): δ 7.81-7.76 (d, *J* = 20.5 Hz, 1H), 7.35-7.30 (m, 5H), 5.03-4.97 (m, 2H), 4.61-4.55 (m, 1H), 4.32-4.16 (m, 2H), 4.08-3.87 (m, 2H), 3.65-3.59 (m, 1H), 2.21-2.11 (m, 2H), 1.98 (s, 3H), 1.91-1.75 (m, 2H), 1.37 (s, 9H).

**Mass m/z:** 435.0 [$M^{+}$+1].

Synthesis of (*S*)-1-((*S*)-3-acetoxy-2-(benzyloxycarbonylamino)-propanoyl)-pyrrolidine-2-carboxylic acid (**3**):

To a solution of compound **2** (1 g, 2.30 mmol) in $CH_2Cl_2$ (5 mL) was added 20% TFA-DCM (10mL) and stirred at RT for 2h. The reaction mixture was diluted with water (10 mL) and extracted with EtOAc (2 x 15mL). The organic layer was dried over anhydrous $Na_2SO_4$ and concentrated under reduced pressure to yield compound **3** (800 mg, 92%).
**$^1$H-NMR:** (200 MHz, DMSO-*d*$_6$): δ 12.58 (br s, 1H), 7.81-7.77 (d, *J*= 8.0 Hz, 1H), 7.35-7.27 (m, 5H), 5.04-4.96 (m, 2H), 4.66-4.60 (m, 1H), 4.32-4.24 (m, 2H), 4.04-3.86 (m, 1H), 3.66-3.59 (t, *J* = 12.6 Hz, 2H), 2.17-2.07 (m, 3H), 1.98-1.80 (m, 4H).
**Mass m/z:** 379.0 [$M^{+}$+1].

Synthesis of (2*S*,3*R*)-methyl 2-((*S*)-1-((*S*)-1-((*R*)-3-acetoxy-2-(benzyloxycarbonylamino)-propanoyl)-pyrrolidine-2-carbonyl)pyrrolidine-2-carboxamido)-3-hydroxybutanoate (**5**):

Compound **3** (1.0 g, 2.64 mmol) was dissolved in $CH_2Cl_2$ (10 mL), NMM (0.32 g, 3.17 mmol) and IBCF (0.41 g, 3.04 mmol) were added to the reaction mixture at -15 °C and stirred for 30 minutes under inert atmosphere. A mixture of (2*S*,3*R*)-methyl 3-hydroxy-2-((*S*)-pyrrolidine-2-carboxamido)-butanoate (**4**) (0.73 g, 3.17 mmol) and NMM (0.35 mL) in DMF (3 mL) were added drop wise to the reaction mixture at -15 °C and stirring was continued for another 3 h at RT. The reaction mixture was diluted with DCM (200 mL), washed with water (20 mL), citric acid solution (2 x 20 mL) and brine (2 x 50 mL). The separated organic layer was dried over anhydrous $Na_2SO_4$ and concentrated under reduced pressure. The crude residue obtained was purified by silica gel column chromatography eluting with 5% $CH_3OH$/EtOAc to afford compound (**5**) (0.29 g, 19%).
**$^1$H-NMR:** (500 MHz, DMSO-*d*$_6$): δ 7.83-7.81 (m, 1H), 7.72-7.70 (m, 1H), 7.36-7.35 (m, 5H), 5.07-5.01 (m, 2H), 4.99-4.93 (m, 1H), 4.58 (s, 1H), 4.50-4.48 (m, 1H), 4.26-4.22 (m, 2H), 4.07-4.00 (m, 2H), 3.89-3.86 (m, 1H), 3.61-3.55 (m, 5H), 3.53 (s, 1H), 3.39 (s, 1H), 2.12 (s, 1H), 1.98 (s, 3H), 1.94-1.83 (m, 4H), 1.81-1.80 (m, 3H), 1.05 (d, *J*= 6.5 Hz, 3H).
**Mass m/z:** 591.0 [$M^{+}$+1].

Synthesis of benzyl-(*R*)-1-((*S*)-2-((S)-2-((2*S*,3*R*)-1-(aminooxy)-3-hydroxy-1-oxobutan-2-ylcarbamoyl)-pyrrolidine-1-carbonyl)-pyrrolidin-1-yl)-3-hydroxy-1-oxopropan-2-ylcarbamate (**6**):

A solution of methanolic ammonia (3 mL) was added to compound **5** (0.28 g, 0.47 mmol) and stirred at RT for 18 h. The volatiles were evaporated under reduced pressure to afford compound **6** (0.21 g, 82.3%).
**$^1$H-NMR:** (500 MHz, DMSO-*d*$_6$): δ 7.38-7.31 (m, 5H), 7.26 (s, 1H), 7.10-7.03 (m, 2H), 6.65 (br s, 1H), 5.04-5.01 (m, 2H), 4.98-4.84 (m, 1H), 4.76-4.75 (m, 1H), 4.61 (s, 1H), 4.38-4.31 (m, 2H), 4.02-4.00 (m, 2H), 3.77-3.74 (m, 1H), 3.67-3.56 (m, 3H), 3.44-3.37 (m, 2H), 2.14-1.86 (m, 8H), 1.01-1.00 (m, 3H).
**Mass m/z**: 550 [$M^{+}$+1].

Synthesis of (*S*)-*N*-((2*S*,3*R*)-1-amino-3-hydroxy-1-oxobutan-2-yl)-1-((*S*)-1-((*S*)-2-amino-3-hydroxypropanoyl)-pyrrolidine-2-carbonyl)-pyrrolidine-2-carboxamide (**CM-1**):

To a solution of compound **6** (0.21 g, 0.39 mmol) in methanol (5 mL) was added 10% Pd/C (30 mg) and the reaction mixture was stirred under hydrogen atmosphere for 2 h. The reaction mixture was filtered over celite, solvent was evaporated *in vacuo,* and the crude residue obtained was triturated with diethyl ether to yield **CM-1** (130 mg, 83.3%).
**$^1$H-NMR:** (500 MHz, DMSO-*d*$_6$) (Rotamers): δ 7.39 (d, *J* = 8.0 Hz, 1H), 7.08-7.03 (m, 2H), 6.65 (br s, 1H), 4.89-4.85 (m, 1H), 1.61-1.59 (m, 1H), 4.39-4.38 (m, 1H), 4.02-4.00 (m, 2H), 3.68-3.52 (m, 4H), 3.43-3.36 (m, 2H), 3.22-3.10 (m, 2H), 2.19-2.13 (m, 1H), 2.07-1.98 (m, 1H), 1.93-1.81 (m, 5H), 1.75 (s, 2H), 1.01-1.00 (m, 3H).
**LCMS m/z:** 400.2 [$M^{+}$+1].
**HPLC Purity:** 99.27%.

Synthesis of (*S*)-1-(benzyloxycarbonyl) pyrrolidine-2-carboxylic acid (**8**):

To a stirred solution of (*S*)-pyrrolidine-2-carboxylic acid (7) (2.0 g, 17.39mmol) in THF: $H_2O$ (20 mL, 1:1) were added $Na_2CO_3$ (2.76 g, 26.08mmol) and Cbz-Cl (3.54 g, 20.80mmol) and stirred at RT for 18 h. The reaction mixture was washed with EtOAc (10 mL) and the aqueous layer was acidified with 3N HCl and extracted with EtOAc (2 x 20 mL). The combined organic layer was washed with brine, dried over anhydrous $Na_2SO_4$ and concentrated under reduced pressure to yield compound **8** (3.0 g, 69.7%).
**$^1$H-NMR:** (500 MHz, DMSO-*d*$_6$): δ 12.62 (br s, 1H), 7.36-7.22 (m, 5H), 5.12-5.00 (m, 2H), 4.24-4.15 (dd, *J* = 5.0, 36.0

Hz, 1H), 3.46-3.31 (m, 2H), 2.25-2.15 (m, 1H), 1.94-1.79 (m, 3H).
**Mass m/z**: 250.0 [$M^+$+1].

Synthesis of (S)-benzyl 2-((2S, 3R)-3-hydroxy-1-methoxy-1-oxobutan-2-ylcarbamoyl)pyrrolidine-1-carboxylate (**9**):

Compound **8** (5.0 g, 20.08 mmol) was dissolved in $CH_2Cl_2$ (50 mL), NMM (2.43 mL, 22.08 mmol) and IBCF (2.74 mL, 23.09 mmol) were added and stirred at -15 °C for 30 minutes under inert atmosphere. A mixture of (2*S*,3*R*)-methyl 2-amino-3-hydroxybutanoate (2.93 g, 22.08 mmol) and NMM (2.43 mL, 22.08 mmol) in DMF (15 mL) were added drop wise at -15 °C. The resultant reaction mixture was stirred at RT for 3 h. It was diluted with DCM (200 mL) and the organic layer was washed with water (50 mL), brine (50 mL), dried over anhydrous $Na_2SO_4$ and concentrated under reduced pressure. The obtained crude was purified by silica gel column chromatography eluting with 30% EtOAc/Hexane to afford compound **9** (3.1 g, 42%).
**$^1$H-NMR:** (500 MHz, DMSO-$d_6$)(Rotamers): δ 7.98-7.94 (m, 1H), 7.35-7.27 (m, 5H), 5.09-4.94 (m, 3H), 4.44 (dd, *J*= 5.5, 8.5 Hz, 1H), 4.29-4.27 (m, 1H), 4.12 (s, 1H), 3.62 (s, 3H), 3.44-3.30 (m, 2H), 2.20-2.08 (m, 1H), 1.87-1.78 (m, 3H), 1.08-0.94 (2d, 3H).
**Mass m/z:** 365.0 [$M^+$+1].

Reference Example 2 - Synthesis of (*S*)-*N*-((*S*)-1-amino-3-hydroxy-1-oxopropan-2-yl)-1-((*S*)-1-((2*S*,3*R*)-2-amino-3-hydroxybutanoyl)pyrrolidine-2-carbonyl)pyrrolidine-2-carboxamide (CM-2):

The following reaction sequence was used (Scheme B) to synthesize (*S*)-*N*-((*S*)-1-amino-3-hydroxy-1-oxopropan-2-yl)-1-((*S*)-1-((2*S*,3*R*)-2-amino-3-hydroxybutanoyl) pyrrolidine-2-carbonyl) pyrrolidine-2-carboxamide (CM-2):

Scheme B. Synthesis of CM-2.

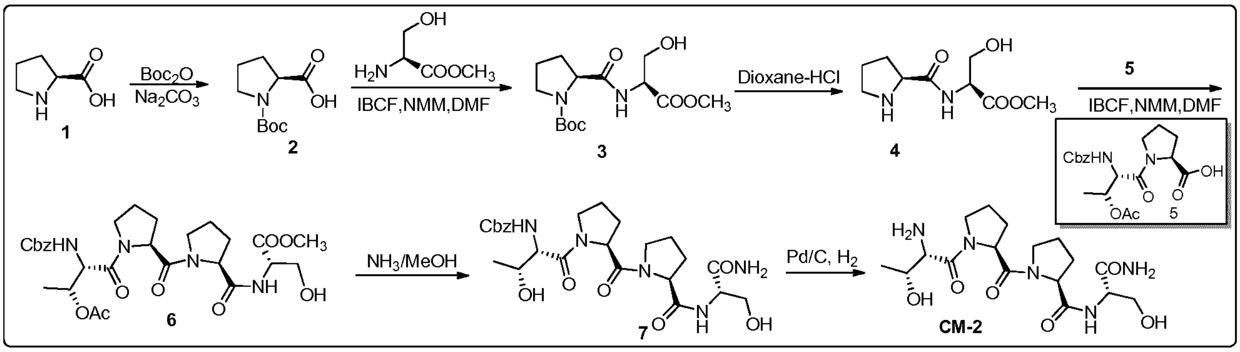

1 2 3 4 5 6 7 CM-2

Synthesis of (*S*)-1-(tert-butoxycarbonyl)-pyrrolidine-2-carboxylic acid (**2**):

To an ice cold stirred solution of (*S*)-pyrrolidine-2-carboxylic acid (**1**) (3.0 g, 26.08 mmol) in THF:$H_2O$ (60 mL, 1:1) were added $Na_2CO_3$ (5.52 g, 52.16 mmol), $Boc_2O$ (6.25 g, 26.69 mmol) and stirred at RT for 16 h. The reaction mixture was diluted with water and washed with EtOAc (50 mL). The aqueous layer was acidified with 2N HCl and extracted with EtOAc (2 x 100 mL). The combined organic layer was dried over anhydrous $Na_2SO_4$ and concentrated under reduced pressure to yield the (*S*)-1-(*tert*-butoxycarbonyl)-pyrrolidine-2-carboxylic acid (**2**) (4.8 g, 86%).
**$^1$H-NMR:** (500 MHz, DMSO-$d_6$): δ 12.49 (br s, 1H), 4.08-4.03 (m, 1H), 3.36-3.24 (m, 2H), 2.22-2.11 (m, 1H), 1.87-1.76 (m, 3H), 1.39 (s, 9H).
**Mass m/z:** 216.0 [$M^+$+1].

Synthesis of (*S*)-tert-butyl 2-((*S*)-3-hydroxy-1-methoxy-1-oxopropan-2-ylcarbamoyl)-pyrrolidine-1-carboxylate (**3**):

Compound **2** (2.0 g, 9.00 mmol) was dissolved in $CH_2Cl_2$ (10 mL) cooled to -15 °C, NMM (1.12 mL, 10.2 mmol) and IBCF (1.26 mL, 1.15 mmol) were added and stirred at 0 °C for 20 minutes. A mixture of (*S*)-methyl 2-amino-3-hydroxypropanoate (1.59 g, 10.2 mmol) and NMM (1.12 mL) in DMF (3 mL) were added drop wise at -15 °C and the resultant reaction mixture was stirred at RT for 1 h. It was diluted with DCM (200 mL), water (50 mL) and washed with 2N HCl (20 mL) and brine (2 x 50 mL). The separated organic layer was dried over anhydrous $Na_2SO_4$ and concentrated under reduced pressure. The crude residue obtained was purified by silica gel column chromatography eluting with 20% EtOAc/Hexane to afford compound **3** (2.3 g) as a syrup.
**Mass m/z:** 317.0 [$M^+$+1].

Synthesis of (*S*)-methyl 3-hydroxy-2-((*S*)-pyrrolidine-2-carboxamido)propionate (**4**):

(*S*)-Tert-butyl-2-((*S*)-3-hydroxy-1-methoxy-1-oxopropan-2-ylcarbamoyl)-pyrrolidine-1-carboxylate (**3**) (500 mg, 1.58 mmol) was dissolved in 1,4-dioxane (3 mL) and a HCl solution in dioxane (3.16 mL, 3.16 mmol) was added stirred at RT for 4 h. The volatiles were evaporated under reduced pressure to afford compound **4** (280 mg) as solid.
**$^1$H-NMR:** (200 MHz, DMSO-$d_6$): $\delta$ 9.99 (br s, 1H), 9.12-9.08 (m, 1H), 8.53 (br s, 1H), 5.48 (br s, 2H), 4.43-4.22 (m, 2H), 3.82-3.67 (m, 4H), 3.56 (s, 3H), 2.36-2.27 (m, 1H), 1.93-1.86 (m, 3H).
**Mass m/z:** 217.0 [$M^+$+1].

Synthesis of (*S*)-methyl 2-((*S*)-1-((*S*)-1-((2*R*, 3*S*)-3-acetoxy-2-(benzyloxycarbonylamino)-butanoyl)-pyrrolidine-2-carbonyl)-pyrrolidine-2-carboxamido)-3-hydroxypropanoate (**6**):

(2*S*)-1-((2*R*)-3-acetoxy-2-(benzyloxycarbonylamino)-butanoyl)-pyrrolidine-2-carboxylic acid (**5**) (1.3 g, 2.62 mmol) was dissolved in $CH_2Cl_2$ (15 mL), NMM (0.43 mL) and IBCF (0.51 mL) was added at -10 °C and stirred for 30 minutes under inert atmosphere. A mixture of (*S*)-methyl-3-hydroxy-2-((*S*)-pyrrolidine-2-carboxamido)-propionate (**4**) (992 mg, 3.93 mmol) and NMM (0.43 mL) in DMF (5 mL) were added drop wise to the reaction mixture and stirring was continued for another 3 h at RT. The reaction mixture was diluted with DCM (200 mL), washed with water (20 mL), citric acid solution (2 x 20 mL) and brine (2 x 50 mL). The separated organic layer was dried over anhydrous $Na_2SO_4$ and concentrated under reduced pressure. The obtained crude material was purified by silica gel column chromatography eluting with 5% $CH_3OH/CH_2Cl_2$ to afford compound **6** (270 mg, 17.5%).
**$^1$H-NMR:** (500 MHz, DMSO-$d_6$): $\delta$ 8.13 (d, *J* = 8.0 Hz, 1H), 7.74 (d, *J* = 7.5 Hz, 1H), 7.38-7.31 (m, 5H), 5.08-4.96 (m, 3H), 4.85-4.82 (m, 1H), 4.56 (d, *J* = 8.0 Hz, 1H), 4.44-4.42 (m, 2H), 4.27 (d, *J* = 7.0 Hz, 1H), 4.10 (d, *J* = 10.5 Hz, 2H), 3.81-3.78 (m, 1H), 3.72-3.70 (m, 1H), 3.61-3.59 (m, 3H), 3.54-3.50 (m, 2H), 2.16-2.14 (m, 1H), 2.05-2.01 (m, 1H), 1.90 (s, 3H), 1.87-1.86 (m, 3H), 1.85-1.84 (m, 3H), 1.21-1.20 (d, *J* = 6.0 Hz, 3H).
**Mass m/z:** 591.0 [$M^+$+1].

Synthesis of Benzyl-(2*R*,3*S*)-1-((*S*)-2-((*S*)-2-((*S*)-1-(aminooxy)-3-hydroxy-1-oxopropan-2-ylcarbamoyl) pyrrolidine-1-carbonyl)pyrrolidin-1-yl)-3-hydroxy-1-oxobutan-2-ylcarbamate (**7**):

To a solution of compound **6** (250 g, 0.42 mmol) in $CH_3OH$ (2 mL) was added MeOH-$NH_3$ (10 mL) and was stirred at RT for 16 h. The volatiles were evaporated under reduced pressure to afford compound **7** (190 mg, 84%).
**$^1$H-NMR:** (500 MHz, DMSO-$d_6$): $\delta$ 7.60 (d, *J* = 7.5 Hz, 1H), 7.35-7.30 (m, 5H), 7.18 (d, *J* = 7.0 Hz, 1H), 7.11-7.06 (m, 2H), 5.05-4.97 (m, 2H), 4.82-4.81 (m, 1H), 4.60-4.59 (m, 2H), 4.33-4.31 (m, 1H), 4.15-4.08 (m, 2H), 3.81-3.79 (m, 1H), 3.72-3.64 (m, 2H), 3.59-3.53 (m, 4H), 2.14 (s, 1H), 2.03 (d, *J* = 9.0 Hz, 1H), 1.95-1.85 (m, 5H), 1.75 (s, 1H), 1.10 (d, *J* = 6.5 Hz, 3H).
**Mass m/z:** 550.0 [$M^+$+1].

Synthesis of (*S*)-N-((*S*)-1-amino-3-hydroxy-1-oxopropan-2-yl)-1-((*S*)-1-((2*S*,3*R*)-2-amino-3-hydroxybutanoyl) pyrrolidine-2-carbonyl)-pyrrolidine-2-carboxamide (**CM-2**):

To a solution of compound **7** (190 mg, 0.35 mmol) in methanol (5 mL) was added 10% Pd/C (50 mg) and the reaction mixture was stirred under hydrogen atmosphere for 2 h. The reaction mixture was filtered through a celite pad, solvent was evaporated *in vacuo* and the crude was purified by column chromatography on basic alumina using 0-5% $CH_3OH$ in $CH_2Cl_2$ as eluent to yield **CM-2** (130 mg, 73%).
**$^1$H-NMR:** (500 MHz, DMSO-$d_6$): $\delta$ 7.65-7.60 (m, 1H), 7.12-7.03 (m, 2H), 4.81 (br s, 1H), 4.58-4.57 (m, 1H), 4.49 (m, 1H), 4.38-4.19 (m, 1H), 4.10-4.06 (m, 1H), 3.69-3.62 (m, 2H), 3.59-3.56 (m, 4H), 3.49-3.45 (m, 2H), 3.37-3.26 (m, 2H), 2.19-2.15 (m, 1H), 2.09-1.99 (m, 1H), 1.95-1.84 (m, 5H), 1.75 (s, 1H), 1.06 (d, *J* = 13.0 Hz, 3H).
**LCMS m/z:** 400.8 [$M^+$+1],
**HPLC Purity:** 97.71%.

Reference Example 3 - Synthesis of (*S*)-*N*-((*S*)-1-amino-3-hydroxy-1-oxopropan-2-yl)-1-((*S*)-1-((*S*)-2-amino-3-hydroxy-propanoyl)-pyrraolidine-2-carbonyl)-pyrrolidine-2-carboxamide (CM-3):

The following reaction sequence was used (Scheme C) to synthesize (*S*)-*N*-((*S*)-1-amino-3-hydroxy-1-oxopropan-2-yl)-1-((*S*)-1-((*S*)-2-amino-3-hydroxy-propanoyl)-pyrrolidine-2-carbonyl)-pyrrolidine-2-carboxamide (CM-3):

Scheme C. Synthesis of CM-3.

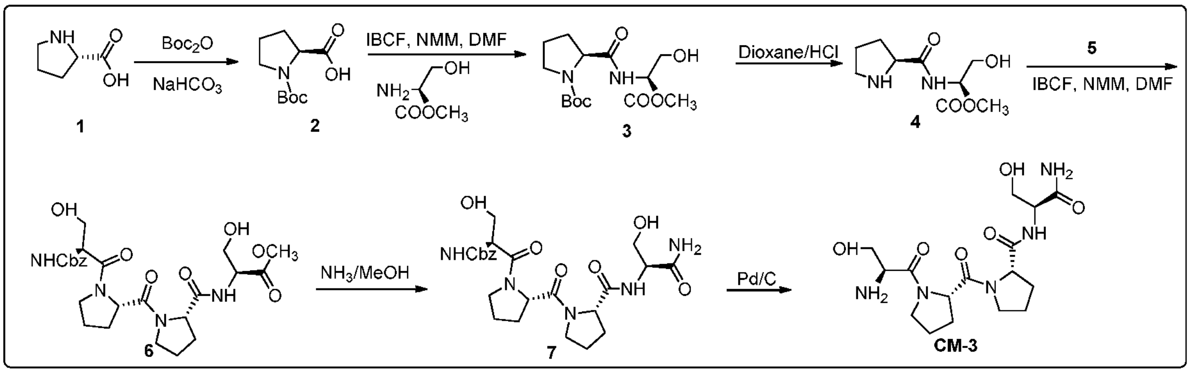


Synthesis of (*S*)-1-(*tert*-butoxycarbonyl)-pyrrolidine-2-carboxylic acid **(2):**

To a stirred solution of (*S*)-pyrrolidine-2-carboxylic acid (3.0 g, 26.08mmol) in THF:$H_2O$ (60 mL, 1:1) at 0 °C were added $Na_2CO_3$ (5.52 g, 52.16 mmol) and $Boc_2O$ (6.25 g, 26.69mmol) and stirred at RT for 16 h. The reaction mixture was diluted with water and washed with EtOAc (50 mL). The aqueous layer was acidified with 2N HCl and extracted with EtOAc (2 x 50 mL). The combined organic layer was dried over anhydrous $Na_2SO_4$ and concentrated under reduced pressure to yield the (*S*)-1-(*tert*-butoxycarbonyl)-pyrrolidine-2-carboxylic acid **2** (4.8 g, 85.7%).
**$^1$H-NMR:** (500 MHz, DMSO-$d_6$): δ 12.49 (br s, 1H), 4.08-4.03 (m, 1H), 3.36-3.24 (m, 2H), 2.22-2.11 (m, 1H), 1.87-1.76 (m, 3H), 1.39 (s, 9H).
**Mass m/z:** 216.0 [$M^+$+1],

Synthesis of (*S*)-tert-butyl 2-((*S*)-3-hydroxy-1-methoxy-1-oxopropan-2-ylcarbamoyl) pyrrolidine-1-carboxylate **(3):**

Compound 2 (2.0 g, 9.00mmol) was dissolved in $CH_2Cl_2$ (10 mL) cooled to -15 °C, NMM (1.12 mL, 10.2 mmol) and IBCF (1.26 mL, 1.15 mmol) were added and stirred at 0 °C for 20 minutes. A mixture of (*S*)-methyl-2-amino-3-hydroxypropanoate (1.59 g, 10.2mmol) and NMM (1.12 mL) in DMF (3 mL) were added drop wise at -15 °C. The resultant reaction mixture was stirred at RT for 1 h. The reaction mixture was diluted with DCM (200 mL) and water (25 mL) and was washed with 2N HCl (20 mL) and brine (10 mL). The separated organic layer was dried over anhydrous $Na_2SO_4$ and concentrated under reduced pressure. The obtained crude material was purified by silica gel column chromatography eluting with 20% EtOAc/Hexane to afford compound **3** (2.3 g) as solid.
**Mass m/z:** 317.0 [$M^+$+1].

Synthesis of (*S*)-methyl 3-hydroxy-2-((5)-pyrrolidine-2-carboxamido) propanoate **(4):**

To a solution of (*S*)-tert-butyl-2-((*S*)-3-hydroxy-1-methoxy-1-oxopropan-2-ylcarbamoyl) pyrrolidine-1-carboxylate 3 (500 mg, 1.58 mmol) in 1,4-dioxane (3 mL) was added a solution of HCl in dioxane (3.16mL, 3.16 mmol) and stirred at RT for 4 h. The volatiles were evaporated under reduced pressure to afford compound 4 (280 mg) as solid.
**$^1$H-NMR:** (200 MHz, DMSO-$d_6$): δ 9.99 (br s, 1H), 9.12-9.08 (m, 1H), 8.53 (br s, 1H), 5.48 (br s, 2H), 4.43-4.22 (m, 2H), 3.82-3.67 (m, 4H), 3.56 (s, 3H), 2.36-2.27 (m, 1H), 1.93-1.86 (m, 3H).
**Mass m/z:** 217.0 [$M^+$+1].

Synthesis of (*S*)-methyl 2-((*S*)-1-((*S*)-1-((*S*)-2-(benzyloxycarbonylamino)-3-hydroxypropanoyl)-pyrrolidine-2-carbonyl)-pyrrolidine-2-carboxamido)-3-hydroxypropanoate (**6**):

(*S*)-1-((*S*)-3-Acetoxy-2-(benzyloxycarbonylamino)-propanoyl)-pyrrolidine-2-carboxylic acid **(5)** (400 mg, 1.05 mmol) was dissolved in $CH_2Cl_2$ (2 mL), NMM (0.13 mL) and IBCF (0.14 mL) were added at -15 °C and stirred for 30 minutes under inert atmosphere. A mixture of (*S*)-methyl-3-hydroxy-2-((*S*)-pyrrolidine-2-carboxamido)-propanoate hydrochloride **(4)** (293 mg, 1.16 mmol) and NMM (0.13 mL) in DMF (2 mL) were added drop wise to the reaction mixture and stirring was continued for another 3 h at RT. The reaction mixture was diluted with DCM (200 mL), washed with water (20 mL) and brine (10 mL). The separated organic layer was dried over anhydrous $Na_2SO_4$ and concentrated under reduced pressure. The obtained crude material was purified by silica gel column chromatography eluting with 5% $CH_3OH/CH_2Cl_2$ to afford compound **6** (80 mg, 13%).
**$^1$H-NMR:** (500 MHz, DMSO-$d_6$): δ 8.09 (d, *J* = 7.5 Hz, 1H), 7.71 (d, *J* = 8.0 Hz, 1H), 7.36-7.31 (m, 6H), 5.07-4.99 (m,

3H), 4.59-4.58 (m, 2H), 4.41-4.40 (m, 1H), 4.29-4.24 (m, 3H), 3.86 (t, *J* = 9.5 Hz, 1H), 3.72-3.68 (m, 1H), 3.64-3.57 (m, 3H), 3.40-3.38 (m, 3H), 2.14-2.01 (m, 2H), 1.98 (s, 3H), 1.90-1.80 (m, 6H).
**Mass m/z:** 535.0 [$M^{+}$+1].

Synthesis of Benzyl-(*S*)-1-((*S*)-2-((*S*)-2-((*S*)-1-amino-3-hydroxy-1-oxopropan-2-ylcarbamoyl) pyrrolidine-1-carbonyl) pyrrolidin-1-yl)-3-hydroxy-1-oxopropan-2-ylcarbamate (7):

To a solution of (*S*)-methyl-2-((*S*)-1-((*S*)-1-((*S*)-2-(benzyloxycarbonylamino)-3-hydroxypropanoyl)-pyrrolidine-2-carbonyl)-pyrrolidine-2-carboxamido)-3-hydroxypropanoate **(6)** (60 mg, 1.04 mmol) in MeOH was added MeOH-$NH_3$ (3 mL) was stirred at RT for 16 h. The volatiles were evaporated under reduced pressure to afford compound 7 (30 mg, 55%).
**$^1$H-NMR:** (500 MHz, DMSO-$d_6$): δ 7.60 (d, *J* = 7.5 Hz, 1H), 7.36-7.31 (m, 6H), 7.11-7.06 (m, 2H), 5.04-4.98 (m, 2H), 4.82-4.74 (m, 2H), 4.61-4.59 (m, 1H), 4.36-4.30 (m, 2H), 4.10-4.07 (m, 1H), 3.67-3.65 (m, 2H), 3.59-3.55 (m, 6H), 3.44-3.40 (m, 2H), 1.95-1.92 (m, 6H).
**Mass m/z:** 520.0 [$M^{+}$+1].

Synthesis of (*S*)-*N*-((*S*)-1-amino-3-hydroxy-1-oxopropan-2-yl)-1-((*S*)-1-((*S*)-2-amino-3-hydroxy-propanoyl)-pyrrolidine-2-carbonyl)-pyrrolidine-2-carboxamide **(CM-3):**

Benzyl-(*S*)-1-((*S*)-2-((*S*)-2-((*S*)-1-amino-3-hydroxy-1-oxopropan-2-ylcarbamoyl) pyrrolidine-1-carbonyl)pyrrolidin-1-yl)-3-hydroxy-1-oxopropan-2-ylcarbamate 7 (300 mg, 0.57 mmol) was dissolved in methanol (8 mL), 10% Pd/C (50 mg) was added and reaction mixture was stirred under hydrogen atmosphere for 2 h. The reaction mixture was filtered and the filtrate was concentrated under reduced pressure to yield **CM-3** (150 mg, 68%).
**$^1$H-NMR:** (500 MHz, DMSO-$d_6$) (Rotamers): δ 7.62 (d, *J* = 8.0 Hz, 1H), 7.24 (br s, 1H), 7.14-7.07 (m, 2H), 4.87-4.82 (m, 2H), 4.59-4.57 (m, 1H), 4.37-4.31 (m, 2H), 4.11-4.07 (m, 2H), 3.70-3.39 (m, 8H), 2.17-2.01 (m, 2H), 1.95-1.79 (m, 6H).
**LCMS m/z:** 386.4 [$M^{+}$+1].
**HPLC Purity:** 98.45%.

Example 4 - Synthesis of (*R*)-*N*-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-benzylpyrrolidine-2-carboxamide (CM-4A) and (*S*)-*N*-((2*S*,3*R*)-1-amino-3-hydroxy-1-oxobutan-2-yl)-1-((*S*)-1-((2*S*,3*R*)-2-amino-3-hydroxybutanoyl)-pyrrolidine-2-carbonyl)-2-benzylpyrrolidine-2-carboxamide (CM-4B):

The following reaction sequence was used (Scheme D) to synthesize (R)-N-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-benzylpyrrolidine-2-carboxamide (CM-4A) and (*S*)-*N*-((2*S*,3*R*)-1-amino-3-hydroxy-1-oxobutan-2-yl)-1-((*S*)-1-((2*S*,3*R*)-2-amino-3-hydroxybutanoyl)-pyrrolidine-2-carbonyl)-2-benzylpyrrolidine-2-carboxamide (CM-4B):

Scheme D. Synthesis of CM-4A and CM-4B.

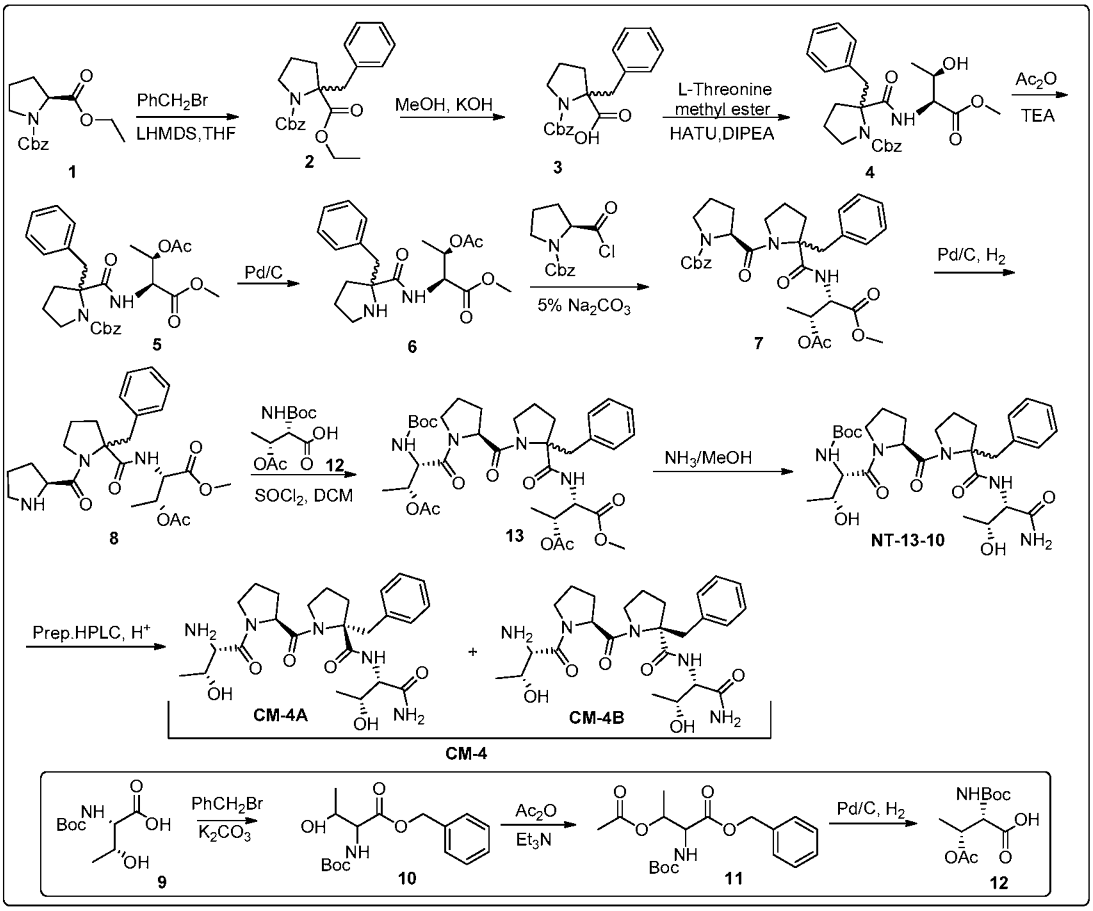


Synthesis of 1-benzyl 2-ethyl 2-benzylpyrrolidine-1,2-dicarboxylate **(2):**

To a solution of (*S*)-1-benzyl 2-ethyl pyrrolidine-1, 2-dicarboxylate **1** (10 g, 36.10mmol) in THF (150mL) under inert atmosphere was added LiHMDS (1M in THF) (43.3mL, 43.3mmol) at -25 °C and stirred for 2 h. Benzyl bromide (5.17mL, 43.26mmol) was added drop wise at -25 °C to the reaction mixture. It was allowed to warm to RT and stirred for 2h. The reaction mixture was cooled to 5 °C, quenched with saturated $NH_4Cl$ solution and the aqueous layer was extracted with EtOAc (2 x 200mL). The combined organic extracts were dried over anhydrous $Na_2SO_4$ and concentrated under reduced pressure. The crude residue obtained was purified by silica gel column chromatography eluting with 5% EtOAc/hexane to afford compound **2** (13 g, 75%) as liquid.
**$^1$H-NMR:** (200 MHz, DMSO-$d_6$): δ 7.47-7.32 (m, 5H), 7.27-7.16 (m, 3H), 7.07-7.04 (m, 2H), 5.29-5.06 (m, 2H), 4.16-3.89 (m, 2H), 3.57-3.33 (m, 2H), 3.02-2.78 (m, 2H), 2.13-1.89 (m, 2H), 1.56-1.51 (m, 1H), 1.21-1.04 (m, 3H), 0.93-0.79 (m, 1H).
**Mass m/z:** 368.2 [$M^+$+1].

Synthesis of 1-benzyl 2-ethyl 2-benzylpyrrolidine-1,2-dicarboxylate **(3):**

To a stirred solution of compound **2** (8.0 g, 21.79mmol) in $CH_3OH$ (20mL) was added 2N aqueous KOH (20mL) and heated up to 100 °C and stirred for 16 h. The volatiles were evaporated under reduced pressure. The residue obtained was diluted with ice cold water (50mL) and washed with ether (50mL). The aqueous layer was acidified to pH~2 using HCl solution and extracted with EtOAc (2 x 100mL). The combined organic layer was dried over anhydrous $Na_2SO_4$ and concentrated under reduced pressure to afford compound 3 (6 g, 81%) as an off white solid.
**$^1$H-NMR:** (200 MHz, DMSO-$d_6$): δ 12.71 (br s, 1H), 7.40-7.30 (m, 5H), 7.25-7.19 (m, 3H), 7.07-7.00 (m, 2H), 5.27-5.02 (m, 2H), 3.59-3.32 (m, 2H), 3.02-2.83 (m, 2H), 2.13-1.91 (m, 2H), 1.58-1.49 (m, 1H), 0.90-0.77 (m, 1H).
**Mass m/z:** 340.1 [$M^+$+1].

Synthesis of Benzyl 2-benzyl-2-((2*S*,3*R*)-3-hydroxy-1-methoxy-1-oxobutan-2-ylcarbamoyl) pyrrolidine-1-carboxylate **(4):**

To a suspension of 2-benzyl-1-(benzyloxycarbonyl) pyrrolidine-2-carboxylic acid (1.0 g, 2.94mmol), L-threonine methyl ester (471 mg, 3.53mmol) in DMF (20mL) was added HATU (1.12 g, 2.94mmol) and. DIPEA (1.58mL, 8.84mmol) at 5 °C. The reaction mixture was stirred at RT for 16 h. It was diluted with EtOAc (150mL) and washed with water (2 x 30mL). The organic layer was washed with brine, dried over $Na_2SO_4$, concentrated and purified by silica gel column chromatography 50% EtOAc/Hexane as eluent to yield compound 4 (1.0 g, 74%).
**$^1$H-NMR:** (200 MHz, DMSO-*d*$_6$): δ 7.62-7.59 (m, 1H), 7.44-7.31 (m, 5H), 7.21-7.18 (m, 3H), 7.06-6.99 (m, 2H), 5.25-5.24 (m, 1H), 5.12-4.94 (m, 2H), 4.30 (s, 1H), 4.15-4.08 (m, 1H), 3.66-3.64 (m, 3H), 3.63-3.49 (m, 2H), 3.14 (s, 1H), 2.89 (s, 1H), 2.09-2.02 (m, 2H), 1.56-1.51 (m, 1H), 1.09-0.98 (m, 4H).
**Mass m/z:** 455.1 [$M^+$+1], 477.3 [M+Na].

Synthesis of Benzyl 2-((2*S*,3*R*)-3-acetoxy-1-methoxy-1-oxobutan-2-ylcarbamoyl)-2-benzylpyrrolidine-1-carboxylate **(5)**

2-Benzyl-2-((2*S*,3*R*)-3-hydroxy-1-methoxy-1-oxobutan-2-ylcarbamoyl) pyrrolidine-1-carboxylate (3 g, 6.60mmol) was dissolved in THF (30mL), $Et_3N$ (1.11mL, 7.92mmol) and $Ac_2O$ (742 mg, 7.26mmol) were added at RT. The reaction mixture was stirred at RT for 2 h. The volatiles were evaporated under reduced pressure and the residue obtained was diluted with $CH_2Cl_2$ and washed with dil.HCl. The combined organic extracts were dried over $Na_2SO_4$ and concentrated under reduced pressure. The crude residue was purified by column chromatography using 30% EtOAc/Hexane as eluent to yield compound **5** (2.5 g, 76%).
**$^1$H-NMR:** (500 MHz, DMSO-*d*$_6$) (Rotamers): δ 8.15-7.71 (m, 1H), 7.42-7.04 (m, 10H), 5.30-5.19 (m, 2H), 5.11-5.09 (m, 1H), 4.99-4.93 (m, 1H), 4.67-4.62 (m, 1H), 3.66-3.64 (m, 3H), 3.55-3.46 (m, 2H), 3.38-3.35 (m, 1H), 2.88-2.69 (m, 1H), 2.17-2.00 (m, 2H), 1.98-1.92 (m, 3H), 1.56-1.46 (m, 1H), 1.23-1.17 (m, 3H), 1.02-0.86 (m, 1H).
**LCMS m/z:** 497.4 [$M^+$+1].

Synthesis of (2S, 3*R*)-methyl 3-acetoxy-2-(2-benzylpyrrolidine-2-carboxamido) butanoate **(6):**

To a stirring solution of compound **5** (4 g, 8.06mmol) in Ethanol (50mL) was added 10% Pd/C (1.2 g) and the reaction mixture was stirred under $H_2$ atmosphere (balloon pressure) for 4 h. It was filtered through celite pad and the filtrate was concentrated under reduced pressure to yield compound **6** (2.2 g, 75%).
**$^1$H-NMR:** (500 MHz, DMSO-*d*$_6$) (Rotamers): δ 8.22-8.17 (m, 1H), 7.24-7.16 (m, 5H), 5.17 (t, *J* = 11.5 Hz, 1H), 4.48-4.42 (m, 1H), 3.60-3.54 (s, 3H), 3.20 (t, *J* = 13.5 Hz, 1H), 3.06-2.97 (m, 1H), 2.82-2.68 (m, 3H), 2.08-2.02 (m, 1H), 1.89 (s, 3H), 1.72-1.51 (m, 3H), 1.10 (2d, 3H).
**LCMS m/z:** 363 [$M^+$+1], 385 [M+Na].

Synthesis of (*S*)-benzyl 2-(2-((2*S*,3*R*)-3-acetoxy-1-methoxy-1-oxobutan-2-ylcarbamoyl)-2-benzylpyrrolidine-1-carbonyl)pyrrolidine-1-carboxylate (7):

To a stirred solution of compound 6 (1 g, 2.76mmol) and $Na_2CO_3$ (732 mg, 6.90mmol)in $CH_2Cl_2$:$H_2O$ (20mL, 1:1) was added a solution of acid chloride [To a solution of (*S*)-1-(benzyloxycarbonyl) pyrrolidine-2-carboxylic acid (825 mg, 3.31mmol) in $CH_2Cl_2$ (20mL) was added $SOCl_2$ (0.60mL) drop wise at 0 °C and was refluxed for 2 h. The volatiles were removed under reduced pressure to yield (*S*)-benzyl 2-(chlorocarbonyl) pyrrolidine-1-carboxylate] in $CH_2Cl_2$ and the reaction mixture was stirred at RT for 2 h. The volatiles were evaporated under reduced pressure. The residue was diluted with $CH_2Cl_2$ (100 mL), filtered and the filtrate was concentrated under vacuo. The crude residue was purified by column chromatography using 60% EtOAc/Hexane as eluent to afford compound 7 (750 mg, 45%).
**$^1$H-NMR:** (500 MHz, DMSO-*d*$_6$) (Rotamers): δ 7.36-7.23 (m, 8H), 7.15-7.12 (m, 3H), 5.21-5.15 (m, 2H), 5.04-4.92 (m, 1H), 4.57-4.50 (m, 2H), 3.88 (d, *J* = 14.5 Hz, 1H), 3.65 (s, 3H), 3.54-3.46 (m, 3H), 3.21-3.13 (m, 1H), 3.02-2.90 (m, 2H), 2.19-2.02 (m, 4H), 1.97 (s, 3H), 1.89 (s, 1H), 1.77-1.65 (m, 1H), 1.17 (s, 2H), 1.06 (s, 2H).
**Mass m/z:** 594.1 [$M^+$+1],

Synthesis of (2*S*,3*R*)-methyl 3-acetoxy-2-(2-benzyl-1-((*S*)-pyrrolidine-2-carbonyl) pyrrolidine-2-carboxamido) butanoate **(8):**

To a solution of compound **7** (200 mg, 0.336 mmol) in EtOAc (15 mL) was added 10% Pd/C (40 mg) was added under inert atmosphere and stirred for 12h under $H_2$ atmosphere (balloon pressure). The reaction mixture was filtered through celite pad and concentrated under reduced pressure. The obtained residue was triturated with ether (10 mL) to afford compound **8** (125 mg, 81%) as solid.

**$^1$H-NMR:** (500 MHz, $CDCl_3$) (Rotamers): δ 7.88-7.87 (d, 1H, J= 8.5), 7.30-7.26 (m, 2H), 7.24-7.21 (m, 1H), 7.13-7.12 (d, 2H, J = 7), 5.44-5.43 (m, 1H), 4.76-4.74 (m, 1H), 3.94-3.92 (m, 1H), 3.84-3.81 (m, 1H), 3.75 (s, 3H), 3.50 (m, 1H), 3.26-3.12 (m, 3H), 2.90-2.88 (m, 1H), 2.23-2.15 (m, 4H), 2.04 (s, 3H), 1.87-1.77 (m, 5H), 1.27-1.24 (m, 3H).
**Mass m/z:** 460(M+1).

Synthesis of Benzyl 2-(tert-butoxycarbonylamino)-3-hydroxybutanoate **(10):**

To a solution of 2-(tert-butoxycarbonylamino)-3-hydroxybutanoic acid (3.0 g, 13.69mmol) in DMF (50mL) was added $K_2CO_3$ (3.73 g, 27.39mmol) and stirred at RT for 15 min. (Bromomethyl)benzene (2.81 g, 16.43mmol) was added and stirred at RT for 6 h. The reaction mixture was diluted with water (50mL) and extracted with EtOAc (2 x 50mL). The combined organic layer was washed with brine (50mL), dried over anhydrous $Na_2SO_4$ and concentrated under reduced pressure. The crude material was purified by silica gel column chromatography using 20% EtOAc/hexane as eluent to afford benzyl 2-(tert-butoxycarbonylamino)-3-hydroxybutanoate **10** (2.8 g, 66%).
**$^1$H-NMR:** (500 MHz, DMSO-*d*$_6$): δ 7.37-7.30 (m, 5H), 6.60 (d, *J* = 8.5 Hz, 1H), 5.18-5.08 (m, 2H), 4.76 (d, *J* = 7 Hz, 1H), 4.08-4.00 (m, 2H), 1.38 (s, 9H), 1.09 (d, *J* = 6.0 Hz, 3H).
**Mass m/z:** 310.0 [$M^+$+1], 210 [$M^+$-De Boc].

Synthesis of benzyl 3-acetoxy-2-(tert-butoxycarbonylamino) butanoate **(11):**

To a stirred solution of benzyl 2-(tert-butoxycarbonylamino)-3-hydroxybutanoate (2.8 g, 9.06mmol) in THF (80mL) was added $Ac_2O$ (1.1 g, 10.87mmol), $Et_3N$ (1.51mL, 10.87mmol) and DMAP (280 mg) and stirred at RT for 15 min. The volatiles were removed under reduced pressure. The residue obtained was diluted with EtOAc (150mL) and washed with cold 0.5N HCl solution (2 x 20mL). The organic layer was washed with brine, dried over anhydrous $Na_2SO_4$ and concentrated under reduced pressure to afford 3-acetoxy-2-(tert-butoxycarbonylamino)butanoate **11** (2.8 g, 88%).
**$^1$H-NMR:** (500 MHz, DMSO-*d*$_6$): δ 7.35-7.34 (m, 5H), 7.27-7.25 (d, *J* = 8.5 Hz, 1H), 5.18-5.06 (m, 3H), 4.34-4.32 (m, 1H), 1.90 (s, 3H), 1.39 (s, 9H), 1.16 (d, *J* = 3 Hz, 3H).
**Mass m/z:** 252 [$M^+$+1-De Boc].

Synthesis of (2*S*, 3*R*)-3-acetoxy-2-(tert-butoxycarbonylamino) butanoic acid **(12):**

Benzyl-3-acetoxy-2-(tert-butoxycarbonylamino) butanoate **11** (1.4 g, 3.98mmol) was dissolved in EtOAc (40mL), 10% Pd/C (600 mg) was added and reaction mixture was stirred under hydrogen atmosphere for 16 h. The reaction mixture was filtered over celite, solvent was evaporated in *vacuo* and the crude residue was triturated with hexane to yield (2*S, 3R)-3*-acetoxy-2-(tert-butoxycarbonylamino) butanoic acid **12** (0.7 g, 70%).
**$^1$H-NMR:** (500 MHz, DMSO-*d*$_6$): δ 12.78 (br s, 1H), 6.94 (d, *J* = 9.5 Hz, 1H), 5.16-5.14 (m, 1H), 4.17-4.15 (m, 1H), 1.95 (s, 3H), 1.39 (s, 9H), 1.10 (d, *J* = 6.0 Hz, 3H).
**Mass m/z:** 260.0 [M-1].

Synthesis of (2*S*,3*R*)-methyl 3-acetoxy-2-(1-((*S*)-1-((2*S*,3*R*)-3-acetoxy-2-((*tert*-butoxycarbonyl)-amino)butanoyl)pyrrolidine-2-carbonyl)-2-benzylpyrrolidine-2-carboxamido)butanoate **(13):**

To a solution of compound (2*S*,3*R*)-3-acetoxy-2-(tert-butoxycarbonylamino) butanoic acid **12** (199 mg, 0.76mmol) in $CH_2Cl_2$ (6mL) was under inert atmosphere were added IBCF (125 mg, 0.91mmol) and NMM (154 mg, 1.52mmol) at -15 °C and stirred for 1 h. A solution of (2*S*,3*R*)-methyl 3-acetoxy-2-(2-benzyl-1-((*S*)-pyrrolidine-2-carbonyl) pyrrolidine-2-carboxamido) butanoate **8** (350 mg, 0.76mmol) in DMF (2mL) was added to the reaction mixture and stirred for 1h at -15° C. The resultant reaction mixture was allowed to warm to RT and stirred for 19h. The reaction mixture was extracted with EtOAc and the separated organic layer was washed with water (20mL), followed by brine (20mL), dried over $Na_2SO_4$ and concentrated under reduced pressure. The crude material was purified by preparative HPLC to afford compound **13** (100 mg, 20%).
**$^1$H-NMR:** (500 MHz, $CD_3OD$) (Rotamers): δ 7.30-7.24 (m, 3H), 7.15-7.13 (m, 2H), 4.62-4.55 (m, 2H), 4.29-3.97 (m, 1H), 3.98-3.79 (m, 4H), 3.75 (s, 3H), 3.62-3.22 (m, 2H), 3.23 (d, *J* = 13.5 Hz, 1H), 3.00-2.95 (q, 1H), 2.37-2.31 (m, 1H), 2.23-2.10 (m, 2H), 2.02-1.88 (m, 3H), 1.46-1.28 (m, 2H), 0.97 (d, *J* = 7.0 Hz, 6H).

Synthesis of *tert*-butyl ((2*S*,3*R*)-1-((*S*)-2-(2-(((2*S*,3*R*)-1-amino-3-hydroxy-1-oxobutan-2-yl)carbamoyl)-2-benzylpyrrolidine-1-carbonyl)pyrrolidin-1-yl)-3-hydroxy-1-oxobutan-2-yl)carbamate **(NT-13-10).**

A solution of compound **13** (100 mg, 0.153mmol) in methanolic-$NH_3$ (10mL) was stirred in a sealed tube at RT for 72 h. The reaction mixture was concentrated under reduced pressure. The obtained crude residue was washed with

ether (2 x 2mL) to afford **NT-13-10** (85 mg).

Synthesis of (*R*)-N-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-benzylpyrrolidine-2-carboxamide **(CM-4A)** and (*S*)-N-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-benzylpyrrolidine-2-carboxamide **(CM-4B):**

**NT-13-10** (85 mg) was further purified by chiral preparative HPLC to afford two isomers which on treatment with dioxane HCl and evaporation afforded 15 mg each of **CM-4A** and **CM-4B.**
**$^1$H-NMR:** (500 MHz, $CD_3OD$) (Rotamers): δ 7.33-7.26 (m, 3H), 7.16 (s, 2H), 4.55-4.54 (m, 1H), 4.39 (s, 1H), 4.14 (s, 1H), 4.01-3.98 (m, 1H), 3.91-3.71 (m, 3H), 3.59 (s, 2H), 3.25-3.16 (m, 1H), 3.04-3.00 (m, 1H), 2.33-2.10 (m, 3H), 2.01-1.91 (m, 2H), 1.86-1.80 (m, 1H), 1.46-1.44 (m, 1H), 1.34-1.29 (m, 1H), 1.25-1.19 (m, 3H), 0.99-0.97 (d, *J* = 14.0 Hz, 3H).
**Mass m/z:** 503 $[M^+]$
**HPLC Purity:** 98.1%.

Synthesis of (*R*)-*N*-((2*S*,3*R*)-1-amino-3-hydroxy-1-oxobutan-2-yl)-1-((*S*)-1-((2*S*,3*R*)-2-amino-3-hydroxybutanoyl)-pyrrolidine-2-carbonyl)-2-benzylpyrrolidine-2-carboxamide trifluoroacetate salt **(CM-4A-TFA):**

To a solution of **CM-4A** in methanol was added excess trifluoroacetic acid. The resultant precipitate was collected by filtration, washed with methanol, and dried in vacuo to yield **CM-4A-TFA.**

Example 5 - Synthesis of (*R*)-*N*-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-(4-methylbenzyl)pyrrolidine-2-carboxamide (CM-5A) and (*S*)-*N*-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-methylbenzyl) pyrrolidine-2-carboxamide (CM-5B):

The following reaction sequence was used (Scheme E) to synthesize (R)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-methylbenzyl) pyrrolidine-2-carboxamide (CM-5A) and (*S*)-*N*-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-methylbenzyl) pyrrolidine-2-carboxamide (CM-5B):

Scheme E. Synthesis of CM-5A and CM-5B:

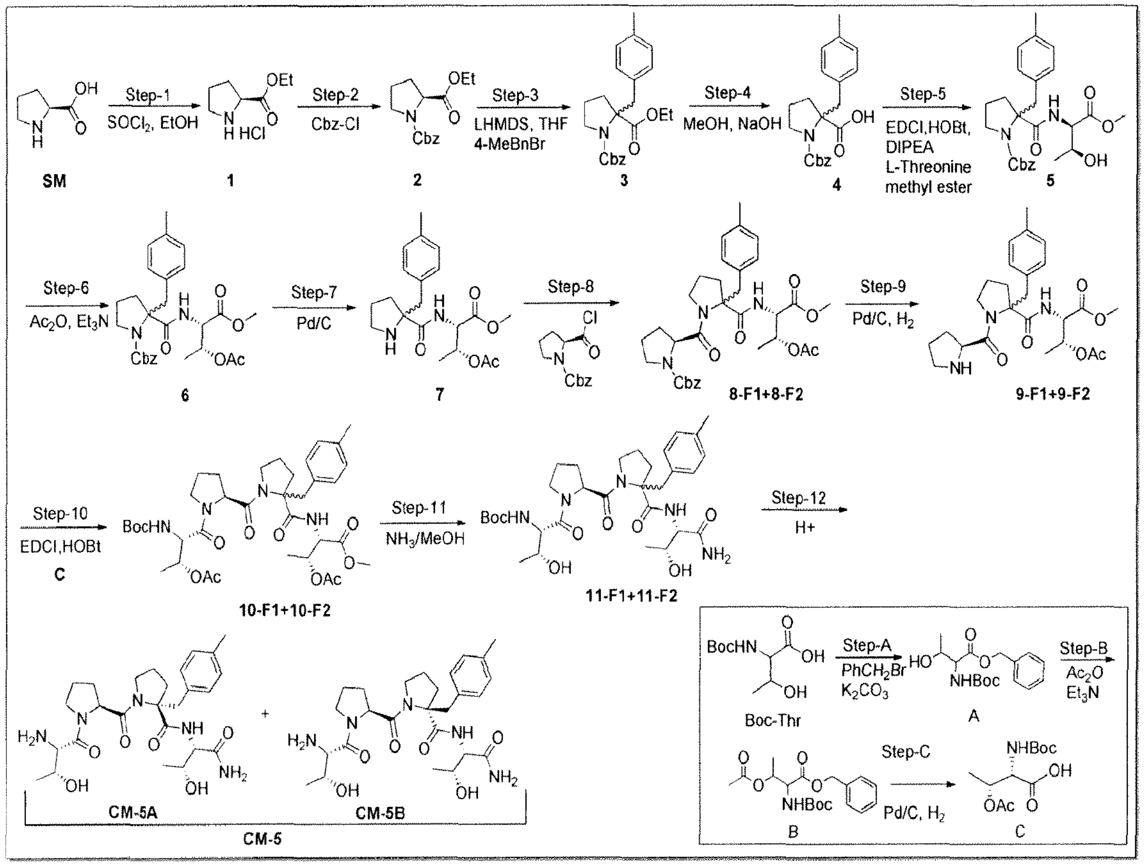


Synthesis of (*S*)-ethyl pyrrolidine-2-carboxylate hydrochloride (1):

To a stirring solution of L-proline (SM) (110 g, 956.5 mmol) in ethanol was added thionyl chloride (141 ml, 1911.3 mmol) and refluxed for 16 h. The reaction mixture was brought to RT and concentrated under vacuum to afford compound 1 as hydrochloride salt (170 g, 99 %).

Synthesis of (*S*)-1-benzyl 2-ethyl pyrrolidine-1,2-dicarboxylate (**2**):

To a stirring solution of compound 1 (170 g, 947 mmol) in $CH_2Cl_2$ was added $Et_3N$ (398 ml, 2.83 mol). After being stirred for 30 min, Cbz-Cl (1.136 mol) was added to the reaction mixture and stirring was continued for another 12 h at RT. The reaction mixture was washed with water and extracted with $CH_2Cl_2$. The organic layer was dried over anhydrous $Na_2SO_4$ and concentrated under vacuum. The crude was purified by column chromatography to afford compound 2 (230 g, 88 %).
**$^1$H-NMR:** (200 MHz, DMSO-$d_6$): δ 7.42-7.31(m, 5H), 5.09 (m, 2H), 4.32-4.23(m, 1H), 4.08-3.98(m, 2H), 3.50-3.38 (m, 2H), 2.30-2.18 (m, 1H), 1.90-1.81(m, 3H), 1.10-1.04 (t, 3H).
**Mass m/z:** 278 [$M^+$+1],

Synthesis of 1-benzyl 2-ethyl 2-(4-methylbenzyl) pyrrolidine-1, 2-dicarboxylate (3):

To a stirring solution of compound **2** (5 g, 0.018 mol) in THF (40 mL) under inert atmosphere was added LiHMDS (1M in THF) (36 mL, 0.036 mol) at -78 °C and stirred for 30 min. To this 4-Methyl benzyl bromide (5 g, 0.027 mol) was added drop wise at -30 °C and it was allowed to warm to RT and stirred for 2 h. The reaction mixture was cooled to 5 °C, quenched with saturated $NH_4Cl$ solution and the aqueous layer was extracted with EtOAc (2 x 100 mL). The combined organic extracts were dried over anhydrous $Na_2SO_4$ and concentrated under reduced pressure. The crude residue obtained was purified by silica gel column chromatography eluting with 20% EtOAc/hexane to afford compound 3 (5.2 g, 76 %) as liquid.
**$^1$H-NMR:** (500 MHz, DMSO-$d_6$): δ 7.47-7.32 (m, 5H), 7.17-7.12 (m, 1H), 7.05-7.01 (m, 1H), 6.95-6.90 (m, 2H), 5.25-5.00

(m, 2H), 4.16-4.12 (m, 1H), 4.00-3.89 (m, 1H), 3.52 (d, 1H), 3.38-3.33 (m, 1H), 2.99-2.89 (m, 2H), 2.23 (s, 3H), 2.12-1.90 (m, 1H), 1.56-1.51 (m, 1H), 1.05-1.01 (m, 3H), 1.00-0.97 (m, 1H), 0.92-0.89 (m, 1H).

Synthesis of 1-((benzyloxy) carbonyl)-2-(4-methylbenzyl) pyrrolidine-2-carboxylic acid **(4):**

To a stirring solution of compound **3** (5.2 g, 0.0136 mol) in methanol (25 mL) and water (15 mL) was added 2N aqueous NaOH (2 g, 0.052 mol) and heated to 85 °C for 4 h. The volatiles were evaporated under reduced pressure and obtained residue was diluted with ice cold water (50mL) and washed with ether (50 mL). The aqueous layer was acidified to pH~2 using 2N HCl and extracted with $CH_2Cl_2$ (2 x 100mL). The combined organic layers were dried over anhydrous $Na_2SO_4$ and concentrated under reduced pressure to afford compound **4** (4.3 g, 89%) as an off white solid.
**$^1$H-NMR:** (400 MHz, $CDCl_3$): δ 7.45-7.38 (m, 5H), 7.09-6.89 (m, 4H), 5.35-5.26 (m, 2H), 3.67-3.47 (m, 2H), 3.14-2.99 (m, 2H), 2.42-2.39 (m, 1H), 2.29 (s, 3H), 2.22-1.99 (m, 1H), 1.74-1.61 (m, 1H), 1.31-1.22 (m, 1H).
**LCMS (ESI):** 354 [$M^+$+1].

Synthesis of benzyl 2-(((2R, 3S)-3-hydroxy-1-methoxy-1-oxobutan-2-yl) carbamoyl)-2-(4-methylbenzyl) pyrrolidine-1-carboxylate **(5):**

To a stirring solution of compound **4** (4.3 g, 12.1 mmol) in $CH_2Cl_2$ (40 mL) were added EDCI. HCl (2.5 g, 13.4 mmol) followed by HOBt (1.82 g, 13.4 mmol) and DIPEA (6.4 mL, 36.5 mmol) at 0 °C. After being stirred for 10 min, L-threonine methyl ester (2.2 g, 13.4 mmol) was added to the reaction mixture and stirred for another 4 h at RT. After consumption of the starting material (by TLC), the reaction was diluted with $CH_2Cl_2$ (150 mL) and washed with water (2 x 30 mL). The organic layer was washed with brine, dried over $Na_2SO_4$, concentrated and purified by silica gel column chromatography eluting with 2% MeOH/$CH_2Cl_2$ to afford compound **5** (4.1 g, 72%).
**$^1$H-NMR:** (500 MHz, $CDCl_3$): δ 7.44-7.31 (m, 5H), 7.09-6.85 (m, 5H), 5.32-5.25 (m, 1H), 5.05-4.94 (m, 2H), 4.25-4.20 (m, 1H), 4.15-4.08 (m, 1H), 3.66-3.64 (m, 2H), 3.45-3.41 (m, 2H), 3.14-3.09 (m, 1H), 2.89-2.84 (m, 1H), 2.20 (s, 3H), 2.05-2.02 (m, 2H), 1.55-1.51 (m, 1H), 1.09-0.98 (m, 4H).
**LCMS (ESI):** 469 [$M^+$+1].

Synthesis of benzyl 2-(((2S, 3R)-3-acetoxy-1-methoxy-1-oxobutan-2-yl) carbamoyl)-2-(4-methylbenzyl) pyrrolidine-1-carboxylate **(6):**

To a stirring solution of compound **5** (4.1 g, 8.75 mmol) in $CH_2Cl_2$ (40 mL) was added $Et_3N$ (3.0 mL, 21.9 mmol) followed by $Ac_2O$ (1.0 g, 10.5 mmol) at 0 °C and stirred for 1 h. To this DMAP (25 mg, catalytic) was added and stirred at RT for 2 h. After consumption of the starting material (by TLC), the reaction mixture was diluted with water and extracted with EtOAc (2x 100 mL). The separated organic layer was dried over anhydrous $Na_2SO_4$, filtered and concentrated under vacuum to afford compound **6** (4.2 g, crude). This was directly used for the next step without further purification.
**$^1$H-NMR:** (500 MHz, $CDCl_3$): δ 7.42-7.30 (m, 5H), 7.09-6.85 (m, 5H), 5.25-5.21 (m, 2H), 5.11-5.09 (m, 1H), 4.67-4.62 (m, 1H), 3.66-3.64 (m, 2H), 3.55-3.46 (m, 3H), 3.15-3.10 (m, 1H), 2.92-2.85 (m, 1H), 2.20 (s, 3H), 2.05-1.95 (m, 5H), 1.56-1.46 (m, 1H), 1.15-1.11 (m, 4H).
**LCMS (ESI):** 511 [$M^+$+1].

Synthesis of (2*S*,3*R*)-methyl 3-acetoxy-2-(2-(4-methylbenzyl) pyrrolidine-2-carboxamido) butanoate (7):

To a stirring solution of compound **6** (4.2 g, 8.22 mmol) in methanol (35 mL) was added 10% Pd/C (1.0 g) under $N_2$ atmosphere. The reaction mixture was stirred under $H_2$ atmosphere (balloon pressure) for 3 h. After consumption of the starting material (by TLC), the reaction was filtered through celite pad and the filtrate was concentrated under reduced pressure to afford compound 7 (3.0 g, 100%) as syrup.
**$^1$H-NMR:** (500 MHz, $CDCl_3$): δ 8.22 (br s, 1H), 7.09-7.06 (m, 4H), 5.23-5.19 (m, 1H), 4.50-4.42 (m, 1H), 3.60 (d, 3H), 3.20 (m, 1H), 3.00-2.97 (m, 1H), 2.75-2.68 (m, 2H), 2.28 (s, 3H), 2.05-2.02 (m, 1H), 1.90 (d, 3H), 1.65-1.51 (m, 3H), 1.10 (dd, 3H).
**LCMS m/z:** 377 [$M^+$+1].

Synthesis of (2*S*)-benzyl 2-(2-(((2*S*,3*R*)-3-acetoxy-1-methoxy-1-oxobutan-2-yl) carbamoyl)-2-(4-methylbenzyl) pyrrolidine-1-carbonyl) pyrrolidine-1-carboxylate **(8):**

To a stirring solution of compound 7 (3.0 g, 7.97 mmol) in $CH_2Cl_2$ (30 mL) and water (20 mL) was added $Na_2CO_3$ (2.1 g, 19.9 mmol) and stirred at 0 °C for 5 min. To this acid chloride [To a solution of (*S*)-1-(benzyloxycarbonyl)

pyrrolidine-2-carboxylic acid (2.3 g, 9.56 mmol) in $CH_2Cl_2$ (10 mL) was added $SOCl_2$ (1.4 mL) drop wise at 0 °C and was refluxed for 2 h. The volatiles were removed under reduced pressure] and the reaction mixture was stirred at RT for 2 h. The separated organic layer was concentrated under vacuum and obtained isomers were separated by washing with diethyl ether (2x20 mL) to afford compound 8-F1 (1.6 g) and compound 8-F2 (2.6 g).

Analytical data for Compound 8-F1:

**$^1$H-NMR:** (400 MHz, $CDCl_3$): δ 7.39-7.28 (m, 5H), 7.15-6.85 (m, 5H), 5.21-5.05 (m, 2H), 5.04-4.92 (m, 1H), 4.65-4.50 (m, 1H), 4.53-4.45 (m, 1H), 3.65 (s, 3H), 3.54-3.46 (m, 4H), 3.21-3.13 (m, 2H), 2.25-2.16 (m, 4H), 2.05-2.00 (m, 2H), 1.95-1.85 (m, 4H), 1.56-1.51 (m, 2H), 1.15 (dd, 3H).
**LCMS m/z:** 608 [$M^+$+1].
**UPLC (Purity):** 97%.

Analytical data for Compound 8-F2:

**$^1$H-NMR:** (400 MHz, $CDCl_3$): δ 8.50 (br s, 1H), 7.36-7.23 (m, 5H), 7.15-6.85 (m, 5H), 5.21-5.05 (m, 2H), 5.04-4.92 (m, 1H), 4.65-4.50 (m, 1H), 4.53-4.45 (m, 1H), 3.65 (s, 3H), 3.54-3.46 (m, 4H), 3.21-3.13 (m, 2H), 2.25-2.16 (m, 4H), 2.05-2.00 (m, 2H), 1.95-1.85 (m, 4H), 1.56-1.51 (m, 2H), 1.15 (dd, 3H).
**LCMS m/z:** 608 [$M^+$+1]
**UPLC (Purity):** 96%.

Synthesis of (2S, 3R)-methyl 3-acetoxy-2-((R)-2-(4-methylbenzyl)-1-((S)-pyrrolidine-2-carbonyl) pyrrolidine-2-carboxamido) butanoate **(9-F1):**

To a stirring solution of compound **8-F1** (1.6 g, 2.63 mmol) in MeOH (20 mL) and EtOAc (20 mL) was added 10% Pd/C (0.5 g) under inert atmosphere and stirred for 4 h under $H_2$ atmosphere (balloon pressure). The reaction mixture was filtered through celite pad and concentrated under reduced pressure to afford compound **9-F1** (1.1 g, 92%).
**$^1$H-NMR:** (400 MHz, $CDCl_3$): δ 8.23 (dd, 1H), 7.20-6.85 (m, 5H), 5.20-5.13 (m, 1H), 4.63-4.59 (m, 1H), 3.85-3.81 (m, 1H), 3.65-3.61 (m, 5H), 3.32-3.25 (m, 2H), 3.12-3.05 (m, 3H), 2.75-2.71 (m, 1H), 2.25 (s, 3H), 2.15-2.13 (m, 2H), 2.00 (d, 3H), 1.77 (m, 3H), 1.27 (dd, 4H).
**LCMS m/z:** 474 [$M^+$+1].

Synthesis of (2S, 3R)-methyl 3-acetoxy-2-((S)-2-(4-methylbenzyl)-1-((S)-pyrrolidine-2-carbonyl) pyrrolidine-2-carboxamido) butanoate **(9-F2):**

To a stirring solution of compound **8-F2** (2.6 g, 4.20 mmol) in MeOH (25 mL) was added 10% Pd/C (1.0 g) under inert atmosphere and stirred for 3 h under $H_2$ atmosphere (balloon pressure). The reaction mixture was filtered through celite pad and concentrated under reduced pressure to afford compound **9-F2** (1.1 g, 55%).
**$^1$H-NMR:** (400 MHz, $CDCl_3$): δ 8.23 (dd, 1H), 7.20-6.85 (m, 5H), 5.20-5.13 (m, 1H), 4.63-4.59 (m, 1H), 3.85-3.81 (m, 1H), 3.65-3.61 (m, 5H), 3.32-3.25 (m, 2H), 3.12-3.05 (m, 3H), 2.75-2.71 (m, 1H), 2.25 (s, 3H), 2.15-2.13 (m, 2H), 2.00 (d, 3H), 1.77 (m, 3H), 1.27 (dd, 4H).
**LCMS m/z:** 474 [$M^+$+1],

Synthesis of Benzyl 2-(tert-butoxycarbonylamino)-3-hydroxybutanoate **(A):**

To a solution of 2-(tert-butoxycarbonylamino)-3-hydroxybutanoic acid (50 g, 228.3mmol) in DMF (500 mL) was added $K_2CO_3$ (63 g, 456.6 mmol) and stirred at RT for 15 min. To this Benzyl bromide (46.83 g, 273.9 mmol) was added and stirred at RT for 6 h. The reaction mixture was diluted with water (500mL) and extracted with EtOAc (2 x 750mL). The combined organic layers were washed with brine (50mL), dried over anhydrous $Na_2SO_4$ and concentrated under reduced pressure. The crude material was purified by silica gel column chromatography eluting with 20% EtOAc/hexane to afford benzyl 2-(tert-butoxycarbonylamino)-3-hydroxybutanoate **A** (52 g, 73 %).
**$^1$H-NMR:** (500 MHz, DMSO-$d_6$): δ 7.37-7.30 (m, 5H), 6.60 (d, 1H), 5.18-5.08 (m, 2H), 4.76 (d, 1H), 4.08-4.00 (m, 2H), 1.38 (s, 9H), 1.09 (d, 3H).
**Mass m/z:** 310.0 [$M^+$+1], 210 [$M^+$-De Boc].

Synthesis of benzyl 3-acetoxy-2-(tert-butoxycarbonylamino) butanoate (B):

To a stirring solution of compound A (52 g, 168.2 mmol) in $CH_2Cl_2$ (500mL) was added $Ac_2O$ (20.5 g, 201.9mmol),

$Et_3N$ (25.4 g, 252.4mmol) and DMAP (3.5 g) and stirred at RT for 2 h. The volatiles were removed under reduced pressure. The residue obtained was diluted with EtOAc (750mL) and washed with cold 0.5 N HCl solution (2 x 200mL). The organic layer was washed with brine, dried over anhydrous $Na_2SO_4$ and concentrated under reduced pressure to afford 3-acetoxy-2-(tert-butoxycarbonylamino) butanoate B (52 g, 88%).
**$^1$H-NMR:** (500 MHz, DMSO-$d_6$): δ 7.35-7.34 (m, 5H), 7.27-7.25 (d, 1H), 5.18-5.06 (m, 3H), 4.34-4.32 (m, 1H), 1.90 (s, 3H), 1.39 (s, 9H), 1.16 (d, 3H).
**Mass m/z:** 252 [$M^+$+1-De Boc]

Synthesis of (2*S*,3*R*)-3-acetoxy-2-(tert-butoxycarbonylamino) butanoic acid **(C):**

To a stirring solution of compound B (52 g, 148.1mmol) in MeOH (1 L) was added 10% Pd/C under $N_2$ atmosphere and reaction mixture was stirred under hydrogen atmosphere for 16 h. The reaction mixture was filtered through a pad of celite, obtained filtrate was evaporated under vacuum and the crude residue was triturated with hexane to yield (2*S*, *3R)-3*-acetoxy-2-(tert-butoxycarbonylamino) butanoic acid C (35 g, 90%).
**$^1$H-NMR:** (500 MHz, DMSO-$d_6$): δ 12.78 (br s, 1H), 6.94 (d, 1H), 5.16-5.14 (m, 1H), 4.17-4.15 (m, 1H), 1.95 (s, 3H), 1.39 (s, 9H), 1.10 (d, 3H).
**Mass m/z:** 260.0 [M-1].

Synthesis of (2*S*,3*R*)-methyl 3-acetoxy-2-((*R*)-1-((*S*)-1-((2*S*,3*R*)-3-acetoxy-2-((*tert*-butoxycarbonyl) amino) butanoyl) pyrrolidine-2-carbonyl)-2-(4-methylbenzyl) pyrrolidine-2-carboxamido) butanoate **(10-F1):**

To a stirring solution of compound **C** (0.66 g, 2.55 mmol) in $CH_2Cl_2$ (15 mL) were added EDCI (0.49 g, 2.55 mmol), HOBt (0.345 g, 2.55 mmol) followed by DIPEA (1.2 mL, 6.96 mmol) at 0 °C and stirred for 10 min. To this compound **9-F1** (1.1 g, 2.32 mmol) was added and stirred for 15h. The reaction mixture was extracted with EtOAc (2 x 75 ml) and the separated organic layer was washed with water (200 mL), followed by brine (200 mL), dried over $Na_2SO_4$ and concentrated under reduced pressure. The crude material was purified by column chromatography eluting with 70% EtOAc/Hexane to afford compound **10-F1** (1.6 g, 100%) as syrup.
**$^1$H-NMR:** (500 MHz, $CDCl_3$): δ 7.24-6.91 (m, 5H), 5.42-5.01 (m, 2H), 4.73-4.68 (m, 1H), 4.54-4.41 (m, 1H), 3.90-3.85 (m, 1H), 3.67 (s, 3H), 3.65-3.60 (m, 2H), 3.59-3.55 (m, 1H), 3.59-3.55 (m, 1H), 3.30 (s, 3H), 3.12-3.05 (m, 1H), 2.33 (s, 3H), 2.05 (s, 3H), 2.00-1.96 (m, 4H), 1.94-1.85 (m, 4H), 1.43 (s, 9H), 1.25 (d, 3H), 1.15 (d, 3H).
**LCMS (ESI):** *m*/*z* 717 [$M^+$+1].

Synthesis of (2*S*,3*R*)-methyl 3-acetoxy-2-((*S*)-1-((*S*)-1-((2*S*,3*R*)-3-acetoxy-2-((*tert*-butoxycarbonyl) amino) butanoyl) pyrrolidine-2-carbonyl)-2-(4-methylbenzyl) pyrrolidine-2-carboxamido) butanoate **(10-F2):**

To a stirring solution of compound C (0.66 g, 2.55 mmol) in $CH_2Cl_2$ (10 mL) were added EDCI. HCl (0.48 g, 2.55 mmol), HOBt (0.345 g, 2.55 mmol) followed by DIPEA (1.2 mL, 6.96 mmol) at 0 °C and stirred for 10 min. To this compound **9-F2** (1.1 g, 2.32 mmol) was added and stirred for 15h. The reaction mixture was extracted with EtOAc (2 x 75 ml) and the separated organic layer was washed with water (200 mL), followed by brine (200 mL), dried over $Na_2SO_4$ and concentrated under reduced pressure. The crude material was purified by column chromatography eluting with 70% EtOAc/Hexane to afford compound **10-F2** (0.8 g, 50%) as syrup.
**$^1$H-NMR:** (400 MHz, DMSO-d6): δ 7.24-6.91 (m, 5H), 5.42-5.01 (m, 2H), 4.73-4.68 (m, 1H), 4.54-4.41 (m, 1H), 3.90-3.85 (m, 1H), 3.67 (s, 3H), 3.65-3.60 (m, 2H), 3.59-3.55 (m, 1H), 3.59-3.55 (m, 1H), 3.30 (s, 3H), 3.12-3.05 (m, 1H), 2.33 (s, 3H), 2.05 (s, 3H), 2.00-1.96 (m, 4H), 1.94-1.85 (m, 4H), 1.43 (s, 9H), 1.25 (d, 3H), 1.15 (d, 3H).
**LCMS (ESI):** *m*/*z* 717 [$M^+$+1].

Synthesis of tert-butyl ((2*S*,3*R*)-1-((*S*)-2-((*R*)-2-(((2*S*,3*R*)-1-amino-3-hydroxy-1-oxobutan-2-yl) carbamoyl)-2-(4-methylbenzyl) pyrrolidine-1-carbonyl) pyrrolidin-1-yl)-3-hydroxy-1-oxobutan-2-yl) carbamate **(11-F1):**

A mixture of compound 10-F1 (1.6 g, 2.20 mmol) and methanolic.$NH_3$ (20 mL) was taken in a autoclave and heated to 70 °C for 16 h. After consumption of the starting material (by TLC), the reaction mixture was concentrated under reduced pressure to afford compound 11-F1 (0.6 g, 46%).
**$^1$H-NMR:** (400 MHz, DMSO-$d_6$): δ 7.32 (br s, 1H), 7.15-7.10 (m, 2H), 7.05-6.91 (m, 3H), 6.65 (br s, 1H), 4.75-4.59 (m, 2H), 4.24-3.45 (m, 8H), 3.35 (s, 1H), 3.05-3.00 (m, 1H), 2.32 (s, 3H), 2.26-1.85 (m, 6H), 1.75 (s, 6H), 1.60-1.55 (m, 1H), 1.47 (s, 9H), 1.15 (d, 3H).
**LCMS (ESI):** *m*/*z* 618.5 [$M^+$+1].

Synthesis of tert-butyl ((2*S*,3*R*)-1-((*S*)-2-((*S*)-2-(((2*S*,3*R*)-1-amino-3-hydroxy-1-oxobutan-2-yl) carbamoyl)-2-(4-methylbenzyl) pyrrolidine-1-carbonyl)pyrrolidin-1-yl)-3-hydroxy-1-oxobutan-2-yl) carbamate **(11-F2):**

A mixture of compound 10-F2 (0.8 g, 1.11 mmol) and methanolic.$NH_3$ (20 mL) was stirred at RT for 16 h. After consumption of the starting material (by TLC & LCMS), the reaction mixture was concentrated under reduced pressure to afford compound 11-F2 (0.4 g, 58%).

**$^1$H-NMR:** (400 MHz, DMSO-$d_6$): δ 7.32 (br s, 1H), 7.15-7.10 (m, 2H), 7.05-6.91 (m, 3H), 6.65 (br s, 1H), 4.75-4.59 (m, 2H), 4.24-3.45 (m, 8H), 3.35 (s, 1H), 3.05-3.00 (m, 1H), 2.32 (s, 3H), 2.26-1.85 (m, 6H), 1.75 (s, 6H), 1.60-1.55 (m, 1H), 1.47 (s, 9H), 1.15 (d, 3H).

**LCMS (ESI):** *m/z* 618.5 [$M^+$+1].

Synthesis of (*R*)-N-((2*S*,3*R*)-1-amino-3-hydroxy-1-oxobutan-2-yl)-1-((*S*)-1-((2*S*,3*R*)-2-amino-3 -hydroxybutanoyl) pyrrolidine-2-carbonyl)-2-(4-methylbenzyl) pyrrolidine-2-carboxamide (CM-5A):

A stirring solution of compound 11-F1 (0.25 g, 0.40 mmol) in 2N-HCl in 1,4 Dioxane (7 mL) at RT for 2 h. The reaction mixture was concentrated under reduced pressure and purified by prep-HPLC to afford CM-5A (0.1 g, 44%) as hydrochloride salt.

**$^1$H-NMR:** (400 MHz, $D_2O$): δ 7.35 (t, 2H), 7.25 (d, 2H), 4.95-4.90 (m, 1H), 4.43-4.35 (m, 4H), 3.94-3.89 (m, 1H), 3.87-3.78 (m, 2H), 3.63 (d, 1H), 3.49-3.40 (m, 1H), 3.25 (d, 1H), 2.56-2.34 (m, 5H), 2.28-2.20 (m, 4H), 1.75-1.71 (m, 1H), 1.56 (d, 3H), 1.32 (d, 3H), 1.15-1.11 (m, 1H).

**LCMS (ESI)** *m/z***:** 518 [$M^+$+1].

**UPLC Purity:** 99%.

**Optical rotation [$\alpha^{25}{}_D$]:** +30.51 (C=1% in water).

**Prep- HPLC:** $R_t$= 7.56 min (Chiralpak IA, 250 x 4.6mm, 5μ; mobile phase (A) 0.1% TFA in *n*-Hexane (B) EtOH (4/1): A: B (80:20); flow Rate: 1.00 mL/min).

Synthesis of (*S*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-methylbenzyl) pyrrolidine-2-carboxamide (CM-5B):

A stirring solution of compound 11-F2 (0.4 g, 0.64 mmol) in 2N-HCl in 1,4 Dioxane (5 mL) at RT for 2 h. The reaction mixture was concentrated under reduced pressure to give crude; which was washed with EtOAc and *n*-pentane to afford CM-5B (0.15 g, 42%) as hydrochloride salt.

**$^1$H-NMR:** (400 MHz, $D_2O$): δ 7.35 (t, 2H), 7.25 (d, 2H), 4.95-4.90 (m, 1H), 4.43-4.35 (m, 4H), 3.94-3.89 (m, 1H), 3.87-3.78 (m, 2H), 3.63 (d, 1H), 3.49-3.40 (m, 1H), 3.25 (d, 1H), 2.56-2.34 (m, 5H), 2.28-2.20 (m, 4H), 1.75-1.71 (m, 1H), 1.56 (d, 3H), 1.32 (d, 3H), 1.15-1.11 (m, 1H).

**LCMS (ESI)** *m/z***:** 518 [$M^+$+1].

**UPLC Purity:** 96%.

**Optical rotation [$\alpha^{25}{}_D$]:** -124.6 (C=1% in water).

**Prep- HPLC:** $R_t$ = 9.68 min (Chiralpak IA, 250 x 4.6mm, 5μ; mobile phase (A) 0.1% TFA in *n*-Hexane (B) EtOH (4/1): A: B (80:20); flow Rate: 1.00 mL/min).

Example 6 - Synthesis of (*R*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-fluorobenzyl) pyrrolidine-2-carboxamide (CM-6A) and (*S*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-fluorobenzyl) pyrrolidine-2-carboxamide (CM-6B):

The following reaction sequence was used (Scheme F) to synthesize (R)-N-((2S,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-fluorobenzyl) pyrrolidine-2-carboxamide (CM-6A) and (*S*)-N-((2S,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-fluorobenzyl) pyrrolidine-2-carboxamide (CM-6B):

Scheme F. Synthesis of CM-6A and CM6B:

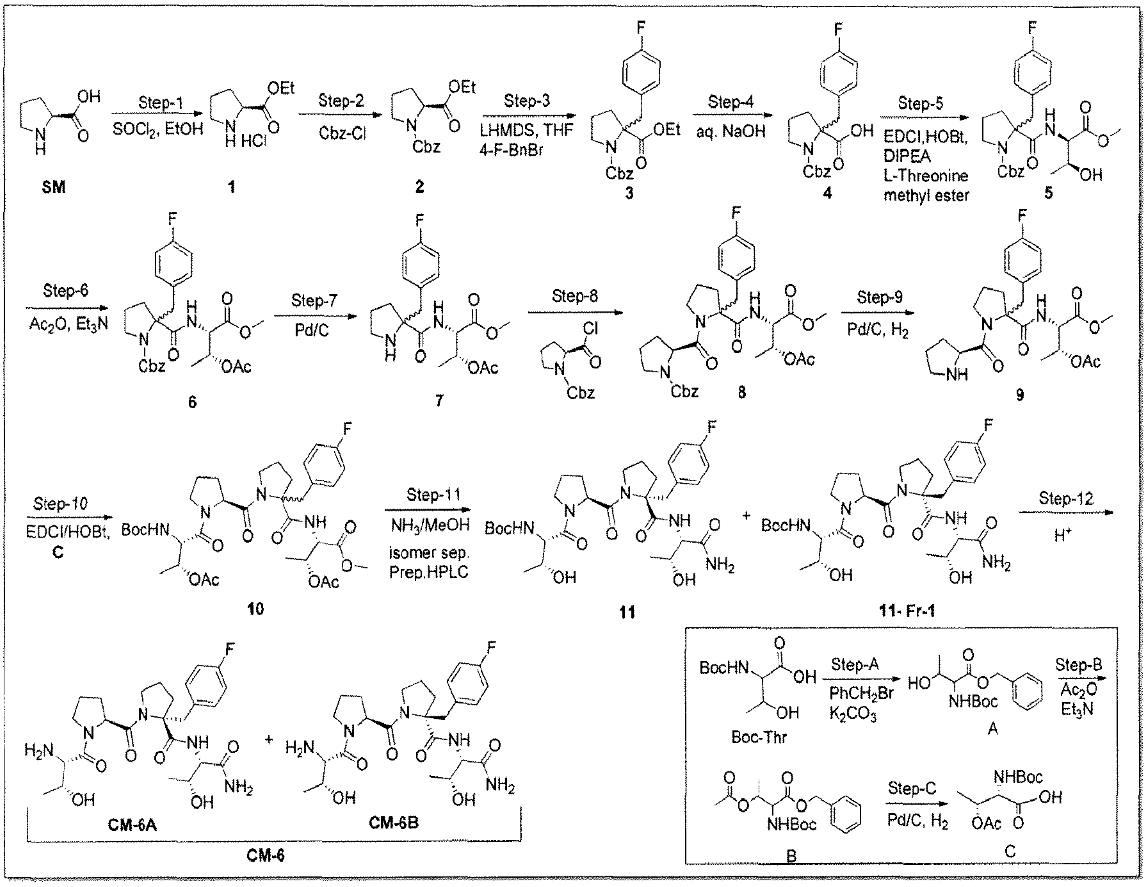


Synthesis of (S)-ethyl pyrrolidine-2-carboxylate hydrochloride (1):

To a stirring solution of L-proline **(SM)** (110 g, 956.5 mmol) in ethanol was added thionyl chloride (141 ml, 1911.3 mmol) and refluxed for 16 h. The reaction mixture was brought to RT and concentrated under vacuum to afford compound 1 as hydrochloride salt (170 g, 99 %).

Synthesis of (*S*)-1-benzyl 2-methyl pyrrolidine-1,2-dicarboxylate **(2):**

To a stirring solution of compound **1** (170 g, 947 mmol) in $CH_2Cl_2$ was added $Et_3N$ (398 ml, 2.83 mol). After being stirred for 30 min, Cbz-Cl (1.136 mol) was added to the reaction mixture and stirring was continued for another 12 h at RT. The reaction mixture was washed with water and extracted with $CH_2Cl_2$. The organic layer was dried over anhydrous $Na_2SO_4$ and concentrated under vacuum. The crude was purified by column chromatography to afford compound 2 (230 g, 88 %).
**$^1$H-NMR:** (200 MHz, DMSO-$d_6$): δ 7.42-731(m, 5H), 5.09 (m, 2H), 4.32-4.23(m, 1H), 4.08-3.98(m, 2H), 3.50-3.38 (m, 2H), 2.30-2.18 (m, 1H), 1.90-1.81(m, 3H), 1.10-1.04 (t, 3H).
**Mass m/z:** 278 [$M^+$+1].

Synthesis of 1-benzyl 2-ethyl 2-(4-fluorobenzyl) pyrrolidine-1,2-dicarboxylate **(3):**

To a stirring solution of compound **2** (10 g, 36.0 mmol) in dry THF (100 mL) under inert atmosphere was added LiHMDS (1M in THF) (72.1 mL, 72.1 mmol) at -20 °C and stirred for 1 h. To this 4-fluorobenzyl bromide (5.34 mL, 43.2 mmol) was added drop wise at -20 °C and it was allowed to warm to RT and stirred for 1 h. The reaction mixture was cooled to 5 °C, quenched with aqueous $NH_4Cl$ solution and the aqueous layer was extracted with EtOAc (2 x 100 mL). The combined organic extracts were dried over anhydrous $Na_2SO_4$ and concentrated under reduced pressure. The crude residue obtained was purified by silica gel column chromatography eluting with 30% EtOAc/hexane to afford compound 3 (10 g, 72 %).
**$^1$H-NMR:** (400 MHz, DMSO-$d_6$) (Rotamers): δ 7.46-7.32 (m, 5H), 7.15-7.01 (m, 4H), 5.18-5.09 (m, 2H), 4.16-3.91 (m, 2H), 3.53 (d, 1H), 3.62 (d, 1H), 3.38-3.32 (m, 1H), 2.97-2.81 (m, 1H), 2.14-1.91 (m, 2H), 1.65-1.51 (m, 1H), 1.25 (t, 2H),

1.09 (t, 1H), 0.99-0.85 (m, 1H).
**LCMS (ESI):** 386 [$M^+$+1].

Synthesis of 1-((benzyloxy) carbonyl)-2-(4-fluorobenzyl) pyrrolidine-2-carboxylic acid **(4):**

To a stirring solution of compound **3** (10 g, 25.9 mmol) in methanol (50 mL) and water (50 mL) was added 2N aqueous NaOH (3.11 g, 77.8 mmol) and heated to 70 °C for 16 h. The volatiles were evaporated under reduced pressure and obtained residue was diluted with ice cold water (50mL) and washed with ether (50 mL). The aqueous layer was acidified to pH~2 using aqueous HCl and extracted with EtOAc (2 x 100mL). The combined organic layers were washed with brine, dried over anhydrous $Na_2SO_4$ and concentrated under reduced pressure to give crude; which was purified by silica gel column chromatography eluting with 2% MeOH/$CH_2Cl_2$ to afford compound **4** (9 g, 97%).

Synthesis of benzyl 2-(4-fluorobenzyl)-2-(((2R, 3S)-3-hydroxy-1-methoxy-1-oxobutan-2-yl) carbamoyl) pyrrolidine-1-carboxylate **(5):**

To a stirring solution of compound **4** (9.5 g, 26.6 mmol) in $CH_2Cl_2$ (95 mL) were added EDCI. HCl (7.61 g, 39.87 mmol) followed by HOBt (5.42 g, 39.87 mmol) and DIPEA (14.6 mL, 79.7 mmol) at 0 °C under $N_2$ atmosphere. After being stirred for 10 min, hydrochloride salt of L-threonine methyl ester (5.4 g, 31.89 mmol) was added to the reaction mixture and stirred for another 16 h at RT. After consumption of the starting material (by TLC), the reaction was diluted with water (100 mL) and extracted with $CH_2Cl_2$ (2x 100 mL). The organic layer was dried over $Na_2SO_4$ and concentrated to give crude; which was purified by silica gel column chromatography eluting with 2% MeOH/$CH_2Cl_2$ to afford compound **5** (13 g, crude).

Synthesis of benzyl 2-(((2*S*,3*R*)-3-acetoxy-1-methoxy-1-oxobutan-2-yl) carbamoyl)-2-(4-fluorobenzyl) pyrrolidine-1-carboxylate **(6):**

To a stirring solution of compound **5** (13 g, 27.5 mmol) in $CH_2Cl_2$ (130 mL) was added $Et_3N$ (9.58 mL, 68.7 mmol) followed by $Ac_2O$ (4.13 mL, 41.26 mmol) at 0 °C and stirred for 1 h. To this DMAP (0.13 g) was added and stirred at RT for 16 h. After consumption of the starting material (by TLC), the reaction was diluted with water (100 mL) and extracted with $CH_2Cl_2$ (2x 100 mL). The organic layer was dried over $Na_2SO_4$ and concentrated to give crude; which was purified by silica gel column chromatography eluting with 30% EtOAc/Hexane to afford compound **6** (10 g, 71%).
**$^1$H-NMR:** (500 MHz, DMSO-$d_6$) (Rotamers): $\delta$ 7.22-7.14 (m, 5H), 7.11-6.98 (m, 4H), 5.26-5.21 (m, 2H), 5.09 (t, 1H), 4.67-4.63 (m, 1H), 3.66-3.64 (m, 3H), 3.69-3.65 (m, 2H), 3.19-3.16 (m, 1H), 2.92-2.85 (m, 1H), 2.10-2.02 (m, 1H), 2.98 (s, 4H), 1.56-1.46 (m, 1H), 1.20-1.14 (m, 3H), 1.12 (t, 1H).
**LCMS (ESI):** 515 [$M^+$+1].
**Mass m/z:** 515 [$M^+$+1],

Synthesis of (2*S*,3*R*)-methyl 3-acetoxy-2-(2-(4-fluorobenzyl) pyrrolidine-2-carboxamido) butanoate **(7):**

To a stirring solution of compound **6** (10 g, 19.4 mmol) in methanol (100 mL) was added wet 10% Pd/C (2 g) under $N_2$ atmosphere. The reaction mixture was stirred under $H_2$ atmosphere (balloon pressure) for 4 h. After consumption of the starting material (by TLC), the reaction was filtered through celite pad and the filtrate was concentrated under reduced pressure to afford compound 7 (6.7 g, 91%).

Synthesis of (2*S*)-benzyl 2-(2-(((2*S*,3*R*)-3-acetoxy-1-methoxy-1-oxobutan-2-yl) carbamoyl)-2-(4-fluorobenzyl) pyrrolidine-1-carbonyl)pyrrolidine-1-carboxylate **(8):**

To a stirring solution of compound 7 (6.7 g, 17.6 mmol) in $CH_2Cl_2$ (67 mL) and water (67 mL) was added $Na_2CO_3$ (4.67 g, 44.0 mmol) and stirred at 0 °C for 5 min. To this acid chloride [To a solution of (*S*)-1-(benzyloxycarbonyl) pyrrolidine-2-carboxylic acid (20.6 g, 82.8mmol) in $CH_2Cl_2$ (20mL) was added $SOCl_2$ (3.19 mL, 44.0 mmol) drop wise at 0 °C and was refluxed for 2 h. The volatiles were removed under reduced pressure to yield (*S*)-benzyl 2-(chlorocarbonyl) pyrrolidine-1-carboxylate] (5.26 g, 21.15 mmol) and the reaction mixture was stirred at RT for 2 h. The separated organic layer was concentrated under vacuum and obtained crude material was purified by column chromatography eluting with 2% MeOH/$CH_2Cl_2$ to afford compound 8 (8.9 g, 83%).
**LCMS m/z:** 612 [$M^+$+1].
**Mass m/z:** 612 [$M^+$+1],

Synthesis of (2*S*,3*R*)-methyl 3-acetoxy-2-(2-(4-fluorobenzyl)-1-((*S*)-pyrrolidine-2-carbonyl) pyrrolidine-2-carboxamido) butanoate **(9):**

To a stirring solution of compound **8** (8.9 g, 14.5 mmol) in MeOH (180 mL) was added wet 10% Pd/C (1.8 g) under inert atmosphere and stirred for 4 h under $H_2$ atmosphere (balloon pressure). The reaction mixture was filtered through celite pad and concentrated under reduced pressure to afford compound **9** (6.9 g, crude).
**$^1$H-NMR:** (400 MHz, $CD_3OD$) (Rotamers): δ 7.22-7.14 (m, 2H), 7.03 (t, 2H), 5.39-5.34 (m, 1H), 4.52 (t, 1H), 3.78-3.72 (m, 2H), 3.74 (d, 3H), 3.35 (s, 2H), 3.29-3.24 (m, 1H), 3.18-3.12 (m, 1H), 2.85-2.83 (m, 1H), 2.31-2.25 (m, 2H), 2.18-2.15 (m, 1H), 2.06 (d, 3H), 1.89-1.75 (m, 3H), 1.71-1.65 (m, 1H), 1.52-1.40 (m, 1H), 1.29 (dd, 3H).
**Mass m/z:** 478 [$M^+$+1].

Synthesis of Benzyl 2-(tert-butoxycarbonylamino)-3-hydroxybutanoate (A):

To a solution of 2-(tert-butoxycarbonylamino)-3-hydroxybutanoic acid **(Boc-Thr)** (50 g, 228.3mmol) in DMF (500 mL) was added $K_2CO_3$ (63 g, 456.6 mmol) and stirred at RT for 15 min. To this Benzyl bromide (46.83 g, 273.9 mmol) was added and stirred at RT for 6 h. The reaction mixture was diluted with water (500mL) and extracted with EtOAc (2 x 750mL). The combined organic layers were washed with brine (50mL), dried over anhydrous $Na_2SO_4$ and concentrated under reduced pressure. The crude material was purified by silica gel column chromatography eluting with 20% EtOAc/hexane to afford benzyl 2-(tert-butoxycarbonylamino)-3-hydroxybutanoate **A** (52 g, 73 %).
**$^1$H-NMR:** (500 MHz, DMSO-$d_6$): δ 7.37-7.30 (m, 5H), 6.60 (d, 1H), 5.18-5.08 (m, 2H), 4.76 (d, 1H), 4.08-4.00 (m, 2H), 1.38 (s, 9H), 1.09 (d, 3H).
**Mass m/z:** 310.0 [$M^+$+1], 210 [$M^+$-De Boc].

Synthesis of benzyl 3-acetoxy-2-(tert-butoxycarbonylamino) butanoate (B):

To a stirring solution of compound **A** (52 g, 168.2 mmol) in $CH_2Cl_2$ (500mL) was added $Ac_2O$ (20.5 g, 201.9mmol), $Et_3N$ (25.4 g, 252.4mmol) and DMAP (3.5 g) and stirred at RT for 2 h. The volatiles were removed under reduced pressure. The residue obtained was diluted with EtOAc (750mL) and washed with cold 0.5 N HCl solution (2 x 200mL). The organic layer was washed with brine, dried over anhydrous $Na_2SO_4$ and concentrated under reduced pressure to afford 3-acetoxy-2-(tert-butoxycarbonylamino) butanoate **B** (52 g, 88%).
**$^1$H-NMR:** (500 MHz, DMSO-$d_6$): δ 7.35-7.34 (m, 5H), 7.27-7.25 (d, 1H), 5.18-5.06 (m, 3H), 4.34-4.32 (m, 1H), 1.90 (s, 3H), 1.39 (s, 9H), 1.16 (d, 3H).
**Mass m/z:** 252 [$M^+$+1-De Boc].

Synthesis of (2*S*,3*R*)-3-acetoxy-2-(tert-butoxycarbonylamino) butanoic acid (C):

To a stirring solution of compound **B** (52 g, 148.1mmol) in MeOH (1 L) was added 10% Pd/C under $N_2$ atmosphere and reaction mixture was stirred under hydrogen atmosphere for 16 h. The reaction mixture was filtered through a pad of celite, obtained filtrate was evaporated under vacuum and the crude residue was triturated with hexane to yield (2*S*,3*R*)-3-acetoxy-2-(*tert*-butoxycarbonylamino) butanoic acid **C** (35 g, 90%).
**$^1$H-NMR:** (500 MHz, DMSO-$d_6$): δ 12.78 (br s, 1H), 6.94 (d, 1H), 5.16-5.14 (m, 1H), 4.17-4.15 (m, 1H), 1.95 (s, 3H), 1.39 (s, 9H), 1.10 (d, 3H).
**Mass m/z:** 260.0 [$M^+$-1].

Synthesis of (2*S*,3*R*)-methyl 3-acetoxy-2-(1-((*S*)-1-((2*S*,3*R*)-3-acetoxy-2-((*tert*-butoxycarbonyl) amino) butanoyl) pyrrolidine-2-carbonyl)-2-(4-fluorobenzyl) pyrrolidine-2-carboxamido) butanoate **(10):**

To a stirring solution of compound **C** (4.53 g, 17.35 mmol) in $CH_2Cl_2$ (69 mL) were added EDCI. HCl (4.14 g, 21.69 mmol), HOBt (2.95 g, 21.7 mmol) followed by DIPEA (7.99 mL, 43.4 mmol) at 0 °C and stirred for 10 min. To this compound **9** (6.9 g, 14.46 mmol) was added and stirred for 16 h. The reaction mixture was diluted with water (75 mL) and extracted with EtOAc (2 x 75ml). The separated organic layer was dried over $Na_2SO_4$ and concentrated under reduced pressure. The crude material was purified by column chromatography eluting with 2% MeOH/$CH_2Cl_2$ to afford compound **10** (8.0 g, 77%).
**$^1$H-NMR:** (400 MHz, DMSO-d6) (Rotamers): δ 7.35-7.29 (m, 1H), 7.23 (m, 1H), 7.13 (d, 2H), 7.08-7.05 (m, 1H), 5.30-5.12 (m, 2H), 4.67-4.34 (m, 3H), 3.98-3.70 (m, 3H), 3.65 (s, 1H), 3.49-3.45 (m, 1H), 3.25-3.10 (m, 1H), 2.32-1.96 (m, 8H), 1.75-1.62 (m, 2H), 1.49 (d, 9H), 1.32-1.29 (m, 3H), 1.25-1.20 (m, 3H).
**LCMS m/z:** 621 [$M^+$+1].

Synthesis of *tert*-butyl ((2*S*,3*R*)-1-((2*S*)-2-(2-(((2*S*,3*R*)-1-amino-3-hydroxy-1-oxobutan-2-yl) carbamoyl)-2-(4-fluorobenzyl) pyrrolidine-1-carbonyl) pyrrolidin-1-yl)-3-hydroxy-1-oxobutan-2-yl) carbamate **(11):**

A stirring mixture of compound **10** (4 g, 5.55 mmol) and methanolic-$NH_3$ (20 mL) was taken in a sealed tube and stirred for 48 h. The reaction mixture was concentrated under vacuum to give crude; which was purified by silica gel column chromatography eluting with 2% MeOH/$CH_2Cl_2$ to afford compound **11** (1.6 g) as mixture. This crude material was submitted for chiral prep-purification.

Chiral preparative HPLC of Isomers:

The isomers of compound **11** (1.6 g, crude) were separated by chiral prep HPLC to obtain Compound **11-F1** (0.6 g) and Compound **11-F2** (0.25 g).

Analytical data for Compound 11-F1:

**$^1$H-NMR:** (400 MHz, DMSO-d6): δ 8.15 (br s, 1H), 7.39-7.35 (m, 2H), 7.24 (s, 1H), 7.11 (t, 2H), 7.04-7.00 (m, 1H), 6.79-6.74 (m, 2H), 4.75 (d, 1H), 4.21 (t, 2H), 4.02-3.95 (m, 1H), 3.81-3.75 (m, 2H), 3.69-3.56 (m, 2H), 3.53-3.49 (m, 1H), 3.27-3.09 (m, 2H), 2.29-2.10 (m, 1H), 2.05-1.91 (m, 5H), 1.69-1.51 (m, 1H), 1.40 (s, 9H), 1.19-1.11 (m, 6H), 0.92-0.84 (m, 1H).
**HPLC:** 85%.
**LCMS (ESI):** *m/z* 622 [$M^+$+1].
**Chiral HPLC:** $R_t$ = 10.69 min (Chiralpak IA, 250 x 4.6mm, 5μ; mobile phase (A) 0.1% TFA in *n*-Hexane (B) EtOH (4/1): A: B (80:20); flow Rate: 1.00 mL/min).

Analytical data for Compound 11-F2:

**$^1$H-NMR:** (400 MHz, DMSO-d6): δ 8.15 (br s, 1H), 7.39-7.35 (m, 2H), 7.24 (s, 1H), 7.11 (t, 2H), 7.04-7.00 (m, 1H), 6.79-6.74 (m, 2H), 4.75 (d, 1H), 4.21 (t, 2H), 4.02-3.95 (m, 1H), 3.81-3.75 (m, 2H), 3.69-3.56 (m, 2H), 3.53-3.49 (m, 1H), 3.27-3.09 (m, 2H), 2.29-2.10 (m, 1H), 2.05-1.91 (m, 5H), 1.69-1.51 (m, 1H), 1.40 (s, 9H), 1.19-1.11 (m, 6H), 0.92-0.84 (m, 1H).
**HPLC:** 84%.
**LCMS (ESI):** *m/z* 622 [$M^+$+1].
**Chiral HPLC:** $R_t$ = 18.55 min (Chiralpak IA, 250 x 4.6mm, 5μ; mobile phase (A) 0.1% TFA in *n*-Hexane (B) EtOH (4/1): A: B (80:20); flow Rate: 1.00 mL/min).

Synthesis of (*R*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-fluorobenzyl) pyrrolidine-2-carboxamide (CM-6A):

To a stirring solution of compound **11-F1** (0.6 g, 0.96 mmol) in $CH_2Cl_2$ (12 mL) was added 4M-HCl in 1,4 dioxane (6 mL) at 0 °C and stirred at RT for 1 h. The reaction mixture was concentrated under vacuum to give crude; which was further washed with EtOAc and dried under vacuum to afford CM-6A (0.28 g, 52%) as hydrochloride salt.
**$^1$H-NMR:** (400 MHz, $D_2O$): δ 7.41-7.32 (m, 2H), 7.21 (t, 2H), 4.95-4.90 (m, 1H), 4.43-4.35 (m, 4H), 3.94-3.62 (m, 5H), 3.27 (d, 1H), 2.56-2.50 (m, 1H), 2.35-2.29 (m, 1H), 2.14-2.05 (m, 4H), 1.81-1.75 (m, 1H), 1.35 (d, 3H), 1.32 (d, 3H), 1.19-1.11 (m, 1H).
**LCMS (ESI)** *m/z***:** 522 [$M^+$+1].
**UPLC Purity:** 96%.
**Chiral HPLC:** $R_t$ = 7.73 min (Chiralpak IA, 250 x 4.6mm, 5μ; mobile phase (A) 0.1% TFA in *n*-Hexane (B) EtOH (4/1): A: B (80:20); flow Rate: 1.00 mL/min).
**Optical rotation [$\alpha^{20}_D$]:** +7.15 (C=1% in water).

Synthesis of (S)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-fluorobenzyl) pyrrolidine-2-carboxamide (CM-6B):

To a stirring solution of compound **11-F2** (0.25 g, 0.40 mmol) in $CH_2Cl_2$ (5 mL) was added 4M-HCl in 1,4 Dioxane (2.5 mL) at 0 °C and stirred at RT for 3 h. The reaction mixture was concentrated under vacuum to give crude; which was further washed with EtOAc and dried under vacuum to afford CM-6B (0.1 g, 45%) as hydrochloride salt.
**$^1$H-NMR:** (400 MHz, $D_2O$): δ 7.41-7.32 (m, 2H), 7.21 (t, 2H), 4.95-4.90 (m, 1H), 4.43-4.35 (m, 4H), 3.94-3.62 (m, 5H), 3.27 (d, 1H), 2.56-2.50 (m, 1H), 2.35-2.29 (m, 1H), 2.14-2.05 (m, 4H), 1.81-1.75 (m, 1H), 1.35 (d, 3H), 1.32 (d, 3H),

1.19-1.11 (m, 1H).
**LCMS (ESI)** *m/z***:** 522 [$M^{+}$+1].
**UPLC Purity:** 98.38%.
**Chiral HPLC:** $R_t$ = 11.28 min (Chiralpak IA, 250 x 4.6mm, 5μ; mobile phase (A) 0.1% TFA in *n*-Hexane (B) EtOH (4/1): A: B (80:20); flow Rate: 1.00 mL/min).
**Optical rotation [$\alpha^{20}{}_{D}$]:** -102.77 (C=1% in water).

Example 7 - Synthesis of (*R*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-(trifluoromethyl) benzyl) pyrrolidine-2-carboxamide (CM-7A) and (*S*)-N-((2*S*,3*R*)-1-amino-3-_hydroxy-1-oxobutan-2-yl)-1-((*S*)-1-((2*S*,3*R*)-2-amino-3-hydroxybutanoyl)pyrrolidine-2-carbonyl)-2-(4-(trifluoromethyl) benzyl) pyrrolidine-2-carboxamide (CM-7B):

The following reaction sequence was used (Scheme G) to synthesize (*R*)-N-((2S,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-(trifluoromethyl) benzyl) pyrrolidine-2-carboxamide (CM-7A) and (*S*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(4-(trifluoromethyl) benzyl) pyrrolidine-2-carboxamide (CM-7B):

Scheme G. Synthesis of CM-7A and CM-7B:

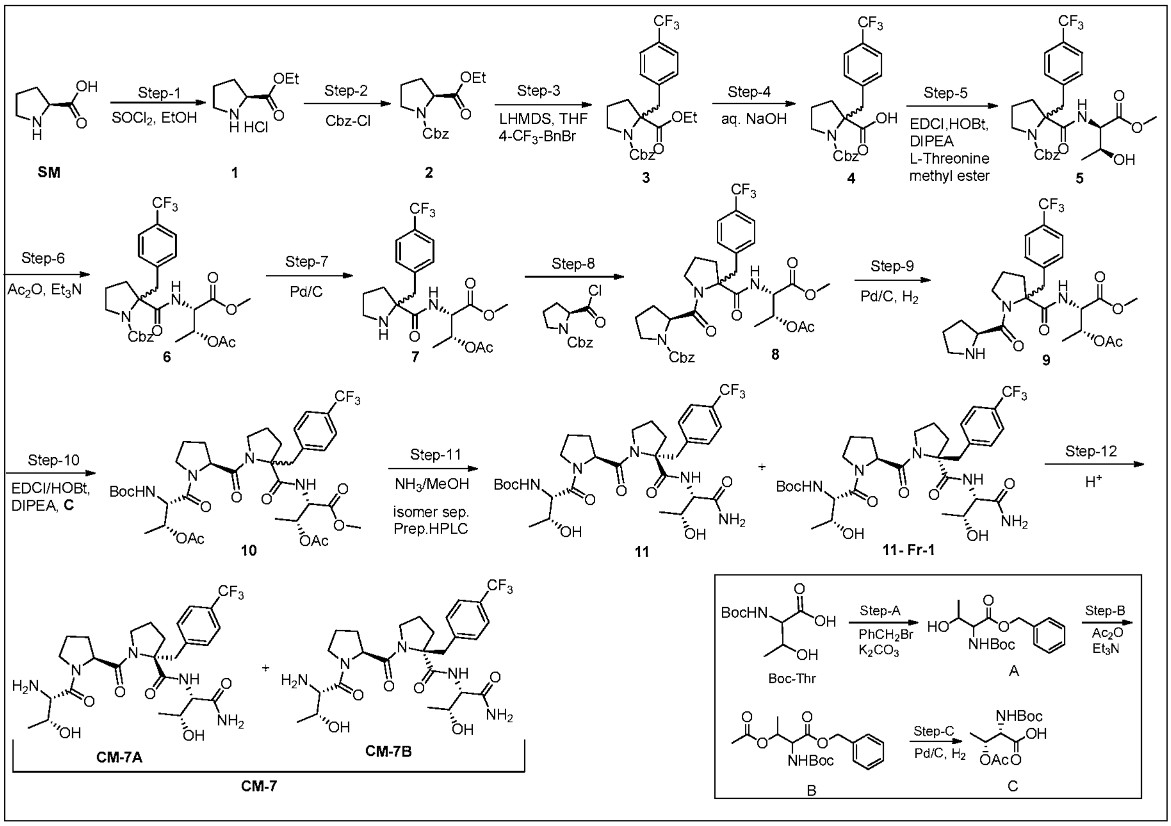


Synthesis of (*S*)-ethyl pyrrolidine-2-carboxylate hydrochloride **(1):**

To a stirring solution of L-proline **(SM)** (110 g, 956.5 mmol) in ethanol was added thionyl chloride (141 ml, 1911.3 mmol) and refluxed for 16 h. The reaction mixture was brought to RT and concentrated under vacuum to afford compound 1 as hydrochloride salt (170 g, 99 %).

Synthesis of (*S*)-1-benzyl 2-methyl pyrrolidine-1,2-dicarboxylate **(2):**

To a stirring solution of compound **1** (170 g, 947 mmol) in $CH_2Cl_2$ was added $Et_3N$ (398 ml, 2.83 mol). After being stirred for 30 min, Cbz-Cl (1.136 mol) was added to the reaction mixture and stirring was continued for another 12 h at RT. The reaction mixture was washed with water and extracted with $CH_2Cl_2$. The organic layer was dried over anhydrous $Na_2SO_4$ and concentrated under vacuum. The crude was purified by column chromatography to afford

compound **2** (230 g, 88 %).
**$^1$H-NMR:** (200 MHz, DMSO-$d_6$): δ 7.42-7.31(m, 5H), 5.09 (m, 2H), 4.32-4.23(m, 1H), 4.08-3.98(m, 2H), 3.50-3.38 (m, 2H), 2.30-2.18 (m, 1H), 1.90-1.81(m, 3H), 1.10-1.04 (t, 3H).
**Mass m/z:** 278 [$M^+$+1],

Synthesis of 1-benzyl 2-ethyl 2-(4-(trifluoromethyl) benzyl) pyrrolidine-1, 2-dicarboxylate **(3):**

To a stirring solution of compound **2** (5 g, 0.018 mol) in THF (50 mL) under inert atmosphere was added LiHMDS (1M in THF) (27 mL, 0.026 mol) at -78 °C and stirred for 30 min. To this 1-(bromomethyl)-4-(trifluoromethyl)benzene (3.3 mL, 0.021 mol) was added drop wise at -40 °C and it was allowed to warm to RT and stirred for 3 h. The reaction mixture was cooled to 5 °C, quenched with aqueous $NH_4Cl$ solution and the aqueous layer was extracted with EtOAc (2 x 100 mL). The combined organic extracts were dried over anhydrous $Na_2SO_4$ and concentrated under reduced pressure. The crude residue obtained was purified by silica gel column chromatography eluting with 10% EtOAc/hexane to afford compound **3** (6 g, 76 %) as liquid.
**$^1$H-NMR:** (500 MHz, DMSO-$d_6$): δ 7.64-7.58 (m, 2H), 7.44-7.25 (m, 7H), 5.22-5.12 (m, 2H), 4.16-4.12 (m, 1H), 4.05-3.89 (m, 1H), 3.62 (d, 1H), 3.48-3.39 (m, 1H), 3.16-3.10 (m, 1H), 2.99-2.89 (m, 1H), 2.19-1.99 (m, 2H), 1.63-1.58 (m, 1H), 1.19 (t, 2H), 1.05 (t, 1H), 0.92-0.89 (m, 1H).

Synthesis of 1-((benzyloxy) carbonyl)-2-(4-(trifluoromethyl) benzyl) pyrrolidine-2-carboxylic acid **(4):**

To a stirring solution of compound **3** (6 g, 0.014 mol) in methanol (50 mL) and water (15 mL) was added 2N aqueous NaOH (1.1 g, 0.027 mol) and heated to 70 °C for 3 h. The volatiles were evaporated under reduced pressure and obtained residue was diluted with ice cold water (50mL) and washed with ether (50 mL). The aqueous layer was acidified to pH~2 using 2N HCl and extracted with EtOAc (2 x 100mL). The combined organic layers were dried over anhydrous $Na_2SO_4$ and concentrated under reduced pressure to afford compound **4** (4 g, 71%) as an off white solid.
**$^1$H-NMR:** (400 MHz, DMSO-$d_6$): δ 7.59-7.22 (m, 10H), 5.24-5.19 (m, 1H), 5.05-5.00 (m, 1H), 3.65 (d, 1H), 3.35-3.20 (m, 1H), 3.19 (t, 1H), 3.01-2.89 (m, 1H), 2.00-1.97 (m, 2H), 1.65-1.61 (m, 1H), 0.85-0.77 (m, 1H).
**LCMS (ESI):** 406 [$M^+$-1].
**Mass *m/z*:** 406 [$M^+$-1].

Synthesis of benzyl 2-(((2R, 3S)-3-hydroxy-1-methoxy-1-oxobutan-2-yl) carbamoyl)-2-(4-(trifluoromethyl) benzyl) pyrrolidine-1-carboxylate **(5):**

To a stirring solution of compound **4** (4 g, 9.80 mmol) in $CH_2Cl_2$ (40) were added EDCI (2.8 g, 14.7 mmol) followed by HOBt (2.6 g, 19.6 mmol) and DIPEA (3.5 mL, 19.6 mmol) at 0 °C. After being stirred for 10 min, hydrochloride salt of L-threonine methyl ester (2 g, 11.8 mmol) was added to the reaction mixture and stirred for another 16 h at RT. After consumption of the starting material (by TLC), the reaction was diluted with $CH_2Cl_2$ (150 mL) and washed with aqueous $NaHCO_3$ (2 x 30 mL) followed by citric acid (2 x 30 mL). The organic layer was dried over $Na_2SO_4$ and concentrated to afford compound **5** (5 g, crude).

Synthesis of benzyl 2-(((2*S*,3*R*)-3-acetoxy-1-methoxy-1-oxobutan-2-yl) carbamoyl)-2-(4-(trifluoromethyl) benzyl) pyrrolidine-1-carboxylate **(6):**

To a stirring solution of compound **5** (5 g, 9.57 mmol) in $CH_2Cl_2$ (50 mL) was added $Et_3N$ (4 mL, 28.7 mmol) followed by $Ac_2O$ (2.7 mL, 28.7mmol) at 0 °C and stirred for 1 h. To this DMAP (0.1 g) was added and stirred at RT for 16 h. The volatiles were evaporated under reduced pressure and obtained crude material was purified by column chromatography eluting with 20% EtOAc/Hexane to afford compound **6** (3.4 g, 63%) as light yellow liquid.
**$^1$H-NMR:** (500 MHz, DMSO-$d_6$) (Rotamers): δ 7.62-7.50 (m, 2H), 7.43-7.24 (m, 7H), 5.27-5.21 (m, 2H), 5.11-5.09 (m, 1H), 4.67-4.62 (m, 1H), 3.66-3.64 (m, 4H), 3.55-3.46 (m, 2H), 2.92-2.85 (m, 2H), 2.05-1.95 (m, 1H), 1.99 (s, 3H), 1.56-1.46 (m, 1H), 1.15-1.11 (m, 4H).
**LCMS (ESI):** 565 [$M^+$+1].

Synthesis of (2*S*,3*R*)-methyl 3-acetoxy-2-(2-(4-(trifluoromethyl) benzyl) pyrrolidine-2-carboxamido) butanoate **(7):**

To a stirring solution of compound **6** (3.4 g, 6.02 mmol) in methanol (20 mL) was added wet 10% Pd/C (0.34 g) under $N_2$ atmosphere. The reaction mixture was stirred under $H_2$ atmosphere (balloon pressure) for 6 h. After consumption of the starting material (by TLC), the reaction was filtered through celite pad and the filtrate was concentrated under reduced pressure to afford compound 7 (2.1 g, 77%) as thick syrup.

**$^1$H-NMR:** (400 MHz, DMSO-$d_6$) (Rotamers): δ 8.22 (br s, 1H), 7.62-7.56 (m, 2H), 7.45-7.39 (m, 2H), 5.23-5.19 (m, 1H), 4.50-4.42 (m, 1H), 3.60 (d, 3H), 3.05-3.00 (m, 1H), 2.97-2.85 (m, 3H), 2.18-2.09 (m, 1H), 1.95 (d, 3H), 1.65-1.51 (m, 3H), 1.10 (d, 2H), 0.75 (d, 1H).
**LCMS m/z:** 431 [$M^+$+1],

Synthesis of (2*S*)-benzyl 2-(2-(((2*S*,3*R*)-3-acetoxy-1-methoxy-1-oxobutan-2-yl) carbamoyl)-2-(4-(trifluoromethyl) benzyl) pyrrolidine-1-carbonyl)pyrrolidine-1-carboxylate (8):

To a stirring solution of compound 7 (4.5 g, 10.4 mmol) in $CH_2Cl_2$ (60 mL) and water (40 mL) was added $Na_2CO_3$ (2.2 g, 20.9 mmol) and stirred at 0 °C for 5 min. (*S*)-benzyl 2-(chlorocarbonyl) pyrrolidine-1-carboxylate (3.3 g, 12.5 mmol) was added and the reaction mixture was stirred at RT for 2 h. The separated organic layer was concentrated under vacuum and obtained crude material was purified by column chromatography eluting with 50% EtOAc/Hexane to afford compound 8 (4.5 g, 65%) as yellow liquid. (*S*)-benzyl 2-(chlorocarbonyl) pyrrolidine-1-carboxylate was synthesized as follows. To a solution of (*S*)-1-(benzyloxycarbonyl) pyrrolidine-2-carboxylic acid (20.6 g, 82.8mmol) in $CH_2Cl_2$ (20mL) was added $SOCl_2$ (20.5 g, 172.6 mmol) drop wise at 0 °C and was refluxed for 2 h. The volatiles were removed under reduced pressure to yield (*S*)-benzyl 2-(chlorocarbonyl) pyrrolidine-1-carboxylate.
**$^1$H-NMR:** (400 MHz, DMSO-$d_6$) (Rotamers): δ 8.50 (br s, 1H), 7.79-7.65 (m, 2H), 7.52-7.37 (m, 7H), 5.42-5.05 (m, 3H), 4.79-4.59 (m, 1H), 4.15 (q, 1H), 3.78 (s, 3H), 3.69-3.50 (m, 3H), 3.21-3.13 (m, 1H), 2.45-2.16 (m, 2H), 2.10 (s, 6H), 2.05-2.00 (m, 2H), 1.95-1.85 (m, 1H), 1.44-1.19 (m, 6H).
**LCMS m/z:** 662 [$M^+$+1].

Synthesis of (2*S*,3*R*)-methyl 3-acetoxy-2-(1-((*S*)-pyrrolidine-2-carbonyl)-2-(4-(trifluoromethyl) benzyl) pyrrolidine-2-carboxamido) butanoate **(9):**

To a stirring solution of compound **8** (4.5 g, 6.8 mmol) in MeOH (45 mL) was added wet 10% Pd/C (0.45 g) under inert atmosphere and stirred for 16 h under $H_2$ atmosphere (balloon pressure). The reaction mixture was filtered through celite pad and concentrated under reduced pressure to afford compound **9** (3.5 g, crude) as semi solid.

Synthesis of Benzyl 2-(*tert*-butoxycarbonylamino)-3-hydroxybutanoate **(A):**

To a solution of 2-(tert-butoxycarbonylamino)-3-hydroxybutanoic acid **(Boc-Thr)** (50 g, 228.3mmol) in DMF (500 mL) was added $K_2CO_3$ (63 g, 456.6 mmol) and stirred at RT for 15 min. To this Benzyl bromide (46.83 g, 273.9 mmol) was added and stirred at RT for 6 h. The reaction mixture was diluted with water (500mL) and extracted with EtOAc (2 x 750mL). The combined organic layers were washed with brine (50mL), dried over anhydrous $Na_2SO_4$ and concentrated under reduced pressure. The crude material was purified by silica gel column chromatography eluting with 20% EtOAc/hexane to afford benzyl 2-(tert-butoxycarbonylamino)-3-hydroxybutanoate **A** (52 g, 73 %).
**$^1$H-NMR:** (500 MHz, DMSO-$d_6$): δ 7.37-7.30 (m, 5H), 6.60 (d, 1H), 5.18-5.08 (m, 2H), 4.76 (d, 1H), 4.08-4.00 (m, 2H), 1.38 (s, 9H), 1.09 (d, 3H).
**Mass m/z:** 310.0 [$M^+$+1], 210 [$M^+$-De Boc].

Synthesis of benzyl 3-acetoxy-2-(*tert*-butoxycarbonylamino) butanoate (**B**):

To a stirring solution of compound **A** (52 g, 168.2 mmol) in $CH_2Cl_2$ (500mL) was added $Ac_2O$ (20.5 g, 201.9mmol), $Et_3N$ (25.4 g, 252.4mmol) and DMAP (3.5 g) and stirred at RT for 2 h. The volatiles were removed under reduced pressure. The residue obtained was diluted with EtOAc (750mL) and washed with cold 0.5 N HCl solution (2 x 200mL). The organic layer was washed with brine, dried over anhydrous $Na_2SO_4$ and concentrated under reduced pressure to afford 3-acetoxy-2-(tert-butoxycarbonylamino) butanoate **B** (52 g, 88%).
**$^1$H-NMR:** (500 MHz, DMSO-$d_6$): δ 7.35-7.34 (m, 5H), 7.27-7.25 (d, 1H), 5.18-5.06 (m, 3H), 4.34-4.32 (m, 1H), 1.90 (s, 3H), 1.39 (s, 9H), 1.16 (d, 3H).
**Mass m/z:** 252 [$M^+$+1-De Boc].

Synthesis of (2*S*,3*R*)-3-acetoxy-2-(tert-butoxycarbonylamino) butanoic acid (**C**):

To a stirring solution of compound **B** (52 g, 148.1mmol) in MeOH (1 L) was added 10% Pd/C under $N_2$ atmosphere and reaction mixture was stirred under hydrogen atmosphere for 16 h. The reaction mixture was filtered through a pad of celite, obtained filtrate was evaporated under vacuum and the crude residue was triturated with hexane to yield (2*S*,3*R*)-3-acetoxy-2-(tert-butoxycarbonylamino) butanoic acid **C** (35 g, 90%).
**$^1$H-NMR:** (500 MHz, DMSO-$d_6$): δ 12.78 (br s, 1H), 6.94 (d, 1H), 5.16-5.14 (m, 1H), 4.17-4.15 (m, 1H), 1.95 (s, 3H), 1.39

(s, 9H), 1.10 (d, 3H).
**Mass m/z:** 260.0 [M-1].

Synthesis of (2*S*,3*R*)-methyl 3-acetoxy-2-(1-((*S*)-1-((2*S*,3*R*)-3-acetoxy-2-((*tert*-butoxycarbonyl)amino)butanoyl)pyrrolidine-2-carbonyl)-2-(4-(trifluoromethyl)benzyl) pyrrolidine-2-carboxamido)butanoate (**10**):

To a stirring solution of compound **C** (1.78 g, 6.83 mmol) in $CH_2Cl_2$ (30 mL) were added EDCI (1.63 g, 8.53 mmol), HOBt (1.53 g, 11.38 mmol) followed by DIPEA (2 mL, 14.2 mmol) at 0 °C and stirred for 10 min. To this compound **9** (3 g, 5.69 mmol) was added and stirred for 16 h. The reaction mixture was extracted with EtOAc (2 x 75ml) and the separated organic layer was washed with aqueous NaHCO3 (100mL), aqueous citric acid (100 mL) followed by brine (100 mL), dried over $Na_2SO_4$ and concentrated under reduced pressure. The crude material was purified by column chromatography eluting with 70% EtOAc/Hexane to afford compound **10** (2.1 g, 51%) as an off-white solid.
**$^1$H-NMR:** (400 MHz, DMSO-d6) (Rotamers): δ 7.71-7.55 (m, 3H), 7.34-7.05 (m, 2H), 5.35-5.20 (m, 1H), 5.09-5.01 (m, 1H), 4.78-4.35 (m, 3H), 3.90-3.85 (m, 2H), 3.67 (s, 4H), 3.39-3.25 (m, 1H), 3.25-3.05 (m, 1H), 2.21-1.96 (m, 12H), 1.39 (d, 9H), 1.35-1.25 (m, 8H), 0.85 (d, 1H).

Synthesis of *tert*-butyl ((2*S*,3*R*)-1-((2*S*)-2-(2-(((2*S*,3*R*)-1-amino-3-hydroxy-1-oxobutan-2-yl)carbamoyl)-2-(4-(trifluoromethyl)benzyl)pyrrolidine-1-carbonyl)pyrrolidin-1-yl)-3-hydroxy-1-oxobutan-2-yl) carbamate (**11**):

To a stirring solution of compound **10** (1 g, 1.29 mmol) in MeOH (10 mL) was added methanolic-$NH_3$ (20 mL) at 0 °C and stirred at RT for 24 h. The reaction mixture was concentrated under vacuum to afford compound **11** (0.87 g, crude) as an off-white solid.

Chiral preparative HPLC of Isomers:

The isomers of compound **11** (0.3 g, crude) were separated by chiral prep HPLC to obtain Compound **11** (0.3 g) and Compound **11-F2** (0.15 g).

Analytical data for Compound **11:**

**$^1$H-NMR:** (400 MHz, DMSO-d6): δ 7.59-7.51 (m, 4H), 7.24 (s, 1H), 7.02 (s, 1H), 6.79-6.60 (m, 2H), 5.09 (d, 1H), 4.77-4.74 (m, 1H), 4.64-4.61 (m, 1H), 4.21 (t, 2H), 3.98 (dd, 1H), 3.85-3.80 (m, 2H), 3.71-3.60 (m, 3H), 3.25-3.19 (m, 2H), 2.21-1.85 (m, 5H), 1.75-1.70 (m, 1H), 1.44 (s, 10H), 1.21 (d, 3H), 1.09 (d, 3H), 0.91-0.85 (m, 1H).
**HPLC:** 93%.
**LCMS (ESI):** *m*/*z* 671.6 [$M^+$+1].
**Chiral HPLC:** $R_t$ = 11.70 min (Chiralpak IA, 250 x 4.6mm, 5μ; mobile phase (A) *n*-Hexane (B) EtOH (4/1): A: B (80:20); flow Rate: 1.00 mL/min).

Analytical data for Compound 11-F2:

**$^1$H-NMR:** (400 MHz, DMSO-d6): δ 7.59-7.51 (m, 4H), 7.24 (s, 1H), 7.02 (s, 1H), 6.79-6.60 (m, 2H), 5.09 (d, 1H), 4.77-4.74 (m, 1H), 4.64-4.61 (m, 1H), 4.21 (t, 2H), 3.98 (dd, 1H), 3.85-3.80 (m, 2H), 3.71-3.60 (m, 3H), 3.25-3.19 (m, 2H), 2.21-1.85 (m, 5H), 1.75-1.70 (m, 1H), 1.44 (s, 10H), 1.21 (d, 3H), 1.09 (d, 3H), 0.91-0.85 (m, 1H).
**HPLC:** 91%.
**LCMS (ESI):** *m*/*z* 671.6 [$M^+$+1].
**Chiral HPLC:** $R_t$ = 19.68 min (Chiralpak IA, 250 x 4.6mm, 5μ; mobile phase (A) *n*-Hexane (B) EtOH (4/1): A: B (80:20); flow Rate: 1.00 mL/min).

Synthesis of (R)-N-((2S, 3R)-1-amino-3-hydroxy-1-oxobutan-2-yl)-1-((S)-1-((2S, 3R)-2-amino-3-hydroxybutanoyl)pyrrolidine-2-carbonyl)-2-(4-(trifluoromethyl)benzyl) pyrrolidine-2-carboxamide (CM-7A):

To a stirring solution of compound **11-F1** (0.3 g, 0.45 mmol) in $CH_2Cl_2$ (5 mL) was added 4N-HCl in 1,4 Dioxane (1 mL) at 0 °C and stirred at RT for 3 h. The reaction mixture was concentrated under vacuum to afford CM-7A (0.15 g, 59 %) as hydrochloride salt.
**$^1$H-NMR:** (400 MHz, $D_2O$): δ 7.89 (d, 2H), 7.57 (d, 2H), 4.95-4.90 (m, 1H), 4.43-4.35 (m, 4H), 3.94-3.79 (m, 5H), 3.35 (q, 2H), 2.56-2.50 (m, 1H), 2.35-2.29 (m, 2H), 2.14-2.05 (m, 3H), 1.81-1.75 (m, 1H), 1.35 (d, 3H), 1.32 (d, 3H), 1.19-1.11 (m, 1H).
**LCMS (ESI)** *m*/*z:* 572.6 [$M^+$+1].

**Mass m/z:** 572.4 [$M^{+}$+1].
**UPLC Purity:** 97%.
**Optical rotation [$\alpha^{20}{}_{D}$]:** +14.86 (C=1% in water).

Synthesis of (*S*)-N-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-(4-(trifluoromethyl)benzyl) pyrrolidine-2-carboxamide (CM-7B):

To a stirring solution of compound **11-F2** (0.15 g, 0.22 mmol) in $CH_2Cl_2$ (5 mL) was added 4N-HCl in 1,4 Dioxane (0.5 mL) at 0 °C and stirred at RT for 3 h. The reaction mixture was concentrated under vacuum to afford CM-7B (140 mg, 59 %) as hydrochloride salt.
**$^{1}$H-NMR:** (400 MHz, $D_2O$): δ 7.89 (d, 2H), 7.57 (d, 2H), 4.95-4.90 (m, 1H), 4.43-4.35 (m, 4H), 3.94-3.79 (m, 5H), 3.35 (q, 2H), 2.56-2.50 (m, 1H), 2.35-2.29 (m, 2H), 2.14-2.05 (m, 3H), 1.81-1.75 (m, 1H), 1.35 (d, 3H), 1.32 (d, 3H), 1.19-1.11 (m, 1H).
**LCMS (ESI)** *m/z:* 572.8 [$M^{+}$+1].
**Mass m/z:** 572.4 [$M^{+}$+1].
**UPLC Purity:** 97.67%.
**Optical rotation [$\alpha^{20}{}_{D}$]:** -105.51 (C=1% in water).

Example 8 - Synthesis of (*R*)-N-((2*S*,3*R*)-1-amino-3-hydroxy-1-oxobutan-2-yl)-1-((*S*)-1-((2*S*, 3*R*)-2-amino-3-hydroxybutanoyl)pyrrolidine-2-carbonyl)-2-(3-methylbenzyl)pyrrolidine-2-carboxamide (CM-8A) and (*S*)-N-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-(3-methylbenzyl)pyrrolidine-2-carboxamide (CM-8B):

The following reaction sequence was used (Scheme H) to synthesize (*R*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(3-methylbenzyl) pyrrolidine-2-carboxamide (CM-8A) and (*S*)-N-((2*S*, 3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(3-methylbenzyl) pyrrolidine-2-carboxamide (CM-8B):

Scheme H. Synthesis of CM-8A and CM-8B:

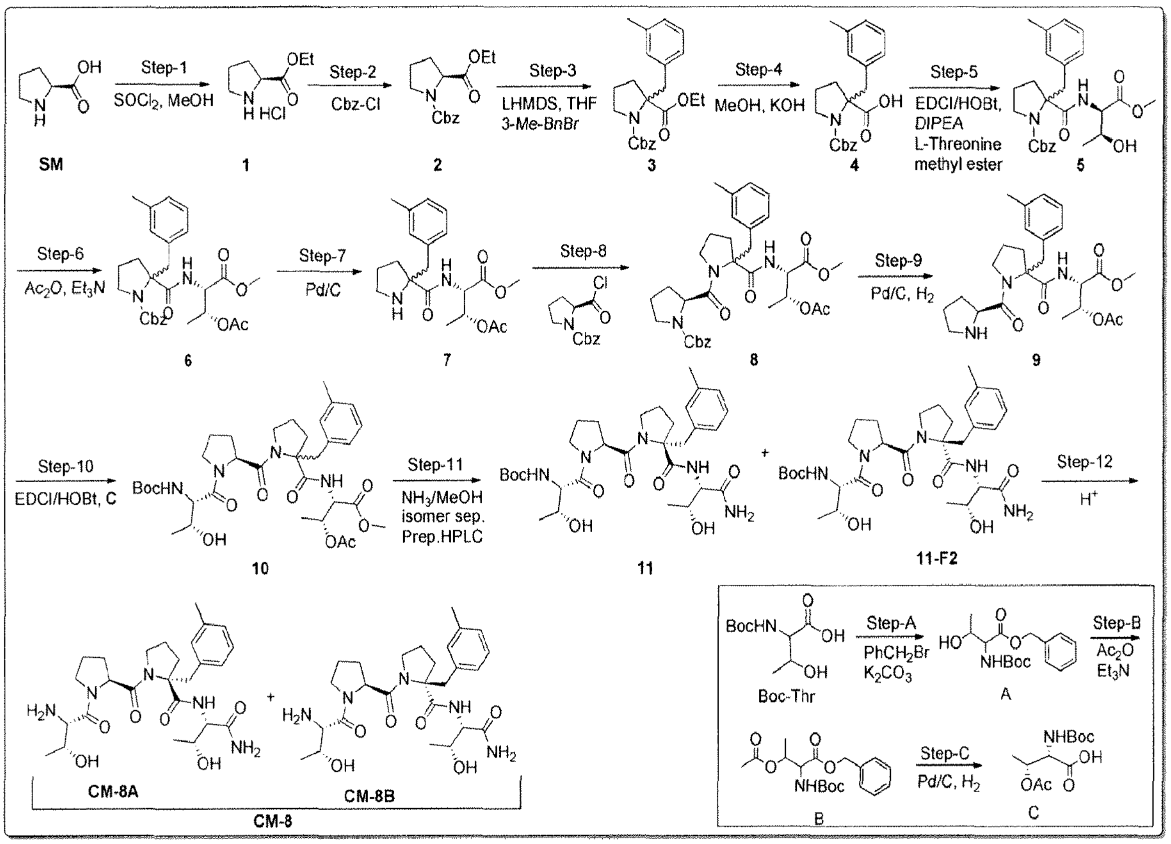

Synthesis of (*S*)-ethyl pyrrolidine-2-carboxylate hydrochloride (1):

To a stirring solution of L-proline (**SM**) (110 g, 956.5 mmol) in ethanol was added thionyl chloride (141 ml, 1911.3 mmol) and refluxed for 16 h. The reaction mixture was brought to RT and concentrated under vacuum to afford compound 1 as hydrochloride salt (170 g, 99 %).

Synthesis of (*S*)-1-benzyl 2-methyl pyrrolidine-1,2-dicarboxylate (**2**):

To a stirring solution of compound 1 (170 g, 947 mmol) in $CH_2Cl_2$ was added $Et_3N$ (398 ml, 2.83 mol). After being stirred for 30 min, Cbz-Cl (1.136 mol) was added to the reaction mixture and stirring was continued for another 12 h at RT. The reaction mixture was washed with water and extracted with $CH_2Cl_2$. The organic layer was dried over anhydrous $Na_2SO_4$ and concentrated under vacuum. The crude was purified by column chromatography to afford compound 2 (230 g, 88 %).
**$^1$H-NMR:** (200 MHz, DMSO-$d_6$): δ 7.42-7.31(m, 5H), 5.09 (m, 2H), 4.32-4.23(m, 1H), 4.08-3.98(m, 2H), 3.50-3.38 (m, 2H), 2.30-2.18 (m, 1H), 1.90-1.81(m, 3H), 1.10-1.04 (t, 3H).
**Mass m/z:** 278 [$M^+$+1].

Synthesis of 1-benzyl 2-ethyl 2-(3-methylbenzyl) pyrrolidine-1, 2-dicarboxylate (**3**):

To a stirring solution of compound **2** (5 g, 0.018 mol) in THF (50 mL) under inert atmosphere was added LiHMDS (1M in THF) (37 mL, 0.036 mol) at -78 °C and stirred for 30 min. To this 3-Methyl benzyl bromide (4 g, 0.021 mol) was added drop wise at -40 °C and it was allowed to warm to RT and stirred for 2 h. The reaction mixture was cooled to 5 °C, quenched with saturated $NH_4Cl$ solution and the aqueous layer was extracted with EtOAc (2 x 100 mL). The combined organic extracts were dried over anhydrous $Na_2SO_4$ and concentrated under reduced pressure. The crude residue obtained was purified by silica gel column chromatography eluting with 20% EtOAc/hexane to afford compound **3** (6 g, 87 %) as liquid.
**$^1$H-NMR:** (500 MHz, DMSO-$d_6$): δ 7.47-7.32 (m, 5H), 7.17-7.12 (m, 1H), 7.05-7.01 (m, 1H), 6.95-6.90 (m, 2H), 5.25-5.00 (m, 2H), 4.16-4.12 (m, 1H), 4.00-3.89 (m, 1H), 3.52 (d, 1H), 3.38-3.33 (m, 1H), 2.99-2.89 (m, 2H), 2.23 (s, 3H), 2.12-1.90 (m, 1H), 1.56-1.51 (m, 1H), 1.05-1.01 (m, 3H), 1.00-0.97 (m, 1H), 0.92-0.89 (m, 1H).
**LCMS (ESI):** 382 [$M^+$+1].
**HPLC (Purity):** 99%.

Synthesis of 1-((benzyloxy) carbonyl)-2-(3-methylbenzyl) pyrrolidine-2-carboxylic acid (**4**):

To a stirring solution of compound **3** (6 g, 0.015 mol) in methanol (30 mL) and water (15 mL) was added 2N aqueous NaOH (2 g, 0.052 mol) and heated to 85 °C for 6 h. The volatiles were evaporated under reduced pressure and obtained residue was diluted with ice cold water (50mL) and washed with ether (50 mL). The aqueous layer was acidified to pH~2 using 2N HCl and extracted with EtOAc (2 x 100mL). The combined organic layers were dried over anhydrous $Na_2SO_4$ and concentrated under reduced pressure to afford compound **4** (3.3 g, 78%) as an off white solid.
**$^1$H-NMR:** (400 MHz, DMSO-$d_6$): δ 12.71 (br s, 1H), 7.40-7.30 (m, 5H), 7.15-7.11 (m, 1H), 7.07-7.00 (m, 1H), 6.97-6.85 (m, 2H), 5.27-5.20 (m, 1H), 5.05-5.00 (m, 1H), 3.65 (d, 1H), 3.35-3.20 (m, 1H), 3.00-2.85 (m, 2H), 2.26 (s, 3H), 2.00-1.97 (m, 2H), 1.65-1.61 (m, 1H), 0.85-0.77 (m, 1H).
**HPLC (Purity):** 99.78%.

Synthesis of benzyl 2-(((2*R*,3*S*)-3-hydroxy-1-methoxy-1-oxobutan-2-yl) carbamoyl)-2-(3-methylbenzyl) pyrrolidine-1-carboxylate (**5**):

To a stirring solution of compound **4** (3.3 g, 9.0 mmol) in $CH_2Cl_2$ (30) were added EDCI. HCl (2.2 g, 11.4 mmol) followed by HOBt (1.82 g, 13.5 mmol) and DIPEA (6.1 mL, 27.0 mmol)
at 0 °C. After being stirred for 10 min, hydrochloride salt of L-threonine methyl ester (1.95 g, 11.2 mmol) was added to the reaction mixture and stirred for another 16 h at RT. After consumption of the starting material (by TLC), the reaction was diluted with EtOAc (150 mL) and washed with water (2 x 30 mL). The organic layer was washed with brine, dried over $Na_2SO_4$, concentrated and purified by silica gel column chromatography eluting with 2% MeOH/$CH_2Cl_2$ to afford compound **5** (3.15 g, 73%).
**$^1$H-NMR:** (500 MHz, DMSO-$d_6$): δ 7.64 (br s, 1H), 7.44-7.31 (m, 5H), 7.12-7.00 (m, 2H), 6.95-6.85 (m, 2H), 5.32-5.25 (m, 1H), 5.05-4.94 (m, 2H), 4.25-4.20 (m, 1H), 4.15-4.08 (m, 1H), 3.66-3.64 (m, 2H), 3.45-3.41 (m, 2H), 3.14-3.09 (m, 1H), 2.89-2.84 (m, 1H), 2.20 (s, 3H), 2.05-2.02 (m, 2H), 1.55-1.51 (m, 1H), 1.09-0.98 (m, 4H).
**LCMS (ESI):** 469 [$M^+$+1].

Synthesis of benzyl 2-(((2*S*,3*R*)-3-acetoxy-1-methoxy-1-oxobutan-2-yl)carbamoyl)-2-(3-methylbenzyl)pyrrolidine-1-carboxylate (**6**):

To a stirring solution of compound **5** (3.1 g, 6.4 mmol) in $CH_2Cl_2$ (30 mL) was added $Et_3N$ (1.4 mL, 5.6 mmol) followed by $Ac_2O$ (0.8 mL, 8.4 mmol) at 0 °C and stirred for 1 h. To this DMAP (0.1 g) was added and stirred at RT for 6 h. The volatiles were evaporated under reduced pressure and obtained crude material was purified by column chromatography eluting with 20% EtOAc/Hexane to afford compound **6** (3.15 g, 92.5%).
**$^1$H-NMR:** (400 MHz, DMSO-$d_6$) (Rotamers): δ 8.25 (br s, 1H), 7.42-7.30 (m, 5H), 7.12-7.00 (m, 2H), 6.98-6.89 (m, 2H), 5.25-5.21 (m, 2H), 5.11-5.09 (m, 1H), 4.67-4.62 (m, 1H), 3.66-3.64 (m, 2H), 3.55-3.46 (m, 3H), 3.15-3.10 (m, 1H), 2.92-2.85 (m, 1H), 2.20 (s, 3H), 2.05-1.95 (m, 5H), 1.56-1.46 (m, 1H), 1.15-1.11 (m, 4H).
**LCMS (ESI):** 511 [$M^+$+1].
**HPLC (Purity):** 98%.

Synthesis of (2*S*,3*R*)-methyl 3-acetoxy-2-(2-(3-methylbenzyl) pyrrolidine-2-carboxamido) butanoate (**7**):

To a stirring solution of compound **6** (3.15 g, 5.97 mmol) in methanol (30 mL) was added wet 10% Pd/C (1.0 g) under $N_2$ atmosphere. The reaction mixture was stirred under $H_2$ atmosphere (balloon pressure) for 4 h. After consumption of the starting material (by TLC), the reaction was filtered through celite pad and the filtrate was concentrated under reduced pressure to afford compound **7** (2.35 g, 99.5%) as syrup.
**$^1$H-NMR:** (400 MHz, DMSO-$d_6$) (Rotamers): δ 8.22 (br s, 1H), 7.19-7.16 (m, 1H), 7.00-6.95 (m, 3H), 5.23-5.19 (m, 1H), 4.50-4.42 (m, 1H), 3.60 (d, 3H), 3.20 (m, 1H), 3.00-2.97 (m, 1H), 2.75-2.68 (m, 2H), 2.28 (s, 3H), 2.05-2.02 (m, 1H), 1.90 (d, 3H), 1.65-1.51 (m, 3H), 1.10 (dd, 3H).
**LCMS m/z:** 377 [$M^+$+1].
**HPLC (Purity):** 98.5%.

Synthesis of (2*S*)-benzyl 2-(2-(((2*S*,3*R*)-3-acetoxy-1-methoxy-1-oxobutan-2-yl) carbamoyl)-2-(3-methylbenzyl) pyrrolidine-1-carbonyl) pyrrolidine-1-carboxylate (**8**):

To a stirring solution of compound **7** (2.35 g, 6.30 mmol) in $CH_2Cl_2$ (60 mL) and water (40 mL) was added $Na_2CO_3$ (2.0 g, 18.8 mmol) and stirred at 0 °C for 5 min. (*S*)-benzyl 2-(chlorocarbonyl) pyrrolidine-1-carboxylate (2.12 g, 7.6 mmol) was added and the reaction mixture stirred at RT for 2 h. The separated organic layer was concentrated under vacuum and obtained crude material was purified by column chromatography eluting with 3% MeOH/$CH_2Cl_2$ to afford compound 8 (2.5 g, 63%). (*S*)-benzyl 2-(chlorocarbonyl) pyrrolidine-1-carboxylate was synthesized as follows. To a solution of (*S*)-1-(benzyloxycarbonyl) pyrrolidine-2-carboxylic acid (20.6 g, 82.8mmol) in $CH_2Cl_2$ (20mL) was added $SOCl_2$ (20.5 g, 172.6 mmol) drop wise at 0 °C and was refluxed for 2 h. The volatiles were removed under reduced pressure to yield (*S*)-benzyl 2-(chlorocarbonyl) pyrrolidine-1-carboxylate.
**$^1$H-NMR:** (400 MHz, DMSO-$d_6$) (Rotamers): δ 8.50 (br s, 1H), 7.36-7.23 (m, 5H), 7.15-6.85 (m, 5H), 5.21-5.05 (m, 2H), 5.04-4.92 (m, 1H), 4.65-4.50 (m, 1H), 4.53-4.45 (m, 1H), 3.65 (s, 3H), 3.54-3.46 (m, 4H), 3.21-3.13 (m, 2H), 2.25-2.16 (m, 4H), 2.05-2.00 (m, 2H), 1.95-1.85 (m, 4H), 1.56-1.51 (m, 2H), 1.15 (dd, 3H).
**LCMS m/z:** 608 [$M^+$+1].
**HPLC (Purity):** 91.3%.

Synthesis of (2*S*,3*R*)-methyl 3-acetoxy-2-(2-(3-methylbenzyl)-1-((*S*)-pyrrolidine-2-carbonyl) pyrrolidine-2-carboxamido) butanoate (**9**):

To a stirring solution of compound **8** (2.5 g, 4.0 mmol) in MeOH (30 mL) was added wet 10% Pd/C (0.5 g) under inert atmosphere and stirred for 4 h under $H_2$ atmosphere (balloon pressure). The reaction mixture was filtered through celite pad and concentrated under reduced pressure to afford compound **9** (1.56 g, 79.5%).
**$^1$H-NMR:** (400 MHz, DMSO-$d_6$) (Rotamers): δ 8.23 (dd, 1H), 7.20-6.85 (m, 5H), 5.20-5.13 (m, 1H), 4.63-4.59 (m, 1H), 3.85-3.81 (m, 1H), 3.65-3.61 (m, 5H), 3.32-3.25 (m, 2H), 3.12-3.05 (m, 3H), 2.75-2.71 (m, 1H), 2.25 (s, 3H), 2.15-2.13 (m, 2H), 2.00 (d, 3H), 1.77 (m, 3H), 1.27 (dd, 4H).
**LCMS m/z:** 474 [$M^+$+1].
**HPLC (Purity):** 87.3%.

Synthesis of Benzyl 2-(tert-butoxycarbonylamino)-3-hydroxybutanoate (**A**):

To a solution of 2-(*tert*-butoxycarbonylamino)-3-hydroxybutanoic acid (Boc-Thr) (50 g, 228.3mmol) in DMF (500 mL) was added $K_2CO_3$ (63 g, 456.6 mmol) and stirred at RT for 15 min. To this Benzyl bromide (46.83 g, 273.9

mmol) was added and stirred at RT for 6 h. The reaction mixture was diluted with water (500mL) and extracted with EtOAc (2 x 750mL). The combined organic layers were washed with brine (50mL), dried over anhydrous $Na_2SO_4$ and concentrated under reduced pressure. The crude material was purified by silica gel column chromatography eluting with 20% EtOAc/hexane to afford benzyl 2-(tert-butoxycarbonylamino)-3-hydroxybutanoate **A** (52 g, 73 %).
**$^1$H-NMR:** (500 MHz, DMSO-$d_6$): δ 7.37-7.30 (m, 5H), 6.60 (d, 1H), 5.18-5.08 (m, 2H), 4.76 (d, 1H), 4.08-4.00 (m, 2H), 1.38 (s, 9H), 1.09 (d, 3H).
**Mass m/z:** 310.0 [$M^+$+1], 210 [$M^+$-De Boc].

Synthesis of benzyl 3-acetoxy-2-(tert-butoxycarbonylamino) butanoate (**B**)::

To a stirring solution of compound **A** (52 g, 168.2 mmol) in $CH_2Cl_2$ (500mL) was added $Ac_2O$ (20.5 g, 201.9mmol), $Et_3N$ (25.4 g, 252.4mmol) and DMAP (3.5 g) and stirred at RT for 2 h. The volatiles were removed under reduced pressure. The residue obtained was diluted with EtOAc (750mL) and washed with cold 0.5 N HCl solution (2 x 200mL). The organic layer was washed with brine, dried over anhydrous $Na_2SO_4$ and concentrated under reduced pressure to afford 3-acetoxy-2-(tert-butoxycarbonylamino) butanoate **B** (52 g, 88%).
**$^1$H-NMR:** (500 MHz, DMSO-$d_6$): δ 7.35-7.34 (m, 5H), 7.27-7.25 (d, 1H), 5.18-5.06 (m, 3H), 4.34-4.32 (m, 1H), 1.90 (s, 3H), 1.39 (s, 9H), 1.16 (d, 3H).
**Mass m/z:** 252 [$M^+$+1-De Boc].

Synthesis of (2*S*,3*R*)-3-acetoxy-2-(tert-butoxycarbonylamino) butanoic acid (C):

To a stirring solution of compound **B** (52 g, 148.1mmol) in MeOH (1 L) was added 10% Pd/C under $N_2$ atmosphere and reaction mixture was stirred under hydrogen atmosphere for 16 h. The reaction mixture was filtered through a pad of celite, obtained filtrate was evaporated under vacuum and the crude residue was triturated with hexane to yield (2*S*,3*R*)-3-acetoxy-2-(tert-butoxycarbonylamino) butanoic acid **C** (35 g, 90%).
**$^1$H-NMR:** (500 MHz, DMSO-$d_6$): δ 12.78 (br s, 1H), 6.94 (d, 1H), 5.16-5.14 (m, 1H), 4.17-4.15 (m, 1H), 1.95 (s, 3H), 1.39 (s, 9H), 1.10 (d, 3H).
**Mass m/z:** 260.0 [M-1].

Synthesis of (2*S*,3*R*)-methyl 3-acetoxy-2-(1-((*S*)-1-((2*S*,3*R*)-2-((*tert*-butoxycarbonyl)amino)-3-hydroxybutanoyl)pyrrolidine-2-carbonyl)-2-(3-methylbenzyl) pyrrolidine-2-carboxamido) butanoate (**10**):

To a stirring solution of compound C (10 g, 0.02 mol) in $CH_2Cl_2$ (100 mL) were added EDCI (5 g, 0.02 mol), HOBt (5 g, 0.04 mol) followed by DIPEA (10.6 mL, 0.06 mol) at 0 °C and stirred for 10 min. To this compound **9** (7 g, 0.025 mol) was added and stirred for 15h. The reaction mixture was extracted with EtOAc (2 x 75ml) and the separated organic layer was washed with water (200mL), followed by brine (200mL), dried over $Na_2SO_4$ and concentrated under reduced pressure. The crude material was purified by column chromatography eluting with 4% MeOH/$CH_2Cl_2$ to afford compound **10** (10 g, 67%) as syrup.
**$^1$H-NMR:** (400 MHz, DMSO-d6) (Rotamers): δ 7.24-6.91 (m, 5H), 5.42-5.01 (m, 2H), 4.73-4.68 (m, 1H), 4.54-4.41 (m, 1H), 3.90-3.85 (m, 1H), 3.67 (s, 3H), 3.65-3.60 (m, 2H), 3.59-3.55 (m, 1H), 3.59-3.55 (m, 1H), 3.30 (s, 3H), 3.12-3.05 (m, 1H), 2.33 (s, 3H), 2.05 (s, 3H), 2.00-1.96 (m, 4H), 1.94-1.85 (m, 4H), 1.43 (s, 9H), 1.25 (d, 3H), 1.15 (d, 3H).
**LCMS (ESI):** *m/z* 675 [$M^+$+1].
**HPLC:** 71%.

Synthesis of *tert*-butyl ((2*S*,3*R*)-1-((2S)-2-(2-(((2*S*,3*R*)-1-amino-3-hydroxy-1-oxobutan-2-yl)carbamoyl)-2-(3-methylbenzyl)pyrrolidine-1-carbonyl)pyrrolidin-1-yl)-3-hydroxy-1-oxobutan-2-yl) carbamate (**11**):

To a stirring solution of compound 10 (10 g, 0.014 mol) in MeOH (20 mL) was added methanolic-$NH_3$ (100 mL) and taken in a sealed tube. The reaction mixture was stirred at RT for 20 h. The reaction mixture was concentrated under reduced pressure. The obtained crude material was purified by silica gel column chromatography eluting with 4% MeOH/$CH_2Cl_2$ to afford compound 11 (8.0 g, 99% yield).
**$^1$H-NMR:** (400 MHz, DMSO-d6) (Rotamers): δ 7.32 (br s, 2H), 7.15-7.10 (m, 4H), 7.05-6.91 (m, 4H), 6.65 (br s, 2H), 4.75-4.59 (m, 2H), 4.24-3.45 (m, 8H), 3.35 (s, 1H), 3.05-3.00 (m, 1H), 2.32 (s, 3H), 2.26-1.85 (m, 6H), 1.75 (s, 6H), 1.60-1.55 (m, 1H), 1.47 (s, 9H), 1.15 (d, 3H), 1.05 (d, 3H).
**LCMS (ESI):** *m/z* 618 [$M^+$+1].

Chiral preparative HPLC of Isomers:

The isomers of compound **11** (8.0 g, 12.9 mmol) were separated by chiral prep HPLC to obtain Compound **11-F1** (0.4 g) and Compound **11-F2** (0.25 g).

Analytical data for Compound 11-F1:

**HPLC:** 91.1%.
**LCMS (ESI):** *m/z* 618 [$M^+$+1].
**Chiral HPLC:** $R_t$ = 9.20 min (Chiralpak IA, 250 x 4.6mm, 5μ; mobile phase (A) 0.1% TFA in *n*-Hexane (B) EtOH (4/1): A: B (80:20); flow Rate: 1.00 mL/min).

Analytical data for Compound 11-F2:

**HPLC:** 84%.
**LCMS (ESI):** *m/z* 618 [$M^+$+1].
**Chiral HPLC:** $R_t$ = 13.39 min (Chiralpak IA, 250 x 4.6mm, 5μ; mobile phase (A) 0.1% TFA in *n*-Hexane (B) EtOH (4/1): A: B (80:20); flow Rate: 1.00 mL/min).

Synthesis of (*R*)-N-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-(3-methylbenzyl) pyrrolidine-2-carboxamide (CM-8A):

To a stirring solution of compound 12-F1 (0.4 g, 0.60 mmol) in $CH_2Cl_2$ (20 mL) was added 2N-HCl in 1,4 Dioxane (2 mL) at 0 °C and stirred at RT for 3 h. The reaction mixture was concentrated under reduced pressure and obtained material was triturated with EtOAc and dried under vacuum to afford CM-8A (0.22 g, 66 %) as hydrochloride salt.
**$^1$H-NMR:** (400 MHz, $D_2O$): δ 7.35 (t, 1H), 7.25 (s, 1H), 7.21 (d, 1H), 7.15 (d, 1H), 4.95-4.90 (m, 1H), 4.43-4.35 (m, 4H), 3.94-3.89 (m, 1H), 3.87-3.78 (m, 2H), 3.63 (d, 1H), 3.49-3.40 (m, 1H), 3.25 (d, 1H), 2.56-2.34 (m, 5H), 2.28-2.20 (m, 4H), 1.75-1.71 (m, 1H), 1.56 (d, 3H), 1.32 (d, 3H), 1.15-1.11 (m, 1H).
**LCMS (ESI)** *m/z:* 518 [$M^+$+1].
**HPLC Purity:** 97%.
**Optical rotation [$\alpha^{25}_D$]:** +18.8 (C=1% in water).

Synthesis of (*S*)-N-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-(3-methylbenzyl) pyrrolidine-2-carboxamide (CM-8B):

To a stirring solution of compound 12-F2 (0.25 g, 0.40 mmol) in $CH_2Cl_2$ (20 mL) was added 2N-HCl in 1,4 Dioxane (2 mL) at 0 °C and stirred at RT for 3 h. The reaction mixture was concentrated under reduced pressure and obtained material was triturated with EtOAc and dried under vacuum to afford CM-8B (130 mg, 62 %) as hydrochloride salt.
**$^1$H-NMR:** (400 MHz, $D_2O$): δ 7.35-7.23 (m, 2H), 7.12-7.00 (m, 2H), 4.45-4.40 (m, 1H), 4.32-4.10 (m, 3H), 3.94-3.75 (m, 3H), 3.60 (d, 1H), 3.25 (d, 1H), 3.14-3.11 (m, 1H), 2.56-2.05 (m, 10H), 1.85-1.79 (m, 2H), 1.45 (d, 3H), 1.25 (d, 3H).
**LCMS (ESI)** *m/z:* 518 [$M^+$+1].
**HPLC Purity:** 92%.
**Optical rotation [$\alpha^{25}_D$]:** -104.9 (C=1% in water).

Example 8 - Synthesis of (*R*)-N-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-(3-fluorobenzyl)pyrrolidine-2-carboxamide (CM-9A) and (*S*)-N-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-(3-fluorobenzyl) pyrrolidine-2-carboxamide (CM-9B):

The following reaction sequence was used (Scheme I) to synthesize (*R*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(3-fluorobenzyl) pyrrolidine-2-carboxamide (CM-9A) and (*S*)-*N*-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(3-fluorobenzyl) pyrrolidine-2-carboxamide (CM-9B):

Scheme I. Synthesis of CM-9A and CM-9B:

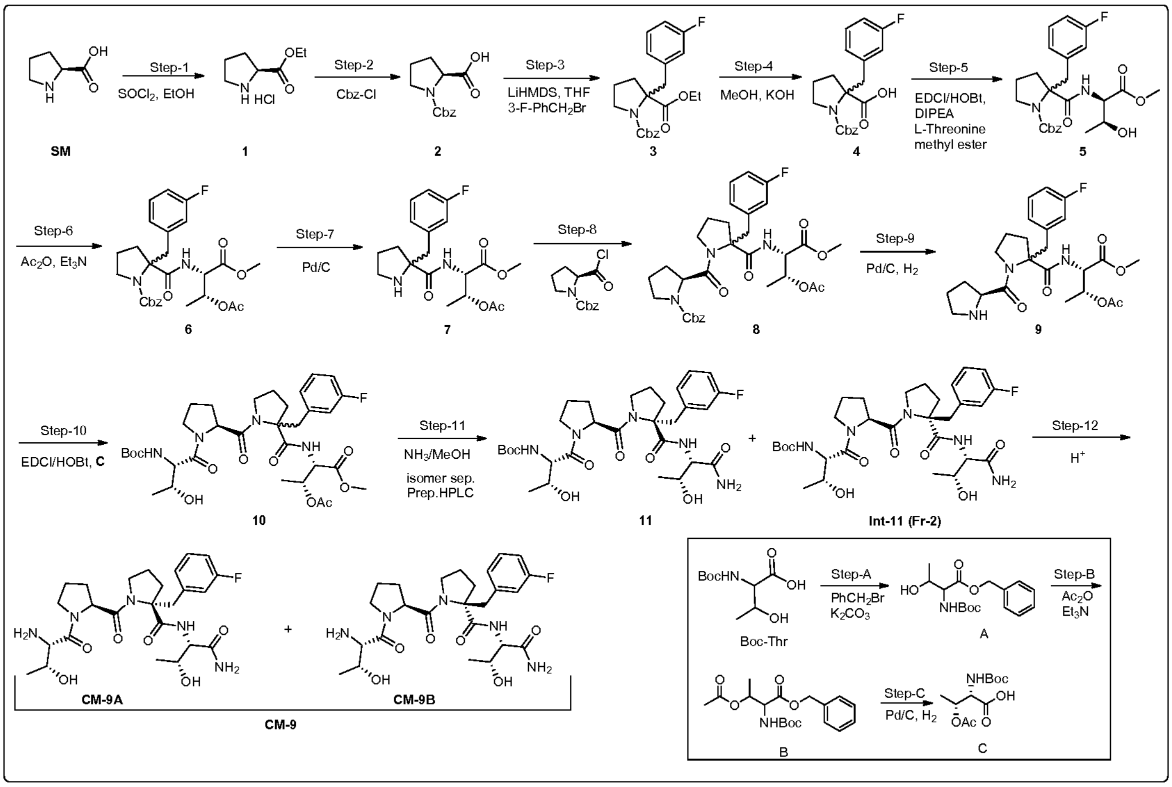


Synthesis of (*S*)-ethyl pyrrolidine-2-carboxylate hydrochloride (**1**):

To a stirring solution of L-proline (**SM**) (110 g, 956.5 mmol) in ethanol was added thionyl chloride (141 ml, 1911.3 mmol) and refluxed for 16 h. The reaction mixture was brought to RT and concentrated under vacuum to afford compound **1** as hydrochloride salt (170 g, 99 %).

Synthesis of (*S*)-1-benzyl 2-methyl pyrrolidine-1,2-dicarboxylate (**2**):

To a stirring solution of compound **1** (170 g, 947 mmol) in $CH_2Cl_2$ was added $Et_3N$ (398 ml, 2.83 mol). After being stirred for 30 min, Cbz-Cl (1.136 mol) was added to the reaction mixture and stirring was continued for another 12 h at RT. The reaction mixture was washed with water and extracted with $CH_2Cl_2$. The organic layer was dried over anhydrous $Na_2SO_4$ and concentrated under vacuum. The crude was purified by column chromatography to afford compound **2** (230 g, 88 %).
**$^1$H-NMR:** (200 MHz, DMSO-*d*$_6$): δ 7.42-7.31(m, 5H), 5.09 (m, 2H), 4.32-4.23(m, 1H), 4.08-3.98(m, 2H), 3.50-3.38 (m, 2H), 2.30-2.18 (m, 1H), 1.90-1.81(m, 3H), 1.10-1.04 (t, 3H).
**Mass m/z:** 278 [M$^+$+1].

Synthesis of 1-benzyl 2-ethyl 2-(3-fluorobenzyl) pyrrolidine-1, 2-dicarboxylate (**3**):

To a stirring solution of compound **2** (10 g, 0.018 mol) in THF (100 mL) under inert atmosphere was added LiHMDS (1M in THF) (76 mL, 0.076 mol) at -78 °C and stirred for 1 h. To this 3-Fluorobenzyl bromide (5.6 mL, 0.045 mol) was added drop wise at -30 °C and stirred for 3 h. The reaction mixture was cooled to 5 °C, quenched with saturated $NH_4Cl$ solution and the aqueous layer was extracted with EtOAc (2 x 100 mL). The combined organic extracts were dried over anhydrous $Na_2SO_4$ and concentrated under reduced pressure. The crude residue obtained was purified by silica gel column chromatography eluting with 12% EtOAc/hexane to afford compound **3** (11 g, 78 %) as liquid.
**$^1$H-NMR:** (400 MHz, DMSO-*d*$_6$): δ 7.64-7.58 (m, 2H), 7.44-7.25 (m, 7H), 5.22-5.12 (m, 2H), 4.16-4.12 (m, 1H), 4.05-3.89 (m, 1H), 3.62 (d, 1H), 3.48-3.39 (m, 1H), 3.16-3.10 (m, 1H), 2.99-2.89 (m, 1H), 2.19-1.99 (m, 2H), 1.63-1.58 (m, 1H), 1.19 (t, 2H), 1.05 (t, 1H), 0.92-0.89 (m, 1H).
**LCMS (ESI):** 372 [M$^+$+1].

Synthesis of 1-((benzyloxy) carbonyl)-2-(3-fluorobenzyl) pyrrolidine-2-carboxylic acid (**4**):

To a stirring solution of compound **3** (11 g, 0.029 mol) in methanol (50 mL) and water (50 mL) was added 2N aqueous NaOH (3.5 g, 0.089 mol) and heated to 60 °C for 3 h. The volatiles were evaporated under reduced pressure and obtained residue was diluted with ice cold water (50mL) and washed with ether (50 mL). The aqueous layer was acidified to pH~2 using 2N HCl and extracted with EtOAc (2 x 100mL). The combined organic layers were dried over anhydrous $Na_2SO_4$ and concentrated under reduced pressure to afford compound **4** (9.5 g, 90%) as liquid.
**$^1$H-NMR:** (400 MHz, DMSO-*d*$_6$): δ 7.59-7.22 (m, 10H), 5.24-5.19 (m, 1H), 5.05-5.00 (m, 1H), 3.65 (d, 1H), 3.35-3.20 (m, 1H), 3.19 (t, 1H), 3.01-2.89 (m, 1H), 2.00-1.97 (m, 2H), 1.65-1.61 (m, 1H), 0.85-0.77 (m, 1H).
**LCMS (ESI):** 358 [$M^+$+1].

Synthesis of benzyl 2-(3-fluorobenzyl)-2-(((2R, 3S)-3-hydroxy-1-methoxy-1-oxobutan-2-yl) carbamoyl) pyrrolidine-1-carboxylate (**5**):

To a stirring solution of compound **4** (9.5 g, 26.6 mmol) in $CH_2Cl_2$ (100 mL) were added EDCI. HCl (6.1 g, 31.9 mmol) followed by HOBt (5.4 g, 39.9 mmol) and DIPEA (14 mL, 79.8 mmol) at 0 °C. After being stirred for 10 min, hydrochloride salt of L-threonine methyl ester (4.97 g, 29.2 mmol) was added to the reaction mixture and stirred for another 16 h at RT. After consumption of the starting material (by TLC), the reaction was diluted with $CH_2Cl_2$ (150 mL), washed with brine, dried over $Na_2SO_4$ and concentrated to afford compound **5** (13 g, crude).
**LCMS (ESI):** 473.7 [$M^+$+1].

Synthesis of benzyl 2-(((2*S*,3*R*)-3-acetoxy-1-methoxy-1-oxobutan-2-yl)carbamoyl)-2-(3-fluorobenzyl) pyrrolidine-1-carboxylate (**6**):

To a stirring solution of compound **5** (13 g, 27.5 mmol) in $CH_2Cl_2$ (100 mL) was added $Et_3N$ (5.8 mL, 41.3 mmol) followed by $Ac_2O$ (3.23 mL, 33.0 mmol) at 0 °C and stirred for 10 min. To this DMAP (1.2 g) was added and stirred at RT for 2 h. The reaction mixture was washed with citric acid and extracted with $CH_2Cl_2$ (2x 150 mL). The separated organic layer was dried over anhydrous $Na_2SO_4$, filtered and concentrated under reduced pressure to get crude; which was purified by column chromatography eluting with 20% EtOAc/Hexane to afford compound **6** (9.5 g, 67%) as light yellow liquid.
**$^1$H-NMR:** (400 MHz, DMSO-*d*$_6$) (Rotamers): δ 7.39-7.23 (m, 6H), 7.05 (t, 1H), 6.95-6.78 (m, 2H), 5.27-5.21 (m, 2H), 5.11-5.09 (m, 1H), 4.67-4.62 (m, 1H), 3.66-3.64 (m, 4H), 3.55-3.46 (m, 2H), 2.92-2.85 (m, 2H), 2.05-1.95 (m, 1H), 1.99 (s, 3H), 1.56-1.46 (m, 1H), 1.15-1.11 (m, 4H).
**LCMS (ESI):** 515 [$M^+$+1].

Synthesis of (2*S*,3*R*)-methyl 3-acetoxy-2-(2-(3-fluorobenzyl)pyrrolidine-2-carboxamido)butanoate (**7**):

To a stirring solution of compound **6** (9.5 g, 18.4 mmol) in methanol (100 mL) was added wet 10% Pd/C (1.2 g) under $N_2$ atmosphere. The reaction mixture was stirred under $H_2$ atmosphere (balloon pressure) for 3 h. After consumption of the starting material (by TLC), the reaction was filtered through celite pad and the filtrate was concentrated under reduced pressure to afford compound **7** (6.3 g, 90%) as liquid.
**$^1$H-NMR:** (400 MHz, DMSO-*d*$_6$) (Rotamers): δ 8.22 (br s, 1H), 7.62-7.56 (m, 2H), 7.45-7.39 (m, 2H), 5.23-5.19 (m, 1H), 4.50-4.42 (m, 1H), 3.60 (d, 3H), 3.05-3.00 (m, 1H), 2.97-2.85 (m, 3H), 2.18-2.09 (m, 1H), 1.95 (d, 3H), 1.65-1.51 (m, 3H), 1.10 (d, 2H), 0.75 (d, 1H).
**LCMS m/z:** 381 [$M^+$+1].

Synthesis of (2*S*)-benzyl 2-(2-(((2*S*,3*R*)-3-acetoxy-1-methoxy-1-oxobutan-2-yl) carbamoyl)-2-(3-fluorobenzyl) pyrrolidine-1-carbonyl) pyrrolidine-1-carboxylate (**8**):

To a stirring solution of compound 7 (6.2 g, 16.3 mmol) in $CH_2Cl_2$ (50 mL) and water (50 mL) was added $Na_2CO_3$ (4.3 g, 40.7 mmol) and stirred at 0 °C for 5 min. (*S*)-benzyl 2-(chlorocarbonyl) pyrrolidine-1-carboxylate was added (17.93 mmol) and the reaction mixture was stirred at RT for 2 h. The reaction mixture was washed with water and extracted with $CH_2Cl_2$ (2x 100 mL). The separated organic layer was concentrated under vacuum and obtained crude material was purified by column chromatography eluting with 30% EtOAc/Hexane to afford compound 8 (7.3 g, 73%) as liquid. (*S*)-benzyl 2-(chlorocarbonyl) pyrrolidine-1-carboxylate was synthesized as follows. To a solution of (*S*)-1-(benzyloxycarbonyl) pyrrolidine-2-carboxylic acid (4.87 g, 19.5 mmol) in $CH_2Cl_2$ (20mL) was added $SOCl_2$ (3.0 mL, 40.7 mmol) drop wise at 0 °C and was refluxed for 2 h. The volatiles were removed under reduced pressure to yield (*S*)-benzyl 2-(chlorocarbonyl) pyrrolidine-1-carboxylate.

**$^1$H-NMR:** (400 MHz, DMSO-$d_6$) (Rotamers): δ 8.50 (br s, 1H), 7.79-7.65 (m, 2H), 7.52-7.37 (m, 7H), 5.42-5.05 (m, 3H), 4.79-4.59 (m, 1H), 4.15 (q, 1H), 3.78 (s, 3H), 3.69-3.50 (m, 2H), 3.21-3.13 (m, 1H), 2.45-2.16 (m, 2H), 2.10 (s, 6H), 2.05-2.00 (m, 2H), 1.95-1.85 (m, 1H), 1.44-1.19 (m, 6H).
**LCMS m/z:** 612 [$M^+$+1], 613 [$M^+$+2].

Synthesis of (2S, 3R)-methyl 3-acetoxy-2-(2-(3-fluorobenzyl)-1-((S)-pyrrolidine-2-carbonyl)pyrrolidine-2-carboxamido)butanoate (9):

To a stirring solution of compound **8** (7.2 g, 11.78 mmol) in MeOH (80 mL) was added wet 10% Pd/C (1.2 g) under inert atmosphere and stirred for 16 h under $H_2$ atmosphere (balloon pressure). The reaction mixture was filtered through celite pad and concentrated under reduced pressure to afford compound **9** (5.2 g, 92%) as liquid.

Synthesis of Benzyl 2-(*tert*-butoxycarbonylamino)-3-hydroxybutanoate (**A**):

To a solution of 2-(tert-butoxycarbonylamino)-3-hydroxybutanoic acid (**Boc-Thr**)(50 g, 228.3mmol) in DMF (500 mL) was added $K_2CO_3$ (63 g, 456.6 mmol) and stirred at RT for 15 min. To this Benzyl bromide (46.83 g, 273.9 mmol) was added and stirred at RT for 6 h. The reaction mixture was diluted with water (500mL) and extracted with EtOAc (2 x 750mL). The combined organic layers were washed with brine (50mL), dried over anhydrous $Na_2SO_4$ and concentrated under reduced pressure. The crude material was purified by silica gel column chromatography eluting with 20% EtOAc/hexane to afford benzyl 2-(tert-butoxycarbonylamino)-3-hydroxybutanoate **A** (52 g, 73 %).
**$^1$H-NMR:** (500 MHz, DMSO-$d_6$): δ 7.37-7.30 (m, 5H), 6.60 (d, 1H), 5.18-5.08 (m, 2H), 4.76 (d, 1H), 4.08-4.00 (m, 2H), 1.38 (s, 9H), 1.09 (d, 3H).
**Mass m/z:** 310.0 [$M^+$+1], 210 [$M^+$-De Boc].

Synthesis of benzyl 3-acetoxy-2-(*tert*-butoxycarbonylamino) butanoate (**B**):

To a stirring solution of compound **A** (52 g, 168.2 mmol) in $CH_2Cl_2$ (500mL) was added $Ac_2O$ (20.5 g, 201.9mmol), $Et_3N$ (25.4 g, 252.4mmol) and DMAP (3.5 g) and stirred at RT for 2 h. The volatiles were removed under reduced pressure. The residue obtained was diluted with EtOAc (750mL) and washed with cold 0.5 N HCl solution (2 x 200mL). The organic layer was washed with brine, dried over anhydrous $Na_2SO_4$ and concentrated under reduced pressure to afford 3-acetoxy-2-(tert-butoxycarbonylamino) butanoate **B** (52 g, 88%).
**$^1$H-NMR:** (500 MHz, DMSO-$d_6$): δ 7.35-7.34 (m, 5H), 7.27-7.25 (d, 1H), 5.18-5.06 (m, 3H), 4.34-4.32 (m, 1H), 1.90 (s, 3H), 1.39 (s, 9H), 1.16 (d, 3H).
**Mass m/z:** 252 [$M^+$+1-De Boc].

Synthesis of (2*S*,3*R*)-3-acetoxy-2-(tert-butoxycarbonylamino) butanoic acid (C):

To a stirring solution of compound **B** (52 g, 148.1mmol) in MeOH (1 L) was added 10% Pd/C under $N_2$ atmosphere and reaction mixture was stirred under hydrogen atmosphere for 16 h. The reaction mixture was filtered through a pad of celite, obtained filtrate was evaporated under vacuum and the crude residue was triturated with hexane to yield (2*S*,3*R*)-3-acetoxy-2-(*tert*-butoxycarbonylamino) butanoic acid **C** (35 g, 90%).
**$^1$H-NMR:** (500 MHz, DMSO-$d_6$): δ 12.78 (br s, 1H), 6.94 (d, 1H), 5.16-5.14 (m, 1H), 4.17-4.15 (m, 1H), 1.95 (s, 3H), 1.39 (s, 9H), 1.10 (d, 3H).
**Mass m/z:** 260.0 [M-1].

Synthesis of (2*S*,3*R*)-methyl 3-acetoxy-2-(1-((*S*)-1-((2*S*,3*R*)-2-((*tert*-butoxycarbonyl) amino)-3-hydroxybutanoyl) pyrrolidine-2-carbonyl)-2-(3-fluorobenzyl) pyrrolidine-2-carboxamido) butanoate (**10**):

To a stirring solution of compound **C** (3.41 g, 13.0 mmol) in $CH_2Cl_2$ (50 mL) were added EDCI (3.12 g, 16.3 mmol), HOBt (2.2 g, 16.3 mmol) followed by DIPEA (5.7 mL, 32.7 mmol) at 0 °C and stirred for 10 min. To this compound **9** (5.2 g, 10.9 mmol) was added and stirred for 16 h. The reaction mixture was extracted with $CH_2Cl_2$ (2 x 75ml) and the separated organic layer was washed with aqueous $NaHCO_3$ (100mL), aqueous citric acid (100 mL) followed by brine (100 mL), dried over $Na_2SO_4$ and concentrated under reduced pressure. The crude material was purified by column chromatography eluting with 2% MeOH/$CH_2Cl_2$ to afford compound **10** (6.0 g, 77%) as liquid.
**$^1$H-NMR:** (400 MHz, DMSO-d6) (Rotamers): δ 7.71-7.55 (m, 3H), 7.34-7.05 (m, 2H), 5.35-5.20 (m, 1H), 5.09-5.01 (m, 1H), 4.78-4.35 (m, 3H), 3.90-3.85 (m, 2H), 3.67 (s, 3H), 3.39-3.25 (m, 1H), 3.25-3.05 (m, 1H), 2.21-1.96 (m, 12H), 1.39 (d, 9H), 1.35-1.25 (m, 8H), 0.85 (d, 1H).
**LCMS (ESI):** *m*/*z* 478.4 [$M^+$+1], 479.4 [$M^+$+2].

Synthesis of tert-butyl ((2*S*,3*R*)-1-((2*S*)-2-(2-(((2*S*,3*R*)-1-amino-3-hydroxy-1-oxobutan-2-yl) carbamoyl)-2-(3-fluorobenzyl) pyrrolidine-1-carbonyl) pyrrolidin-1-yl)-3-hydroxy-1-oxobutan-2-yl) carbamate (**11**):

A solution of compound **10** (3 g, 4.1 mmol) in methanolic ammonia (50 mL) was stirred at RT for 48 h. The reaction mixture was concentrated under vacuum to give crude; which was purified by silica gel column chromatography eluting with 4% MeOH/$CH_2Cl_2$ to afford compound **11** (1.7 g, crude) as white solid.
**$^1$H-NMR:** (400 MHz, DMSO-d6): δ 7.59-7.51 (m, 4H), 7.24 (s, 1H), 7.02 (s, 1H), 6.79-6.60 (m, 2H), 5.09 (d, 1H), 4.77-4.74 (m, 1H), 4.64-4.61 (m, 1H), 4.21 (t, 2H), 3.98 (dd, 1H), 3.85-3.80 (m, 2H), 3.71-3.60 (m, 3H), 3.25-3.19 (m, 2H), 2.21-1.85 (m, 5H), 1.75-1.70 (m, 1H), 1.44 (s, 10H), 1.21 (d, 3H), 1.09 (d, 3H), 0.91-0.85 (m, 1H).
**LCMS (ESI):** *m*/*z* 622 [$M^+$+1].

Chiral preparative HPLC of Isomers:

The isomers of compound **11** (1.7 g) were separated by chiral prep HPLC to obtain Compound **11** (0.4 g) and Compound **11-F2** (0.3 g).

Analytical data for Compound 11:

**$^1$H-NMR:** (400 MHz, DMSO-d6): δ 7.59-7.51 (m, 4H), 7.24 (s, 1H), 7.02 (s, 1H), 6.79-6.60 (m, 2H), 5.09 (d, 1H), 4.77-4.74 (m, 1H), 4.64-4.61 (m, 1H), 4.21 (t, 2H), 3.98 (dd, 1H), 3.85-3.80 (m, 2H), 3.71-3.60 (m, 3H), 3.25-3.19 (m, 2H), 2.21-1.85 (m, 5H), 1.75-1.70 (m, 1H), 1.44 (s, 10H), 1.21 (d, 3H), 1.09 (d, 3H), 0.91-0.85 (m, 1H).
**UPLC:** 87%.
**LCMS (ESI):** *m*/*z* 622 [$M^+$+1].
**Chiral HPLC:** $R_t$ = 10.32 min (Chiralpak IA, 250 x 4.6mm, 5μ; mobile phase (A) 0.1% TFA in *n*-Hexane (B) EtOH (4/1): A: B (80:20); flow Rate: 1.00 mL/min).

Analytical data for Compound 11-F2:

**$^1$H-NMR:** (400 MHz, DMSO-d6): δ 7.59-7.51 (m, 4H), 7.24 (s, 1H), 7.02 (s, 1H), 6.79-6.60 (m, 2H), 5.09 (d, 1H), 4.77-4.74 (m, 1H), 4.64-4.61 (m, 1H), 4.21 (t, 2H), 3.98 (dd, 1H), 3.85-3.80 (m, 2H), 3.71-3.60 (m, 3H), 3.25-3.19 (m, 2H), 2.21-1.85 (m, 5H), 1.75-1.70 (m, 1H), 1.44 (s, 10H), 1.21 (d, 3H), 1.09 (d, 3H), 0.91-0.85 (m, 1H).
**UPLC:** 91%.
**LCMS (ESI):** *m*/*z* 622 [$M^+$+1].
**Chiral HPLC:** $R_t$ = 15.37 min (Chiralpak IA, 250 x 4.6mm, 5μ; mobile phase (A) 0.1% TFA in *n*-Hexane (B) EtOH (4/1): A: B (80:20); flow Rate: 1.00 mL/min).

Synthesis of (*R*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(3-fluorobenzyl) pyrrolidine-2-carboxamide (CM-9A):

To a stirring solution of compound **11-F1** (0.4 g, 0.64 mmol) in $CH_2Cl_2$ (5 mL) was added 4N-HCl in 1,4 Dioxane (2 mL) at 0 °C and stirred at RT for 2 h. The reaction mixture was concentrated under vacuum. Obtained crude material was washed with EtOAc (20 mL) followed by *n*-pentane (20 mL) and dried under vacuum to afford CM-9A (0.2 g, 59 %) as hydrochloride salt.
**$^1$H-NMR:** (400 MHz, $D_2O$): δ 7.89 (d, 2H), 7.57 (d, 2H), 4.95-4.90 (m, 1H), 4.43-4.35 (m, 4H), 3.94-3.79 (m, 5H), 3.35 (q, 2H), 2.56-2.50 (m, 1H), 2.35-2.29 (m, 2H), 2.14-2.05 (m, 3H), 1.81-1.75 (m, 1H), 1.35 (d, 3H), 1.32 (d, 3H), 1.19-1.11 (m, 1H).
**LCMS (ESI)** *m*/*z:* 522 [$M^+$+1], 523 [$M^+$+2].
**UPLC Purity:** 97%.
**Chiral HPLC Purity:** 99%.
**Optical rotation [$\alpha^{20}{}_D$]:** +15.6 (C=1% in water).

Synthesis of (*S*)-N-((2*S*,3*R*)-1-amino-3-hydroxy-1-oxobutan-2-yl)-1-((*S*)-1-((2*S*,3*R*)-2-amino-3-hydroxybutanoyl) pyrrolidine-2-carbonyl)-2-(3-fluorobenzyl) pyrrolidine-2-carboxamide (CM-9B):

To a stirring solution of compound **11-F2** (0.2 g, 0.32 mmol) in $CH_2Cl_2$ (5 mL) was added 4N-HCl in 1,4 Dioxane (2 mL) at 0 °C and stirred at RT for 2 h. The reaction mixture was concentrated under vacuum. Obtained crude material was washed with EtOAc (20 mL) followed by *n*-pentane (20 mL) and dried under vacuum to afford CM-9B (0.1 g, 59 %) as hydrochloride salt.

**$^1$H-NMR:** (400 MHz, $D_2O$): δ 7.89 (d, 2H), 7.57 (d, 2H), 4.95-4.90 (m, 1H), 4.43-4.35 (m, 4H), 3.94-3.79 (m, 5H), 3.35 (q, 2H), 2.56-2.50 (m, 1H), 2.35-2.29 (m, 2H), 2.14-2.05 (m, 3H), 1.81-1.75 (m, 1H), 1.35 (d, 3H), 1.32 (d, 3H), 1.19-1.11 (m, 1H).
**LCMS (ESI)** *m*/*z:* 522 [$M^+$+1], 523 [$M^+$+2].
**UPLC Purity:** 98%.
**Chiral HPLC Purity:** 98%.
**Optical rotation [$\alpha^{20}{}_D$]:** -101.46 (C=1% in water).

Example 10 - Synthesis of *N*-((2*S*,3*R*)-1-amino-3-hydroxy-1-oxobutan-2-yl)-1-((*S*)-1-((*S*)-2-amino-3-hydroxypropanoyl)pyrrolidine-2-carbonyl)-2-benzylpyrrolidine-2-carboxamide (CM-10):

The following reaction sequence was used (Scheme J) to synthesize *N*-((2*S*,3*R*)-1-amino-3-hydroxy-1-oxobutan-2-yl)-1-((*S*)-1-((*S*)-2-amino-3-hydroxypropanoyl)pyrrolidine-2-carbonyl)-2-benzylpyrrolidine-2-carboxamide (CM-10):

Scheme J. Synthesis of CM-10.

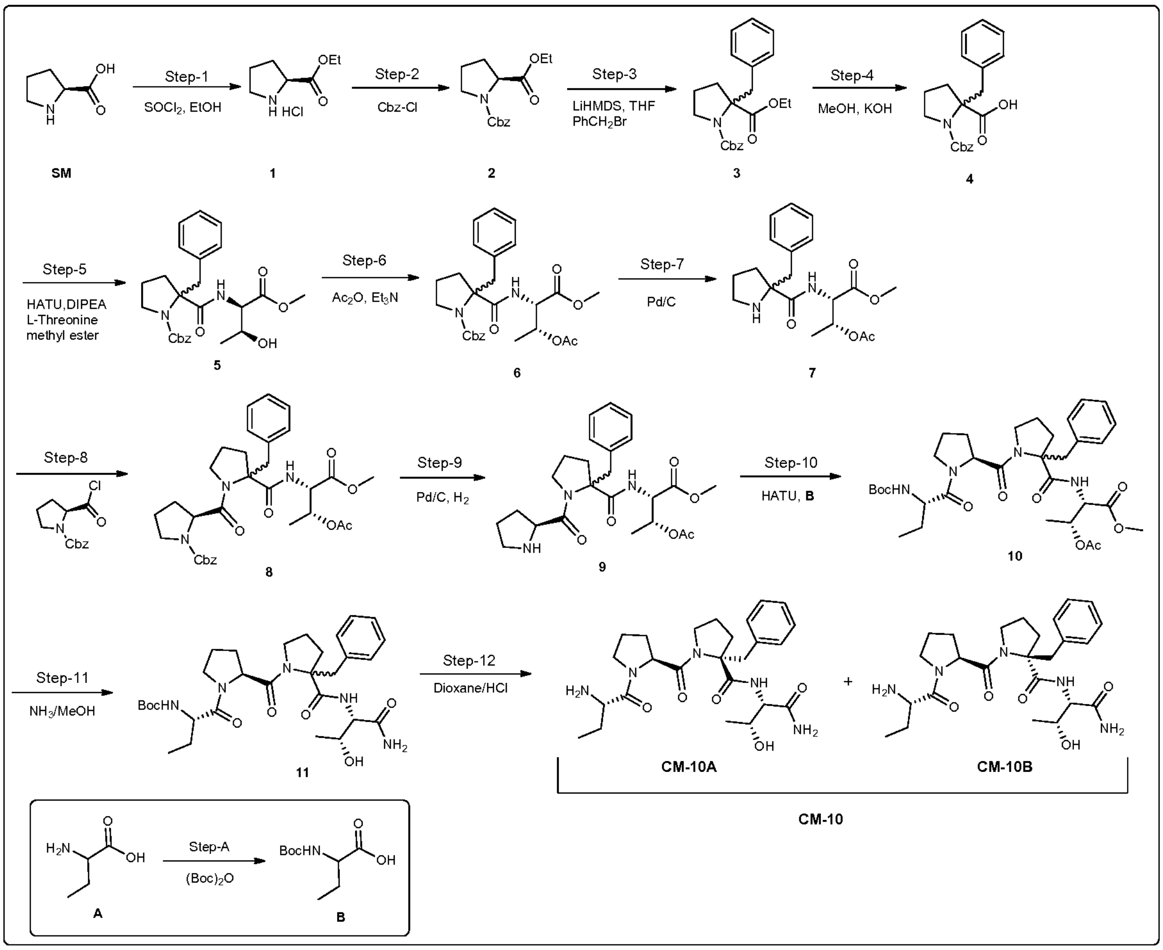


Synthesis of (S)-ethyl pyrrolidine-2-carboxylate hydrochloride (**1**):

To a stirring solution of L-proline (SM) (110 g, 956.5 mmol) in ethanol was added thionyl chloride (141 ml, 1911.3 mmol) and refluxed for 16 h. The reaction mixture was brought to RT and concentrated under vacuum to afford compound **1** as hydrochloride salt (170 g, 99 %).

Synthesis of (S)-1-benzyl 2-methyl pyrrolidine-1,2-dicarboxylate (**2**):

To a stirring solution of compound 1 (170 g, 947 mmol) in $CH_2Cl_2$ was added TEA (398 ml, 2.83 mol) and after 30 min Cbz-Cl (1.136 mol) was added. The reaction mixture was stirred at RT for 12 h. The reaction mixture was washed

with water and extracted with $CH_2Cl_2$. The organic layer was dried over anhydrous $Na_2SO_4$ and concentrated under vacuum. Obtained crude material was purified by column chromatography to afford compound **2** (230 g, 88 %).
**$^1$H-NMR:** (200 MHz, DMSO-$d_6$): δ 7.42-7.31(m, 5H), 5.09 (m, 2H), 4.32-4.23(m, 1H), 4.08-3.98(m, 2H), 3.50-3.38 (m, 2H), 2.30-2.18 (m, 1H), 1.90-1.81(m, 3H), 1.10-1.04 (t, 3H).
**Mass m/z:** 278 [$M^+$+1],

Synthesis of 1-benzyl 2-ethyl 2-benzylpyrrolidine-1,2-dicarboxylate (**3**):

To a solution of (*S*)-1-benzyl 2-ethyl pyrrolidine-1,2-dicarboxylate **2** (87 g, 314 mmol) in THF (800mL) under inert atmosphere was added LiHMDS (1M in THF) (351 mL, 351 mmol) at -25 °C and stirred for 2 h. Benzyl bromide (45 mL, 376 mmol) was added drop wise at -25 °C to the reaction mixture. It was allowed to warm to RT and stirred for 2h. The reaction mixture was cooled to 5 °C, quenched with saturated $NH_4Cl$ solution and the aqueous layer was extracted with EtOAc (2 x 200mL). The combined organic extracts were dried over anhydrous $Na_2SO_4$ and concentrated under reduced pressure. The crude residue obtained was purified by silica gel column chromatography eluting with 5% EtOAc/hexane to afford compound **3** (80 g, 69 %) as liquid.
**$^1$H-NMR:** (200 MHz, DMSO-$d_6$): δ 7.47-7.32 (m, 5H), 7.27-7.16 (m, 3H), 7.07-7.04 (m, 2H), 5.29-5.06 (m, 2H), 4.16-3.89 (m, 2H), 3.57-3.33 (m, 2H), 3.02-2.78 (m, 2H), 2.13-1.89 (m, 2H), 1.56-1.51 (m, 1H), 1.21-1.04 (m, 3H), 0.93-0.79 (m, 1H).
**Mass m/z:** 368.2 [$M^+$+1].

Synthesis of 1-benzyl 2-ethyl 2-benzylpyrrolidine-1,2-dicarboxylate (**4**):

To a stirring solution of compound **3** (125 g, 340 mmol) in $CH_3OH$ (700 mL) was added 2N aqueous NaOH (680 mmol) and heated up to 100 °C for 16 h. The volatiles were evaporated under reduced pressure. The residue obtained was diluted with ice cold water (50 mL) and washed with ether (50 mL). The aqueous layer was acidified to pH~2 using 2N HCl and extracted with EtOAc (2 x 100 mL). The combined organic layers were dried over anhydrous $Na_2SO_4$ and concentrated under reduced pressure to afford compound **4** (90 g, 78%) as an off white solid.
**$^1$H-NMR:** (200 MHz, DMSO-$d_6$): δ 12.71 (br s, 1H), 7.40-7.30 (m, 5H), 7.25-7.19 (m, 3H), 7.07-7.00 (m, 2H), 5.27-5.02 (m, 2H), 3.59-3.32 (m, 2H), 3.02-2.83 (m, 2H), 2.13-1.91 (m, 2H), 1.58-1.49 (m, 1H), 0.90-0.77 (m, 1H).
**Mass m/z:** 340.1 [$M^+$+1].

Synthesis of Benzyl 2-benzyl-2-((2*S*,3*R*)-3-hydroxy-1-methoxy-1-oxobutan-2-ylcarbamoyl) pyrrolidine-1-carboxylate (**5**):

To a stirring suspension of compound **4** (50 g, 147.9 mmol), L-threonine methyl ester (25 g, 147.9 mmol) in DCM (500 mL) was added HATU (56.2 g, 147.9mmol) and DIPEA (64 mL, 36.98mmol) at 5 °C. The reaction mixture was stirred at RT for 3 h. It was diluted with EtOAc (150mL) and washed with water (2 x 30mL). The organic layer was washed with brine, dried over $Na_2SO_4$, concentrated and purified by silica gel column chromatography 50% EtOAc/Hexane as eluent to afford compound **5** (49.6 g, 74%).
**$^1$H-NMR:** (200 MHz, DMSO-$d_6$): δ 7.62-7.59 (m, 1H), 7.44-7.31 (m, 5H), 7.21-7.18 (m, 3H), 7.06-6.99 (m, 2H), 5.25-5.24 (m, 1H), 5.12-4.94 (m, 2H), 4.30 (s, 1H), 4.15-4.08 (m, 1H), 3.66-3.64 (m, 3H), 3.63-3.49 (m, 2H), 3.14 (s, 1H), 2.89 (s, 1H), 2.09-2.02 (m, 2H), 1.56-1.51 (m, 1H), 1.09-0.98 (m, 4H).
**Mass m/z:** 455.1 [$M^+$+1], 477.3 [M+Na].

Synthesis of Benzyl 2-((2*S*,3*R*)-3-acetoxy-1-methoxy-1-oxobutan-2-ylcarbamoyl)-2-benzylpyrrolidine-1-carboxylate (**6**):

To a stirring solution of compound **5** (49 g, 107.9 mmol) in THF (30mL) were added $Et_3N$ (22.7 mL, 161.8 mmol) and $Ac_2O$ (13.2 g, 129.5 mmol) at RT. The reaction mixture was stirred at RT for 2 h. The volatiles were evaporated under reduced pressure and the residue obtained was diluted with $CH_2Cl_2$ and washed with dil.HCl. The combined organic extracts were dried over $Na_2SO_4$ and concentrated under reduced pressure. The crude residue was purified by column chromatography using 30% EtOAc/Hexane as eluent to afford compound **6** (42 g, 80%).
**$^1$H-NMR:** (500 MHz, DMSO-$d_6$) (Rotamers): δ 8.15-7.71 (m, 1H), 7.42-7.04 (m, 10H), 5.30-5.19 (m, 2H), 5.11-5.09 (m, 1H), 4.99-4.93 (m, 1H), 4.67-4.62 (m, 1H), 3.66-3.64 (m, 3H), 3.55-3.46 (m, 2H), 3.38-3.35 (m, 1H), 2.88-2.69 (m, 1H), 2.17-2.00 (m, 2H), 1.98-1.92 (m, 3H), 1.56-1.46 (m, 1H), 1.23-1.17 (m, 3H), 1.02-0.86 (m, 1H).
**LCMS m/z:** 497.4 [$M^+$+1],

Synthesis of (2*S*,3*R*)-methyl-3-acetoxy-2-(2-benzylpyrrolidine-2-carboxamido)-butanoate (**7**):

To a stirring solution of compound **6** (50 g, 100.9 mmol) in methanol (1.5 L) was added 10% Pd/C under $N_2$ atmosphere. The reaction mixture was stirred under $H_2$ atmosphere (balloon pressure) for 4 h. After consumption of the

starting material (by TLC), the reaction was filtered through celite pad and the filtrate was concentrated under reduced pressure to afford compound **7** (28 g, 77%).

**$^1$H-NMR:** (500 MHz, DMSO-$d_6$) (Rotamers): δ 8.22-8.17 (m, 1H), 7.24-7.16 (m, 5H), 5.17 (t, 1H), 4.48-4.42 (m, 1H), 3.60-3.54 (s, 3H), 3.20 (t, 1H), 3.06-2.97 (m, 1H), 2.82-2.68 (m, 3H), 2.08-2.02 (m, 1H), 1.89 (s, 3H), 1.72-1.51 (m, 3H), 1.10 (2d, 3H).

**LCMS m/z:** 363 [$M^+$+1], 385 [M+Na].

Synthesis of (*S*)-benzyl 2-(2-((2*S*,3*R*)-3-acetoxy-1-methoxy-1-oxobutan-2-ylcarbamoyl)-2-benzylpyrrolidine-1-carbonyl)-pyrrolidine-1-carboxylate (**8**):

To a stirring mixture of compound **7** (25 g, 69.1 mmol) and $Na_2CO_3$ (18.3 g, 172.6mmol) in $CH_2Cl_2$:$H_2O$ (200mL, 1:1) was added a solution of (*S*)-benzyl 2-(chlorocarbonyl) pyrrolidine-1-carboxylate in $CH_2Cl_2$ and the reaction mixture was stirred at RT for 2 h. The volatiles were evaporated under reduced pressure. The residue was diluted with $CH_2Cl_2$ (100 mL), filtered and the filtrate was concentrated under vacuum. The crude residue was purified by column chromatography using 60% EtOAc/hexane as eluent to afford compound 8 (30 g, 73%). (*S*)-benzyl 2-(chlorocarbonyl) pyrrolidine-1-carboxylate was prepared as follows. To a solution of (*S*)-1-(benzyloxycarbonyl)pyrrolidine-2-carboxylic acid (20.6 g, 82.8mmol) in $CH_2Cl_2$ (20mL) was added $SOCl_2$ (20.5 g, 172.6 mmol) drop wise at 0 °C and the resultant solution refluxed for 2 h. The volatiles were removed under reduced pressure to yield (*S*)-benzyl 2-(chlorocarbonyl) pyrrolidine-1-carboxylate.

**$^1$H-NMR:** (500 MHz, DMSO-$d_6$) (Rotamers): δ 7.36-7.23 (m, 8H), 7.15-7.12 (m, 3H), 5.21-5.15 (m, 2H), 5.04-4.92 (m, 1H), 4.57-4.50 (m, 2H), 3.88 (d, 1H), 3.65 (s, 3H), 3.54-3.46 (m, 3H), 3.21-3.13 (m, 1H), 3.02-2.90 (m, 2H), 2.19-2.02 (m, 4H), 1.97 (s, 3H), 1.89 (s, 1H), 1.77-1.65 (m, 1H), 1.17 (s, 2H), 1.06 (s, 2H).

**Mass m/z:** 594.1 [$M^+$+1].

Synthesis of (2*S*,3*R*)-methyl 3-acetoxy-2-2-benzyl-1-((*S*)-pyrrolidine-2-carbonyl) pyrrolidine-2-carboxamido)butanoate (**9**):

To a stirring solution of compound **8** (30 g, 50.05 mmol) in MeOH (300 mL) was added 10% Pd/C was added under inert atmosphere and stirred for 12 h under $H_2$ atmosphere (balloon pressure). The reaction mixture was filtered through celite pad and concentrated under reduced pressure. The obtained residue was triturated with ether (10 mL) to afford compound **9** (21 g, 90%) as solid.

**$^1$H-NMR:** (500 MHz, $CDCl_3$) (Rotamers): δ 7.88-7.87 (m, 1H), 7.30-7.26 (m, 2H), 7.24-7.21 (m, 1H), 7.13-7.12 (d, 2H), 5.44-5.43 (m, 1H), 4.76-4.74 (m, 1H), 3.94-3.92 (m, 1H), 3.84-3.81 (m, 1H), 3.75 (s, 3H), 3.50 (m, 1H), 3.26-3.12 (m, 3H), 2.90-2.88 (m, 1H), 2.23-2.15 (m, 4H), 2.04 (s, 3H), 1.87-1.77 (m, 5H), 1.27-1.24 (m, 3H).

**Mass m/z:** 460(M+1).

Synthesis of 2-((tert-butoxycarbonyl)amino)butanoic acid (**B**):

To a stirring solution of 2-aminobutanoic acid **A** (50 g, 0.47 mol) in THF: $H_2O$ (1 L, 1: 1) was added $NaHCO_3$ (80 g, 0.95 mol) and the reaction mixture was cooled to 0 °C. To this Boc-anhydride (124 g, 0.57 mol) was added drop wise and allowed at RT for 16 h. After consumption of the starting material (by TLC), the reaction was washed with ether and aqueous layer was neutralized with aqueous HCl (pH~4-5) and extracted with EtOAc (2x 250 mL). Combined organic extracts were dried over anhydrous $Na_2SO_4$, filtered and concentrated under vacuum to afford compound **B** (40 g, 41%) as colorless thick syrup.

Synthesis of (2S,3R)-methyl 3-acetoxy-2-(2-benzyl-1-((S)-1-((S)-2-((tert-butoxycarbonyl)amino)-3-hydroxypropanoyl)pyrrolidine-2-carbonyl)pyrrolidine-2-carboxamido)butanoate (**10**):

To a stirring mixture of compound **9** (0.6 g, 1.30 mmol) and compound **B** (267 mg, 1.30 mmol) in $CH_2Cl_2$ (10 mL) were added EDCI (248 mg, 1.30 mmol), HOBt (351 mg, 2.60 mmol) followed by DIPEA (167 mg, 1.30 mmol) under argon atmosphere. The resulting reaction mixture was stirred for 3 h and further stirred at RT for 16 h. After consumption of the starting material (by TLC), the reaction was diluted with water and extracted with $CH_2Cl_2$ (2x 150 mL). The separated organic layer was washed with brine, dried over anhydrous $Na_2SO_4$ and concentrated under reduced pressure. The crude material was purified by silica gel column chromatography eluting with 50% EtOAc/Hexane to afford compound **10** (0.5 g, 59%) as an off-white solid.

**$^1$H-NMR:** (400 MHz, DMSO-$d_6$) (Rotamers): δ 7.30-7.24 (m, 6H), 7.12-7.10 (m, 1H), 5.29-5.20 (m, 1H), 4.79-4.60 (m, 2H), 4.55 (t, 1H), 4.38-4.30 (m, 2H), 3.79-3.60 (m, 11H), 2.21-1.87 (m, 11H), 1.36 (s, 9H), 1.25-1.15 (m, 4H).

**LCMS (ESI):** 647(M+1).

Synthesis of *tert*-butyl ((2S)-1-((2S)-2-(2-(((2S,3R)-1-amino-3-hydroxy-1-oxobutan-2-yl)carbamoyl)-2-benzylpyrrolidine-1-carbonyl)pyrrolidin-1-yl)-3-hydroxy-1-oxopropan-2-yl) carbamate (**11**):

A solution of compound 10 (0.5 g, 0.77 mmol) in methanolic-$NH_3$ (5 mL) was taken in a sealed tube and stirred for 24 h. After consumption of the starting material (by TLC), the reaction mixture was concentrated under reduced pressure to give crude. Obtained material was purified by silica gel column chromatography eluting with 10% MeOH/$CH_2Cl_2$ to afford compound 11 (275 mg, 60%) as an off-white solid.
**$^1$H-NMR:** (400 MHz, DMSO-$d_6$) (Rotamers): δ 7.35-7.20 (m, 6H), 7.15 (d, 1H), 7.01-6.97 (m, 2H), 6.77 (d, 1H), 5.09 (d, 1H), 4.75 (t, 2H), 4.39-4.35 (m, 1H), 4.24-4.20 (m, 1H), 3.92 (dd, 1H), 3.74-3.49 (m, 6H), 3.21-3.11 (m, 2H), 2.23-1.91 (m, 6H), 1.80-1.56 (m, 2H), 1.41 (s, 9H), 1.09 (d, 3H), 0.89-0.80 (m, 1H).
**LCMS (ESI):** 590(M+1).
**Mass (m/z):** 590(M+1).

Synthesis of *N*-((2*S*,3*R*)-1-amino-3-hydroxy-1-oxobutan-2-yl)-1-((*S*)-1-((*S*)-2-amino-3-hydroxypropanoyl)pyrrolidine-2-carbonyl)-2-benzylpyrrolidine-2-carboxamide (**CM-10**):

To a stirring solution of compound 11 (175 mg, 0.29 mmol) in dioxane (3 mL) was added 4N-HCl in 1,4 Dioxane (2 mL) under $N_2$ atmosphere. The reaction mixture was stirred at RT for 2 h. The reaction mixture was concentrated under reduced pressure, diluted with diethyl ether and further stirred for another 15 min. Ether layer was decanted and dried under vacuum to afford CM-10 (108 mg, 74 %) as an off-white solid.
**$^1$H-NMR:** (400 MHz, DMSO-$d_6$) (Rotamers): δ 8.29 (br s, 3H), 7.39-7.20 (m, 7H), 7.07-7.01 (m, 1H), 5.45-5.40 (m, 1H), 5.06 (d, 1H), 4.79-4.76 (m, 1H), 4.27-4.20 (m, 2H), 4.01 (d, 1H), 3.91-3.85 (m, 4H), 3.69-3.55 (m, 5H), 3.29-3.21 (m, 2H), 2.10-1.87 (m, 6H), 1.08 (s, 3H).
**LCMS (ESI):** 490(M+2).

Example 11 - Synthesis of (*R*)-*N*-((2*S*,3*R*)-1-amino-3-hydroxy-1-oxobutan-2-yl)-1-((S)-1-((*2S,3R*)-2-amino-3-hydroxybutanoyl)pyrrolidine-2-carbonyl)-2-(4-methoxybenzyl)pyrrolidine-2-carboxamide (CM-11A) and (*S*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(4-methoxybenzyl)pyrrolidine-2-carboxamide (CM-11B):

The following reaction sequence was used (Scheme K) to synthesize (R)-N-((*2S*,*3R*)-1-amino-3-hydroxy-1-oxobutan-2-yl)-1-((S)-1-((*2S,3R*)-2-amino-3-hydroxybutanoyl)pyrrolidine-2-carbonyl)-2-(4-methoxybenzyl)pyrrolidine-2-carboxamide (CM-11A) and (*S*)-*N*-((*2S*,*3R*)-1-amino-3-hydroxy-1-oxobutan-2-yl)-1-((*S*)-1-((*2S*,*3R*)-2-amino-3-hydroxybutanoyl)pyrrolidine-2-carbonyl)-2-(4-methoxybenzyl)pyrrolidine-2-carboxamide (CM-11B):

Scheme K. Synthesis of CM-11A and CM-11B.

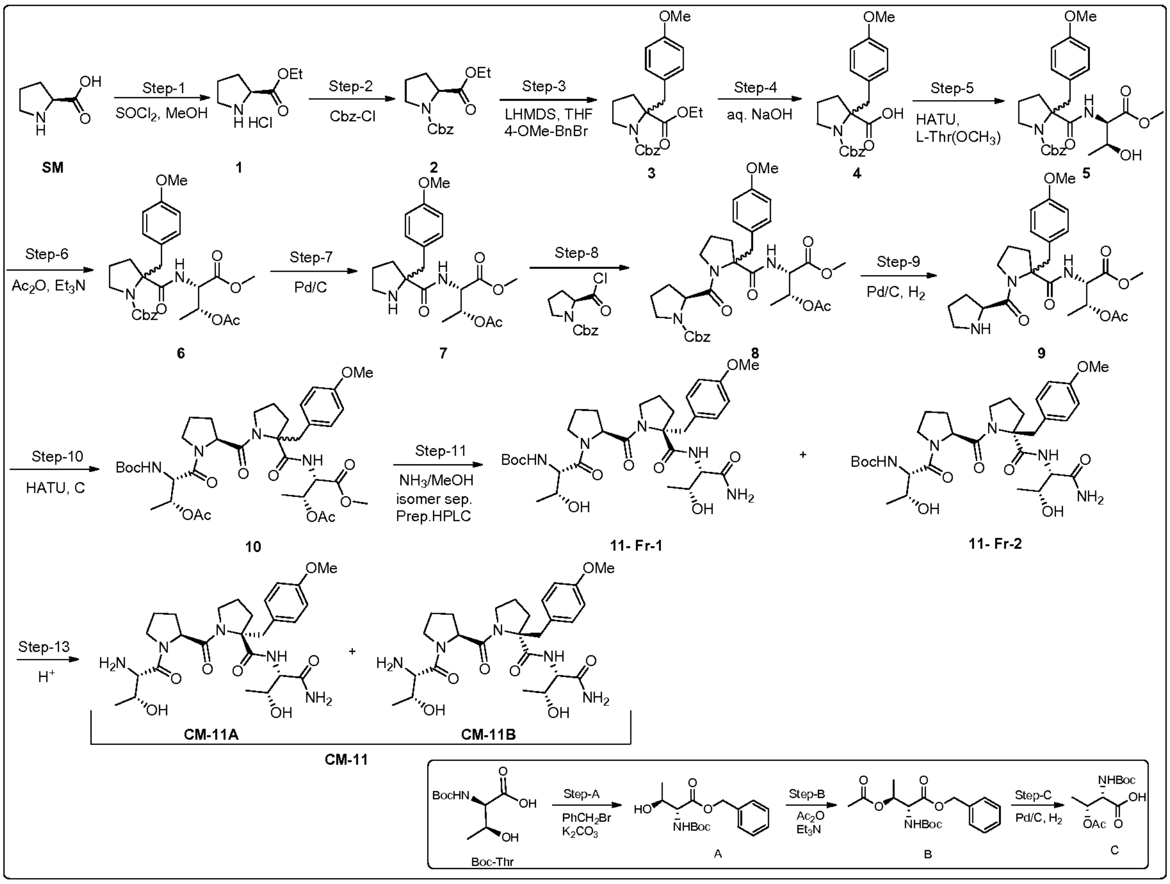


Synthesis of (*S*)-ethyl pyrrolidine-2-carboxylate hydrochloride (**1**):

To a solution of L-proline (SM) (110 g, 956.5 mmol) in ethanol was added thionyl chloride (141 ml, 1911.3 mmol) and refluxed for 16 h. The reaction mixture was brought to RT and concentrated under reduced pressure to afford compound 1 as hydrochloride salt (170 g, 99 %). This material was directly used for the next step without further purification.

Synthesis of (*S*)-1-benzyl 2-ethyl pyrrolidine-1,2-dicarboxylate (**2**):

To a solution of **1** (170 g, 947 mmol) in DCM was added TEA (398 ml, 2.83 mol) and after 30 min Cbz-Cl (1.136 mol) was added. The reaction mixture was stirred at RT for 12 h. The reaction mixture was washed with water and extracted with DCM. The organic layer was dried over anhydrous $Na_2SO_4$ and conc. under reduced pressure. The crude was purified by column chromatography to afford compound 2 (230 g, 88 %).
**$^1$H-NMR:** (200 MHz, DMSO-$d_6$): δ 7.42-7.31(m, 5H), 5.09 (m, 2H), 4.32-4.23(m, 1H), 4.08-3.98(m, 2H), 3.50-3.38 (m, 2H), 2.30-2.18 (m, 1H), 1.90-1.81(m, 3H), 1.10-1.04 (t, 3H).
**Mass m/z:** 278 [$M^+$+1].

Synthesis of 1-benzyl 2-ethyl 2-(4-methoxybenzyl) pyrrolidine-1, 2-dicarboxylate (**3**):

To a solution of compound **2** (10 g, 0.038 mol) in THF (100 mL) under inert atmosphere was added LiHMDS (1M in THF) (76 mL, 0.076 mol) at -25 °C and stirred for 2 h. 4-Methoxy benzyl bromide (11.5 g, 0.057 mol) was added drop wise at -25 °C, allowed to warm to RT and stirred for 2h. The reaction mixture was cooled to 5 °C, quenched with saturated $NH_4Cl$ solution and the aqueous layer was extracted with EtOAc (2 x 200mL). The combined organic extracts were dried over anhydrous $Na_2SO_4$ and concentrated under reduced pressure. The crude residue obtained was purified by silica gel column chromatography eluting with 10% EtOAc/hexane to afford compound **3** (11 g, 75%).
**$^1$H-NMR:** (400 MHz, $CDCl_3$): δ 7.44-7.29 (m, 6H), 7.01 (t, 2H), 6.81-6.79 (m, 2H), 5.32 (t, 1H), 5.15 (d, 1H), 3.82 (d, 5H), 3.55 (d, 1H), 3.50 (s, 2H), 3.02 (d, 2H), 2.14-1.99 (m, 2H), 1.65-1.62 (m, 1H), 1.09-0.95 (m, 1H).

**LCMS (m/z)**:384.3 [$M^+$+1].

Synthesis of 1-((benzyloxy)carbonyl)-2-(4-methoxybenzyl)pyrrolidine-2-carboxylic acid (**4**):

To a stirring solution of compound **3** (1 g, 0.026 mol) in $CH_3OH$ (50 mL) was added 2N aqueous NaOH (25 mL) and heated up to 100 °C for 16 h. The volatiles were evaporated under reduced pressure. The residue obtained was diluted with ice cold water (50mL) and washed with ether (50mL). The aqueous layer was acidified to pH~2 using 2N HCl and extracted with EtOAc (2 x 100mL). The combined organic layers were dried over anhydrous $Na_2SO_4$ and concentrated under reduced pressure to afford compound **4** (10 g, 94%).
**$^1$H-NMR:** (400 MHz, DMSO-*d*$_6$): δ 12.55 (br s, 1H), 7.49-7.65 (m, 5H), 6.99 (t, 2H), 6.78-6.75 (m, 2H), 5.25 (d, 1H), 5.11 (t, 1H), 3.72 (s, 3H), 3.51-3.42 (m, 3H), 2.99-2.95 (m, 2H), 2.15-2.09 (m, 2H), 1.62-1.57 (m, 1H), 0.98-0.94 (m, 1H).
**LCMS (m/z):**368.3 [$M^+$-1]
**HPLC (purity):** 95%.

Synthesis of benzyl 2-(((*2R,3S*)-3-hydroxy-1-methoxy-1-oxobutan-2-yl) carbamoyl)-2-(4-methoxybenzyl) pyrrolidine-1-carboxylate (**5**):

To a suspension of compound **4** (10 g, 0.027 mol), L-threonine methyl ester (4.5 g, 0.027 mol) in DCM (100 mL) was added HATU (10.2 g, 0.027 mol) and DIPEA (11.8 mL, 0.067 mol) at 5 °C. The reaction mixture was stirred at RT for 2 h. The reaction mixture was diluted with DCM (150mL), washed with water (2 x 30mL), brine and dried over $Na_2SO_4$, concentrated and purified by silica gel column chromatography 50% EtOAc/Hexane as eluent to yield compound **5** (7.8 g, 59%).
**$^1$H-NMR:** (400 MHz, DMSO-*d*$_6$): δ 7.47-7.42 (m, 6H), 6.99-6.95 (m, 2H), 6.84-6.77 (m, 2H), 5.35-5.24 (m, 1H), 5.12-4.95 (m, 2H), 4.35-4.32 (m, 1H), 4.19-4.15 (m, 1H), 3.74 (s, 3H), 3.65 (d, 2H), 3.59-3.50 (m, 2H), 3.14-3.05 (m, 1H), 3.92-3.85 (m, 1H), 2.71 (s, 5H), 2.19-2.05 (m, 2H), 1.68-1.60 (m, 1H), 1.14-1.05 (m, 3H).
**LCMS (m/z):**4858.3 [$M^+$+1].

Synthesis of benzyl 2-(((*2S,3R*)-3-acetoxy-1-methoxy-1-oxobutan-2-yl)-carbamoyl)-2-(4-methoxybenzyl) pyrrolidine-1-carboxylate (**6**):

To a stirring solution of compound **5** (7.8 g, 0.016 mol) in DCM (100mL) was added $Et_3N$ (3.36 mL, 0.024 mol) and $Ac_2O$ (1.9 g, 0.019 mol) at RT and stirred for 2 h. The volatiles were evaporated under reduced pressure and the residue obtained was diluted with $CH_2Cl_2$ and washed with dil.HCl. The combined organic extracts were dried over $Na_2SO_4$ and concentrated under reduced pressure. The crude residue was purified by column chromatography using 30% EtOAc/n-hexane as eluent to afford compound **6** (7.6 g, 90%).
**$^1$H-NMR:** (400 MHz, DMSO-*d*$_6$): δ 7.45-7.40 (m, 5H), 6.99-6.95 (m, 2H), 6.82-6.75 (m, 2H), 5.31-5.25 (m, 2H), 4.62-4.55 (m, 1H), 3.74 (s, 3H), 3.65 (t, 2H), 3.09-3.01 (m, 2H), 3.19-3.10 (m, 1H), 2.92-2.87 (m, 1H), 2.21-1.99 (m, 6H), 1.59-1.45 (m, 1H), 1.25-1.15 (m, 4H).
**LCMS (m/z):**527.4 [$M^+$+1].

Synthesis of (*2S,3R*)-methyl 3-acetoxy-2-(2-benzylpyrrolidine-2-carboxamido) butanoate (**7**):

To a stirring solution of compound **6** (7.6 g, 0.014 mol) in methanol (100 mL) was added 10% Pd/C (1.5 g) and the reaction mixture was stirred under $H_2$ atmosphere (balloon pressure) for 12 h. The reaction mixture was filtered through celite pad and the filtrate was concentrated under reduced pressure to afford compound **7** (4.5 g, 80%).
**$^1$H-NMR:** (400 MHz, DMSO-*d*$_6$): δ 8.25 (br s, 1H), 6.81 (t, 2H), 5.2-5.17 (m, 1H), 4.52 (dd, 1H), 3.73 (d, 3H), 3.64 (d, 3H), 3.05 (br s, 1H), 2.86-2.80 (m, 2H), 2.12 (br s, 1H), 1.95 (d, 3H), 1.71-1.62 (br m, 3H), 1.12 (d, 1H), 0.82 (d, 1H).
**LCMS (m/z):**393.3 [$M^+$+1].

Synthesis of (*2S*)-benzyl 2-(2-(((*2S,3R*)-3-acetoxy-1-methoxy-1-oxobutan-2-yl) carbamoyl)-2-(4-methoxybenzyl)pyrrolidine-1-carbonyl)pyrrolidine-1-carboxylate (**8**):

To a stirring solution of compound 7 (4.5 g, 11.4 mmol) and $Na_2CO_3$ (3.0 g, 28.0 mmol) in $CH_2Cl_2$ (54 mL) and $H_2O$ (36 mL) was added a solution of acid chloride (3.4 g, 13.0 mmol) in $CH_2Cl_2$ and the reaction mixture was stirred at RT for 2 h. The volatiles were evaporated under reduced pressure. The residue was diluted with $CH_2Cl_2$ (100 mL), filtered and the filtrate was concentrated under reduced pressure. The crude residue was purified by column chromatography using 30% EtOAc/hexane as eluent to afford compound 8 (7.0 g, 96%).
**$^1$H-NMR:** (400 MHz, DMSO-*d*$_6$): δ 7.45-7.36 (m, 4H), 7.22-6.69 (m, 3H), 5.27-5.05 (m, 2H), 4.69-4.55 (m, 1H), 3.75-3.62

(m, 4H), 3.55-3.47 (m, 3H), 3.25-3.01 (m, 1H), 2.31-1.85 (m, 6H), 1.75-1.45 (m, 1H), 1.22-1.09 (m, 3H).

Synthesis of (*2S*,*3R*)-methyl 3-acetoxy-2-(2-(4-methoxybenzyl)-1-((*S*)-pyrrolidine-2-carbonyl)pyrrolidine-2-carboxamido)butanoate (**9**):

To a solution of compound **8** (7.0 g, 11.2 mmol) in MeOH (100 mL) was added 10% Pd/C (2.0 g) and stirred under $H_2$ atmosphere (balloon pressure) for 12 h. The reaction mixture was filtered through celite pad and concentrated under reduced pressure to afford compound **9** (5.4 g, 99%).
**$^1$H-NMR:** (400 MHz, DMSO-d6): δ 9.25 (br m, 1H), 8.01-7.85 (m, 1H), 7.11-6.99 (m, 2H), 6.85 (d, 2H), 5.29-5.22 (m, 1H), 4.65-4.60 (m, 1H), 4.44-4.22 (m, 1H), 3.72 (s, 3H), 3.55 (s, 3H), 3.49 (d, 3H), 3.39-3.30 (m, 5H), 3.15-3.09 (m, 3H), 2.25-2.20 (m, 1H), 2.02 (s, 9H), 1.65-1.60 (m, 2H), 1.21 (t, 3H).
**LCMS (m/z):**490.4 [$M^+$+1].

Synthesis of (*2S,3R*)-methyl 3-acetoxy-2-(1-((*S*)-1-((*2S*,*3R*)-3-acetoxy-2-((*tert*-butoxycarbonyl)amino)butanoyl)pyrrolidine-2-carbonyl)-2-(4-methoxybenzyl)pyrrolidine-2-carboxamido)butanoate (**10**):

To a solution of compound **9** (5.4 g, 81.1 mmol) in $CH_2Cl_2$ (100 mL) was under inert atmosphere were added EDCI. HCl (3.1 g, 16.0 mmol), HOBt (2.2 g, 16.0 mmol) followed by DIPEA (4.2 g, 33.0 mmol) and compound **C** (3.4 g, 13.0 mmol) at 0 °C. The reaction mixture was stirred for RT for 12 h. The reaction mixture was extracted with EtOAc (2 X 75ml) and the separated organic layer was washed with water (200mL), followed by brine (200mL), dried over $Na_2SO_4$ and concentrated under reduced pressure. The crude material was purified by column chromatography to afford compound **10** (5.8 g, 72%).
**$^1$H-NMR:** (400 MHz, DMSO-d6): δ 7.22-7.01 (m, 2H), 6.85-6.82 (m, 1H), 5.25-5.05 (m, 1H), 4.65-4.20 (m, 2H), 3.85-3.42 (m, 7H), 3.22-3.12 (m, 1H), 2.22-1.85 (m, 8H), 1.52 (d, 6H), 1.29-1.10 (m, 5H).

Synthesis of *tert*-butyl ((*2S,3R*)-1-((*2S*)-2-(2-(((*2S,3R*)-1-amino-3-hydroxy-1-oxobutan-2-yl)carbamoyl)-2-(4-methoxybenzyl)-pyrrolidine-1-carbonyl)pyrrolidin-1-yl)-3-hydroxy-1-oxobutan-2-yl)carbamate (**11**):

A solution of compound **10** (4 g, 5.45 mmol) in methanolic-$NH_3$ (40 mL) was stirred at RT for 36 h. The reaction mixture was concentrated under reduced pressure. The obtained crude material was purified by silica gel column chromatography followed by prep-purification to afford compound 11-Fr-1 (200 mg) and compound-11-Fr-2 (150 mg).

Synthesis of (R)-N-((2S,3R)-1-amino-3-hydroxy-1-oxobutan-2-yl)-1-((S)-1-((2S,3R)-2-amino-3-hydroxybutanoyl)pyrrolidine-2-carbonyl)-2-(4-methoxybenzyl)pyrrolidine-2-carboxamide (**CM-11A**):

To a solution of compound 11-Fr-1 (0.2 g, 0.3 mmol) in DCM (3 mL) was added 4N-HCl in 1,4 dioxane (2 mL) and stirred at RT for 2 h. The reaction mixture was concentrated under reduced pressure to afford **CM-11A** (0.12 g, 66%) as hydrochloride salt.
**$^1$H-NMR:** (400 MHz, $D_2O$): δ 7.32 (d, 2H), 7.05 (d, 2H), 4.99-4.95 (m, 2H), 4.42 (d, 1H), 4.37 (s, 3H), 3.81 (s, 3H), 3.74 (d, 1H), 3.72-3.65 (m, 1H), 3.62 (d, 1H), 3.35-3.30 (m, 1H), 3.29-3.25 (m, 1H), 3.22 (d, 1H), 2.51-2.50 (m, 1H), 2.35-2.30 (m, 1H), 2.19-2.05 (m, 4H), 1.75-1.72 (m, 1H), 1.55 (d, 3H), 1.25 (d, 3H), 1.21-1.15 (m, 1H).
**LCMS (m/z):**534.5 [$M^+$+1].
**HPLC Purity:** 97%.
**Optical rotation [$\alpha^{20}_D$]:** 34.33 (C=1% in water).

Synthesis of (*S*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(4-methoxybenzyl)pyrrolidine-2-carboxamide (**CM-11B**):

To a solution of compound 11-Fr-2 (0.15 g, 0.2 mmol) in DCM (3 mL) was added 4N-HCl in 1,4 Dioxan (2 mL) and stirred at RT for 2 h. The reaction mixture was concentrated under reduced pressure to afford CM-11B (90 mg, 66%) as hydrochloride salt.
**$^1$H-NMR:** (400 MHz, $D_2O$): δ 7.32 (d, 2H), 7.05 (d, 2H), 4.99-4.95 (m, 2H), 4.42 (d, 1H), 4.37 (s, 3H), 3.81 (s, 3H), 3.74 (d, 1H), 3.72-3.65 (m, 1H), 3.62 (d, 1H), 3.35-3.30 (m, 1H), 3.29-3.25 (m, 1H), 3.22 (d, 1H), 2.51-2.50 (m, 1H), 2.35-2.30 (m, 1H), 2.19-2.05 (m, 4H), 1.75-1.72 (m, 1H), 1.55 (d, 3H), 1.25 (d, 3H), 1.21-1.15 (m, 1H).
**LCMS (m/z):**534.5 [$M^+$+1].
**HPLC Purity:** 92%.
**Optical rotation [$\alpha^{20}_D$]:** -108.63 (C=1% in water).

Synthesis of Benzyl 2-(*tert*-butoxycarbonylamino)-3-hydroxybutanoate (**A**):

To a solution of 2-(*tert*-butoxycarbonylamino)-3-hydroxybutanoic acid (**Boc-Thr**)(50 g, 228.3mmol) in DMF (500 mL) was added $K_2CO_3$ (63 g, 456.6 mmol) and stirred at RT for 15 min. Benzyl bromide (46.83 g, 273.9 mmol) was added and stirred at RT for 6 h. The reaction mixture was diluted with water (500mL) and extracted with EtOAc (2 x 750mL). The combined organic layers were washed with brine (50mL), dried over anhydrous $Na_2SO_4$ and concentrated under reduced pressure. The crude material was purified by silica gel column chromatography using 20% EtOAc/hexane as eluent to afford benzyl 2-(tert-butoxycarbonylamino)-3-hydroxybutanoate **A** (52 g, 73 %).
**$^1$H-NMR:** (500 MHz, DMSO-$d_6$): δ 7.37-7.30 (m, 5H), 6.60 (d, *J* = 8.5 Hz, 1H), 5.18-5.08 (m, 2H), 4.76 (d, *J* = 7 Hz, 1H), 4.08-4.00 (m, 2H), 1.38 (s, 9H), 1.09 (d, *J* = 6.0 Hz, 3H).
**Mass m/z:** 310.0 [$M^+$+1], 210 [$M^+$-De Boc].

Synthesis of benzyl 3-acetoxy-2-(*tert*-butoxycarbonylamino)butanoate (**B**):

To a stirred solution of benzyl 2-(tert-butoxycarbonylamino)-3-hydroxybutanoate A (52 g, 168.2 mmol) in DCM (500mL) was added $Ac_2O$ (20.5 g, 201.9mmol), $Et_3N$ (25.4 g, 252.4mmol) and DMAP (3.5 g) and stirred at RT for 2 h. The volatiles were removed under reduced pressure. The residue obtained was diluted with EtOAc (750mL) and washed with cold 0.5 N HCl solution (2 x 200mL). The organic layer was washed with brine, dried over anhydrous $Na_2SO_4$ and concentrated under reduced pressure to afford 3-acetoxy-2-(tert-butoxycarbonylamino) butanoate B (52 g, 88%).
**$^1$H-NMR:** (500 MHz, DMSO-$d_6$): δ 7.35-7.34 (m, 5H), 7.27-7.25 (d, *J* = 8.5 Hz, 1H), 5.18-5.06 (m, 3H), 4.34-4.32 (m, 1H), 1.90 (s, 3H), 1.39 (s, 9H), 1.16 (d, *J* = 3 Hz, 3H).
**Mass m/z:** 252 [$M^+$+1-De Boc].

Synthesis of (2*S*,3*R*)-3-acetoxy-2-(tert-butoxycarbonylamino)butanoic acid (**C**):

Benzyl-3-acetoxy-2-(tert-butoxycarbonylamino)butanoate B (52 g, 148.1 mmol) was dissolved in MeOH (1 L), 10% Pd/C was added and reaction mixture was stirred under hydrogen atmosphere for 16 h. The reaction mixture was filtered over celite, solvent was evaporated under reduced pressure and the crude residue was triturated with hexane to yield (2*S*,3*R*)-3-acetoxy-2-(*tert*-butoxycarbonylamino)butanoic acid C (35 g, 90%).
**$^1$H-NMR:** (500 MHz, DMSO-$d_6$): δ 12.78 (br s, 1H), 6.94 (d, *J* = 9.5 Hz, 1H), 5.16-5.14 (m, 1H), 4.17-4.15 (m, 1H), 1.95 (s, 3H), 1.39 (s, 9H), 1.10 (d, *J* = 6.0 Hz, 3H).
**Mass m/z:** 260.0 [M-1].

Example 12 - Synthesis of (*R*)-*N*-((*2S,3R*)-1-amino-3-hydroxy-1-oxobutan-2-yl)-1-((S)-1-((*2S,3R*)-2-amino-3-hydroxybutanoyl)pyrrolidine-2-carbonyl)-2-(3,5-dibromobenzyl)pyrrolidine-2-carboxamide (CM-12A) and (*S*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3,5-dibromobenzyl)pyrrolidine-2-carboxamide (CM-12B):

The following reaction sequence was used (Scheme L) to synthesize (*R*)-*N*-((*2S,3R*)-1-amino-3-hydroxy-1-oxobutan-2-yl)-1-((S)-1-((*2S,3R*)-2-amino-3-hydroxybutanoyl)pyrrolidine-2-carbonyl)-2-(3,5-dibromobenzyl)pyrrolidine-2-carboxamide (CM-12A) and (*S*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3,5-dibromobenzyl)pyrrolidine-2-carboxamide (CM-12B):

Scheme L. Synthesis of CM-12A and CM-12B:

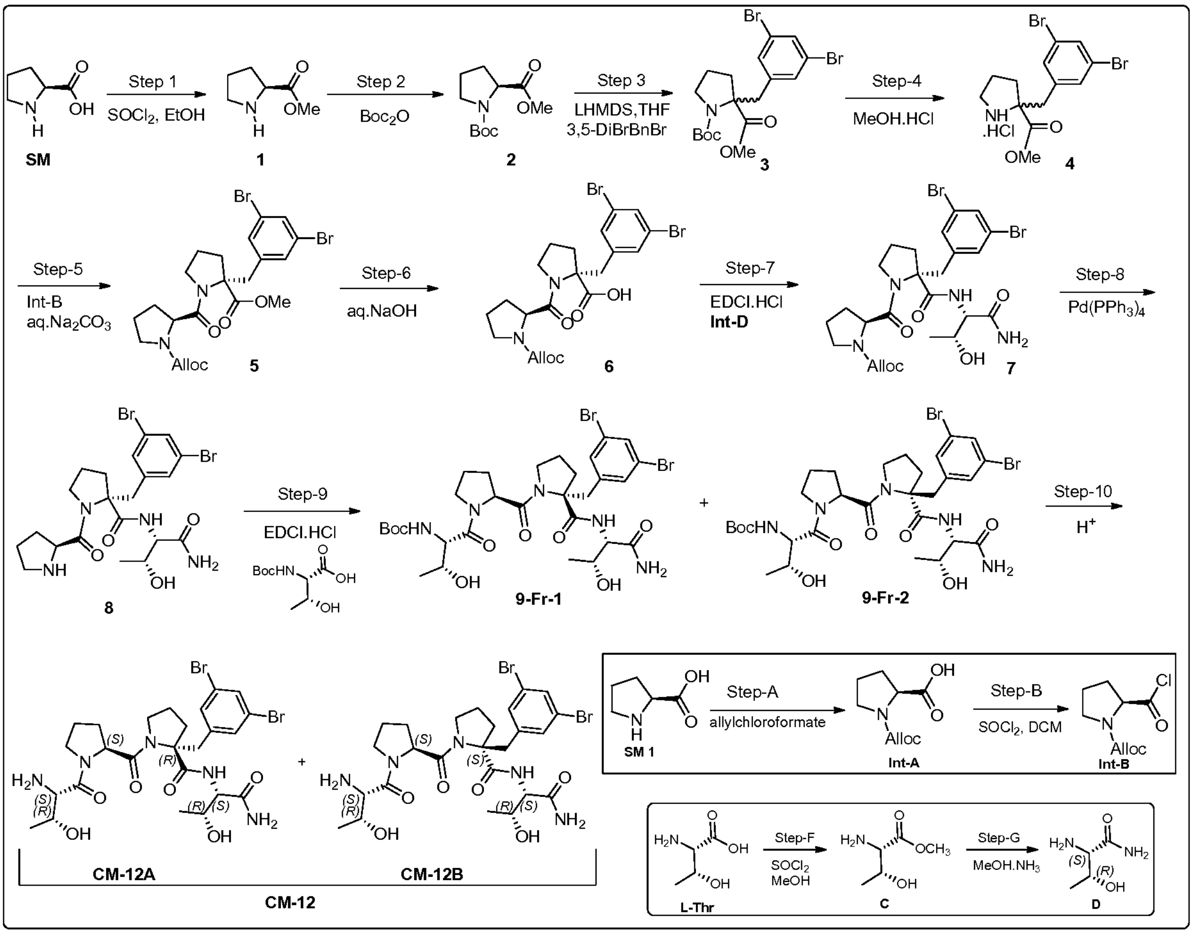


Synthesis of (*S*)-ethyl pyrrolidine-2-carboxylate (**1**):

To a stirring solution of pyrrolidine-2-carboxylic acid (**SM**) (100 g, 0.87 mol) in methanol (800 mL) was slowly added thionyl chloride (76.9 mL, 1.04 mol) at 0 °C. The reaction mixture was heated to reflux for 12 h. After consumption of the starting material (by TLC), the reaction was concentrated under reduced pressure to afford compound **1** (143.9 g, HCl salt).
**$^1$H-NMR:** (400 MHz, $CDCl_3$) (Rotamers): δ 3.89 (s, 3H), 3.68-3.62 (m, 2H), 3.59-3.47 (m, 2H), 2.49-2.37 (m, 1H), 2.27-2.05 (m, 3H).
**LCMS (m/z):** 166 [$M^+$+1].

Synthesis of 1-*tert*-butyl 2-methyl pyrrolidine-1,2-dicarboxylate (**2**):

To a stirring solution of compound **1** (35 g, 0.22 mol) in $CH_2Cl_2$ (175 mL) were added $Et_3N$ (90 mL, 0.65 mol) followed by Boc-anhydride (56.9 mL, 0.26 mol) at 0 °C. The reaction mixture was stirred at RT for 16 h. After consumption of the starting material (by TLC), the reaction was diluted with water (100 mL) and extracted with $CH_2Cl_2$ (2x 100 mL). The organic layer was washed with water, brine, dried over $Na_2SO_4$ and concentrated. Obtained crude material was purified by silica gel column chromatography eluting with 30% EtOAc/*n*-hexane to afford compound **2** (41 g, 95%).
**$^1$H-NMR:** (400 MHz, $CDCl_3$) (Rotamers): δ 4.25-4.21 (m, 1H), 3.75 (s, 3H), 3.57-3.26 (m, 2H), 2.29-2.10 (m, 1H), 1.99-1.75 (m, 3H), 1.45 (s, 9H).
**LCMS (m/z):** 130 [($M^+$+1)-Boc].

Synthesis of 1-tert-butyl 2-methyl 2-(3,5-dibromobenzyl)pyrrolidine-1,2-dicarboxylate (**3**):

To a stirring solution of compound **2** (10 g, 0.043 mol) in THF (50 mL) was added LiHMDS (65.5 mL, 0.065 mol) at -25 °C and stirred for 2 h. To this 3,5-dibromo benzyl bromide (17.1 g, 0.052 mol) was added drop wise at -25

°C and stirred for 2 h at RT. After consumption of the starting material (by TLC), the reaction was quenched with $NH_4Cl$ at 0 °C. The separated organic layer was washed with water and extracted with EtOAc. The separated organic layer was dried over $Na_2SO_4$ and concentrated under reduced pressure to obtain crude product, which was purified by silica gel column chromatography to afford compound **3** (10 g, 50%).
**$^1$H-NMR:** (500 MHz, DMSO-$d_6$): δ 7.72 (s, 1H), 7.41 (d, 2H), 3.69 (d, 3H), 3.37-3.35 (m, 2H), 3.07-3.01 (m, 1H), 2.89-2.81 (m, 1H), 2.08-2.01 (m, 2H), 1.68-1.62 (m, 1H), 1.45 (s, 9H), 1.19-1.12 (m, 1H).
**LCMS (m/z):** 419.1 [($M^+$+1)-Boc].

Synthesis of methyl 2-(3,5-dibromobenzyl)pyrrolidine-2-carboxylate (**4**):

To a stirring solution of compound **3** (10 g, 0.016 mol) in methanol (20 mL) was added MeOH.HCl (80 mL) under $N_2$ atmosphere and stirred for 8 h at RT. The volatiles were evaporated under reduced pressure to afford compound **4** (8 g, 93%) as white color solid.
**$^1$H-NMR:** (400 MHz, DMSO-$d_6$): δ 10.95 (br s, 1H), 9.45 (br s, 1H), 7.81 (s, 1H), 7.65 (s, 2H), 3.75 (s, 3H), 3.55 (d, 1H), 3.39 (d, 1H), 3.22 (br s, 1H), 2.43 (t, 1H), 2.15-2.10 (m, 2H), 1.85-1.81 (m, 1H).
**LCMS (m/z):** 378 [($M^+$-HCl].

Synthesis of (*S*)-1-((Allyloxy)carbonyl)pyrrolidine-2-carboxylic acid (**A**):

To a stirring solution of (*S*)-pyrrolidine-2-carboxylic acid (**SM**) (5 g, 0.043 mol) in 1,4-dioxane (50 mL) and water (100 mL) was added $Na_2CO_3$ (11.5 g, 0.11 mol) followed by allyl Chloroformate (6.2 g, 0.052 mol) at 0 °C and stirred for 12 h. The reaction mixture was extracted with EtOAc (2x 75 mL). The separated organic layer was dried over anhydrous $Na_2SO_4$, filtered and concentrated under reduced pressure to give crude; which was purified silica gel column chromatography to afford compound **A** (3.28 g, 37%).

Synthesis of (*S*)-allyl 2-((*R*)-2-(3,5-dibromobenzyl)-2-(methoxycarbonyl) pyrrolidine-1-carbonyl) pyrrolidine-1-carboxylate (**5**):

To a stirring solution of compound **4** (8 g, 0.019 mol) and $Na_2CO_3$ (5.1 g, 0.048 mol) in DCM:$H_2O$ (150 mL, 3:2) was added a solution of acid chloride **B** and stirred for 2 h at RT. The aqueous layer was extracted with $CH_2Cl_2$ (2x 50 mL). The separated organic layer was washed with water (50 mL), dried over anhydrous $Na_2SO_4$, filtered and concentrated under reduced pressure. Obtained crude material was purified by silica gel column chromatography to afford compound 5 (8 g, 74%). Acid chloride **B** was prepared as follows. To a solution of compound **A** (4.6 g, 0.023 mol) in DCM was added $SOCl_2$ (5.7 g, 0.048 mol) drop wise at 0 °C and the resultant solution refluxed for 2 h; the solvent from the reaction was removed under reduced pressure.
**$^1$H-NMR:** (400 MHz, DMSO-$d_6$): δ 7.75-7.42 (m, 2H), 7.37 (s, 1H), 5.95 (br s, 1H), 9.45 (br s, 1H), 7.81 (s, 1H), 7.65 (s, 2H), 3.75 (s, 3H), 3.55 (d, 1H), 3.39 (d, 1H), 3.22 (br s, 1H), 2.43 (t, 1H), 2.15-2.10 (m, 2H), 1.85-1.81 (m, 1H).
**LCMS (m/z):** 559.3 [($M^+$+1].

Synthesis of (*R*)-1-((*S*)-1-((allyloxy)carbonyl)pyrrolidine-2-carbonyl)-2-(3,5-dibromobenzyl)pyrrolidine-2-carboxylic acid (**6**):

To a stirring solution of compound 5 (8 g, 0.014 mol) in MeOH (50 mL) was added 2N NaOH solution (10 mL) and heated to reflux for 5 h. The volatiles were evaporated under reduced pressure and obtained residue was diluted with water and washed with ether. The aqueous layer was acidified to pH~2 using 2N HCl solutions and extracted with EtOAc. The separated organic layer was dried over anhydrous $Na_2SO_4$, filtered and concentrated under reduced pressure to afford compound 6 (7 g, 90%). This material was directly used for the next step without further purification.
**LCMS (m/z):** 543.3 [($M^+$-1].

Synthesis of (*2S,3R*)-methyl 2-amino-3-hydroxybutanoate (**C**):

To a stirring solution of (*2S*,*3R*)-2-amino-3-hydroxybutanoic acid (200 g, 1.68 mol) in methanol (1.2 L) was added $SOCl_2$ (244 mL, 3.36 mol) drop wise at 0 °C and stirred for 1 h. The resulting reaction mixture was refluxed for 24 h. After consumption of the starting material (by TLC), the reaction mixture was warmed to RT and concentrated under reduced pressure and washed with *n*-hexane (2x 50 mL). The residue was dissolved in EtOH (1 L) and neutralized with $Et_3N$ (471 mL, 3.36 mol) and again stirred for 2 h. The precipitated solid was filtered off; obtained filtrate was concentrated under reduced pressure to afford compound **C** (195 g, 80%).
**$^1$H-NMR:** (400 MHz, DMSO-$d_6$): δ 8.51 (br s, 3H), 4.13-4.10 (m, 1H), 3.91 (br s, 1H), 1.20 (d, 3H).

**LCMS (m/z):** 134.1 [$M^+$+1].

Synthesis of (*2S,3R*)-2-amino-3-hydroxybutanamide (**D**):

A solution of compound **C** (190 g, 1.35 mol) in IPA (2 L) was taken in autoclave and purged $NH_3$ gas (7-8 kg) and stirred at 35 °C for 24 h. Then removed $NH_3$ gas and reaction mixture was concentrated under reduced pressure and added $CH_2Cl_2$ and filtered. Obtained solid was refluxed in EtOH for 1 h at 78 °C. The reaction mass was filtered in heating condition and *n*-hexane was added to the filtrate and again stirred for another 4 h. Obtained precipitated solid was filtered and dried under reduced pressure to afford compound **D** (160 g, 47%).
**$^1$H-NMR:** (500 MHz, DMSO-*d*$_6$): δ 7.38 (br s, 1H), 7.02 (br s, 1H), 4.66 (br s, 1H), 3.77-3.70 (m, 1H), 2.93 (d, 1H), 2.72 (br m, 1H), 1.05 (d, 3H).
**LCMS (m/z):** 119.1 [$M^+$+1].
**UPLC (ELSD purity):** 99.9%.

Synthesis of (*S*)-allyl 2-((*R*)-2-(((*2S,3R*)-1-amino-3-hydroxy-1-oxobutan-2-yl)carbamoyl)-2-(3,5-dibromobenzyl)pyrrolidine-1-carbonyl)pyrrolidine-1-carboxylate (**7**):

To a stirring solution of compound **6** (5 g, 9.19 mmol) in $CH_2Cl_2$ (100 mL) was added HOBt (1.8 g, 13.7 mmol), EDCI.HCl (2.6 g, 13.7 mmol) followed by DIPEA (3.5 g, 27.0 mmol) and compound **D** (1.3 g, 11.0 mmol) at 0 °C. The reaction mixture was stirred at RT for 12 h. After consumption of the starting material (by TLC), the reaction was quenched with water and extracted with $CH_2Cl_2$ (2x 100 mL). The organic layer was dried over anhydrous $Na_2SO_4$ and concentrated under reduced pressure. The crude was purified by column chromatography to afford compound 7 (5 g, 84%).

Synthesis of (*R*)-*N*-((*2S*,*3R*)-1-amino-3-hydroxy-1-oxobutan-2-yl)-2-(3,5-dibromobenzyl)-1-((*S*)-pyrrolidine-2-carbonyl)pyrrolidine-2-carboxamide (**8**):

To a stirring solution of compound **7** (1 g, 1.35 mmol) in THF (50 mL) was added DABCO (1.3 g, 12.4 mmol) followed by $Pd(PPh_3)_4$ (358 mg, 0.3 mmol) at RT and stirred for 30 min. The reaction mixture was diluted with EtOAc (75 mL) and saturated $NaHCO_3$ solution (75 mL). The separated organic layer was dried over anhydrous $Na_2SO_4$ and concentrated under reduced pressure to afford compound **8** (560 mg, 64%). This material was directly used for the next step without further purification.

Synthesis of *tert*-butyl ((2*S*,3*R*)-1-((*S*)-2-((*R*)-2-(((2*S*,3*R*)-1-amino-3-hydroxy-1-oxobutan-2-yl)carbamoyl)-2-(3,5-dibromobenzyl)pyrrolidine-1-carbonyl)pyrrolidin-1-yl)-3-hydroxy-1-oxobutan-2-yl)carbamate (**9**):

To a stirring solution of compound 8 (1.8 g, 3.20 mmol) in $CH_2Cl_2$ (100 mL) was added EDCI.HCl (0.916 g, 4.80 mmol), HOBt (0.65 g, 4.80 mmol) followed by DIPEA (1.7 mL, 9.60 mmol) and (2*S*,3*R*)-2-((*tert*-butoxycarbonyl)amino)-3-hydroxybutanoic acid (0.84 g, 3.80 mmol) at 0 °C. The reaction mixture was stirred at RT for 12 h. The reaction mixture was quenched with water (100 mL) and extracted with $CH_2Cl_2$ (2x 150 mL). The separated organic layers were washed with brine, dried over anhydrous $Na_2SO_4$, filtered and concentrated under reduced pressure to obtain crude product; which was purified by silica gel column chromatography followed by prep-HPLC purification to afford compound **9-Fr-1** (140 mg) and compound **9-Fr-2** (150 mg).

Chiral HPLC data for compound 9-Fr-1:

**$^1$H-NMR:** (400 MHz, DMSO-*d*$_6$): δ 7.71 (d, 3H), 7.21 (s, 1H), 6.99-6.75 (m, 2H), 6.59 (d, 1H), 5.01 (d, 1H), 4.65-4.60 (m, 2H), 4.19 (t, 2H), 3.99 (d, 1H), 3.89-3.85 (m, 2H), 3.65-3.61 (m, 2H), 3.44-3.39 (m, 3H), 2.25-2.15 (m, 1H), 2.02-1.95 (m, 5H), 1.75-1.72 (m, 1H), 1.35 (s, 9H), 1.25 (d, 3H), 1.19 (d, 3H), 0.99-0.95 (m, 1H).
**HPLC:** 99.83%.
**LCMS (m/z):** 762.3 [$M^+$+1].

Chiral HPLC data for compound 9-Fr-2:

**$^1$H-NMR:** (400 MHz, DMSO-*d*$_6$): δ 7.71 (d, 3H), 7.21 (s, 1H), 6.99-6.75 (m, 2H), 6.59 (d, 1H), 5.01 (d, 1H), 4.65-4.60 (m, 2H), 4.19 (t, 2H), 3.99 (d, 1H), 3.89-3.85 (m, 2H), 3.65-3.61 (m, 2H), 3.44-3.39 (m, 3H), 2.25-2.15 (m, 1H), 2.02-1.95 (m, 5H), 1.75-1.72 (m, 1H), 1.35 (s, 9H), 1.25 (d, 3H), 1.19 (d, 3H), 0.99-0.95 (m, 1H).
**HPLC:** 92%.
LCMS (m/z):762.2 [$M^+$+1].

Synthesis of (*R*)-*N*-((*2S,3R*)-1-amino-3-hydroxy-1-oxobutan-2-yl)-1-((*S*)-1-((*2S,3S*)-2-amino-3-hydroxybutanoyl)pyrrolidine-2-carbonyl)-2-(3,5-dibromobenzyl)pyrrolidine-2-carboxamide (**CM-12A**):

To a stirring solution of compound 9-Fr-1 (140 mg, 0.183 mmol) in $CH_2Cl_2$ (3 mL) was added dioxane.HCl (2 mL) at 0 °C under $N_2$ atmosphere. The reaction mixture was stirred at RT for 2 h. The reaction mixture was concentrated under reduced pressure to afford **CM-12A** (120 mg, 93%).
**$^1$H-NMR:** (400 MHz, $D_2O$): δ 7.84 (s, 1H), 7.61 (s, 2H), 4.39 (d, 4H), 3.92-3.85 (m, 4H), 3.65-3.62 (m, 2H), 3.45 (d, 1H), 2.59-2.55 (m, 1H), 2.48-2.44 (m, 2H), 2.15-2.19 (m, 3H), 1.91-1.87 (m, 1H), 1.60 (s, 3H), 1.25 (s, 3H).
**LCMS (m/z)**:662 (M+1).
**HPLC:** 97.55%.
**Optical rotation [$\alpha^{20}{}_D$]:** +29.21 (C=0.5% in water).

Synthesis of (*S*)-*N*-((*2S*,*3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3,5-dibromobenzyl)pyrrolidine-2-carboxamide (**CM-12B**):

To a stirring solution of compound 9-Fr-2 (150 mg, 0.197 mmol) in $CH_2Cl_2$ (3 mL) was added dioxane.HCl (2 mL) at 0 °C under $N_2$ atmosphere. The reaction mixture was stirred at RT for 2 h. The reaction mixture was concentrated under reduced pressure to afford **CM-12B** (130 mg, 94%).
**$^1$H-NMR:** (400 MHz, $D_2O$): δ 7.84 (s, 1H), 7.61 (s, 2H), 4.39 (d, 4H), 3.92-3.85 (m, 4H), 3.65-3.62 (m, 2H), 3.45 (d, 1H), 2.59-2.55 (m, 1H), 2.48-2.44 (m, 2H), 2.15-2.19 (m, 3H), 1.91-1.87 (m, 1H), 1.60 (s, 3H), 1.25 (s, 3H).
**LCMS (m/z):**662 (M+1).
**HPLC:** 92.11%.
**Optical rotation [$\alpha^{20}{}_D$]:** -78.70 (C=0.5% in water).

Example 13 - Synthesis of (*R*)-*N*-((*2S*,*3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3-methoxybenzyl)pyrrolidine-2-carboxamide (CM-13A) and *N*-((*2S,3R*)-1-amino-3-hydroxy-1-oxobutan-2-yl)-1-((*S*)-1-((*2S*,*3R*)-2-amino-3-hydroxybutanoyl)pyrrolidine-2-carbonyl)-2-(3-methoxybenzyl)pyrrolidine-2-carboxamide (CM-13B):

The following reaction sequence was used (Scheme M) to synthesize (*R*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3-methoxybenzyl)pyrrolidine-2-carboxamide (CM-13A) and *N*-((*2S*,*3R*)-1-amino-3-hydroxy-1-oxobutan-2-yl)-1-((*S*)-1-((*2S*,*3R*)-2-amino-3-hydroxybutanoyl)pyrrolidine-2-carbonyl)-2-(3-methoxybenzyl)pyrrolidine-2-carboxamide (CM-13B):

Scheme M. Synthesis of CM-13A and CM-13B:

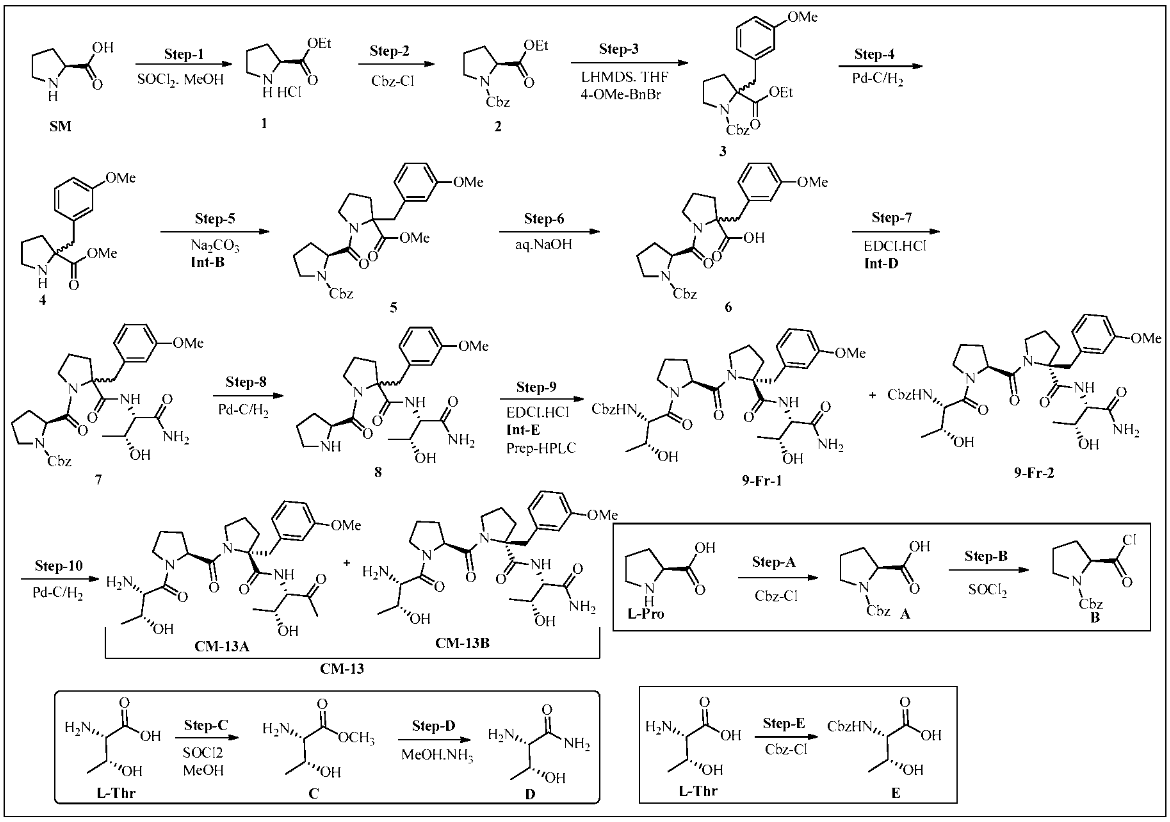


Synthesis of (*S*)-1-((benzyloxy)carbonyl) pyrrolidine-2-carboxylic acid (**A**):

To a stirring solution of (*S*)-pyrrolidine-2-carboxylic acid (250 g, 2.17 mol) in water (1 L) was added $Na_2CO_3$ (576 g, 5.43 mol) and stirred for 1 h. After being cooled to 0 °C, benzylchloroformate (444 g, 2.61 mol) was added drop wise to the reaction mixture and again stirred for 1 h. The resulting reaction mixture was warmed to RT and further stirred for 24 h. After consumption of the starting material (by TLC), the reaction was diluted with water (1 L) and ether (1.5 L). The separated aqueous layer was treated with $PhCH_3$ (1.5 L) and acidified using 6N HCl. The aqueous layer was extracted with EtOAc (3x 1.5 L); Combined organic extracts were washed with brine, dried over anhydrous $Na_2SO_4$, filtered and concentrated under reduced pressure to afford compound **A** (450 g, 84%) as light yellow syrup.
**$^1$H-NMR:** (400 MHz, DMSO-$d_6$): δ 12.71 (br s, 1H), 7.40-7.30 (m, 5H), 5.19-5.01 (m, 2H), 4.25 (dd, 1H), 3.51-3.50 (m, 2H), 2.29-2.15 (m, 1H), 1.89-1.80 (m, 3H).
**LCMS (m/z):** 250 [$M^+$+1].

Synthesis of (*S*)-benzyl 2-(chlorocarbonyl)pyrrolidine-1-carboxylate (**B**):

To a stirring solution of compound **A** (2.5 g, 0.01 mol) in $CH_2Cl_2$ (50 mL) was added $SOCl_2$ (2.7 g, 0.02 mol) at 0 °C and stirred for 2 h. The reaction mixture was concentrated under reduced pressure to afford compound **B** as crude. This material was directly used for the next step without further purification.

Synthesis of (*2S,3R*)-2-(((benzyloxy)carbonyl)amino)-3-hydroxybutanoic acid (**E**):

To a stirring solution of $NaHCO_3$ (529 g, 6.30 mol) in water (1 L) was added (2S,3R)-2-amino-3-hydroxybutanoic acid (250 g, 2.10 mol) at RT and stirred for 30 min. The reaction mixture was cooled to 0 °C, Cbz-Cl (850 mL, 2.52 mol, 50% on $PhCH_3$) was added drop wise to the reaction and stirred for 1 h. The reaction mixture was warmed to RT and again stirred for 28 h. To this MTBE (1L) was added and stirred for 20 min. Separated aqueous layer was stirred with toluene and stirred for 20 min. Aqueous layer was acidified with 1N HCl (pH~1-2) and extracted with EtOAc (3x 1.5 L). The organic layer was washed with brine, dried over anhydrous $Na_2SO_4$ and concentrated under reduced pressure. The crude material was stirred with dicyclohexylamine (819 mL, 4.20 mol) for 4 h to get white solid, filtered and dried. Obtained solid was refluxed with EtOAc (1.5 L) for 1h and then filtered. The solid material was dissolved in water (1 L)

and acidified with dil.$H_2SO_4$ and again stirred for 30 min. The aqueous layer was extracted with EtOAc (3x 1 L). The separated organic layer was washed with brine, dried over anhydrous Na2SO4, filtered and concentrated under reduced pressure. Obtained crude material was triturated with *n*-Hexane for 2 h to get white solid. Obtained solid material was filtered and dried under reduced pressure to afford compound **E** (230 g, 43%).
**$^1$H-NMR:** (400 MHz, DMSO-$d_6$): δ 7.35-7.32 (m, 5H), 6.91 (d, 1H), 5.06 (s, 2H), 4.13-4.09 (m, 1H), 3.99-3.95 (m, 1H), 1.02 (d, 3H).
**LCMS (m/z):**254.1(M+1).
**UPLC (ELSD purity):** 99.82%.

Synthesis of (*2S,3R*)-methyl 2-amino-3-hydroxybutanoate (**C**):

To a stirring solution of (*2S,3R*)-2-amino-3-hydroxybutanoic acid (200 g, 1.68 mol) in methanol (1.2 L) was added $SOCl_2$ (244 mL, 3.36 mol) drop wise at 0 °C and stirred for 1 h. The resulting reaction mixture was refluxed for 24 h. After consumption of the starting material (by TLC), the reaction mixture was warmed to RT and concentrated under reduced pressure and washed with *n*-hexane (2x 50 mL). The residue was dissolved in EtOH (1 L) and neutralized with $Et_3N$ (471 mL, 3.36 mol) and again stirred for 2 h. The precipitated solid was filtered off; obtained filtrate was concentrated under reduced pressure to afford compound **C** (195 g, 80%).
**$^1$H-NMR:** (400 MHz, DMSO-$d_6$): δ 8.51 (br s, 3H), 4.13-4.10 (m, 1H), 3.91 (br s, 1H), 1.20 (d, 3H).
**LCMS (m/z):**134.1 [$M^+$+1].

Synthesis of (*2S,3R*)-2-amino-3-hydroxybutanamide (**D**):

A solution of compound **F** (190 g, 1.35 mol) in IPA (2 L) was taken in autoclave and purged $NH_3$ gas (7-8 kg) and stirred at 35 °C for 24 h. Then removed $NH_3$ gas and reaction mixture was concentrated under reduced pressure and added $CH_2Cl_2$ and filtered. Obtained solid was refluxed in EtOH for 1 h at 78 °C. The reaction mass was filtered in heating condition and *n*-hexane was added to the filtrate and again stirred for another 4 h. Obtained precipitated solid was filtered and dried under reduced pressure to afford compound **D** (160 g, 47%).
**$^1$H-NMR:** (500 MHz, DMSO-$d_6$): δ 7.38 (br s, 1H), 7.02 (br s, 1H), 4.66 (br s, 1H), 3.77-3.70 (m, 1H), 2.93 (d, 1H), 2.72 (br m, 1H), 1.05 (d, 3H).
**LCMS (m/z):** 119.1 [$M^+$+1].
**UPLC (ELSD purity):** 99.9%.

Synthesis of *(S)*-ethyl pyrrolidine-2-carboxylate hydrochloride (**1**):

To a solution of L-proline (**SM**) (110 g, 956.5 mmol) in ethanol was added thionyl chloride (141 ml, 1911.3 mmol) and refluxed for 16 h. The reaction mixture was brought to RT and concentrated under reduced pressure to afford compound **1** as hydrochloride salt (170 g, 99 %).

Synthesis of (*S*)-1-benzyl 2-ethyl pyrrolidine-1,2-dicarboxylate (**2**):

To a solution of **1** (170 g, 947 mmol) in DCM was added TEA (398 ml, 2.83 mol) and after 30 min Cbz-Cl (1.136 mol) was added. The reaction mixture was stirred at RT for 12 h. The reaction mixture was washed with water and extracted with DCM. The organic layer was dried over anhydrous $Na_2SO_4$ and conc. under reduced pressure. The crude was purified by column chromatography to afford compound **2** (230 g, 88 %).
**$^1$H-NMR:** (200 MHz, DMSO-$d_6$): δ 7.42-7.31(m, 5H), 5.09 (m, 2H), 4.32-4.23(m, 1H), 4.08-3.98(m, 2H), 3.50-3.38 (m, 2H), 2.30-2.18 (m, 1H), 1.90-1.81(m, 3H), 1.10-1.04 (t, 3H).
**Mass m/z:** 278 [$M^+$+1].

Synthesis of 1-benzyl 2-ethyl 2-(3-methoxybenzyl) pyrrolidine-1,2-dicarboxylate (3):

To a solution of compound **2** (10 g, 37.9 mmol) in dry THF (100 mL) under inert atmosphere was added LiHMDS (1M in THF) (76 mL, 0.076 mol) at -25 °C and stirred for 2 h. 3-Methoxy benzyl bromide (9.21 g, 45.59 mmol) was added drop wise at -25 °C to the reaction mixture, allowed to warm to RT and stirred for 30 min. The reaction mixture was quenched with saturated $NH_4Cl$ solution and the aqueous layer was extracted with EtOAc (2 x 200mL). The combined organic extracts were dried over anhydrous $Na_2SO_4$ and concentrated under reduced pressure. The crude residue obtained was purified by silica gel column chromatography eluting with 10% EtOAc/hexane to afford compound **3** (10 g, 69%).
**$^1$H-NMR:** (400 MHz, $CDCl_3$): δ 7.41-7.32 (m, 5H), 7.19-7.09 (m, 1H), 6.80 (t, 1H), 6.65 (d, 2H), 5.30 (t, 1H), 5.19 (t, 1H),

3.79-3.70 (m, 5H), 3.55-3.52 (m, 2H), 3.10-3.07 (m, 2H), 2.15-2.08 (m, 1H), 1.79-1.72 (m, 1H), 1.10-0.99 (m, 1H).

Synthesis of methyl 2-(3-methoxybenzyl) pyrrolidine-2-carboxylate (**4**):

To a stirring solution of compound **3** (10 g, 26.17 mmol) in $CH_3OH$ (100 mL) was added Pd/C (3 g) and stirred under $H_2$ atmosphere at RT for 10 h. After consumption of the starting material (by TLC), the reaction mixture was filtered through a pad of celite and washed with MeOH. Obtained filtrate was concentrated under reduced pressure to afford compound **4** (5.3 g, 82%). This material was directly used for the next step without further purification.
**$^1$H-NMR:** (400 MHz, DMSO-$d_6$): δ 7.15 (t, 1H), 6.59-6.55 (m, 3H), 3.71 (s, 3H), 3.55 (s, 3H), 2.99 (d, 1H), 2.85-2.81 (m, 3H), 2.09-2.05 (m, 1H), 1.75-1.67 (m, 3H).
**LCMS (m/z):**250.2 [$M^+$+1].

Synthesis of (*2S)*-benzyl 2-(2-(3-methoxybenzyl)-2-(methoxycarbonyl)pyrrolidine-1-carbonyl)pyrrolidine-1-carboxylate (**5**):

To a suspension of compound **4** (4.3 g, 17.47 mmol) in DCM: $H_2O$ (700 mL, 2:5) was added $Na_2CO_3$ (4.6 g, 43.69 mmol) followed by a solution of **Int-B** (5.2 g, 20.97 mmol) in DCM at RT and stirred for 2 h. The organic layer was washed with brine, dried over $Na_2SO_4$, concentrated under reduced pressure to afford compound **5** (7 g) as crude. This material was directly used for the next step without further purification.

Synthesis of (*S*)-1-(1-((benzyloxy)carbonyl)pyrrolidine-2-carbonyl)-2-(3-methoxybenzyl)pyrrolidine-2-carboxylic acid (**6**):

To a stirring solution of compound **5** (7 g, 14.58 mmol) in MeOH (100 mL) was added 1N NaOH solution (100 mL) and stirred at reflux temperature for 5 h. The volatiles were evaporated under reduced pressure and the residue obtained was diluted with water and adjusted pH~ 2 using saturated citric acid solutions. The aqueous layer was extracted with EtOAc. The combined organic extracts were dried over $Na_2SO_4$ and concentrated under reduced pressure to afford compound **6** (7.6 g) as crude.
**$^1$H-NMR:** (400 MHz, DMSO-$d_6$): δ 12.45 (br s, 1H), 7.85-7.75 (m, 4H), 6.99-6.82 (m, 2H), 5.34-5.29 (m, 1H), 3.74 (d, 1H), 3.65-3.41 (m, 2H), 3.39-3.34 (m, 1H), 3.01 (t, 1H), 2.25-2.21 (m, 1H), 2.01-1.92 (m, 3H), 1.12 (t, 1H).
**LCMS (m/z):**467 [$M^+$+1].

Synthesis of (*S*)-benzyl 2-(2-(((*2S,3R*)-1-amino-3-hydroxy-1-oxobutan-2-yl)carbamoyl)-2-(3-methoxybenzyl)pyrrolidine-1-carbonyl)pyrrolidine-1-carboxylate (7):

To a stirring solution of compound **6** (2 g, 4.29 mmol) in $CH_2Cl_2$ (30 mL) was added EDCI.HCl (1.23 g, 6.43 mmol), HOBt (869 mg, 6.43 mmol) followed by DIPEA (1.66 g, 12.87 mmol) and compound D (506 mg, 4.29 mmol) at 0 °C. The reaction mixture was stirred at RT for 12 h. After consumption of the starting material (by TLC), the reaction was quenched with water and extracted with $CH_2Cl_2$ (2x 100 mL). The organic layer was dried over anhydrous $Na_2SO_4$ and concentrated under reduced pressure. The crude was purified by column chromatography to afford compound **7** (1.5 g, 61%).
**$^1$H-NMR:** (400 MHz, DMSO-$d_6$): δ 7.49-7.37 (m, 6H), 7.11-6.92 (m, 2H), 6.88-6.82 (m, 2H), 5.35-5.01 (m, 2H), 4.89-4.52 (m, 1H), 4.22 (d, 1H), 4.01-3.84 (m, 1H), 3.72 (d, 2H), 3.62-3.55 (m, 4H), 3.22-3.18 (m, 1H), 2.22-1.75 (m, 5H), 1.09 (br s, 3H).

Synthesis of *N*-((*2S,3R*)-1-amino-3-hydroxy-1-oxobutan-2-yl)-2-(3-methoxybenzyl)-1-((*S*)-pyrrolidine-2-carbonyl)pyrrolidine-2-carboxamide (**8**):

To a stirring solution of compound **7** (1.5 g, 2.65 mmol) in MeOH (20 mL) was added 10% Pd/C (0.4 g) and stirred under $H_2$ atmosphere at RT for 6 h. After consumption of the starting material (by TLC), the reaction mixture was filtered through a pad of celite and filtrate was concentrated under reduced pressure to afford compound **8** (1 g, 87%) as white solid.
**$^1$H-NMR:** (400 MHz, DMSO-$d_6$): δ 7.22-7.18 (m, 2H), 7.08-7.01 (m, 1H), 6.98 (br s, 1H), 6.84 (t, 1H), 6.75-6.71 (m, 2H), 5.05 (d, 1H), 4.23 (t, 1H), 5.09 (br s, 1H), 3.95 (d, 1H), 3.74 (s, 4H), 3.61 (t, 2H), 3.09-3.01 (m, 2H), 2.72-2.68 (m, 1H), 2.17-2.01 (m, 3H), 1.85-1.80 (m, 5H), 1.08 (d, 3H).
**LCMS (m/z):** 433 [$M^+$+1].

Synthesis of benzyl ((*2S,3R*)-1-((*S*)-2-(2-(((*2S,3R*)-1-amino-3-hydroxy-1-oxobutan-2-yl)carbamoyl)-2-(3-methoxybenzyl)pyrrolidine-1-carbonyl)pyrrolidin-1-yl)-3-hydroxy-1-oxobutan-2-yl)carbamate (**9**):

To a stirring solution of compound **8** (2 g, 4.62 mmol) in $CH_2Cl_2$ (30 mL) was added HOBt (937 mg, 6.94 mmol), EDCI.HCl (1.32 g, 6.93 mmol) followed by DIPEA (1.79 g, 13.82 mmol) and compound **E** (1.17 g, 4.62 mmol) at 0 °C. The reaction mixture was stirred at RT for 12 h. After consumption of the starting material (by TLC), the reaction was quenched with water and extracted with $CH_2Cl_2$ (2x 100 mL). The organic layer was dried over anhydrous $Na_2SO_4$ and concentrated under reduced pressure. The crude was purified by column chromatography followed by prep-HPLC purification to afford compound **9-Fr-1** (0.5 g) and compound **9-Fr-2** (0.1 g) as white solid.

Chiral HPLC data for compound 9-Fr-1:

**$^1$H-NMR:** (400 MHz, DMSO-$d_6$): δ 7.39 (s, 4H), 7.19-7.15 (m, 2H), 7.09-7.02 (m, 2H), 6.75-6.71 (m, 2H), 5.10-4.99 (m, 2H), 4.76-4.71 (m, 1H), 4.29-4.21 (m, 1H), 4.07-3.87 (m, 2H), 3.71 (d, 2H), 3.65-3.62 (m, 1H), 3.45 (d, 1H), 3.21-3.14 (m, 1H), 2.15-2.01 (m, 5H), 1.72 (br m, 1H), 1.22-1.15 (m, 2H), 1.05 (t, 2H).
**HPLC:** 41.56%.
**LCMS (m/z):** 668 [$M^+$+1].

Chiral HPLC data for compound 9-Fr-2:

**$^1$H-NMR:** (400 MHz, DMSO-$d_6$): δ 7.391 (s, 4H), 7.19-7.15 (m, 2H), 7.09-7.02 (m, 2H), 6.75-6.71 (m, 2H), 5.10-4.99 (m, 2H), 4.76-4.71 (m, 1H), 4.29-4.21 (m, 1H), 4.07-3.87 (m, 2H), 3.71 (d, 2H), 3.65-3.62 (m, 1H), 3.45 (d, 1H), 3.21-3.14 (m, 1H), 2.15-2.01 (m, 5H), 1.72 (br m, 1H), 1.22-1.15 (m, 2H), 1.05 (t, 2H).
**HPLC:** 58.43%.
**LCMS (m/z):** 762.2 [$M^+$+1].

Synthesis of (*R*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3-methoxybenzyl)pyrrolidine-2-carboxamide (**CM-13A**):

To a solution of compound **9-Fr-1** (0.5 g, 0.0.09 mol) in DCM (2 mL) was added ether HCl (2 mL) and stirred at RT for 1 h. The reaction mixture was concentrated under reduced pressure to afford **CM-13A** (45 mg, 84%) as white solid.
**$^1$H-NMR:** (400 MHz, DMSO-$d_6$): δ 8.01 (br s, 3H), 7.35 (d, 1H), 7.21 (t, 1H), 7.09 (s, 1H), 7.01 (s, 1H), 6.89 (d, 1H), 6.69 (s, 2H), 5.49 (br s, 1H), 4.69 (t, 1H), 4.35-4.31 (m, 1H), 4.15 (d, 1H), 4.09 (br m, 1H), 3.91-3.85 (m, 4H), 3.75-3.71 (m, 1H), 3.49 (d, 2H), 3.19 (s, 2H), 3.12 (d, 1H), 2.21-2.15 (m, 1H), 2.12-2.05 (m, 3H), 1.97-1.92 (m, 3H), 1.75-1.71 (m, 1H), 1.41 (d, 3H), 1.09 (d, 3H).
**LCMS (m/z):** 534 [$M^+$+1].
**HPLC Purity:** 94%.

Synthesis of *N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3-methoxybenzyl)pyrrolidine-2-carboxamide (**CM-13B**):

To a solution of compound **9-Fr-2** (0.1 g, 0.187 mmol) in DCM (3 mL) was added ether HCl (2 mL) and stirred at RT for 1 h. The reaction mixture was concentrated under reduced pressure to afford **CM-13B** (80 mg, 75%) as white solid.
**$^1$H-NMR:** (400 MHz, DMSO-$d_6$): δ 8.01 (br s, 3H), 7.35 (d, 1H), 7.21 (t, 1H), 7.09 (s, 1H), 7.01 (s, 1H), 6.89 (d, 1H), 6.69 (s, 2H), 5.49 (br s, 1H), 4.69 (t, 1H), 4.35-4.31 (m, 1H), 4.15 (d, 1H), 4.09 (br m, 1H), 3.91-3.85 (m, 4H), 3.75-3.71 (m, 1H), 3.49 (d, 2H), 3.19 (s, 2H), 3.12 (d, 1H), 2.21-2.15 (m, 1H), 2.12-2.05 (m, 3H), 1.97-1.92 (m, 3H), 1.75-1.71 (m, 1H), 1.41 (d, 3H), 1.09 (d, 3H).
**LCMS (m/z):** 534 [$M^+$+1].
**HPLC Purity:** 92%.

Example 14 - Synthesis of (*R*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3,4,5-trimethoxybenzyl)pyrrolidine-2-carboxamide (CM-14A) and (*S*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3,4,5-trimethoxybenzyl)pyrrolidine-2-carboxamide (CM-14B):

The following reaction sequence was used (Scheme N) to synthesize (*R*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3,4,5-trimethoxybenzyl)pyrrolidine-2-carboxamide (CM-14A) and (*S*)-*N*-((*2S,3R*)-1-amino-3-hydroxy-1-oxobutan-2-yl)-1-((*S*)-1-((*2S,3R*)-2-amino-3-hydroxybutanoyl)pyrrolidine-2-carbonyl)-2-(3,4,5-trimethoxybenzyl)pyrrolidine-2-carboxamide (CM-14B):

Scheme N. Synthesis of CM-14A and CM-14B:

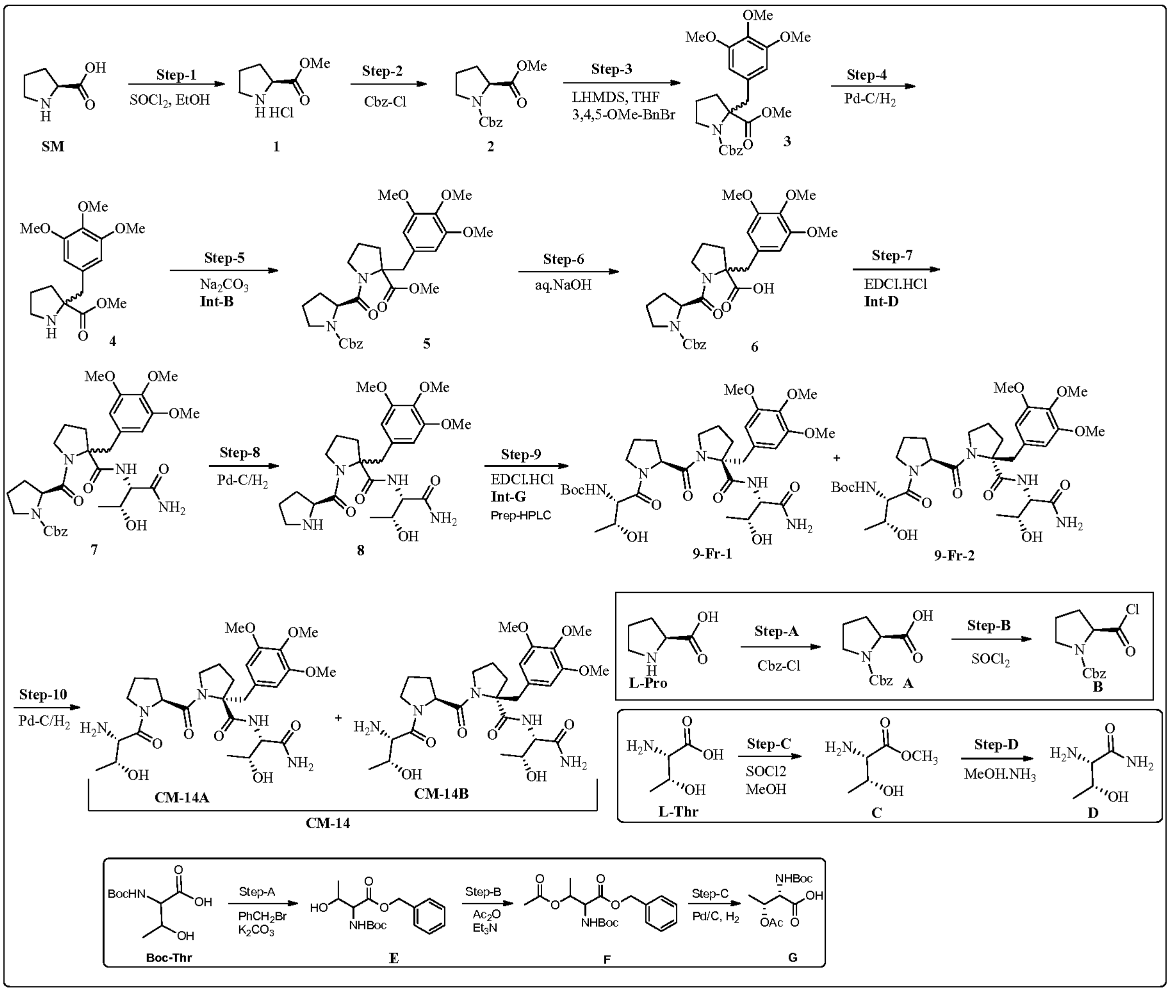


Synthesis of (*S*)-1-((benzyloxy) carbonyl) pyrrolidine-2-carboxylic acid (**A**):

To a stirring solution of (*S*)-pyrrolidine-2-carboxylic acid (250 g, 2.17 mol) in water (1 L) was added $Na_2CO_3$ (576 g, 5.43 mol) and stirred for 1 h. After being cooled to 0 °C, benzylchloroformate (444 g, 2.61 mol) was added drop wise to the reaction mixture and again stirred for 1 h. The resulting reaction mixture was warmed to RT and further stirred for 24 h. After consumption of the starting material (by TLC), the reaction was diluted with water (1 L) and ether (1.5 L). The separated aqueous layer was treated with $PhCH_3$ (1.5 L) and acidified using 6N HCl. The aqueous layer was extracted with EtOAc (3x 1.5 L). Combined organic extracts were washed with brine, dried over anhydrous $Na_2SO_4$, filtered and concentrated under reduced pressure to afford compound **A** (450 g, 84%) as light yellow syrup.
**$^1$H-NMR:** (400 MHz, DMSO-$d_6$): δ 12.71 (br s, 1H), 7.40-7.30 (m, 5H), 5.19-5.01 (m, 2H), 4.25 (dd, 1H), 3.51-3.50 (m, 2H), 2.29-2.15 (m, 1H), 1.89-1.80 (m, 3H).
**LCMS (m/z):** 250 [$M^+$+1].

Synthesis of (*S*)-benzyl 2-(chlorocarbonyl)pyrrolidine-1-carboxylate (**B**):

To a stirring solution of compound **A** (2.5 g, 0.01 mol) in $CH_2Cl_2$ (50 mL) was added $SOCl_2$ (2.7 g, 0.02 mol) at 0 °C and stirred for 2 h. The reaction mixture was concentrated under reduced pressure to afford compound **B** as crude. This material was directly used for the next step without further purification.

Synthesis of (*2S,3R*)-methyl 2-amino-3-hydroxybutanoate (**C**):

To a stirring solution of (*2S,3R*)-2-amino-3-hydroxybutanoic acid (200 g, 1.68 mol) in methanol (1.2 L) was added $SOCl_2$ (244 mL, 3.36 mol) drop wise at 0 °C and stirred for 1 h. The resulting reaction mixture was refluxed for 24 h. After consumption of the starting material (by TLC), the reaction mixture was warmed to RT and concentrated under reduced pressure and decanted with *n*-hexane (2x 50 mL). The residue was dissolved in EtOH (1 L) and neutralized with $Et_3N$ (471 mL, 3.36 mol) and again stirred for 2 h. The precipitated solid was filtered off; obtained filtrate was concentrated under reduced pressure to afford compound **C** (195 g, 80%).
**$^1$H-NMR:** (400 MHz, DMSO-$d_6$): δ 8.51 (br s, 3H), 4.13-4.10 (m, 1H), 3.91 (br s, 1H), 1.20 (d, 3H).
**LCMS (m/z):**134.1 [$M^+$+1].

Synthesis of (*2S,3R*)-2-amino-3-hydroxybutanamide (**D**):

A solution of compound **F** (190 g, 1.35 mol) in IPA (2 L) was taken in autoclave and purged $NH_3$ gas (7-8 kg) and stirred at 35 °C for 24 h. Then removed $NH_3$ gas and reaction mixture was concentrated under reduced pressure and added $CH_2Cl_2$ and filtered. Obtained solid was refluxed in EtOH for 1 h at 78 °C. The reaction mass was filtered in heating condition and *n*-hexane was added to the filtrate and again stirred for another 4 h. Obtained precipitated solid was filtered and dried under reduced pressure to afford compound **D** (160 g, 47%).
**$^1$H-NMR:** (500 MHz, DMSO-$d_6$): δ 7.38 (br s, 1H), 7.02 (br s, 1H), 4.66 (br s, 1H), 3.77-3.70 (m, 1H), 2.93 (d, 1H), 2.72 (br m, 1H), 1.05 (d, 3H).
**LCMS (m/z):** 119.1 [$M^+$+1].
**UPLC (ELSD purity):** 99.9%.

Synthesis of Benzyl 2-(*tert*-butoxycarbonylamino)-3-hydroxybutanoate (**E**):

To a solution of 2-(*tert*-butoxycarbonylamino)-3-hydroxybutanoic acid (50 g, 228.3mmol) in DMF (500 mL) was added $K_2CO_3$ (63 g, 456.6 mmol) and stirred at RT for 15 min. To this Benzyl bromide (46.83 g, 273.9 mmol) was added and stirred at RT for 6 h. The reaction mixture was diluted with water (500mL) and extracted with EtOAc (2 x 750mL). The combined organic layers were washed with brine (50mL), dried over anhydrous $Na_2SO_4$ and concentrated under reduced pressure. The crude material was purified by silica gel column chromatography eluting with 20% EtOAc/hexane to afford benzyl 2-(tert-butoxycarbonylamino)-3-hydroxybutanoate **E** (52 g, 73 %).
**$^1$H-NMR:** (500 MHz, DMSO-$d_6$): δ 7.37-7.30 (m, 5H), 6.60 (d, 1H), 5.18-5.08 (m, 2H), 4.76 (d, 1H), 4.08-4.00 (m, 2H), 1.38 (s, 9H), 1.09 (d, 3H).
**Mass m/z:** 310.0 [$M^+$+1], 210 [$M^+$-De Boc].

Synthesis of benzyl 3-acetoxy-2-(*tert*-butoxycarbonylamino) butanoate (**F**):

To a stirring solution of compound E (52 g, 168.2 mmol) in $CH_2Cl_2$ (500mL) was added $Ac_2O$ (20.5 g, 201.9mmol), $Et_3N$ (25.4 g, 252.4mmol) and DMAP (3.5 g) and stirred at RT for 2 h. The volatiles were removed under reduced pressure. The residue obtained was diluted with EtOAc (750mL) and washed with cold 0.5 N HCl solution (2 x 200mL). The organic layer was washed with brine, dried over anhydrous $Na_2SO_4$ and concentrated under reduced pressure to afford 3-acetoxy-2-(tert-butoxycarbonylamino) butanoate F (52 g, 88%).
**$^1$H-NMR:** (500 MHz, DMSO-$d_6$): δ 7.35-7.34 (m, 5H), 7.27-7.25 (d, 1H), 5.18-5.06 (m, 3H), 4.34-4.32 (m, 1H), 1.90 (s, 3H), 1.39 (s, 9H), 1.16 (d, 3H).
**Mass m/z:** 252 [$M^+$+1-De Boc].

Synthesis of (*2S,3R*)-3-acetoxy-2-(tert-butoxycarbonylamino) butanoic acid (**G**):

To a stirring solution of compound **F** (52 g, 148.1mmol) in MeOH (1 L) was added 10% Pd/C under $N_2$ atmosphere and reaction mixture was stirred under hydrogen atmosphere for 16 h. The reaction mixture was filtered through a pad of celite, obtained filtrate was evaporated under reduced pressure and the crude residue was triturated with hexane to yield (2*S*,3*R*)-3-acetoxy-2-(tert-butoxycarbonylamino) butanoic acid **G** (35 g, 90%).
**$^1$H-NMR:** (500 MHz, DMSO-$d_6$): δ 12.78 (br s, 1H), 6.94 (d, 1H), 5.16-5.14 (m, 1H), 4.17-4.15 (m, 1H), 1.95 (s, 3H), 1.39 (s, 9H), 1.10 (d, 3H).
**Mass m/z:** 260.0 [M-1].

Synthesis of (*S*)-methyl pyrrolidine-2-carboxylate hydrochloride (**1**):

To a solution of L-proline (**SM**) (110 g, 956.5 mmol) in methanol was added thionyl chloride (141 ml, 1911.3 mmol) and refluxed for 16 h. The reaction mixture was brought to RT and concentrated under reduced pressure to afford compound **1** as hydrochloride salt (170 g, 99 %).

Synthesis of (*S*)-1-benzyl 2-methyl pyrrolidine-1,2-dicarboxylate (**2**):

To a solution of 1 (170 g, 947 mmol) in DCM was added TEA (398 ml, 2.83 mol) and after 30 min Cbz-Cl (1.136 mol) was added. The reaction mixture was stirred at RT for 12 h. The reaction mixture was washed with water and extracted with DCM. The organic layer was dried over anhydrous $Na_2SO_4$ and conc. under reduced pressure. The crude was purified by column chromatography to afford compound **2** (230 g, 88 %).
**$^1$H-NMR:** (200 MHz, DMSO-$d_6$): $\delta$ 7.42-7.31(m, 5H), 5.09 (m, 2H), 4.32-4.23(m, 1H), 4.08-3.98(m, 2H), 3.50-3.38 (m, 2H), 2.30-2.18 (m, 1H), 1.90-1.81(m, 3H), 1.10-1.04 (t, 3H).
**Mass m/z:** 278 [$M^+$+1].

Synthesis of 1-benzyl 2-methyl 2-(3,4,5-trimethoxybenzyl) pyrrolidine-1,2-dicarboxylate (**3**):

To a solution of compound **2** (3.4 g, 12.9 mmol) in dry THF (60 mL) under inert atmosphere was added LiHMDS (1M in THF) (25 mL, 25.0 mmol) at -25 °C and stirred for 2 h. 5-(bromomethyl)-1,2,3-trimethoxybenzene (4 g, 15.5 mmol) was added drop wise at -25 °C to the reaction mixture. It was allowed to warm to RT and stirred for 2 h. The reaction mixture was quenched with saturated $NH_4Cl$ solution and the aqueous layer was extracted with EtOAc (2 x 200mL). The combined organic extracts were dried over anhydrous $Na_2SO_4$ and concentrated under reduced pressure. The crude residue obtained was purified by silica gel column chromatography eluting with 15% EtOAc/hexane to afford compound **3** (4.8 g, 84%).
**LCMS (m/z):**444.4 [$M^+$+1].
**HPLC:** 92%.

Synthesis of Methyl 2-(3,4,5-trimethoxybenzyl) pyrrolidine-2-carboxylate (**4**):

To a stirring solution of compound **3** (4.8 g, 10.8 mmol) in $CH_3OH$ (40 mL) was added Pd/C (1.5 g) and stirred under $H_2$ atmosphere at RT for 16 h. After consumption of the starting material (by TLC), the reaction mixture was filtered through a pad of celite and washed with MeOH. Obtained filtrate was concentrated under reduced pressure to afford compound **4** (3.0 g, 90%).
**$^1$H-NMR:** (400 MHz, DMSO-$d_6$): $\delta$ 6.49 (s, 2H), 3.72 (s, 6H), 3.61 (s, 6H), 2.99 (d, 1H), 2.85-2.81 (m, 2H), 2.78 (d, 1H), 2.12-2.07 (m, 1H), 1.79-1.71 (m, 3H).
**LCMS (m/z):** 310.2 [$M^+$+1].

Synthesis of (2*S*)-benzyl 2-(2-(methoxycarbonyl)-2-(3,4,5-trimethoxybenzyl)-pyrrolidine-1-carbonyl)pyrrolidine-1-carboxylate (**5**):

To a suspension of compound **5** (3 g, 9.70 mmol) in DCM: $H_2O$ (45 mL, 5:4) was added $Na_2CO_3$ (2.5 g, 24.20 mmol) followed by a solution of **Int-B** (3.2 g, 11.60 mmol) in DCM at RT and stirred for 2 h. The organic layer was washed with brine, dried over $Na_2SO_4$, concentrated under reduced pressure to afford compound **5** (4.6 g) as crude. This material was directly used for the next step without further purification.
**LCMS (m/z):**541.3 [$M^+$+1].

Synthesis of (*S*)-1-(1-((benzyloxy)carbonyl)pyrrolidine-2-carbonyl)-2-(3,4,5-trimethoxybenzyl) pyrrolidine-2-carboxylic acid (**6**):

To a stirring solution of compound **5** (4.6 g, 8.80 mmol) in MeOH (40 mL) was added 2N NaOH solution (10 mL) and stirred at reflux temperature for 16 h. The volatiles were evaporated under reduced pressure and the residue obtained was diluted with water and adjusted pH~ 2 using 1N HCl solution. The aqueous layer was extracted with EtOAc. The combined organic extracts were dried over $Na_2SO_4$ and concentrated under reduced pressure to afford compound **6** (4 g) as crude.

Synthesis of (*S*)-benzyl 2-(2-(((2*S*,3*R*)-1-amino-3-hydroxy-1-oxobutan-2-yl) carbamoyl)-2-(3,4,5-trimethoxybenzyl) pyrrolidine-1-carbonyl) pyrrolidine-1-carboxylate (**7**):

To a stirring solution of compound **6** (4 g, 7.60 mmol) in $CH_2Cl_2$ (50 mL) was added HOBt (1.5 g, 11.40 mmol), EDCI.HCl (2.10 g, 11.40 mmol) followed by DIPEA (4.1 mL, 22.8 mmol) and compound D (987 mg, 8.00 mmol) at 0 °C. The reaction mixture was stirred at RT for 16 h. After consumption of the starting material (by TLC), the reaction was quenched with water and extracted with $CH_2Cl_2$ (2x 100 mL). The organic layer was dried over anhydrous $Na_2SO_4$ and concentrated under reduced pressure to obtain crude product, which was purified by column chromatography to afford compound **7** (3 g, 63%).
**LCMS (m/z):**625.6 [$M^+$-1].

Synthesis of *N*-((2*S*,3*R*)-1-amino-3-hydroxy-1-oxobutan-2-yl)-1-((*S*)-pyrrolidine-2-carbonyl)-2-(3,4,5-trimethoxybenzyl) pyrrolidine-2-carboxamide (**8**):

To a stirring solution of compound 7 (3 g, 4.79 mmol) in MeOH (30 mL) was added 10% Pd/C (0.7 g) under $N_2$ atmosphere. The reaction mixture was stirred at RT for 16 h under $H_2$ atmosphere. After consumption of the starting material (by TLC), the reaction mixture was filtered through a pad of celite and filtrate was concentrated under reduced pressure to afford compound **8** (2.1 g, 91%) as white solid.

Synthesis of *tert*-butyl ((2*S*,3*R*)-1-((S)-2-(2-(((2*S*,3*R*)-1-amino-3-hydroxy-1-oxobutan-2-yl) carbamoyl)-2-(3,4,5-trimethoxybenzyl) pyrrolidine-1-carbonyl) pyrrolidin-1-yl)-3-hydroxy-1-oxobutan-2-yl) carbamate (**9**):

To a stirring solution of compound **8** (2 g, 4.00 mmol) in $CH_2Cl_2$ (30 mL) was added HOBt (823 mg, 6.00 mmol), EDCI.HCl (1.16 g, 6.00 mmol) followed by DIPEA (2.2 mL, 11.6 mmol) and compound **G** (1.068 g, 4.80 mmol) at 0 °C. The reaction mixture was stirred at RT for 16 h. After consumption of the starting material (by TLC), the reaction was quenched with water and extracted with $CH_2Cl_2$ (2x 100 mL). The organic layer was dried over anhydrous $Na_2SO_4$ and concentrated under reduced pressure. The crude was purified by column chromatography followed by prep-HPLC purification to afford compound **9-Fr-1** (0.12 g) and compound **9-Fr-2** (0.2 g) as white solid.

Chiral HPLC data for compound 9-Fr-1:

**LCMS (m/z):** 594.5 [($M^+$+1)-Boc].
**HPLC:** 94%.

Chiral HPLC data for compound 9-Fr-2:

**LCMS (m/z):** 594.5 [($M^+$+1)-Boc].
**HPLC:** 95%.

Synthesis of (*R*)-*N*-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-(3,4,5-trimethoxybenzyl)pyrrolidine-2-carboxamide (**CM-14A**):

To a solution of compound **9-Fr-1** (0.12 g, 0.17 mmol) in DCM (2.5 mL) was added 1,4-dioxane HCl (2.5 mL) and stirred at RT for 2 h. The reaction mixture was concentrated under reduced pressure to afford CM-14A (80 mg) as HCl salt.
**$^1$H-NMR:** (400 MHz, $D_2O$): δ 6.89 (s, 2H), 4.99 (t, 1H), 4.45-4.40 (m, 4H), 3.98 (s, 6H), 3.85 (s, 4H), 3.71-3.65 (m, 4H), 3.51 (d, 1H), 3.39 (d, 1H), 2.62-2.55 (m, 1H), 2.44-2.37 (m, 1H), 2.24-2.20 (m, 4H), 1.84-1.82 (m, 1H), 1.51 (d, 3H), 1.27 (d, 3H), 1.15-1.09 (m, 1H). LCMS (m/z):594.5 [$M^+$+1]
**HPLC Purity:** 93%.
**Optical rotation [$\alpha^{19.9}{}_D$]:** +21.91 (C=0.5% in water).

Synthesis of (*S*)-*N*-((2*S*,3*R*)-1-amino-3-hydroxy-1-oxobutan-2-yl)-1-((*S*)-1-((2*S*,3*R*)-2-amino-3-hydroxybutanoyl)pyrrolidine-2-carbonyl)-2-(3,4,5-trimethoxybenzyl)pyrrolidine-2-carboxamide (**CM-14B**):

To a solution of compound **9-Fr-2** (0.2 g, 0.20 mmol) in DCM (2.5 mL) was added 1,4-dioxane HCl (2.5 mL) and stirred at RT for 2 h. The reaction mixture was concentrated under reduced pressure to afford **CM-14B** (170 mg) as HCl salt.
**$^1$H-NMR:** (400 MHz, $D_2O$): δ 6.89 (s, 2H), 4.99 (t, 1H), 4.45-4.40 (m, 4H), 3.98 (s, 6H), 3.85 (s, 4H), 3.71-3.65 (m, 4H),

3.51 (d, 1H), 3.39 (d, 1H), 2.62-2.55 (m, 1H), 2.44-2.37 (m, 1H), 2.24-2.20 (m, 4H), 1.84-1.82 (m, 1H), 1.51 (d, 3H), 1.27 (d, 3H), 1.15-1.09 (m, 1H). LCMS (m/z):594.5 [$M^{+}$+1]
**HPLC Purity:** 95%.
**Optical rotation [$\alpha^{19.9}{}_{D}$]:** -92.57(C=0.5% in water).

Example 15 - Drug Metabolism and Pharmacokinetics

This example demonstrates drug metabolism and pharmacokinetics properties of test compounds.
Compounds CM-5A, CM-6A, CM-7A, CM-8A & CM-9A were tested in *in vitro* aqueous solubility, MDCK permeability and metabolic stability assay. Compounds CM-5A, CM-6A & CM-7A were also tested in rat pharmacokinetics. A brief procedure, results and conclusions are summarized below.

**Aqueous solubility:**

Procedure. Kinetic aqueous solubility (*p*ION method) was assessed using below parameters:

- Stock preparation : DMSO
- Buffer : Phosphate free buffer
- pH condition : pH 7.4
- Test concentration : 200 μM with 1% DMSO (n = 3)
- Incubation time : 16 h
- Filtration : 0.22 μ filter plates
- Analysis : LC-MS/MS

Results. Below are the results from the solubility assay:

| Compound | Conc. tested | Mean Solubility (N=3) at pH |
|---|---|---|
| Caffeine | 200 μM | > 200 |
| DES | | 2.6 ± 2.1 |
| CM-5A | | > 200 |
| CM-6A | | > 200 |
| CM-7A | | - |
| CM-8A | | > 200 |
| CM-9A | | 148 ± 42 |

Conclusion. The results suggest that the test compounds have good aqueous solubility at pH 7.4.

**Metabolic Stability:**

Metabolic stability was assessed in liver microsomes as well as in hepatocytes with few compounds.
Procedure for microsomal stability:

- Species : Rat and human
- Test concentration : 1 μM (n = 2)
- Protein concentration : 0.125 mg/mL
- Time points : 0, 5, 15, 30 and 60 minutes
- Controls : Minus NADPH and buffer
- Analysis : Parent compound monitoring LC-MS/MS (Peak area ratios)

Procedure for hepatocyte stability:

- Stock preparation : DMSO

(continued)

| | |
|---|---|
| • Buffer | : Krebs Henseleit Buffer |
| • Total Incubation volume | : 1000 μL |
| • Cell density | : 1 million cells/mL |
| • Test concentration | : 10 μM with 0.5% DMSO (n = 2) |
| • Time points | : 0 and 60 min |
| • Analysis | : LC-MS/MS |

Results:

| Compoun ds | Microsomes/Hepatocytes | % Remaining at 60 minutes | Half-life (min) | Stability Class |
|---|---|---|---|---|
| CM-4A | Rat (M) | 68 | >60 | Unstable |
| | Human (M) | 72 | >60 | Moderate |
| CM-5A | Rat (M) | 95 | >60 | Stable |
| | Human (M) | 86 | >60 | Moderate |
| CM-6A | Rat (M) | 100 | >60 | Stable |
| | Human (M) | 88 | >60 | Moderate |
| CM-6A | Rat (H) | 87 | >60 | Moderate |
| | Human (H) | 98 | >60 | Stable |
| CM-7A | Rat (H) | 76 | >60 | Moderate |
| | Human (H) | 92 | >60 | Stable |
| CM-8A | Rat (M) | 91 | >60 | Stable |
| | Human (M) | 75 | >60 | Moderate |
| CM-9A | Rat (M) | 100 | >60 | Stable |
| | Human (M) | 69 | >60 | Unstable |
| (H): Hepatocytes; (M): Microsomes | | | | |

The metabolic stability classification was based on the below criteria.

| | |
|---|---|
| Stable | % Remaining > 90% |
| Moderate | % Remaining 70-90% |
| Unstable | % Remaining < 70% |

Conclusion. The results suggest that the test compounds have moderate to high stability in microsomes/hepatocytes.

**MDCK Permeability:**

Procedure. Permeability of test compounds was assessed using MDCK (Madin-Darby Canine Kidney Cells) using the below parameters.

| | |
|---|---|
| • Test concentration | : 5 μM (n = 3) |
| • Buffer | : HBSS pH 7.4 |
| • Time points | : 0, 15, 30, 60 and 90 min |
| • Analysis | : Parent compound monitoring using LC-MS/MS |

Results. Below are the results from the MDCK permeability assay:

| Compounds | Concentration (μM) | Papp ($10^{-6}$ cm/sec) | Recovery | Permeability classification |
|---|---|---|---|---|
| Caffeine | 10 | 27.0 ± 1.3 | 100 | High |
| Furosemide | | 0.14 ± 0.01 | 100 | Low |
| CM-4A | 5 | 0.15 | 100 | Low |
| CM-5A | | 0.0 ± 0.0 | 100 | Low |
| CM-6A | | 0.0 ± 0.0 | 100 | Low |
| CM-7A | | 0.11 ± 0.0 | 68 | Low |
| CM-8A | | 0.14 ± 0.01 | 83 | Low |
| CM-9A | | 0.0 ± 0.0 | 100 | Low |

Conclusion. The results suggest that the test compounds have poor/low permeability through MDCK cells. The MDCK permeability classification was based on the below criteria.

| | |
|---|---|
| Low | ≤ 3 x $10^{-6}$ cm/sec |
| Moderate | 3 - 15 x $10^{-6}$ cm/sec |
| High | > 15 x $10^{-6}$ cm/sec |

**Bioavailability of CM-4A**

Procedure. 2-3 month old Male (1 mg/kg IV and 10 mg/kg PO) and male and female (0.1 and 1 mg/kg PO) Sprague Dawley rats were dosed with CM-4A (1 mg/kg IV [tail vein] or 0.1, 1, or 10 mg/kg PO [gastric gavage]; n = 5-6 per group) in sterile saline vehicle and repeated blood draws take at various timepoints via lateral tail vein draws or cardiac sticks using K2-EDTA treated syringes. Data for male and female were combined for 1 mg/kg PO dosing given that no gender difference was seen. Plasma was separated by centrifugation (1,000 x G for 10 min at 4 °C) and plasma samples stored at -80 °C until assay. Samples were stored at -80 °C until assay. On the day of the assay, samples and spiked plasma standards were thawed at 4 °C, extracted in 3 volumes of ice cold acetonitrile, centrifuged at 25,000 x G for 10 min at 4 °C, and the supernatant was loaded into autosampler vials (4 °C). Samples were analyzed using Agilent QQQ LC/MS/MS instrument in multiple reaction monitoring mode (504.3 -> 171.1, 199.1, 386.1 m/z) and the retention time of CM-4A was 9.3 min with and ~10 sec symmetrical peak.

As shown in FIG. 1, CM-4A oral bioavailability is essentially linear from 0.1 to 10 mg/kg.

Example 16 - [$^3$H] MK-801 Binding Assay

This example demonstrates a [$^3$H] MK-801 binding assay that may be used to assess agonistic and/or antagonistic properties of candidate NMDA receptor modulators.

Crude synaptic membranes were prepared from rat forebrains as described in Moskal et al. (2001), "The use of antibody engineering to create novel drugs that target N-methyl-D-aspartate receptors," Curr. Drug Targets, 2:331-45. Male 2-3 month old rats were decapitated without anesthesia by guillotine, and the brains were rapidly removed (~90 sec) and whole cortex and hippocampus dissected on an ice cold platform, frozen on dry ice, and stored at -80 °C. Samples were homogenized in 20 volumes of ice cold 5 mM Tris-HCl pH 7.4 by Brinkman Polytron and pelleted 48,000 x g for 20 min at 4 °C, and washed an additional 3 times as described above. Membranes were then resuspended in 5 mM EDTA and 15 mM Tris-HCl pH 7.4 and incubated for 1 hr at 37 °C, membranes pelleted at 48,000 x g for 20 min at 4°C, snap frozen in liquid nitrogen, and stored at -80 °C. On the day of the experiment, membranes were thawed at room temperature and washed an additional 7 times in ice cold 5 mM Tris-HCl (pH 7.4) as described above. After the last wash, membranes were resuspended in assay buffer (5 mM Tris-acetate pH 7.4), and protein content was determined by the BCA assay.

[$^3$H] MK-801 binding assays were preformed as described in Urwyler et al. (2009), "Drug design, in vitro pharmacology, and structure-activity relationships of 3-acylamino-2-aminopropionic acid derivatives, a novel class of partial agonists at the glycine site on the N-methyl-D-aspartate (NMDA) receptor complex," J. Med. Chem., 52:5093-10. Membrane protein (200 μg) was incubated with varying concentrations of the test compounds ($10^{-3}$ - $10^{-17}$ M) with 50 μM

glutamate for 15 min at 23 °C. Assay tubes were then incubated under non-equilibrium conditions with [$^3$H]MK-801 (5 nM; 22.5 Ci / mmol) for 15 min at 23 °C followed by filtration through Whatman GF/B filters using a Brandel M-24R Cell Harvester. Then the tubes were washed three times with assay buffer (5 mM Tris-acetate PH 7.4), and the filters were analyzed by liquid scintillation to calculate the disintegrations per minute (DPM). Zero levels were determined in the absence of any glycine ligand and in the presence of 30 μM 5,7-Dichlorokynurenic acid (5,7-DCKA). Maximal stimulation was measured in the presence of 1 mM glycine. 50 μM glutamate was present in all samples.

For each data point (i.e., a single concentration of the test compound), the % maximal [$^3$H] MK-801 binding was calculated by the following formula:

$$\% \text{ maximal } [^3\text{H}] \text{ MK-801 binding} = ((\text{DPM}(_{\text{test compound}}) - \text{DPM}_{5,7\text{-DCKA}})/(\text{DPM}_{1\text{ mM glycine}} - \text{DPM}_{5,7\text{-DCKA}})) \times 100\%$$

The efficacy for each compound, expressed as the % increase in [$^3$H] MK-801 binding, is calculated by fitting the data to a "log(agonist) vs. response (three parameters)" equation using Graph Pad Prism, and potency ($EC_{50}$, expressed in pM) and maximal activity (% maximal stimulation) were calculated for each test compound, with the efficacy for the test compound being the best-fit top value (FIGs. 2-4).

**Table 1. [$^3$H] MK-801 Binding Assay Data.**

| Compound | Potency | Efficacy (% Increase in [$^3$H] MK-801 Binding) |
|---|---|---|
| CM-1 | 5 pM | 79% |
| CM-2 | 6 pM | 24% |
| CM-3 | 16 pM | 23% |
| CM-4A | 0.2 pM | 12% |
| CM-4B | 0.2 pM | 12% |
| CM-5 | 196 pM | 19% |
| CM-6 | 0.6 pM | 12% |
| CM-7 | 10617 pM | 13% |
| CM-8 | 6 pM | 16% |
| CM-9 | 7 pM | 15% |
| CM-10 | 0.4 pM | 16% |
| CM-11 | 13 pM | 9% |
| CM-12 | 2211 pM | 13% |
| CM-13 | 0.9 pM | 10% |
| CM-14 | 443 pM | 8% |

Example 17 - NMDA Receptor (NMDAR) Currents

This example demonstrates an assay for determining the effect of test compounds on NMDAR currents.

Experiments were conducted on hippocampal slices from 14-18 day old Sprague-Dawley rats as described in Zhang et al. (2008) "A NMDA receptor glycine site partial agonist, GLYX-13 ("GLYX"), simultaneously enhances LTP and reduces LTD at Schaffer collateral-CA1 synapses in hippocampus," Neuropharmacology, 55:1238-50. Whole cell recordings were obtained from CA1 pyramidal neurons voltage clamped at -60mV, in slices perfused with (artificial cerebrospinal fluid) ACSF containing 0 mM [Mg2+] and 3 mM [Ca2+], plus 10 μM bicuculline and 20 μM CNQX to pharmacologically isolate NMDAR-dependent excitatory postsynaptic currents (EPSCs). Varying concentrations of test compound (10 nM to 1 μM) were bath applied and Schaffer collateral fibers were stimulated with single electrical pulses (80 μs duration) once every 30 s. NMDAR EPSCs were characterized by long rise and decay times, and were fully blocked at the end of each experiment by bath application of the NMDAR-specific antagonist D-2-amino-5-phosphonopentanoic acid (D-AP5; 50 μM). The efficacy of a test compound was calculated as the % increased in NMDAR current from the baseline. The baseline was measured as the NMDAR current before the test compound was applied.

**Table 2. NMDAR Current Assay Data.**

| Compound | Concentration | Efficacy (% Change in NMDAR Current from Baseline) |
|---|---|---|
| CM-1 | 1 μM | 70% |
| CM-2 | NT | NT |
| CM-3 | NT | NT |
| CM-4A | 1 μM | 75% |
| CM-4B | 1 μM | 10% |
| NT = not tested. | | |

FIG. 5 shows a time course of the effect of CM-4A (1 uM) on single shock Schaffer collateral-evoked pharmacologically-isolated NMDA EPSCs recorded in CA1 pyramidal neurons (n=5). (Each point is the mean ± SEM of EPSC peak amplitude or field excitatory postsynaptic potential (fEPSP) of n pyramidal neurons).

Example 18 - Long-term potentiation (LTP) assay procedure 1

This example demonstrates an assay for determining the effect of test compounds on LTP.

Hippocampal slices from 14-18 day old Sprague-Dawley rats were transferred to an interface recording chamber and continuously perfused at 3 ml/min with oxygenated ACSF at 32 ±0.5 °C. Low resistance recording electrodes were made from thin-walled borosilicate glass (1-2 MΩ after filling with ACSF) and inserted into the apical dendritic region of the Schaffer collateral termination field in stratum radiatum of the CA1 region to record field excitatory postsynaptic potentials (fEPSPs). A bipolar stainless steel stimulating electrode (FHC Co.) was placed on Schaffer collateral-commissural fibers in CA3 stratum radiatum, and constant current stimulus intensity adjusted to evoke approximately half-maximal fEPSPs once each 30 s (50-100 pA; 100 ms duration). fEPSP slope was measured by linear interpolation from 20%-80% of maximum negative deflection, and slopes confirmed to be stable to within ±10% for at least 10 min before commencing an experiment. Long-term potentiation (LTP) was induced by a high frequency stimulus train (3x100Hz/500ms; arrow) at Schaffer collateral-CA1 synapses in control (vehicle), untreated slices, or slices pre-treated with test compound (10 nM to 100 μM). Long-term potentiation signals were recorded using a Multiclamp 700B amplifier and digitized with a Digidata 1322 (Axon Instruments, Foster City, CA). Data were analyzed using pClamp software (version 9, Axon Instruments) on an IBM-compatible personal computer. The efficacy was calculated as the % increase in long-term potentiation measured for slices pre-treated with test compound as compared to vehicle.

**Table 3. LTP Assay Data.**

| Compound | Concentration | Efficacy (% Increase from Vehicle) |
|---|---|---|
| CM-1 | NT | NT |
| CM-2 | NT | NT |
| CM-3 | NT | NT |
| CM-4A | 1 uM | 30% |
| CM-4B | 1 uM | 10% |
| NT = not tested. | | |

FIG. 6 shows a dose-response relationship of effects on LTP for CM-2, CM-1, and CM-4A

FIG. 7 shows a dose-response relationship of effects on long-term depression (LTD) of GLYX-13 ("GLYX") versus CM-4A. 1μM of GLYX-13 and CM-4A both significantly reduced the magnitude of LTD, while lower concentrations resulted in smaller magnitude alterations. (Note 0.001 μM represents no drug addition; Each point is mean ± SEM of 8-10 slices).

Example 19 - Long-term potentiation (LTP) assay procedure 2

This example demonstrates an assay for determining the effect of test compounds on LTP.

Assays were conducted as described in Zhang et al. (2008). Sprague-Dawley rats (12-18 days old; Taconic Farms) were deeply anesthetized with isoflurane and decapitated. Brains was removed rapidly, submerged in ice-cold artificial cerebrospinal fluid (ACSF, 2-4 °C), which contained (in mM): 124 NaCl, 4 KCl, 2 MgSO4, 2 CaCl2, 1.25 NaH2PO4,

26 NaHCO3, 10 glucose; at pH 7.4, gassed continuously with 95% O2/5% CO2). Brains were hemisected, the frontal lobes cut off, and individual hemispheres glued using cyanoacrylate adhesive onto a stage immersed in ice-cold ACSF gassed continuously with 95% O2/5% CO2 during slicing. 400 μm thick coronal slices were cut using a Vibratome (Leica VT1200S), and transferred to an interface holding chamber for incubation at room temperature for a minimum of one hr before transferring to a Haas-style interface recording chamber continuously perfused at 3 ml/min with oxygenated ACSF at 32 ± 0.5 °C. Low resistance recording electrodes were made from thin-walled borosilicate glass (1-2 MΩ after filling with ACSF) and inserted into the apical dendritic region of the Schaffer collateral termination field in stratum radiatum of field CA1 region to record field excitatory postsynaptic potentials (fEPSPs). A bipolar stainless steel stimulating electrode (FHC Co.) was placed on Schaffer collateral-commissural fibers in CA3 stratum radiatum, and constant current stimulus intensity adjusted to evoke approximately half-maximal fEPSPs once each 30 s (50-100 pA; 100 μs duration). fEPSP slope was measured before and after induction of LTP by linear interpolation from 20 to 80% of maximum negative deflection, and slopes confirmed to be stable to within ± 10% for at least 15 min before commencing an experiment. Bath application of test compounds (1 μM) was applied 30 min prior to application of Schaffer collateral stimulus trains to elicit LTP. LTP was induced by stimulation of Schaffer collateral axons with four high frequency theta burst stimulus trains of 10 × 100 Hz/5 pulse bursts each, applied at an inter-burst interval of 200 ms. Each train was 2 s in duration, and trains were applied 15 s apart. Signals were recorded using a Multiclamp 700B amplifier and digitized with a Digidata 1322 (Axon Instruments, USA). Data were analyzed using pClamp software (version 9, Axon Instruments) on an IBM-compatible personal computer.

As shown in FIG. 8, CM-6A, CM-7A, CM-8A, and CM-9A (1 μM) increased long-term potentiation after high frequency stimulation of rat Schaffer collateral-evoked NMDA EPSCs recorded in CA1 pyramidal neurons. * P <.05.

Example 20 - Porsolt test procedure 1

This example demonstrates the Porsolt test for assessing test compounds for antidepressant activity.

Experiments were conducted as described in Burgdorf et al. (2009) "The effect of selective breeding for differential rates of 50-kHz ultrasonic vocalizations on emotional behavior in rats," Devel. Psychobiol., 51:34-46. Male Sprague-Dawley rats (2-3 month old) were dosed with test compound (0.3 to 30 mg/kg; intravenously via tail vein injection, or per os via gastric gavage) or vehicle (1 ml/kg sterile saline, or 1 ml/kg DMSO for 2,5-diazaspiro[3.4]octan-1-one) in a blind manner 1 hr before testing. Animals were placed in a 46 cm tall x 20 cm in diameter clear glass tube filled to 30 cm with tap water at room temperature (23°C ± 0.5 °C) for 5 min on the test day. All animals were towel dried after each swimming session by the experimenter. Water was changed after every other animal. Animals were videotaped and total duration (sec) of floating behavior (as defined as the minimal movement required in order to maintain the animal's head above the water) was quantified by a blind experimenter.

**Table 4. Porsolt Assay Data.**

| Compound | Dose, Route | % Reduction in Floating |
|---|---|---|
| CM-1 | 3 mg/kg, i.v. | 90% |
| CM-2 | NT | NT |
| CM-3 | NT | NT |
| CM-4A | 1 mg/kg, p.o. | 84% |
| CM-4B | 1 mg/kg, p.o. | 63% |

NT = not tested.

FIG. 9 shows Mean ± SEM floating time in the rat Porsolt test in 2-3 month old male SD rats dosed with CM-4A (0.1 to 10 mg / kg PO gastric gavage) or sterile saline vehicle (1 ml / kg PO) 1 hr before the first test session. Animals received a single 15 min Porsolt habituation session on the day before the first 5 min Porsolt test. N = 9 - 10 per group. The results demonstrate a dose- and time-dependent antidepressant effect.

FIG. 10 shows Mean ± SEM floating time in the rat Porsolt test in 2-3 month old male SD rats dosed with the AMPA receptor antagonist 2,3-dihydroxy-6-nitro-7-sulfamoyl-benzo[f]quinoxaline-2,3-dione (NBQX) (10 mg/kg IP) or distilled water vehicle (1 ml/kg) 10 min before dosing with CM-4A (1 mg/kg PO) or sterile saline vehicle (1 ml/kg PO gastric gavage) 1 hr before the testing. Animals received a single 15 min Porsolt habituation session on the day before the first 5 min Porsolt test. N = 6 per group. * P < 0.05 Fisher's PLSD post hoc test vs. all other groups. The results demonstrate that the AMPA antagonist NBQX blocks the antidepressant-like effect of CM-4A.

Example 21 - Porsolt test procedure 2

This example demonstrates the Porsolt test for assessing test compounds for antidepressant activity.

The antidepressant-like effects of test compounds were examined with the rat Porsolt test as described in Page et al. (1999) and Burgdorf et al. (2009). Male Sprague Dawley rats (2-3 month old) were used in this study. Animals were dosed with test compounds (0.3 mg/kg IV) or sterile saline vehicle (1 ml / kg IV tail vein) 1 hr before a single test session. A second group of male Sprague Dawley rats (2-3 month old) were dosed with test compounds (0.1 mg/kg PO) or sterile saline vehicle (1 ml / kg PO gastric gavage) 1 hr before the first test session, and re-tested 24 hrs post-dosing. Animals were placed in a 46 cm tall x 20 cm in diameter clear plastic tube filled to 30 cm with tap water at room temperature (22-23 °C) for 15 min 1 day before dosing (habituation) and 5 min on the subsequent test day(s). Water was changed after every other animal. Animals were videotaped and total duration (sec) of floating behavior was quantified by a blind experimenter. CM-6A, CM-7A, CM-8A, and CM-9A were tested in the Porsolt test. CM-5A was not tested in the Porsolt test.

As shown in FIG. 11A, CM-6A, CM-7A, CM-8A, and CM-9A produced an antidepressant-like effect in the Porsolt test as indexed by reduced floating time following a single dose (0.3 mg/kg IV) as compared to vehicle 1 hr post-dosing as indexed by a significant Drug effect ANOVA ($F(2, 24) = 17.5$, $P < 0.0001$), followed by a significant Fisher's PLSD post hoc for each test compound vs. vehicle group (* all P's <0.0001). N = 5-6 rats per group.

As shown in FIGs. 11B and 11C, CM-6A, CM-7A, CM-8A, and CM-9A produced an antidepressant-like effect in the Porsolt test as indexed by reduced floating time following a single dose (0.3 mg/kg IV) as compared to vehicle at 1 hr (FIG. 11B) and 24 hrs (FIG. 11C) post-dosing as indexed by a significant Drug effect repeated measures ANOVA ($F(4, 25) = 58.2$, $P < 0.0001$), followed by a significant Fisher's PLSD post hoc test for test compound at each time point vs. the respective vehicle group (* all P's < 0.0001). n = 6 rats per group.

As shown in FIG. 12, robust Antidepressant-like effects were observed in the rat Porsolt test Mean ± SEM floating time in the rat Porsolt test in 2-3 month old male SD rats dosed with positive control CM-4A (1 mg/kg PO), test compounds (0.1 mg/kg PO) or sterile saline vehicle (1 ml/kg PO gastric gavage) at 1 hr before the testing and 24 hrs post dosing. Animals received a single 15 min Porsolt habituation session on the day before the first 5 min Porsolt test. N = 6 per group. * $P < .05$ Fisher's PLSD post hoc test vs. vehicle.

Example 22 - Rat novelty-induced hypophagia (NIH) test

The rat NIH test was conducted as follows. Animals were food deprived overnight before testing, and lab chow was placed into the center chamber of the open field. After NIH testing, the latency to eat in the animals' home cage was determined as a control. Acute acting antidepressants as well as chronic but not acute SSRI treatment decreased eating latency in the novel cage but not the animals home cage. FIG. 13 shows the Mean ± SEM line latency to eat in the NIH test of 2-3 month old male SD rats treated with CM-4A (1 mg/kg PO) or sterile saline vehicle (1 ml/kg PO) 1 hr before a single 10 min test session. N = 12 per group. * $P < 0.05$. CM-4A (1 mg/kg PO) produces an antidepressant /anxiolytic-like effect in the rat NIH test.

Example 23 - Rat ultrasonic vocalizations (USVs) test

FIGs. 14A and 14B show the results of the rat USVs test. Positive emotional learning was measured during the conditioned stimulus (CS) trials preceding the tickle unconditioned stimulus (UCS) trials (FIG. 14A). Animals received 15 second trials consisting of 6 CS and 6 UCS trials each (3 min total). Running speed for animals to self administer tickling at the end of the 3 min session was also measured. FIG. 14B shows the Mean ± SEM hedonic and aversive USVs in the rat USVs test in 2-3 month old male SD rats treated with CM-4A (1 mg/kg PO) or sterile saline vehicle (1 ml/kg PO) 1 hr before testing. N = 7-8. * $P < 0.05$. The results demonstrate that CM-4A increases positive emotional learning and decreases aversive 20-kHz USVs in the rat USVs test, thereby indicating an antidepressant effect.

Example 24 - Rat open field test

FIG. 15 shows Mean ± SEM line crosses, center compartment crosses, and center compartment time in 2-3 month old male SD rats treated with CM-4A (1 mg/kg PO) or sterile saline vehicle (1 ml/kg PO) 1 hr before a single 10 min test session in the open field. N = 7-8. * $P < 0.05$. The results demonstrate that CM-4A produces an anxiolytic-like effect in the rat open field test without effecting locomotor behavior.

Example 24 - Rat accelerating rota-rod test

FIG. 16 shows Mean ± SEM fall latencies (sec) in the accelerating rota-rod test (4-40 RPM across 300 sec) in 2-3 month old male SD rats pretreated with saline vehicle (1 ml/kg PO), CM-4A (1, 10, or 100 mg/kg PO), or the

positive control ketamine (30 mg/kg SC). Animals were tested immediately before dosing (0 min), 30 min post-dosing, and 60 min post-dosing using a within subjects design. One day before testing, animals received 3 rota-rod habituation sessions with at least 30 min between each session, and rota-rod performance on the last habituation session did not significantly differ from the 0 min timepoint during testing. N =5 for vehicle and CM-4A doses, N = 3 for ketamine. * $P < 0.05$. The results demonstrate that CM-4A (10-100 mg/kg PO) does not produce a sedative / ataxia - like effect in the rat accelerating rota-rod test.

## Claims

1. A compound represented by:

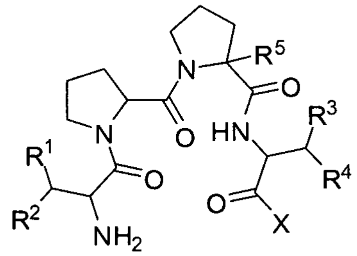

wherein:

$R^1$ is selected from the group consisting of hydrogen, -OH, $C_1$-$C_6$alkyl, and halogen;
$R^2$ and $R^4$ are each independently selected from the group consisting of $C_{1-6}$alkyl, hydrogen, halogen, and -OH;
$R^3$ is selected from the group consisting of -OH, hydrogen, $C_1$-$C_6$alkyl, and halogen;
$R^5$ is -$CH_2$-phenyl (wherein the phenyl is optionally substituted by one, two, or three substituents selected from the group consisting of halogen, $C_{1-3}$alkoxy, and $C_{1-3}$alkyl (optionally substituted by one, two or three halogens)); and
X is selected from the group consisting of $OR^x$ and $NR^xR^x$, wherein $R^X$ is independently selected, for each occurrence, from the group consisting of hydrogen, and $C_1$-$C_6$alkyl; pharmaceutically acceptable salts, stereoisomers, and hydrates thereof.

2. The compound of claim 1, wherein the compound is represented by:

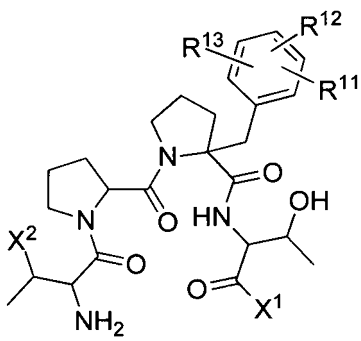

wherein

$R^{11}$, $R^{12}$ and $R^{13}$ are each independently selected from the group consisting of H, halogen, $C_{1-3}$alkoxy, and $C_{1-3}$alkyl (optionally substituted by one, two or three halogens);
$X^1$ is OH or $NH_2$,
$X^2$ is H or OH;

and pharmaceutically acceptable salts, stereoisomers and hydrates thereof.

3. The compound of claim 2, wherein the halogen, for each occurrence, is independently selected from the group

consisting of Cl, Br, and F.

4. The compound of claim 2 or 3, wherein the compound is substantially more efficacious when administered orally to a patient as compared to oral administration to a patient of a peptidyl compound represented by:

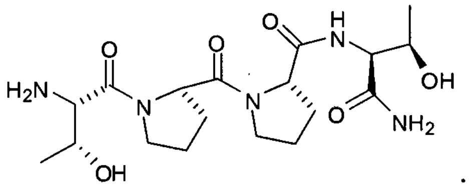

.

5. The compound of any one of claims 2-4, wherein $X^2$ is OH and $X^1$ is $NH_2$.

6. The compound of any one of claims 2-5, wherein each of $R^{11}$, $R^{12}$, and $R^{13}$ is H.

7. The compound of any one of claims 2-6, wherein $R^{11}$ and $R^{13}$ is H, and $R^{12}$ is selected from the group consisting of F, Br, Cl, $CH_3$, $CF_3$, and $-OCH_3$.

8. The compound of claim 1 represented by:

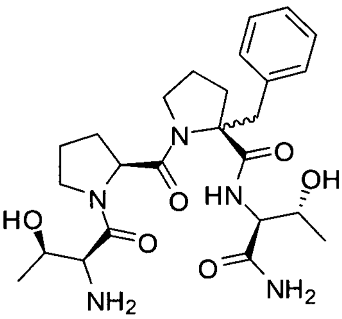

or

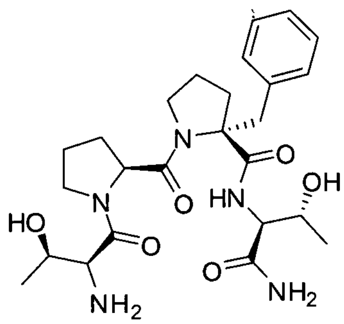

.

9. The compound of claim 1 represented by:

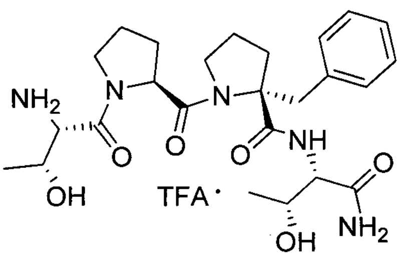

,

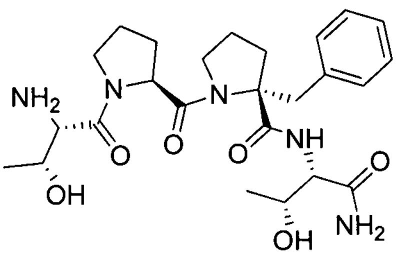

,

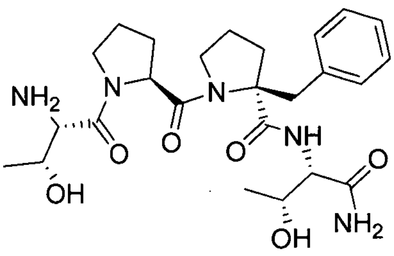

,

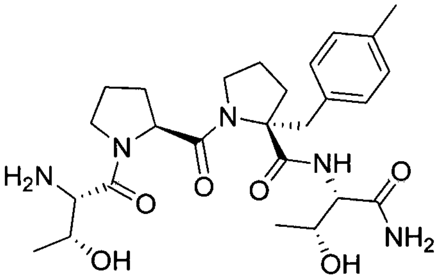

,

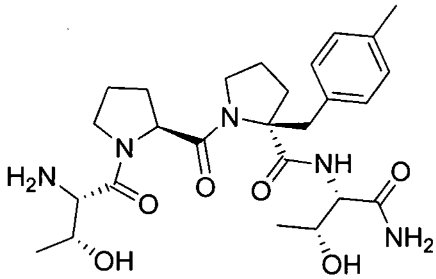

,

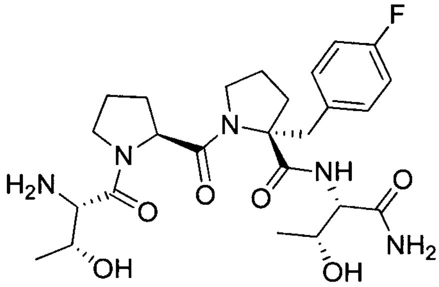,
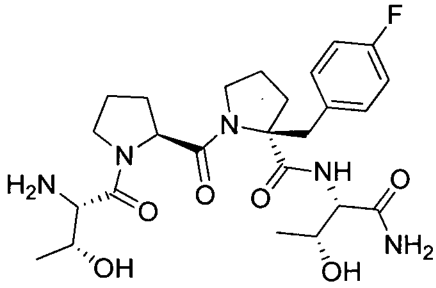,

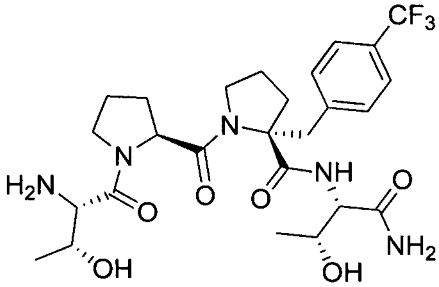,
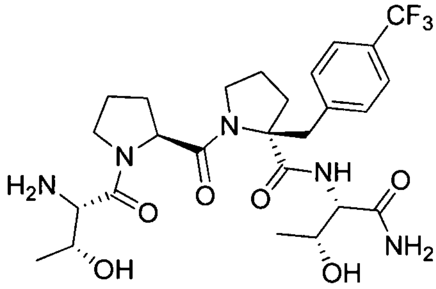,

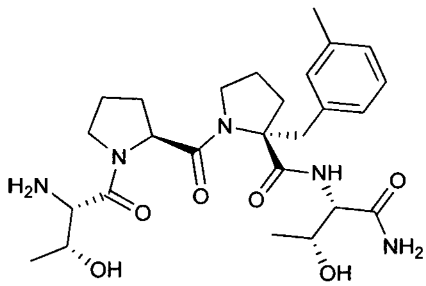,
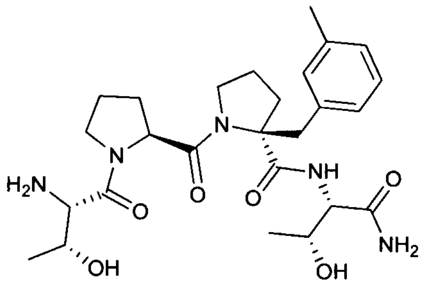,

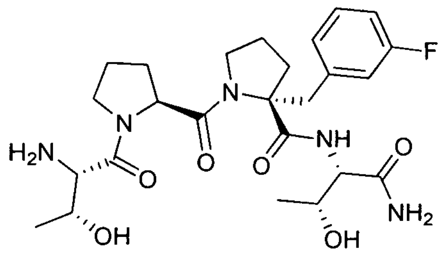,
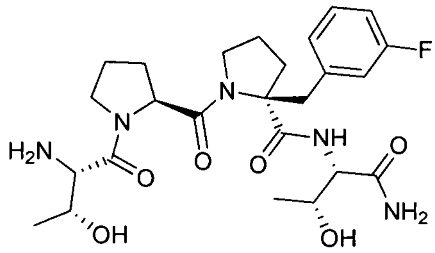,

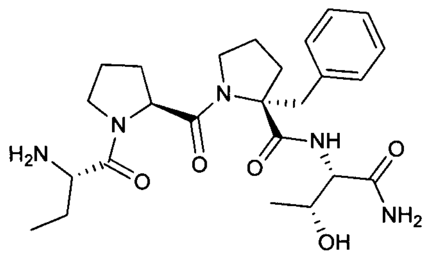,
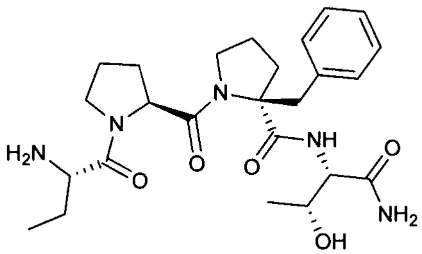,

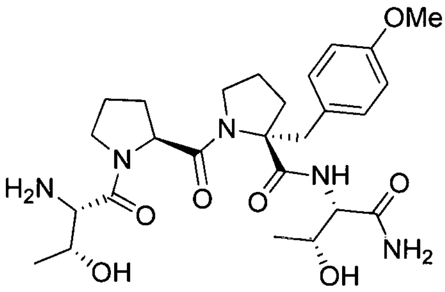, 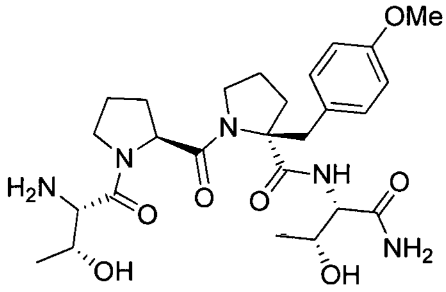,

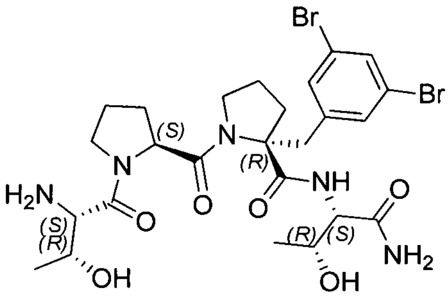, 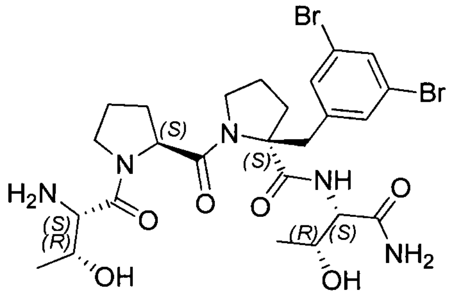,

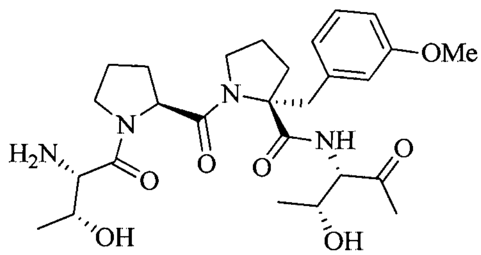, 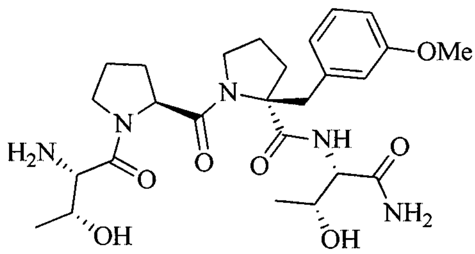,

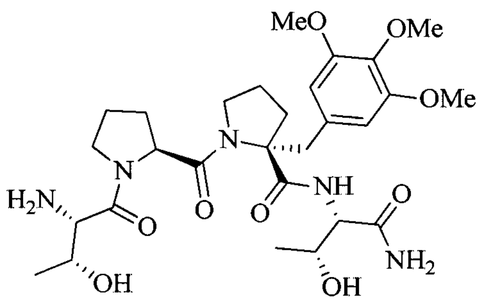, or 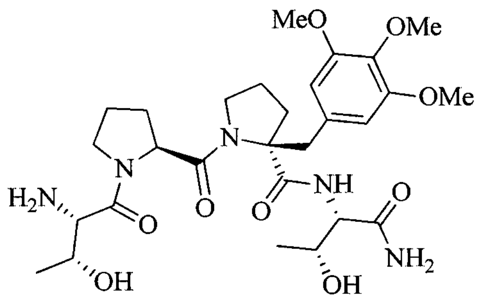.

**10.** A pharmaceutical composition, comprising:
a therapeutically effective amount of a compound of any one of claims 1-9 and a pharmaceutically acceptable carrier, optionally wherein the pharmaceutical composition is suitable for injection.

**11.** A compound of any one of claims 1-9 for use in a method of treating depression, Alzheimer's disease, attention deficit disorder, ADHD, schizophrenia, or anxiety, in a patient in need thereof.

**12.** A compound of any one of claims 2-7 for use in a method of treating depression, Alzheimer's disease, attention deficit disorder, ADHD, schizophrenia, or anxiety, in a patient in need thereof by oral administration.

**13.** A compound of any one of claims 8-9 for use in a method of treating depression, Alzheimer's disease, attention deficit disorder, ADHD, schizophrenia, or anxiety, in a patient in need thereof by subcutaneous or intravenous administration.

**Patentansprüche**

1. Verbindung, dargestellt durch:

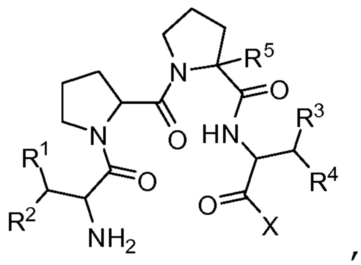

,

worin:

$R^1$ aus der Gruppe bestehend aus Wasserstoff, -OH, $C_{1-6}$-Alkyl und Halogen ausgewählt ist;
$R^2$ und $R^4$ jeweils unabhängig aus der Gruppe bestehend aus $C_{1-6}$-Alkyl, Wasserstoff, Halogen und -OH ausgewählt sind;
$R^3$ aus der Gruppe bestehend aus -OH, Wasserstoff, $C_{1-6}$-Alkyl und Halogen ausgewählt ist;
$R^5$ -$CH_2$-Phenyl ist (wobei das Phenyl gegebenenfalls mit einem, zwei oder drei Substituenten, ausgewählt aus der Gruppe bestehend aus Halogen, $C_{1-3}$-Alkoxy und $C_{1-3}$-Alkyl (gegebenenfalls substituiert mit einer, zwei oder drei Halogengruppen) substituiert ist); und
X aus der Gruppe bestehend aus $OR^x$ und $NR^xR^x$ ausgewählt ist, worin $R^X$ bei jedem Auftreten unabhängig aus der Gruppe bestehend aus Wasserstoff und $C_{1-6}$-Alkyl ausgewählt ist;
pharmazeutisch annehmbare Salze, Stereoisomere und Hydrate davon.

2. Verbindung gemäss Anspruch 1, wobei die Verbindung durch:

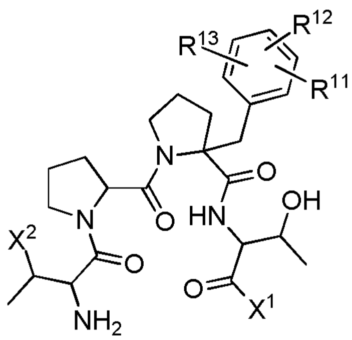

dargestellt ist, worin

$R^{11}$, $R^{12}$ und $R^{13}$ jeweils unabhängig aus der Gruppe bestehend aus H, Halogen, $C_{1-3}$-Alkoxy und $C_{1-3}$-Alkyl (gegebenenfalls substituiert mit einer, zwei oder drei Halogengruppen) ausgewählt sind;
$X^1$ OH oder $NH_2$ ist,
$X^2$ H oder OH ist;
und pharmazeutisch annehmbare Salze, Stereoisomere und Hydrate davon.

3. Verbindung gemäss Anspruch 2, wobei das Halogen bei jedem Auftreten unabhängig aus der Gruppe bestehend aus Cl, Br und F ausgewählt ist.

4. Verbindung gemäss Anspruch 2 oder 3, wobei die Verbindung wesentlich wirksamer ist, wenn sie einem Patienten oral verabreicht wird, verglichen mit der oralen Verabreichung einer Peptidylverbindung an einen Patienten, dargestellt durch:

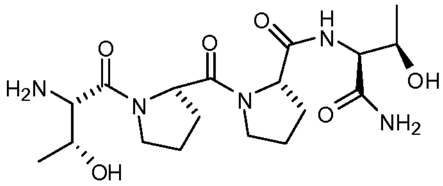

.

5. Verbindung gemäss irgendeinem der Ansprüche 2 bis 4, worin $X^2$ OH ist und $X^1$ $NH_2$ ist.

6. Verbindung gemäss irgendeinem der Ansprüche 2 bis 5, worin jedes $R^{11}$, $R^{12}$ und $R^{13}$ H ist.

7. Verbindung gemäss irgendeinem der Ansprüche 2 bis 6, worin $R^{11}$ und $R^{13}$ H sind und $R^{12}$ aus der Gruppe bestehend aus F, Br, Cl, $CH_3$, $CF_3$ und $-OCH_3$ ausgewählt ist.

8. Verbindung gemäss Anspruch 1, dargestellt durch:

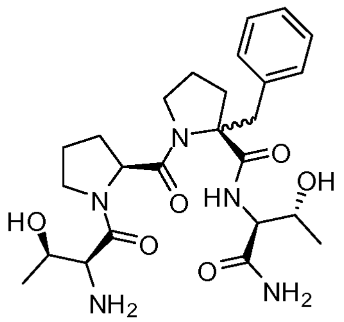

oder

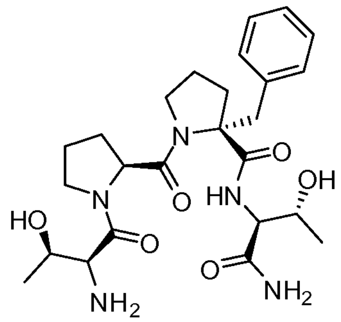

.

9. Verbindung gemäss Anspruch 1, dargestellt durch:

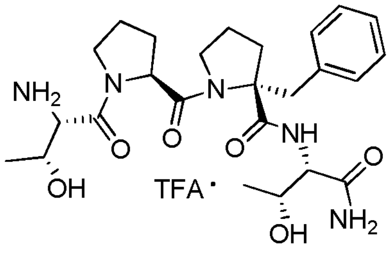

,

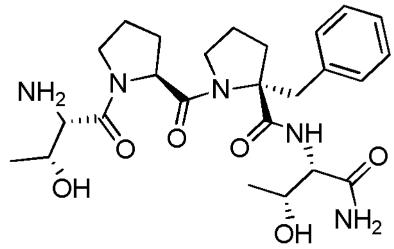

,

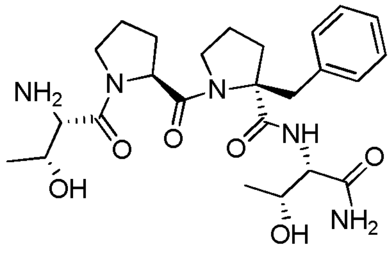

,

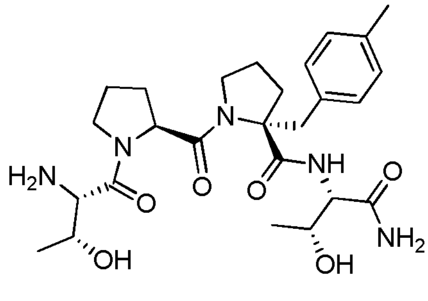

,

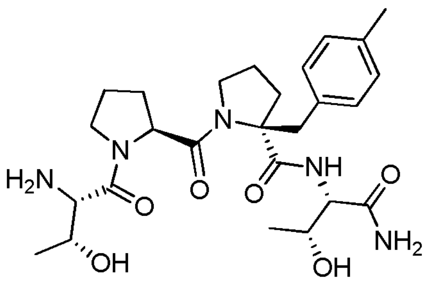 ,

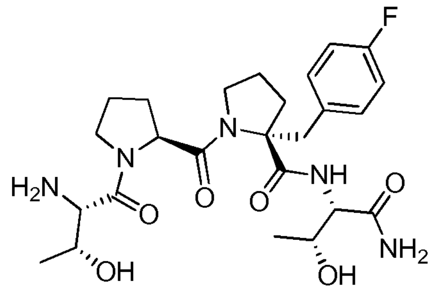 ,

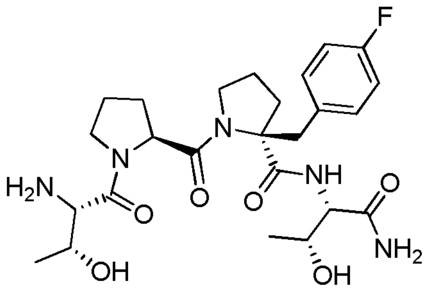 ,

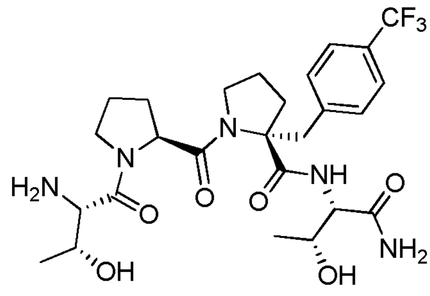 ,

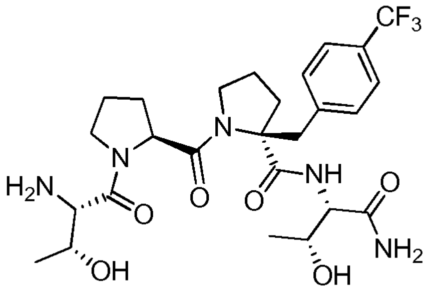 ,

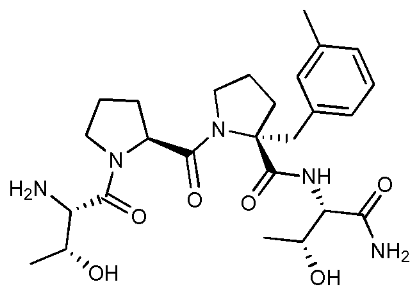 ,

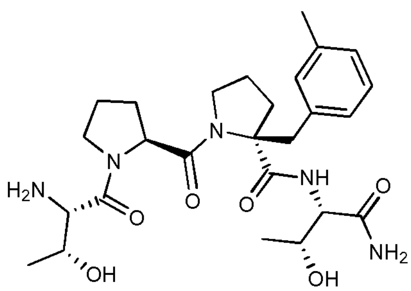 ,

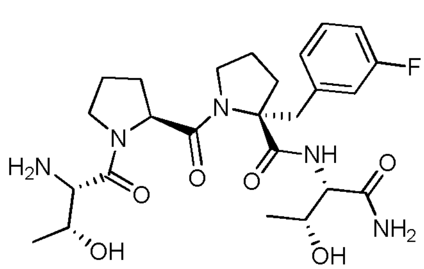 ,

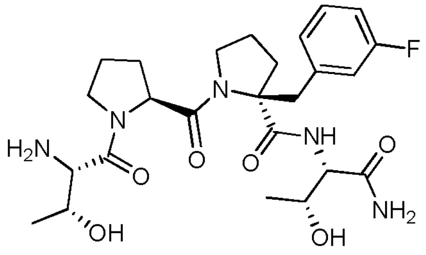 ,

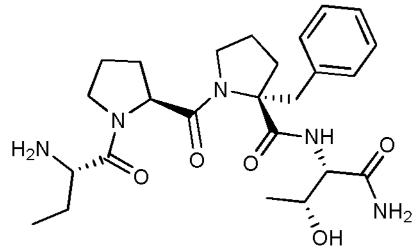 ,

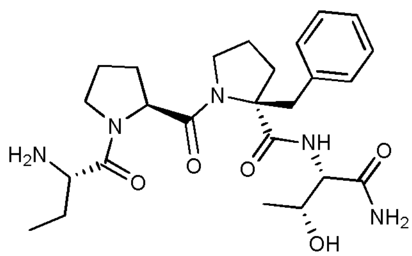

,

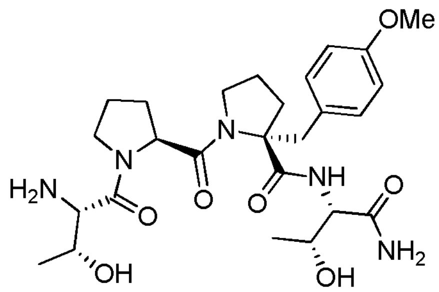

,

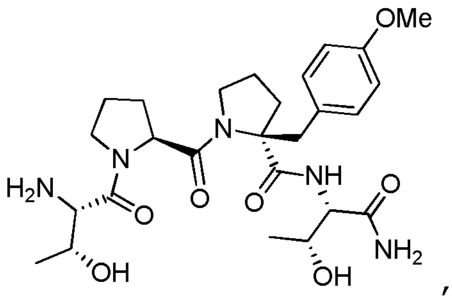

,

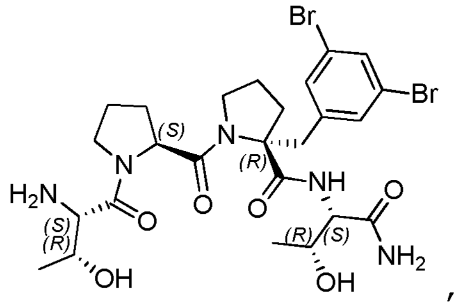

,

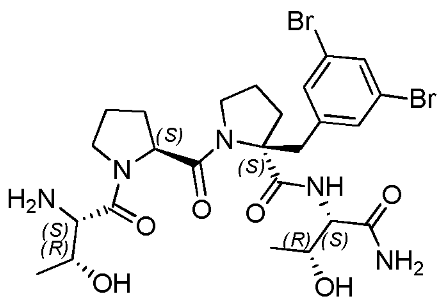

,

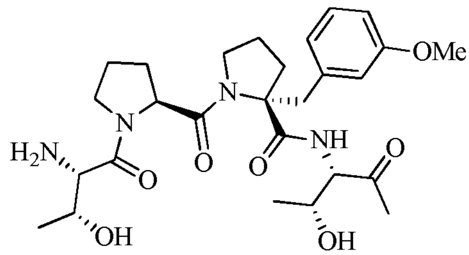

,

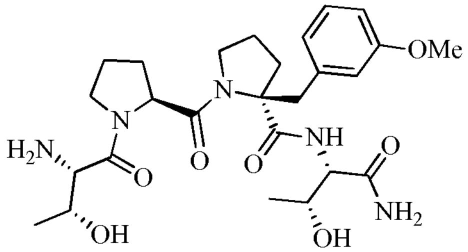

,

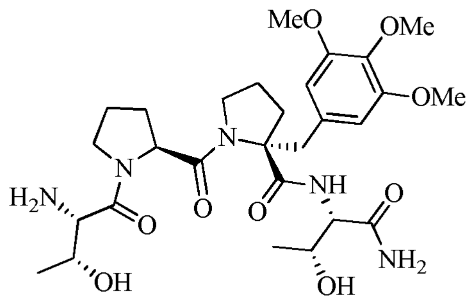

oder

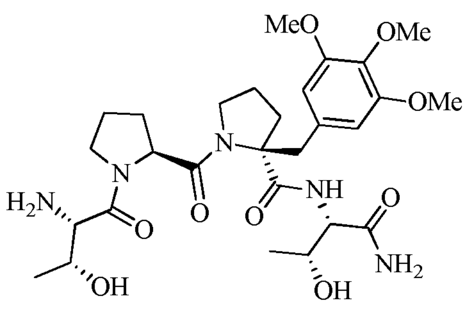

.

**10.** Pharmazeutische Zusammensetzung, die eine therapeutisch wirksame Menge einer Verbindung gemäss irgendeinem der Ansprüche 1 bis 9 und einen pharmazeutisch annehmbaren Träger umfasst, wobei gegebenenfalls die pharmazeutische Zusammensetzung für Injektionen geeignet ist.

**11.** Verbindung gemäss irgendeinem der Ansprüche 1 bis 9 zur Verwendung in einem Verfahren zur Behandlung einer Depression, Alzheimer-Krankheit, Aufmerksamkeitsdefizitstörung, ADHD, Schizophrenie oder Angst bei einem Patienten, der dies benötigt.

**12.** Verbindung gemäss irgendeinem der Ansprüche 2 bis 7 zur Verwendung in einem Verfahren zur Behandlung einer Depression, Alzheimer-Krankheit, Aufmerksamkeitsdefizitstörung, ADHD, Schizophrenie oder Angst bei einem Patienten mit Bedarf daran, durch orale Verabreichung.

**13.** Verbindung gemäss irgendeinem der Ansprüche 8 bis 9 zur Verwendung in einem Verfahren zur Behandlung einer Depression, Alzheimer-Krankheit, Aufmerksamkeitsdefizitstörung, ADHD, Schizophrenie oder Angst bei einem Patienten mit Bedarf daran, durch subkutane oder intravenöse Verabreichung.

## Revendications

**1.** Composé représenté par :

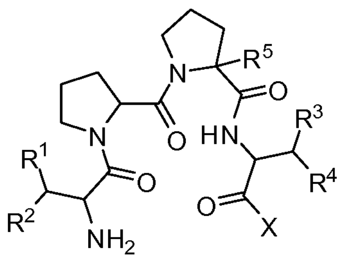

dans lequel :

$R^1$ est choisi parmi le groupe constitué de hydrogène, -OH, $C_1$-$C_6$ alkyle, et halogène;
$R^2$ et $R^4$ sont chacun indépendamment choisis parmi le groupe constitué de $C_{1-6}$ alkyle, hydrogène, halogène, et -OH;
$R^3$ est choisi parmi le groupe constitué de -OH, hydrogène, $C_1$-$C_6$ alkyle, and halogène;
$R^5$ est -$CH_2$-phényle (dans lequel le phényle est optionnellement substitué par un, deux ou trois substituants choisis parmi le groupe constitué de halogène, $C_{1-3}$ alkoxy, et $C_{1-3}$ alkyle (optionnellement substitué par un, deux ou trois halogènes)); et
X est choisi parmi le groupe constitué de $OR^x$ et $NR^xR^x$, dans lesquels $R^X$ est indépendamment choisi, pour chaque occurrence, parmi le groupe constitué de hydrogène, et $C_1$-$C_6$ alkyle;

des hydrates, stéréo-isomères, et sels pharmaceutiquement acceptables de celui-ci.

**2.** Le composé de la revendication 1, dans lequel le composé est représenté par :

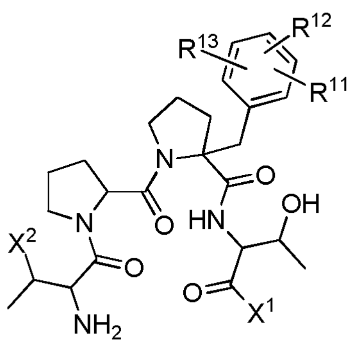

dans lequel

$R^{11}$, $R^{12}$ et $R^{13}$ sont chacun indépendamment choisis parmi le groupe constitué de H, halogène, $C_{1-3}$ alkoxy, et $C_{1-3}$ alkyle (optionnellement substitué par un, deux ou trois halogènes);
$X^1$ est OH ou $NH_2$,
$X^2$ est H ou OH;

et des hydrates, stéréo-isomères, et sels pharmaceutiquement acceptables de celui-ci.

**3.** Le composé de la revendication 2, dans lequel l'halogène, pour chaque occurrence, est indépendamment choisi parmi le groupe constitué de Cl, Br, et F.

**4.** Le composé de la revendication 2 ou 3, dans lequel le composé est substantiellement plus efficace lorsqu'administré oralement à un patient comparé à l'administration orale à un patient d'un composé peptidyle représenté par :

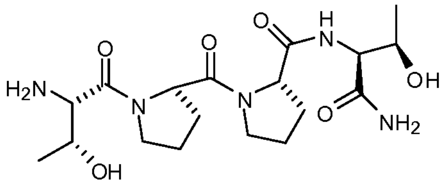
.

**5.** Le composé de l'une quelconque des revendications 2-4, dans lequel $X^2$ est OH et $X^1$ est $NH_2$.

**6.** Le composé de l'une quelconque des revendications 2-5, dans lequel chaque $R^{11}$, $R^{12}$, et $R^{13}$ est H.

**7.** Le composé de l'une quelconque des revendications 2-6, dans lequel $R^{11}$ et $R^{13}$ est H, et $R^{12}$ est choisi parmi le groupe constitué de F, Br, Cl, $CH_3$, $CF_3$, et $-OCH_3$.

**8.** Le composé de la revendication 1 représenté par :

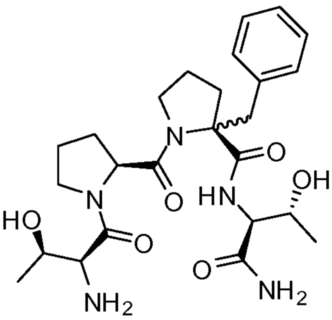
ou
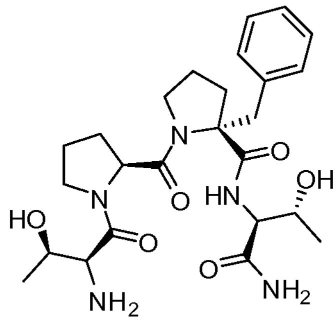
.

**9.** Le composé de la revendication 1 représenté par :

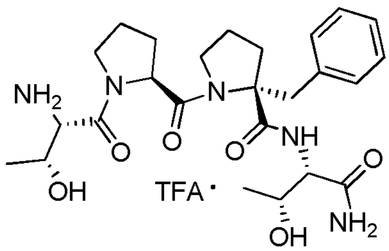
,
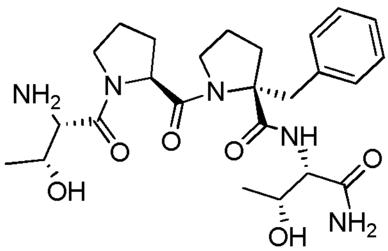
,
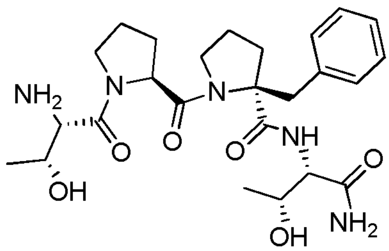
,

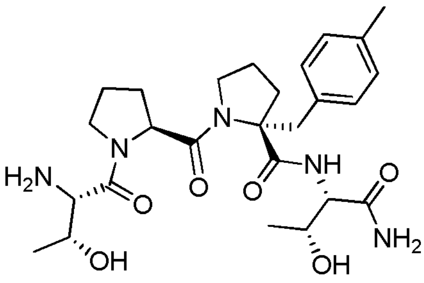,
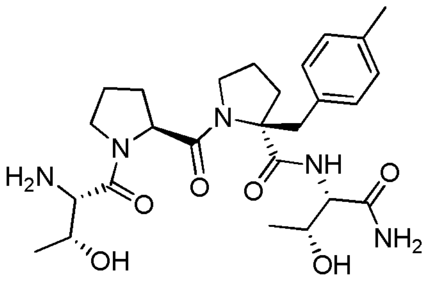,

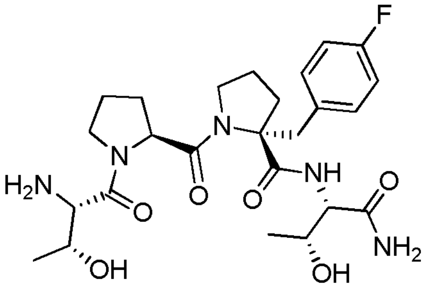,
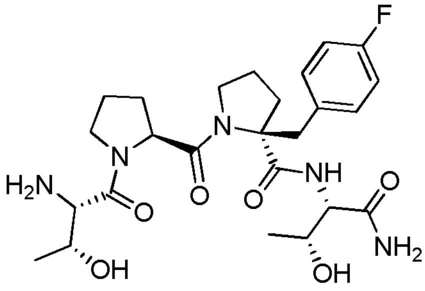,

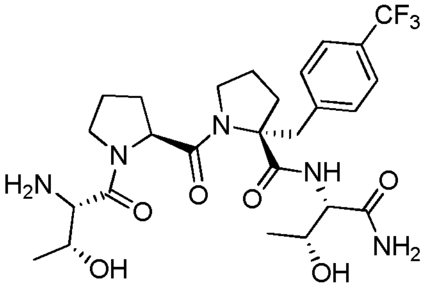,
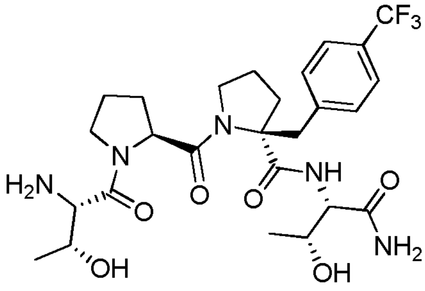,

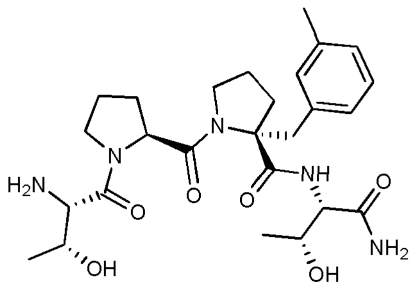,
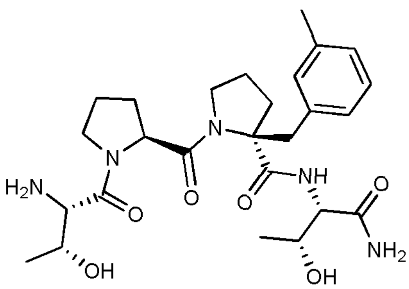,

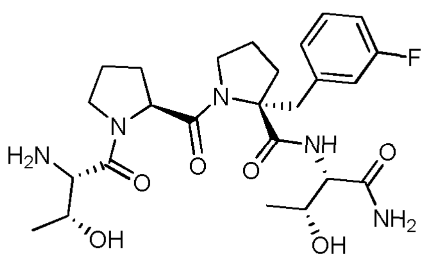,
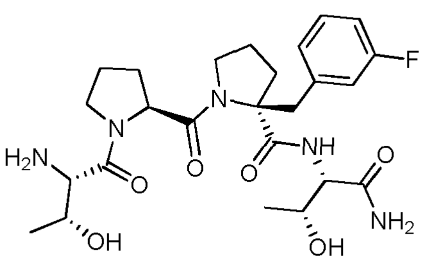,

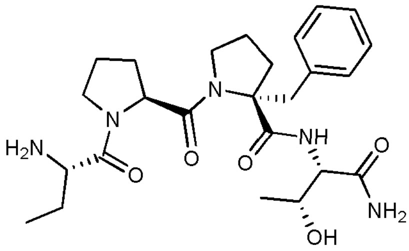
,
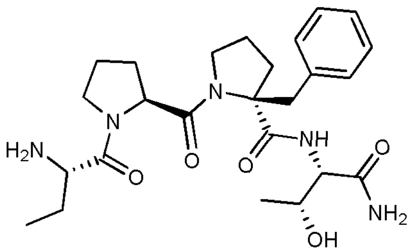
,

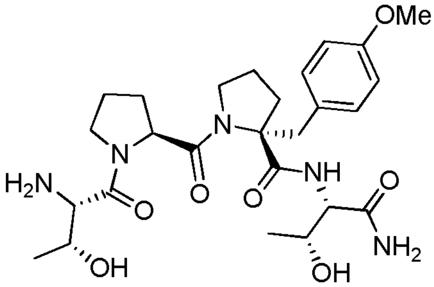
,
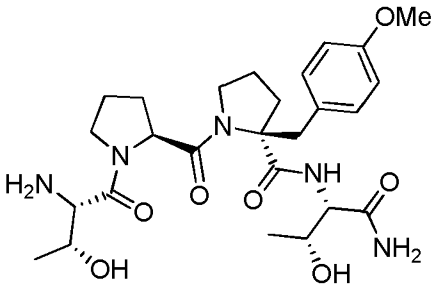
,

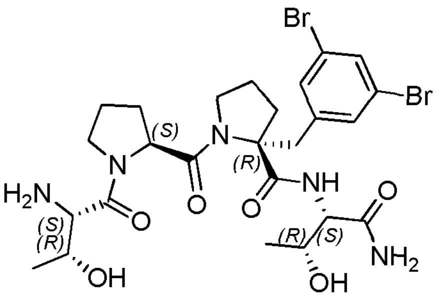
,
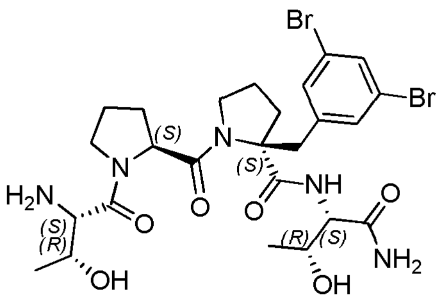
,

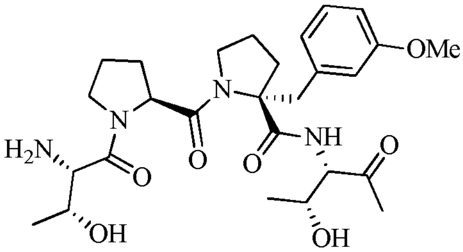
,
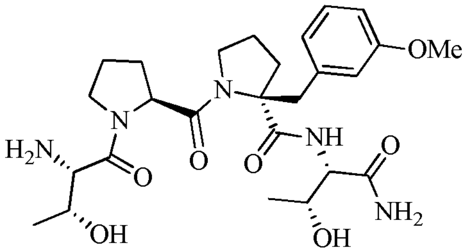
,

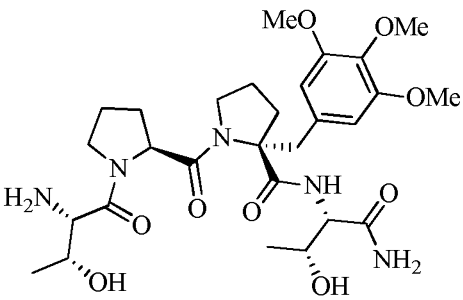
, ou
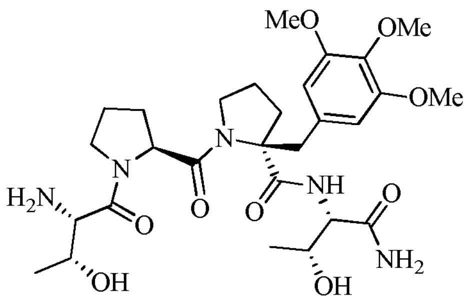
.

**10.** Composition pharmaceutique, comprenant :
une quantité thérapeutiquement efficace d'un composé de l'une quelconque des revendications 1-9 et un support pharmaceutiquement acceptable, optionnellement dans laquelle la composition pharmaceutique est adaptée pour l'injection.

**11.** Composé de l'une quelconque des revendications 1-9 pour l'utilisation dans une méthode de traitement de la dépression, de la maladie d'Alzheimer, d'un trouble de déficit de l'attention, d'un trouble d'hyperactivité avec déficit de l'attention, de la schizophrénie, ou de l'anxiété, dans un patient qui en a besoin.

**12.** Composé de l'une quelconque des revendications 2-7 pour l'utilisation dans une méthode de traitement de la dépression, de la maladie d'Alzheimer, d'un trouble de déficit de l'attention, d'un trouble d'hyperactivité avec déficit de l'attention, de la schizophrénie, ou de l'anxiété, dans un patient qui en a besoin par administration orale.

**13.** Composé de l'une quelconque des revendications 8-9 pour l'utilisation dans une méthode de traitement de la dépression, de la maladie d'Alzheimer, d'un trouble de déficit de l'attention, d'un trouble d'hyperactivité avec déficit de l'attention, de la schizophrénie, ou de l'anxiété, dans un patient qui en a besoin par administration intraveineuse ou sous-cutanée.

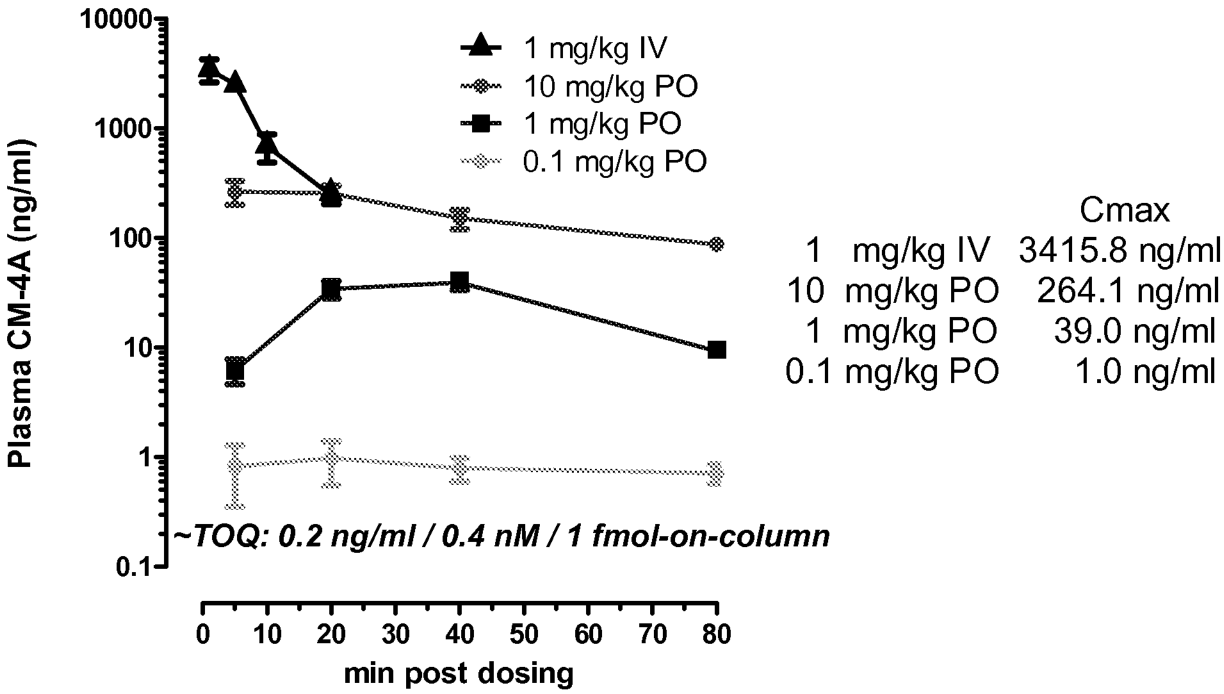
10000
1000
100
10
1
0.1
Plasma CM-4A (ng/ml)
1 mg/kg IV
10 mg/kg PO
1 mg/kg PO
0.1 mg/kg PO
~TOQ: 0.2 ng/ml / 0.4 nM / 1 fmol-on-column
0 10 20 30 40 50 60 70 80
min post dosing
Cmax
1 mg/kg IV 3415.8 ng/ml
10 mg/kg PO 264.1 ng/ml
1 mg/kg PO 39.0 ng/ml
0.1 mg/kg PO 1.0 ng/ml

Fig. 1

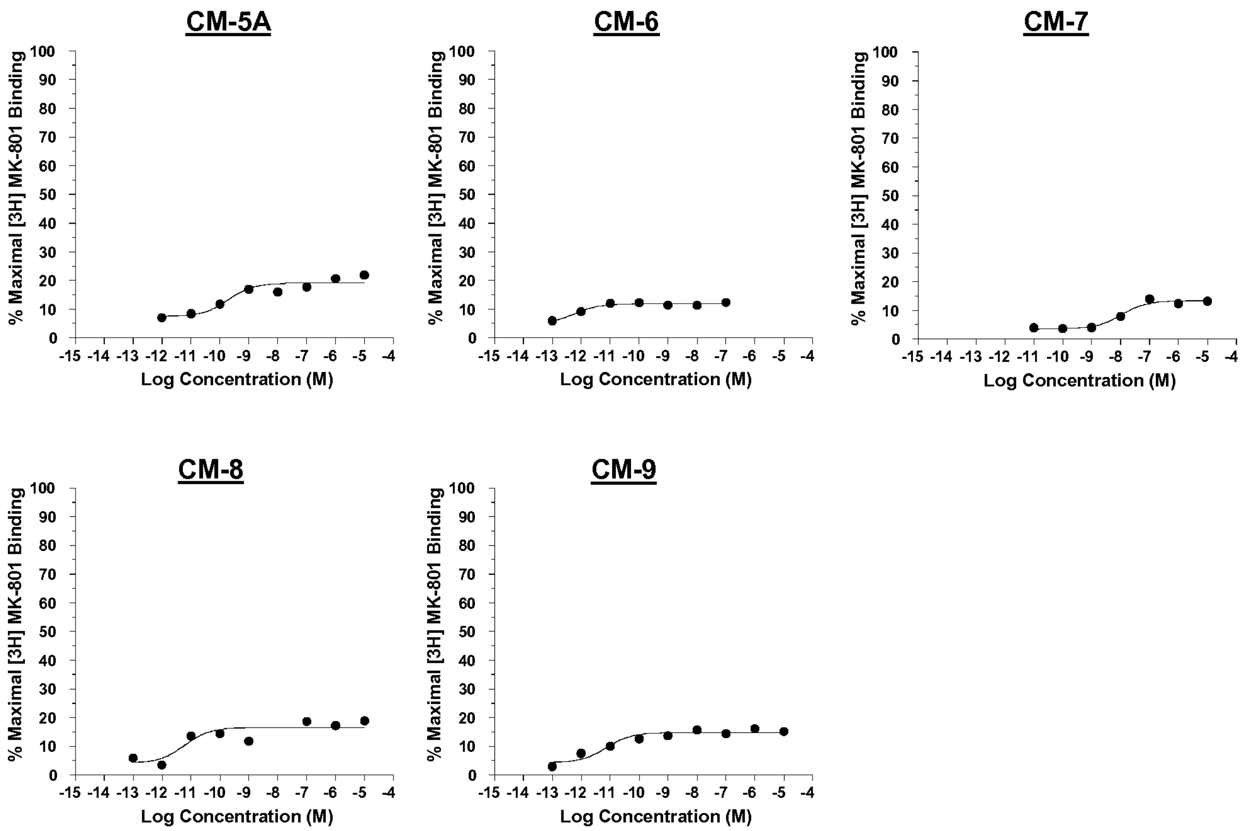
CM-5A
CM-6
CM-7
CM-8
CM-9
% Maximal [3H] MK-801 Binding
Log Concentration (M)
0
10
20
30
40
50
60
70
80
90
100
-15 -14 -13 -12 -11 -10 -9 -8 -7 -6 -5 -4

Fig. 2

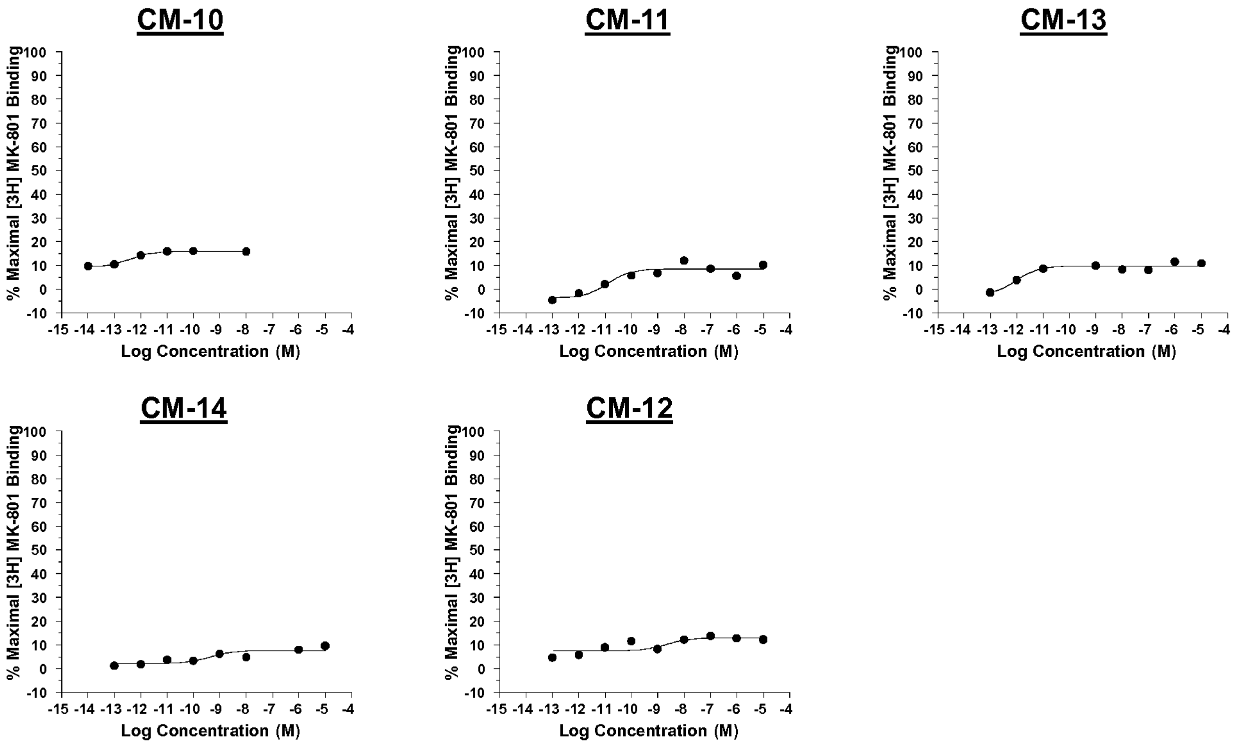
CM-10
CM-11
CM-13
CM-14
CM-12
% Maximal [3H] MK-801 Binding
Log Concentration (M)

Fig. 3

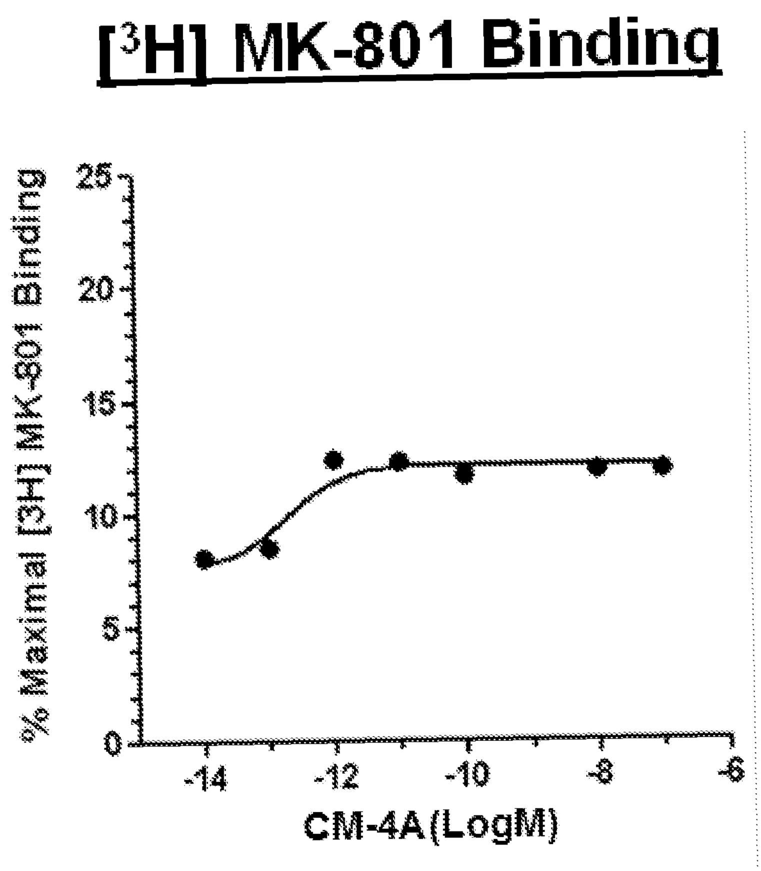
[3H] MK-801 Binding
% Maximal [3H] MK-801 Binding
25
20
15
10
5
0
-14
-12
-10
-8
-6
CM-4A(LogM)

Fig. 4

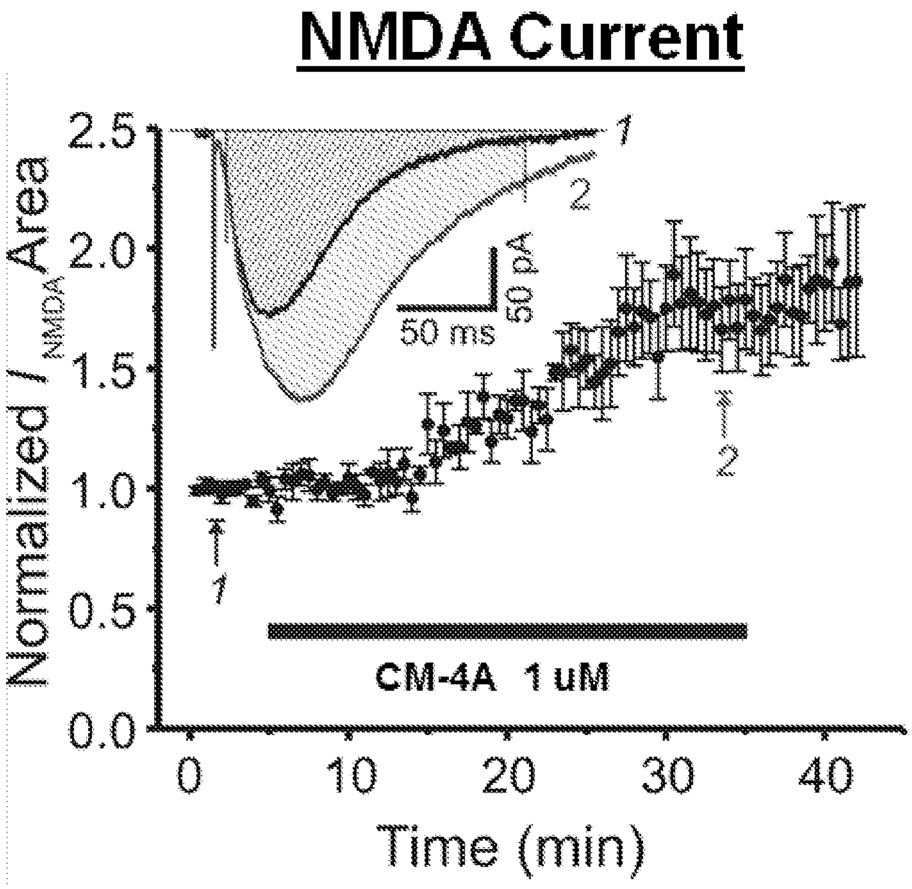
NMDA Current
Normalized $I_{NMDA}$ Area
2.5
2.0
1.5
1.0
0.5
0.0
0
10
20
30
40
Time (min)
1
2
50 ms
50 pA
CM-4A 1 uM

Fig. 5

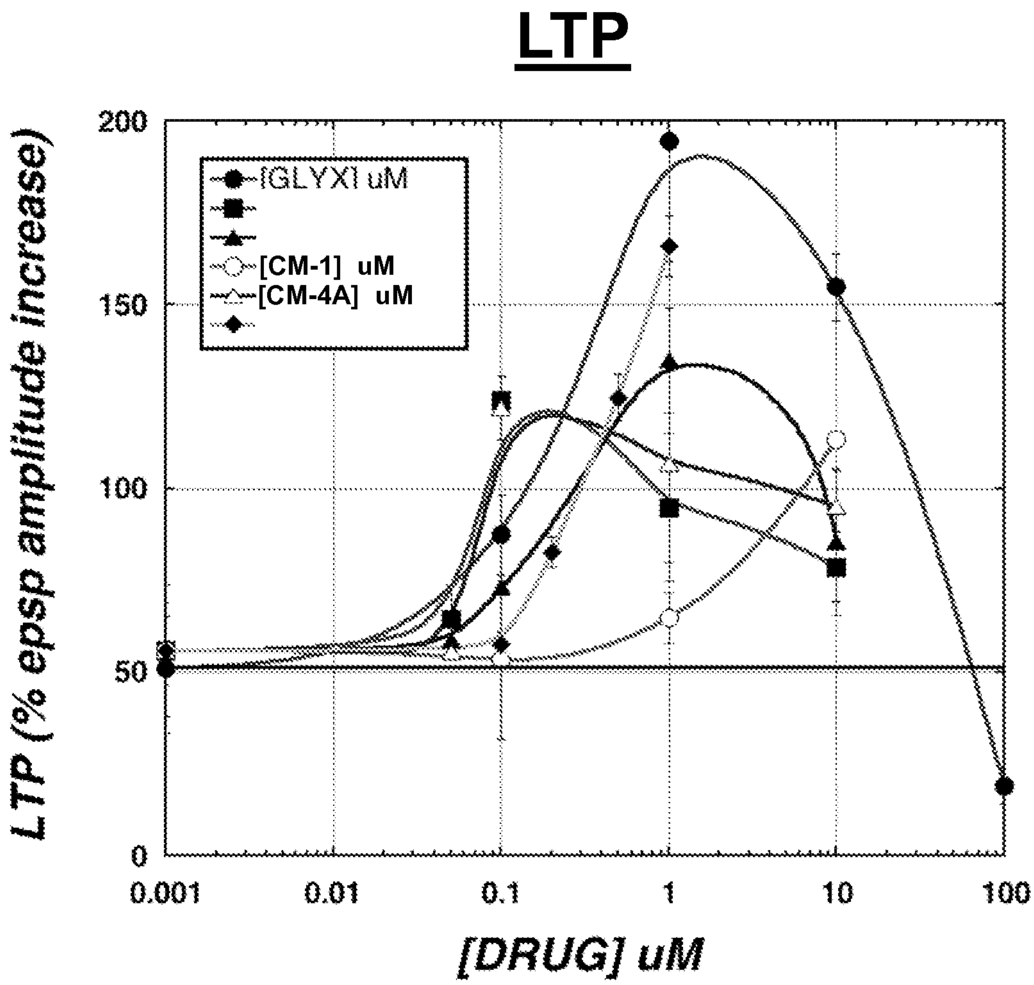
LTP
LTP (% epsp amplitude increase)
200
150
100
50
0
0.001
0.01
0.1
1
10
100
[DRUG] uM
[GLYX] uM
[CM-1] uM
[CM-4A] uM

Fig. 6

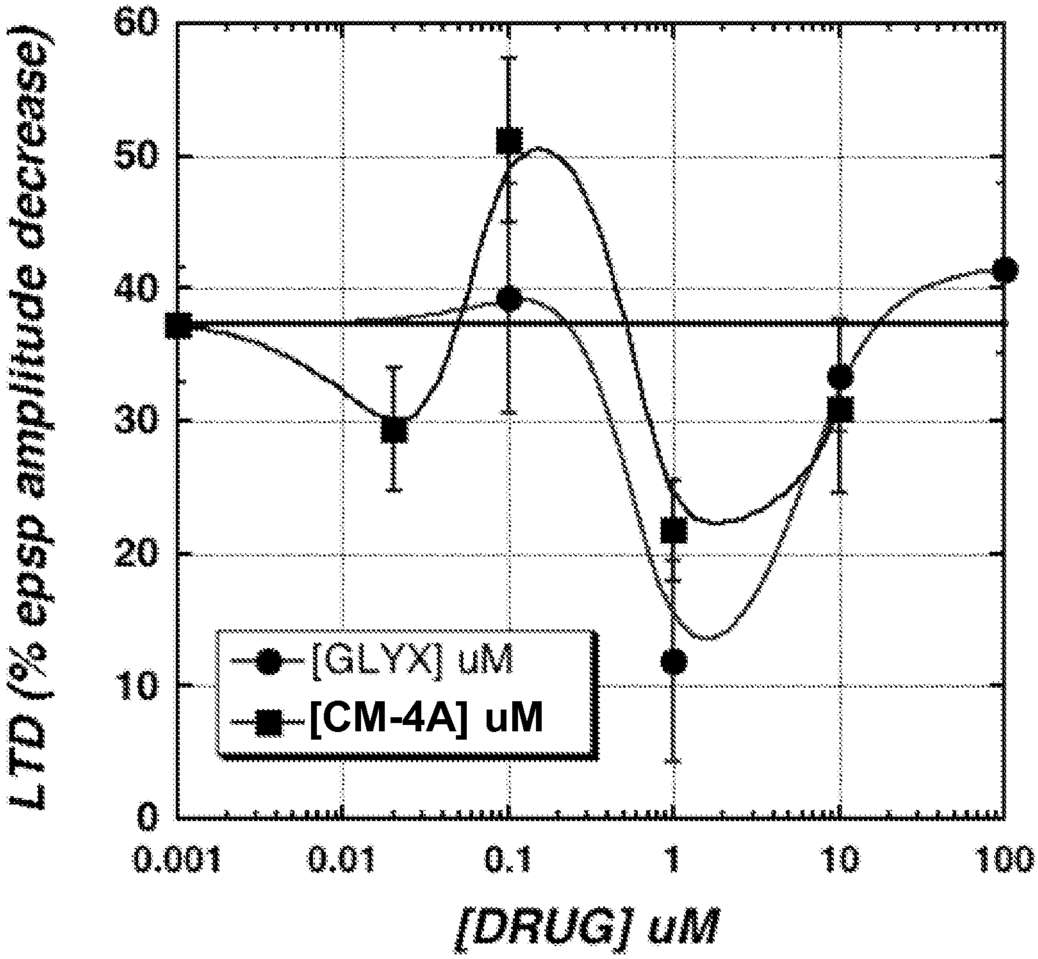
LTD (% epsp amplitude decrease)
60
50
40
30
20
10
0
0.001
0.01
0.1
1
10
100
[DRUG] uM
[GLYX] uM
[CM-4A] uM

Fig. 7

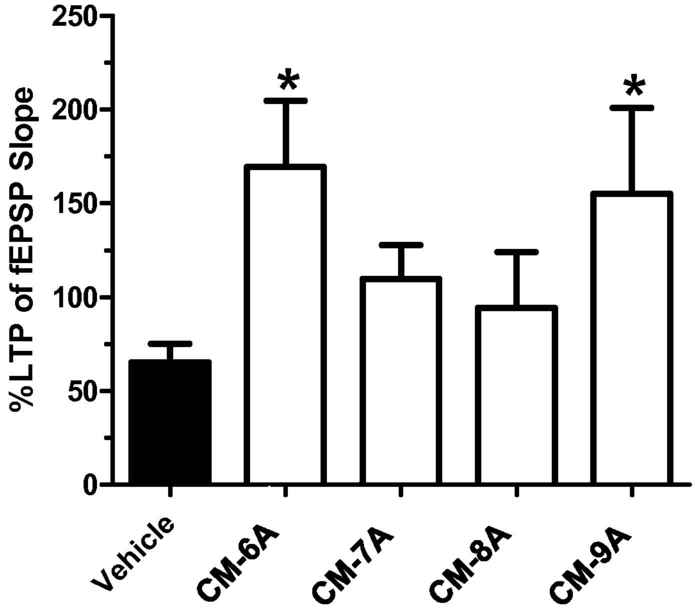
250
200
150
100
50
0
%LTP of fEPSP Slope
*
*
Vehicle
CM-6A
CM-7A
CM-8A
CM-9A

Fig. 8

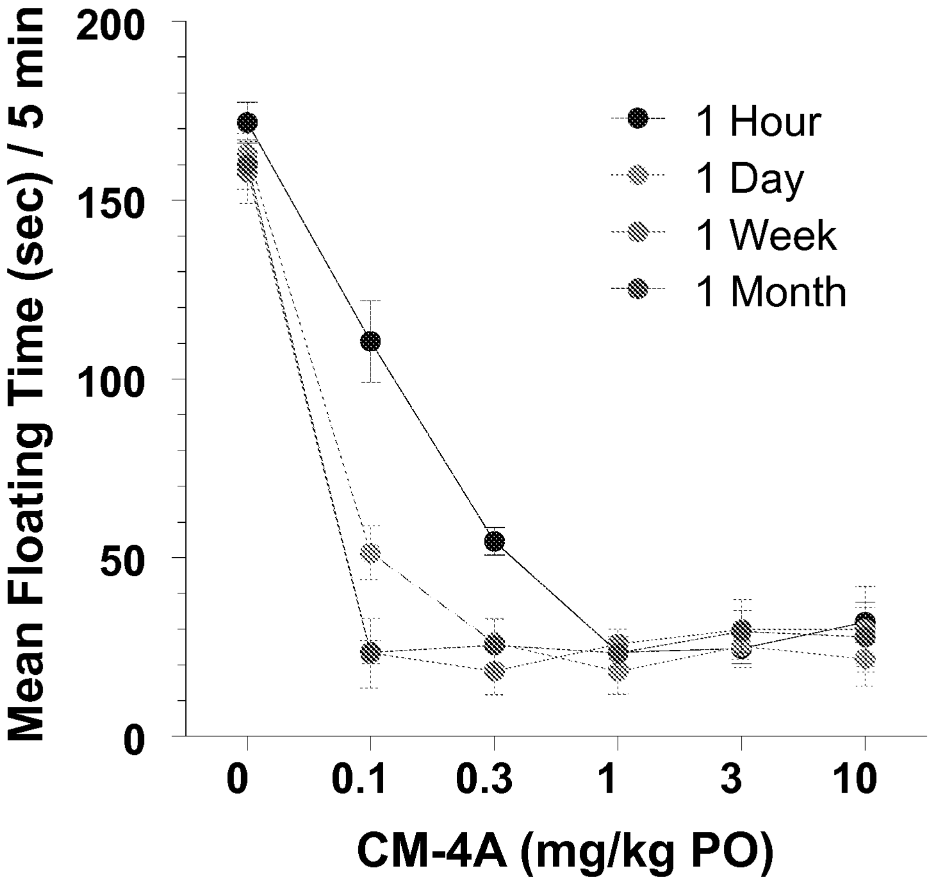
Mean Floating Time (sec) / 5 min
200
150
100
50
0
1 Hour
1 Day
1 Week
1 Month
0
0.1
0.3
1
3
10
CM-4A (mg/kg PO)

Fig. 9

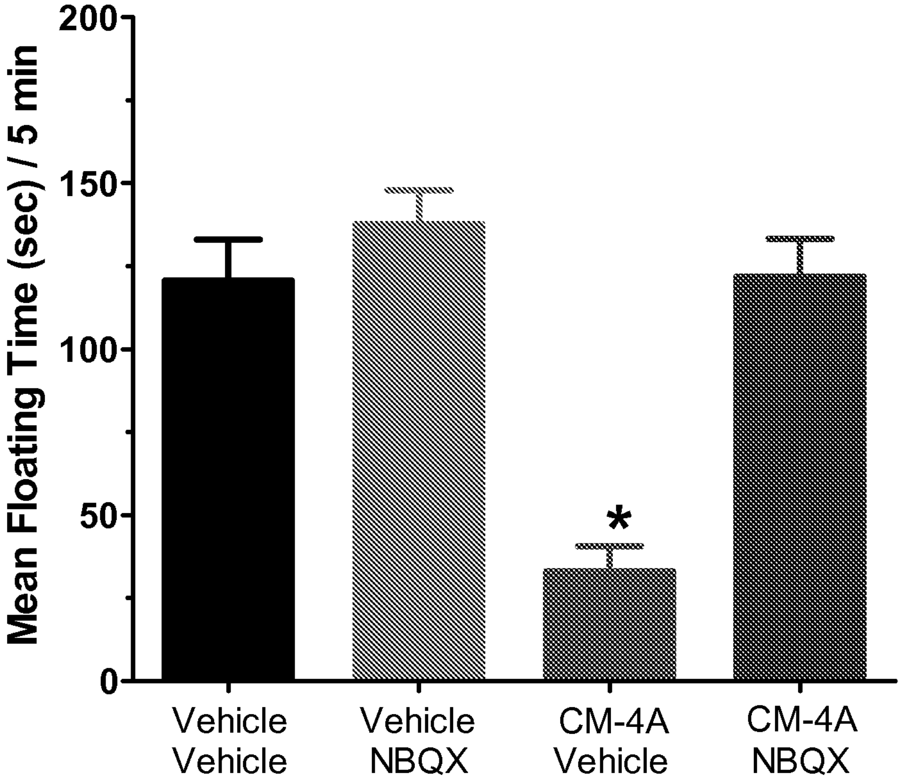
Mean Floating Time (sec) / 5 min
200
150
100
50
0
*
Vehicle Vehicle
Vehicle NBQX
CM-4A Vehicle
CM-4A NBQX

Fig. 10

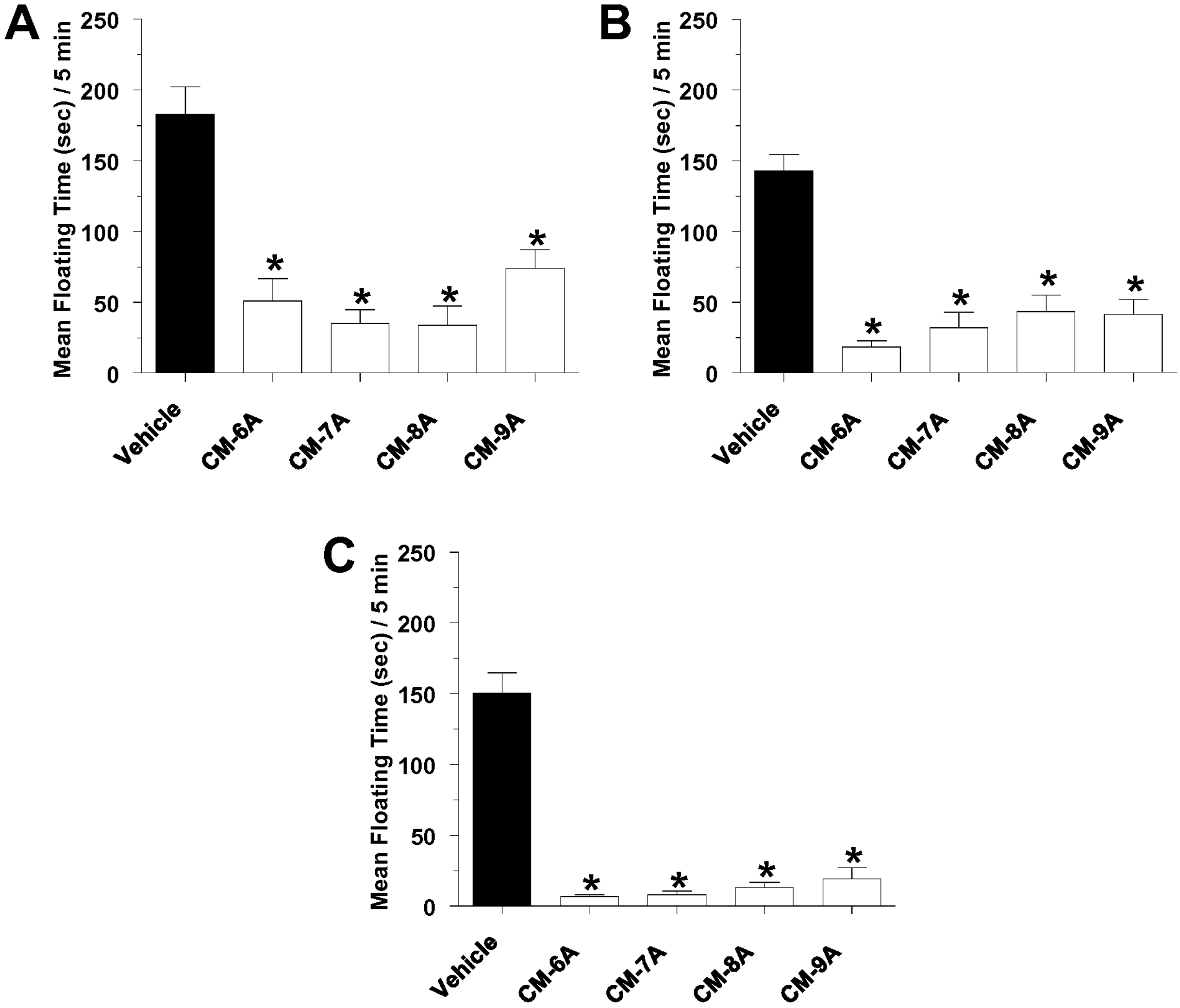
A
B
C
Mean Floating Time (sec) / 5 min
250
200
150
100
50
0
*
Vehicle
CM-6A
CM-7A
CM-8A
CM-9A

Fig. 11

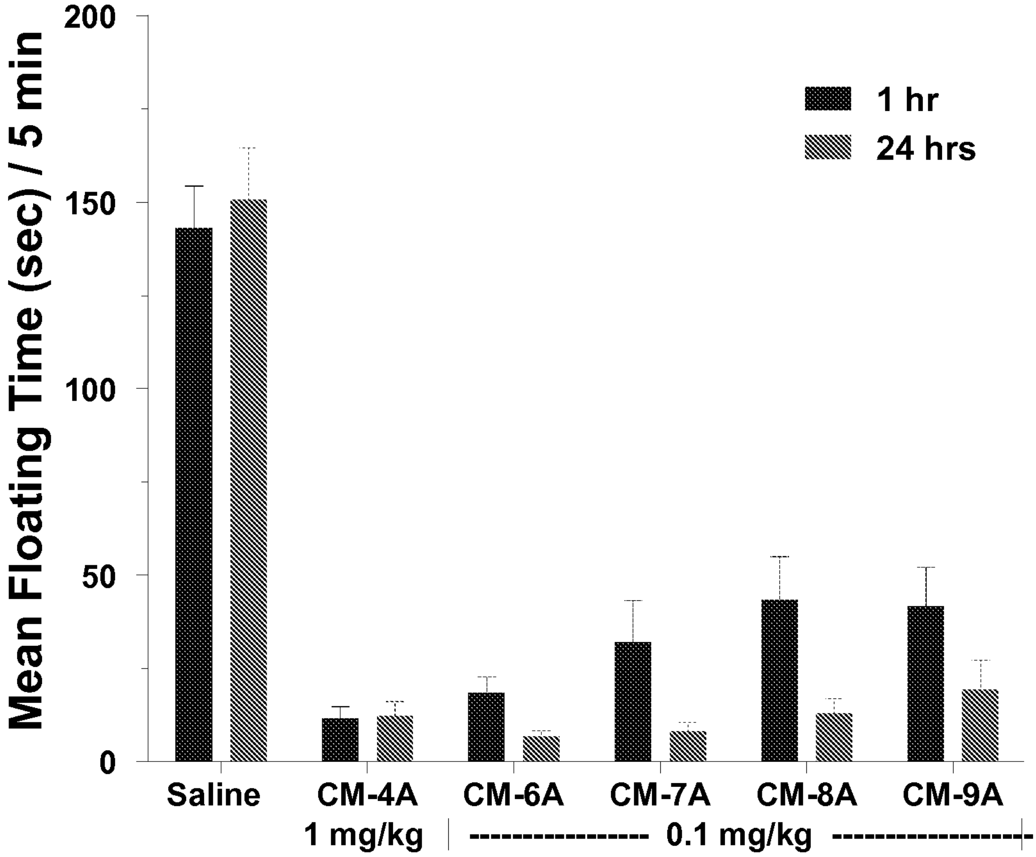
Mean Floating Time (sec) / 5 min
200
150
100
50
0
1 hr
24 hrs
Saline
CM-4A
CM-6A
CM-7A
CM-8A
CM-9A
1 mg/kg
---------------- 0.1 mg/kg -----------------

Fig. 12

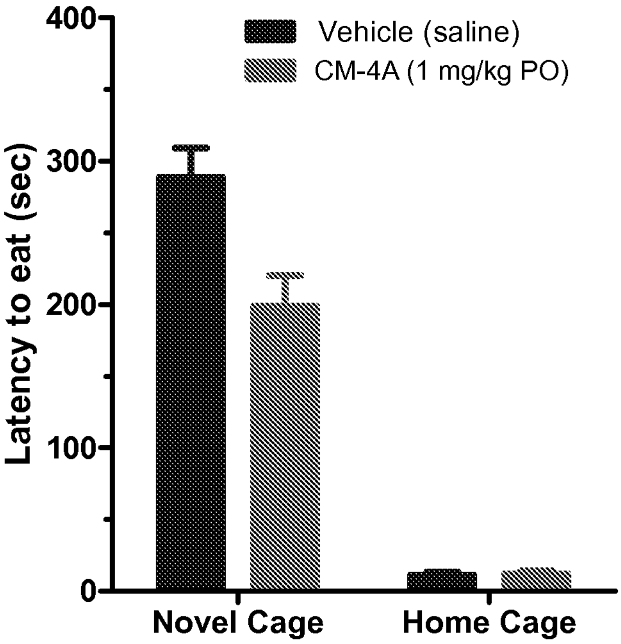
Vehicle (saline)
CM-4A (1 mg/kg PO)
Latency to eat (sec)
400
300
200
100
0
Novel Cage
Home Cage

Fig. 13

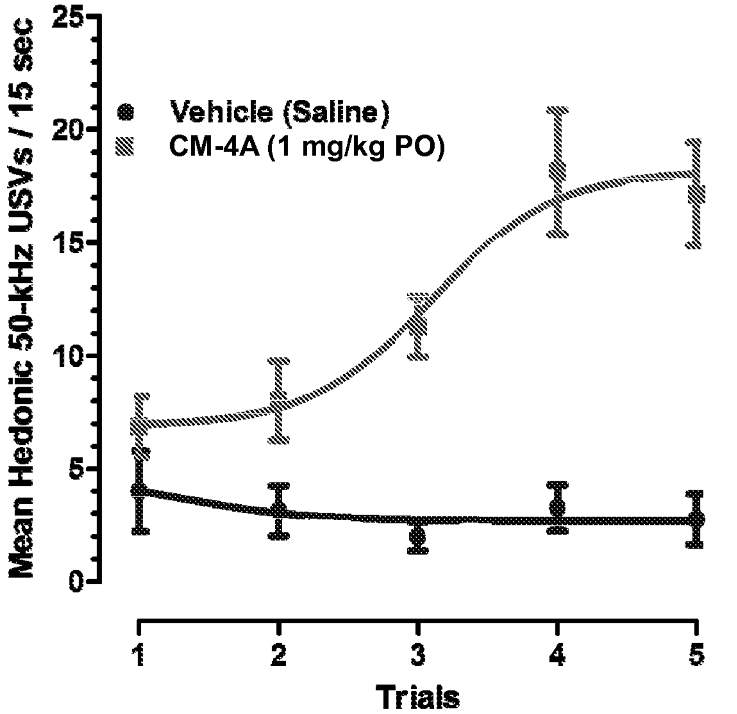
Mean Hedonic 50-kHz USVs / 15 sec
25
20
15
10
5
0
Vehicle (Saline)
CM-4A (1 mg/kg PO)
1
2
3
4
5
Trials

A.

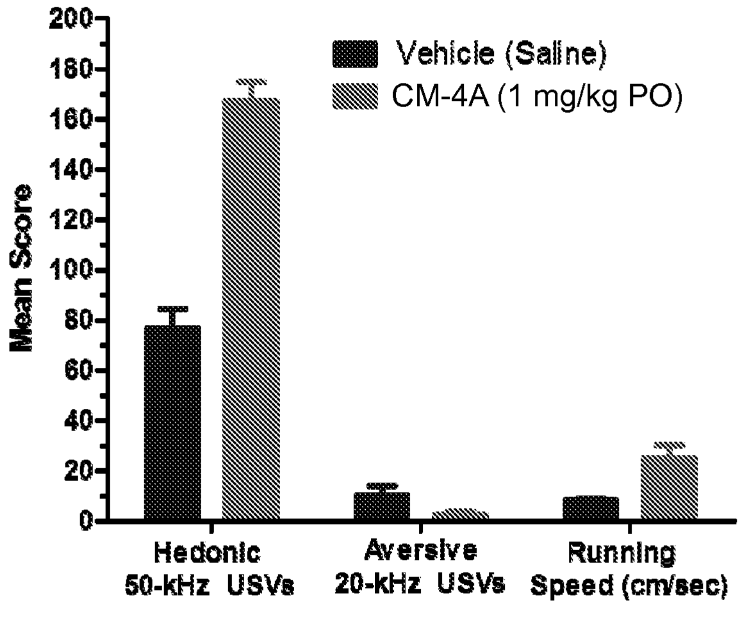
Mean Score
200
180
160
140
120
100
80
60
40
20
0
Vehicle (Saline)
CM-4A (1 mg/kg PO)
Hedonic
50-kHz USVs
Aversive
20-kHz USVs
Running
Speed (cm/sec)

B.

Fig. 14

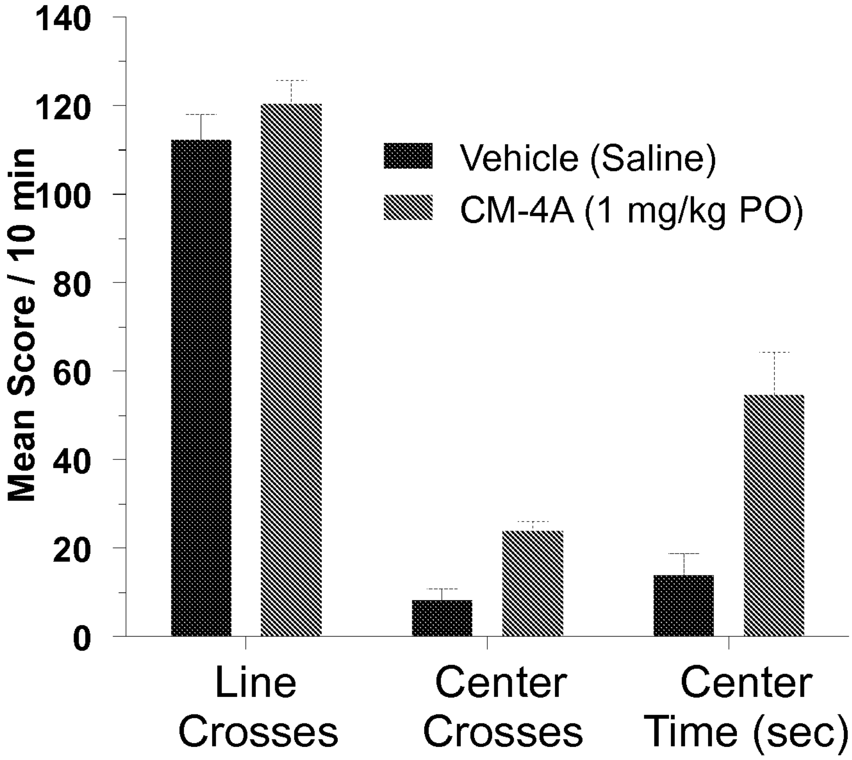
140
120
100
80
60
40
20
0
Mean Score / 10 min
Vehicle (Saline)
CM-4A (1 mg/kg PO)
Line Crosses
Center Crosses
Center Time (sec)

Fig. 15

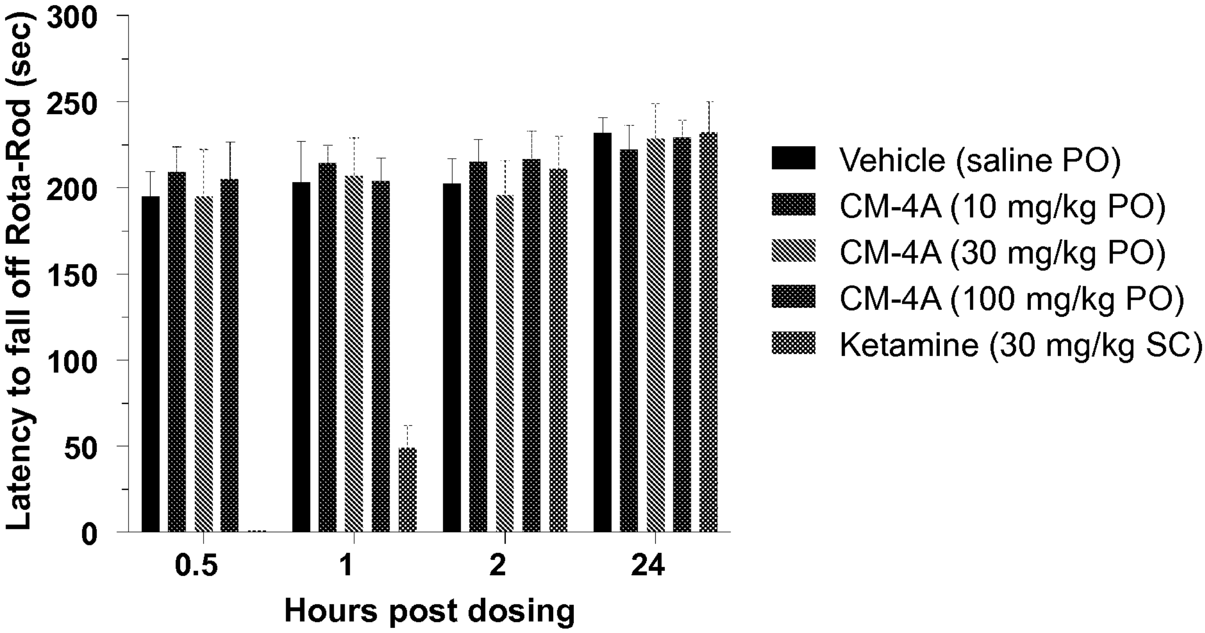
Latency to fall off Rota-Rod (sec)
300
250
200
150
100
50
0
0.5
1
2
24
Hours post dosing
Vehicle (saline PO)
CM-4A (10 mg/kg PO)
CM-4A (30 mg/kg PO)
CM-4A (100 mg/kg PO)
Ketamine (30 mg/kg SC)

Fig. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **FOSTER et al.** *Nature,* 1987, vol. 329, 395-396
- **MAYER et al.** *Trends in Pharmacol. Sci.,* 1990, vol. 11, 254-260
- **COLLINGRIDGE et al.** The NMDA Receptor. Oxford University Press, 1994
- **MOSKAL et al.** The use of antibody engineering to create novel drugs that target N-methyl-D-aspartate receptors. *Curr. Drug Targets,* 2001, vol. 2, 331-45
- **URWYLER et al.** Drug design, in vitro pharmacology, and structure-activity relationships of 3-acylamino-2-aminopropionic acid derivatives, a novel class of partial agonists at the glycine site on the N-methyl-D-aspartate (NMDA) receptor complex. *J. Med. Chem.,* 2009, vol. 52, 5093-10
- **ZHANG et al.** A NMDA receptor glycine site partial agonist, GLYX-13 (''GLYX''), simultaneously enhances LTP and reduces LTD at Schaffer collateral-CA1 synapses in hippocampus. *Neuropharmacology,* 2008, vol. 55, 1238-50
- **BURGDORF et al.** The effect of selective breeding for differential rates of 50-kHz ultrasonic vocalizations on emotional behavior in rats. *Devel. Psychobiol.,* 2009, vol. 51, 34-46